# EUROPEAN PATENT SPECIFICATION

(11) **EP 1 272 456 B1**
(45) Date of publication and mention of the grant of the patent: **27.10.2004**
(21) Application number: 01925029.9
(22) Date of filing: 13.04.2001
(51) Int. Cl.: C07C 243/28, C07C 239/20, C07C 321/14, C07D 215/46, C07D 213/75, C07D 237/20, A61K 31/165, A61K 31/175, A61K 31/18, A61K 31/435, A61K 31/50

(54) **HYDRAZIDE AND ALKOXYAMIDE ANGIOGENESIS INHIBITORS**
HYDRAZID UND ALKOXYAMID ANGIOGENESEINHIBITOREN
INHIBITEURS D'ANGIOGENESE A BASE D'HYDRAZIDE ET D'ALCOXYAMIDE

(30) Priority: 14.04.2000 US 549995; 21.03.2001 US 813008
(43) Date of publication of application: 08.01.2003
(73) Proprietor: Abbott Laboratories, Abbott Park, IL 60064-6050 (US)
(72) Inventor: CRAIG, Richard, A., Racine, WI 53402 (US); KAWAI, Megumi, Libertyville, IL 60048 (US); LYNCH, Linda, M., Pleasant Prairie, WI 53158-2400 (US); PATEL, Jyoti, R., Libertyville, IL 60048 (US); SHEPPARD, George, S., Wilmette, IL 60091 (US); WANG, Jieyi, Lake Bluff, IL 60044 (US); YANG, Fan, Kalamazoo, MI 49001 (US); BA-MAUNG, Nwe, Y., Niles, IL 60714 (US)
(74) Representative: Modiano, Guido, Dr.-Ing.
(86) International application number: PCT/US2001/012274
(87) International publication number: WO 2001/079157

(56) References cited:
- WO-A-99/42436
- DE-A- 19 831 710
- US-A- 5 977 408

## Description

### Technical Field

The present invention relates to substituted hydrazides and N-alkoxyamides which are useful for preventing angiogenesis, methods of making the compounds, compositions containing the compounds, and methods of treatment using the compounds.

### Background of the Invention

Angiogenesis, the fundamental process by which new blood vessels are formed, is essential to a variety of normal body activities (such as reproduction, development and wound repair). Although the process is not completely understood, it is believed to involve a complex interplay of molecules which both stimulate and inhibit the growth of endothelial cells, the primary cells of the capillary blood vessels. Under normal conditions, these molecules appear to maintain the microvasculature in a quiescent state (i.e., one of no capillary growth) for prolonged periods which may last for as long as weeks or in some cases, decades. When necessary, however, (such as during wound repair), these same cells undergo rapid proliferation and turnover within a 5 day period. (*The Journal of Biological Chemistry,* **267**: 10931-10934 (1987), *Science,* **235**: 442-447 (1987)).

Although angiogenesis is a highly regulated process under normal conditions, many diseases (characterized as "angiogenic diseases") are driven by persistent unregulated angiogenesis. For example, ocular angiogenesis has been implicated as the most common cause of blindness and dominates approximately 20 eye diseases. In certain existing conditions such as arthritis, newly formed capillary blood vessels invade the joints and destroy cartilage. In diabetes, new capillaries formed in the retina invade the vitreous, bleed, and cause blindness. Growth and metastasis of solid tumors are also angiogenesis-dependent *(Cancer Research,* **46**: 467-473 (1986), *Journal of the National Cancer Institute,* **82**: 4-6 (1989).
Because the pivotal role played by angiogenesis in tumor formation, metastasis, other disease conditions such as arthritis, inflammation, macular degeneration of age, and diabetic retinopathy, agents which inhibit angiogenesis have been the subject of active current research for their clinical potential.

In *Proc. Natl. Acad. Sci. USA,* **94:** 6099-6103 (1997) and *Chemistry and Biology,* **4**(6): 461-471 (1997) it is reported that both AGM-1470 and ovalicin, a sequiterpene isolated from the fungus *Pseudorotium ocalis* have been found to covalently inactivate a common bifunctional protein, type 2-methionine aminopeptidase (MetAP2) and is concluded that MetAP2 plays a critical role in the proliferation of endothelial cells and may serve as a promising target for the development of new anti-angiogenic drugs.
The literature has thus established a casual link between inhibition of MetAP2 and the resultant inhibition of endothelial cell proliferation and angiogenesis. There is a need for discovery of new agents which inhibit MetAP2 for their potential as new drugs in combating angiogenesis and disease conditions which depend upon angiogenesis for their development.

### Summary of The Invention

In its principle embodiment, the present invention provides a compound of formula (1), 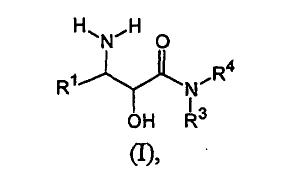 or a therapeutically acceptable salt or prodrug thereof, wherein
R¹ is selected from the group consisting of alkyl, aryl, arylalkyl, cycloalkyl, (cycloalkyl)alkyl, (heterocycle)alkyl, and R⁵S-(alkylene)-;
wherein each group is drawn with its right-hand end being the end that is attached to the parent molecular moiety;
R³ is selected from the group consisting of hydrogen, alkyl, and arylalkyl;
R⁴ is selected from the group consisting of -NR⁶R⁷, and -OR⁸;
wherein each group is drawn with its left-hand end being the end that is attached to the parent molecular moiety;
R⁵ is selected from the group consisting of alkyl, aryl, arylalkyl, cycloalkyl, and (cycloalkyl)alkyl;
R⁶ and R⁷ are independently selected from the group consisting of hydrogen, alkanoyl, alkenyl, alkenyloxyalkyl, alkoxyalkyl, alkoxycarbonylalkyl, alkyl, alkylsulfanylalkyl, aryl, arylalkanoyl, arylalkoxyalkyl, arylalkoxycarbonyl, arylalkyl, aryloxyalkyl, (aryl)oyl, arylsulfonyl, carboxyalkyl, cycloalkyl, (cycloalkyl)alkyl, (cycloalkyl)alkanoyl, (cycloalkyl)oyl, haloalkanoyl, haloalkyl, heterocycle, (heterocycle)alkanoyl, (heterocycle)oyl, hydroxyalkyl, a nitrogen protecting group, and -C(O)NR⁹R¹⁰; or
R⁶ and R⁷ together are arylalkylidene; or
R⁶ and R⁷, together with the nitrogen atom to which they are attached, form a heterocycle;
R⁸ is selected from the group consisting of hydrogen, alkanoylalkyl, alkenyl, alkoxycarbonylalkyl, alkyl, amidoalkyl, aryl, arylalkyl, arylalkoxycarbonylalkyl, (aryl)oylalkyl, carboxyalkyl, and (cycloalkyl)alkyl; and
R⁹ and R¹⁰ are independently selected from the group consisting of hydrogen, alkyl and aryl.

In another embodiment, the present invention provides a compound of formula (II) 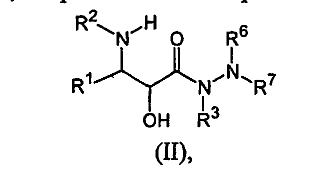 or a therapeutically acceptable salt or prodrug thereof, wherein R¹, R³, R⁶, and R⁷ are as previously defined.

In a preferred embodiment, the present invention provides a compound of formula (II) wherein R¹ is R⁵S-(alkylene)-, R³ is hydrogen, one of R⁶ and R⁷ is hydrogen, and the other is (aryl)oyl.

In another embodiment. the present invention provides a compound of formula (III) 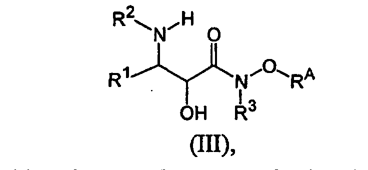 or a therapeutically acceptable salt or prodrug thereof, wherein R¹ and R³ are as previously defined and R^{A} is selected from the group consisting of aryl, alkyl, and arylalkyl.

In another embodiment, the present invention provides a pharmaceutical composition comprising a compound of formula (I) or a therapeutically acceptable salt or prodrug thereof, in combination with a therapeutically acceptable carrier.

In another embodiment, the present invention provides a method of inhibiting angiogenesis in a mammal in recognized need of such treatment comprising adminstering to the mammal a therapeutically acceptable amount of a compound of formula (I).

### Detailed Description of The Invention

Compounds of the present invention comprise hydrazines and hydroxylamines substituted with substituted 2-hydroxy-3-amino alkanoic acids.

Compounds of the present invention exist as stereoisomers, wherein asymmetric or chiral centers are present. These compounds are designated by the symbols "R" or "S" depending on the configuration of substituents around the chiral carbon atom. The present invention contemplates various stereoisomers and mixtures thereof. Stereoisomers include enantiomers and diastereomers, and mixtures of enantiomers or diastereomers are designated (RS). Individual stereoisomers of compounds of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

Because carbon-carbon double bonds may exist in the present compounds, the invention contemplates various geometric isomers and mixtures thereof resulting from the arrangement of substituents around these carbon-carbon double bonds. These substituents are designated as being in the E or Z configuration wherein the term "E" represents higher order substituents on opposite sides of the carbon-carbon double bond, and the term "Z" represents higher order substituents on the same side of the carbon-carbon double bond.

When used throughout this specification and the appended claims, the following terms have the meanings indicated:

The term "alkanoyl" refers to an alkyl group attached to the parent molecular moiety through a carbonyl group. The alkanoyl groups of this invention can be unsubstituted or substituted with one, two, or three substituents independently selected from the group consisting of alkoxycarbonyl, alkylsulfanyl, amino, and hydroxy.

The term "alkanoylalkyl" refers to an alkanoyl group attached to the parent molecular moiety through an alkyl group.

The term "alkenyl" refers to a monovalent straight or branched chain groups having from two to fourteen carbon atoms containing at least one carbon-carbon double bond.

The term "alkenyloxy" refers to an alkenyl group attached to the parent molecular moiety through an oxygen atom.

The term "alkenyloxyalkyl" refers to an alkenyloxy group attached to the parent molecular moiety through an alkyl group.

The term "alkoxy" refers to an alkyl group attached to the parent molecular moiety through an oxygen atom.

The term "alkoxyalkyl" refers to an alkoxy group attached to the parent molecular moiety through an alkyl group.

The term "alkoxycarbonyl" refers to an alkoxy group attached to the parent molecular moiety through a carbonyl group.

The term "alkoxycarbonylalkyl" refers to an alkoxycarbonyl group attached to the parent molecular moiety through an alkyl group.

The term "alkyl" refers to a monovalent group derived from a straight or branched chain saturated hydrocarbon having from one to fourteen carbons by the removal of a single hydrogen atom.

The term "alkylene" refers to a saturated divalent hydrocarbon group derived from a straight or branched chain saturated hydrocarbon having from one to fourteen carbons by the removal of two hydrogen atoms.

The term "alkylsulfanyl" refers to an alkyl group attached to the parent molecular moiety through a sulfur atom.

The term "alkylsulfanylalkyl" refers to an alkylsulfanyl group attached to the parent molecular moiety through an alkyl group.

The term "alkylsulfonyl" refers to an alkyl group attached to the parent molecular moiety through a sulfonyl group.

The term "amido" refers to an amino group attached to the parent molecular moiety through a carbonyl group.

The term "amidoalkyl" refers to an amido group attached to the parent molecular moiety through an alkyl group.

The term "amino" refers to -NR^{a}R^{b}, wherein R^{a} and R^{b} are independently selected from the group consisting of hydrogen, unsubstituted alkanoyl, alkoxycarbonylalkyl, alkyl, aryl, arylalkyl, (aryl)oyl, cycloalkyl, and (cycloalkyl)alkyl. The aryl and the aryl part of the arylalkyl and the (aryl)oyl can be unsubstituted or substituted with one, two, three, four, or five substituents independently selected from the group consisting of alkoxy, alkoxycarbonyl, cyano, halo, haloalkoxy, haloalkyl, and nitro.

The term "aminoalkoxy" refers to an amino group attached to the parent molecular moiety through an alkoxy group.

The term "aminoalkyl" refers to an amino group attached to the parent molecular moiety through an alkyl group.

The term "aminosulfonyl" refers to an amino group attached to the parent molecular moiety through a sulfonyl group.

The term "aryl" refers to phenyl, naphthyl, dihydronaphthyl, tetrahydronaphthyl, indanyl, and indenyl. Aryl groups having an unsaturated or partially saturated ring fused to an aromatic ring can be attached through either the saturated or unsaturated part of the group. The aryl groups of this invention can be unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo. The aryl groups of this invention can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro.

The term "arylalkanoyl" refers to an aryl group attached to the parent molecular moiety through an alkanoyl group.

The term "arylalkoxy" refers to an aryl group attached to the parent molecular moiety through an alkoxy group.

The term "arylalkoxyalkyl" refers to an arylalkoxy group attached to the parent molecular moiety through an alkyl group.

The term "arylalkoxycarbonyl" refers to an arylalkoxy group attached to the parent molecular moiety through a carbonyl group.

The term "arylalkoxycarbonylalkyl" refers to an arylalkoxycarbonyl group attached to the parent molecular moiety through an alkyl group.

The term "arylalkyl" refers to an aryl group attached to the parent group through an alkyl group. The alkyl part of the arylalkyl can be unsubstituted or substituted with a cyano group.

The term "arylalkylidene" refers to =CHR^{c}, wherein R^{c} is aryl.

The term "arylalkylsulfanyl" refers to an arylalkyl group attached to the parent molecular moiety through a sulfur atom.

The term "aryloxy" refers to an aryl group attached to the parent molecular moiety through an oxygen atom.

The term "aryloxyalkyl" refers to an aryloxy group attached to the parent molecular moiety through an alkyl group. The alkyl part of the aryloxyalkyl can be unsubstituted or substituted with a hydroxy group.

The term "(aryl)oyl" refers to an aryl group attached to the parent molecular moiety through a carbonyl group.

The term "(aryl)oylalkyl" represnts an (aryl)oyl group attached to the parent molecular moiety through an alkyl group.

The term "arylsulfonyl" refers to an aryl group attached to the parent molecular moiety through a sulfonyl group.

The term "carbonyl" refers to -C(O)-.

The term "carboxy" refers to -CO₂H.

The term "carboxyalkyl" refers to a carboxy group attached to the parent molecular moiety through an alkyl group.

The term "cyano" refers to -CN.

The term "cycloalkyl" refers to a monovalent saturated cyclic hydrocarbon having from three to ten carbon atoms.

The term "cycloalkylalkanoyl" refers to a cycloalkyl group attached to the parent molecular moiety through an alkanoyl group.

The term "(cycloalkyl)alkyl" refers to a cycloalkyl group attached to the parent molecular moiety through an alkyl group.

The term "(cycloalkyl)alkylsulfanyl" refers to a (cycloalkyl)alkyl group attached to the parent molecular moiety through a sulfur atom.

The term "(cycloalkyl)oyl" refers to a cycloalkyl group attached to the parent molecular moiety through a carbonyl group.

The term "cycloalkylsulfanyl" refers to a cycloalkyl group attached to the parent molecular moiety through a sulfur atom.

The term "halo" or "halogen" refers to F, Cl, Br, or I.

The term "haloalkanoyl" refers to a haloalkyl group attached to the parent molecular moiety through a carbonyl group.

The term "haloalkoxy" refers to a haloalkyl group attached to the parent molecular moiety through an oxygen atom.

The term "haloalkyl" refers to an alkyl group substituted by one, two, three, or four halogen atoms.

The term "haloalkylsulfanyl" refers to a haloalkyl group attached to the parent molecular moiety through a sulfur atom.

The term "heterocycle" refers to a five-, six- or seven-membered ring containing one, two or three heteroatoms independently selected from the group consisting of nitrogen, oxygen and sulfur. The five-membered ring has zero to two double bonds and the six- and seven-membered rings have zero to three double bonds. The term "heterocycle" also includes bicyclic groups in which the heterocycle ring is fused to a phenyl group or a cycloalkyl group. The heterocycle groups of the present invention can be attached through a carbon atom or a nitrogen atom in the group. Examples of heterocycles include, but are not limited to, benzodioxolyl, benzothiazolyl, benzothienyl, chromenyl, dihydropyridazinyl, furyl, isoxazolyl, morpholinyl, piperazinyl, piperidinyl, pyrazinyl, pyrazolyl, pyridazinyl, pyridinyl, pyrimidinyl, pyrrolidinyl, pyrrolyl, quinolinyl, quinoxolinyl, tetrahydrobenzothienyl, tetrahydrofuryl, thiazolidinyl, thiazolyl, thienyl, and the like. The heterocycle groups of this invention can be unsubstituted or substituted with one, two, three, or four, substituents independently selected from the group consisting of alkanoyl, alkoxy, alkoxyalkyl, alkoxycarbonyl, alkyl, alkylsulfanylalkyl, amino, aminosulfonyl, carboxy, halo, haloalkoxy, haloalkyl, hydroxy, nitro, oxo, and thioxo. The heterocycle groups of this invention can be further substituted with an aryl, arylalkyl, aryloxy, aryloxycarbonyl, or (heterocycle)alkenyl group, wherein the aryl, the aryl portion of the aryloxy, the arylalkyl, and the aryloxycarbonyl, and the heterocycle portion of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro.

The term "(heterocycle)alkanoyl" refers to a heterocycle group attached to the parent molecular moiety through an alkanoyl group.

The term "(heterocycle)alkenyl" refers to a heterocycle group attached to the parent molecular moiety through an alkenyl group.

The term "(heterocycle)alkyl" refers to a heterocycle group attached to the parent molecular moiety through an alkyl group.

The term "(heterocycle)oyl" refers to a heterocycle group attached to the parent molecular moiety through a carbonyl group.

The term "hydroxy" refers to -OH.

The term "hydroxyalkoxy" refers to a hydroxyalkyl group attached to the parent molecular moiety through an oxygen atom.

The term "hydroxyalkyl" refers to an alkyl group substituted with one, two, or three hydroxy groups.

The term "nitro" refers to -NO₂.

The term "nitrogen protecting group" refers to groups intended to protect an amino group against undersirable reactions during synthetic procedures. Common N-protecting groups comprise acyl groups such as formyl, acetyl, propionyl, pivaloyl, tert-butylacetyl, 2-chloroacetyl, 2-bromoacetyl, trifluoroacetyl, trichloroacetyl, phthalyl, orthonitrophenoxyacetyl, α-chlorobutyryl, benzoyl, 4-chlorobenzoyl, 4-bromobenzoyl, and 4-nitrobenzoyl; sulfonyl groups such as benzenesulfonyl, and para-toluenesulfonyl; carbamate forming groups such as benzyloxycarbonyl, para-chlorobenzyloxycarbonyl, p-methoxybenzyloxycarbonyl, tert-butyloxycarbonyl (Boc), benzyloxycarbonyl (Cbz), and the like.

The term "oxo" refers to (=O).

The term "sulfonyl" refers to -SO₂-.

The term "thioxo" refers to (=S).

The compounds of the present invention can exist as therapeutically acceptable salts. The term "therapeutically acceptable salt," as used herein, represents salts or zwitterionic forms of the compounds of the instant invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate,trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of therapeutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-ephenamine, and N,N'-dibenzylethylenediamine. Other representative organic amines useful for the formation of base addition salts include ethylenediamine, ethanolamine, diethanolamine, piperidine, and piperazine.

The present compounds can also exist as therapeutically acceptable prodrugs. The term "therapeutically acceptable prodrug," refers to those prodrugs or zwitterions which are suitable for use in contact with the tissues of patients without undue toxicity, irritation, and allergic response, are commensurate with a reasonable benefit/risk ratio, and are effective for their intended use. The term "prodrug," refers to compounds which are rapidly transformed *in vivo* to parent compounds of formula (I) for example, by hydrolysis in blood.

In accordance with methods of treatment and pharmaceutical compositions of the invention, the compounds can be administered alone or in combination with other anti-angiogenesis agents. When using the compounds, the specific therapeutically effective dose level for any particular patient will depend upon factors such as the disorder being treated and the severity of the disorder; the activity of the particular compound used; the specific composition employed; the age, body weight, general health, sex, and diet of the patient; the time of administration; the route of administration; the rate of excretion of the compound employed; the duration of treatment; and drugs used in combination with or coincidently with the compound used. The compounds can be administered orally, parenterally, osmotically (nasal sprays), rectally, vaginally, or topically in unit dosage formulations containing carriers, adjuvants, diluents, vehicles, or combinations thereof. The term "parenteral" includes infusion as well as subcutaneous, intravenous, intramuscular, and intrasternal injection.

Parenterally adminstered aqueous or oleaginous suspensions of the compounds can be formulated with dispersing, wetting, or suspending agents. The injectable preparation can also be an injectable solution or suspension in a diluent or solvent. Among the acceptable diluents or solvents employed are water, saline, Ringer's solution, buffers, monoglycerides, diglycerides, fatty acids such as oleic acid, and fixed oils such as monoglycerides or diglycerides.

The anti-angiogenesis effect of parenterally administered compounds can be prolonged by slowing their absorption. One way to slow the absorption of a particular compound is administering injectable depot forms comprising suspensions of crystalline, amorphous, or otherwise water-insoluble forms of the compound. The rate of absorption of the compound is dependent on its rate of dissolution which is, in turn, dependent on its physical state. Another way to slow absorption of a particular compound is administering injectable depot forms comprising the compound as an oleaginous solution or suspension. Yet another way to slow absorption of a particular compound is administering injectable depot forms comprising microcapsule matrices of the compound trapped within liposomes, microemulsions, or biodegradable polymers such as polylactide-polyglycolide, polyorthoesters or polyanhydrides. Depending on the ratio of drug to polymer and the composition of the polymer, the rate of drug release can be controlled.

Transdermal patches can also provide controlled delivery of the compounds. The rate of absorption can be slowed by using rate controlling membranes or by trapping the compound within a polymer matrix or gel. Conversely, absorption enhancers can be used to increase absorption.

Solid dosage forms for oral administration include capsules, tablets, pills, powders, and granules. In these solid dosage forms, the active compound can optionally comprise diluents such as sucrose, lactose, starch, talc, silicic acid, aluminum hydroxide, calcium silicates, polyamide powder, tableting lubricants, and tableting aids such as magnesium stearate or microcrystalline cellulose. Capsules, tablets and pills can also comprise buffering agents, and tablets and pills can be prepared with enteric coatings or other release-controlling coatings. Powders and sprays can also contain excipients such as talc, silicic acid, aluminum hydroxide, calcium silicate, polyamide powder, or mixtures thereof. Sprays can additionally contain customary propellants such as chlorofluorohydrocarbons or substitutes therefor.

Liquid dosage forms for oral administration include emulsions, microemulsions, solutions, suspensions, syrups, and elixirs comprising inert diluents such as water. These compositions can also comprise adjuvants such as wetting, emulsifying, suspending, sweetening, flavoring, and perfuming agents.

Topical dosage forms include ointments, pastes, creams, lotions, gels, powders, solutions, sprays, inhalants, and transdermal patches. The compound is mixed under sterile conditions with a carrier and any needed preservatives or buffers. These dosage forms can also include excipients such as animal and vegetable fats, oils, waxes, paraffins, starch, tragacanth, cellulose derivatives, polyethylene glycols, silicones, bentonites, silicic acid, talc and zinc oxide, or mixtures thereof. Suppositories for rectal or vaginal administration can be prepared by mixing the compounds with a suitable non-irritating excipient such as cocoa butter or polyethylene glycol, each of which is solid at ordinary temperature but fluid in the rectum or vagina. Ophthalmic formulations comprising eye drops, eye ointments, powders, and solutions are also contemplated as being within the scope of this invention.

The total daily dose of the compounds administered to a host in single or divided doses can be in amounts from about 0.1 to about 200 mg/kg body weight or preferably from about 0.25 to about 100 mg/kg body weight. Single dose compositions can contain these amounts or submultiples thereof to make up the daily dose.

### Determination of Biological Activity

Assays for the inhibition of catalytic activities of MetAP2 were performed in 96-well microtiter plates. Compounds to be tested were dissolved in dimethyl-sulfoxide at 10 mM and diluted ten-fold in assay buffer (50 mM HEPES, pH 7.4, 100 mM NaCl). Ten microliters of solution of each compound to be tested for inhibition were introduced into each cell of the plate. Zero inhibition of enzyme activity was taken to be the result obtained in cells in which 10 mL of assay buffer was placed. A mixture totaling 90 mL per well and made up of of 84 mL of assay buffer containing 100 mM MnCl₂, 1 mL of L-amino acid oxidase (Sigma Catalog No. A-9378, ∼11 mg/mL), 1 mL of horseradish peroxidase (Sigma Catalog No. P-8451, dissolved in assay buffer at a concentration of 10 mg/mL), 1 mL of the tripeptide Met-Ala-Ser (Bachem) dissolved in assay buffer at concentration of 50 mM, 1 mL of *ortho*-dianisidine (Sigma Catalog No. D-1954, freshly made solution in water at a concentration of 10 mg/mL), and MetAP1 at final concentration of 6 mg/ml or MetAP2 at a final concentration of 1.5 mg/mL was rapidly mixed and added to each cell containing test or control compound. The absorbence at 450 nanometers was measured every 20 seconds over a period of twenty minutes using an automaic plate reader (Molecular Devices, CA, USA). The Vmax in mOD/min, calculated for each well, was used to represent MetAP1 or MetAP2 activity. The IC₅₀ for each inhibitor was obtained by plotting the remaining activity *versus* inhibitor concentrations. All of the compounds of the invention displayed IC₅₀'s below 15 µM. Preferred compounds of the invention displayed IC₅₀'s below 1 µM, and most preferred compounds displayed IC₅₀'s below 0.1 µM. Thus, the compounds are useful for treating diseases caused or exacerbated by angiogenesis.

### Synthetic Methods

Abbreviations which have been used in the descriptions of the scheme and the examples that follow are: THF for tetrahydrofuran, PDC for pyridinium dichromate, DMSO for dimethylsulfoxide, DME for 1,2-dimethoxyethane, DCC for 1,3-diicyclohexylcarbodiimide, DIC for 1,3-diisopropylcarbodiimide, HOBT for 1-hydroxybenzotriazole, HOAT for 1-hydroxy-7-azabenzotriazole, EDCI for 1-(3-dimethylaminopropyl)-3-ethylcarbodiimide hydrochloride, PyBOP for benzotriazol-1-yloxytripyrrolidinophosphonium hexafluorophosphate, DBU for 1,8-diazabicyclo(5.4.0)undec-7-ene, NMM for N-methylmorpholine, DMA for N,N-dimethylacetamide, NMP for N-methylpyrrolidinone, BOC-ON for (2-(tert-butoxycarbonyloxyimino)-2-phenylacetonitrile, and DMF for N,N dimethylformaide.

The compounds and processes of the present invention will be better understood in connection with the following synthetic schemes which illustrate the methods by which the compounds of the invention may be prepared. Starting materials can be obtained from commercial sources or prepared by well-established literature methods known to those of ordinary skill in the art. The groups R¹, R³, R⁴, R⁵, and R⁶ are defined above. It will be readily apparent to one of ordinary skill in the art that the compounds defined above can be synthesized by substitution of the appropriate reactants and agents in the syntheses shown below.

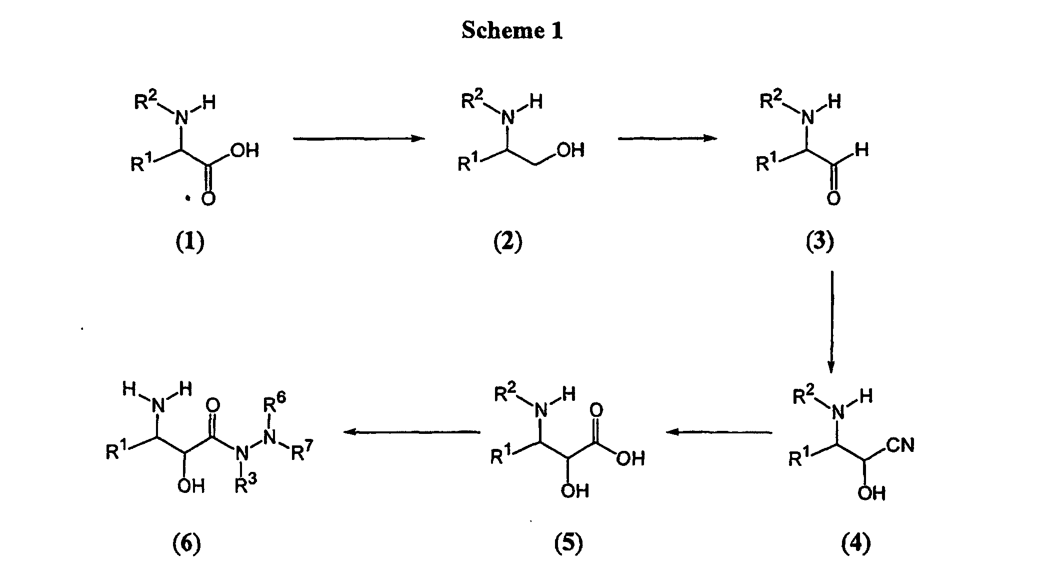

As shown in Scheme **1**, compounds of formula (**1**) (R² is a nitrogen protecting group) can be converted to compounds of formula (**2**) by treatment with a reducing agent. Representative reducing agents include sodium borohydride, calcium borohydride, lithium borohydride, zinc borohydride, lithium aluminum hydride, borane, sodium cyanoborohydride, diisobutylaluminum hydride, and sodium bis(2-methoxyethoxy)aluminum hydride (Red-Al®). Examples of solvents used in these reactions include toluene, dichloromethane, ethanol, THF, dioxane, diethyl ether, or mixtures thereof. The reaction temperature is about -78 °C to 60 °C and depends on the method chosen. Reaction times are typically about 0.5 to 24 hours. Compounds of formula (**2**) can be converted to compounds of formula (**3**) by treatment with an oxidizing reagent. Representative oxidizing agents include Dess-Martin periodinane, PDC, MnO₂ DMSO/oxalyl chloride, and DMSO/SO₃-pyridine/triethylamine. Examples of solvents used in these reactions include DMSO, dichloromethane, chloroform, and THF. The reaction temperature is about -78 °C to 60 °C and depends on the method chosen. Reaction times are typically about 0.5 to 24 hours.

Conversion of compounds of formula (**3**) to compounds of formula (**4**) can be accomplished by the addition of sodium bisulfite, followed by the addition of sodium cyanide or potassium cyanide. Examples of solvents used in these reactions include water, ethyl acetate, and acetonitrile. The reaction temperature is about -10 °C to 60 °C and depends on the method chosen. Reaction times are typically about 2-36 hours. Compounds of formula (**4**) can be converted to compounds of formula (**5**) by hydrolysis with aqueous acid or by hydrolysis with aqueous base followed by acidification. Representative aqueous acids include HBr, HCl, HOAc, and H₂SO₄, and representative aqueous bases include NaOH, KOH, and Ba(OH)₂. Examples of solvents used in these reactions include dioxane, water, ethylene glycol, and DME. The reaction temperature is about 25 °C to 150 °C and depends on the method chosen. Reaction times are typically about 2-36 hours.

Conversion of compounds of formula (**5**) to compounds of formula (**6**) can be accomplished by coupling with substituted hydrazines (HNR³NR⁶R⁷) in the presence of a carbonyl activating group such as DCC, DIC, HOBT, HOAT, EDCI, and PyBOP, and base. Representative bases include NMM, diisopropylethylamine, and DBU. Examples of solvents used in these reactions include dichloromethane, chloroform, DMA, THF, and NMP. The reaction temperature is about -10 °C to 60 °C and depends on the method chosen. Reaction times are typically about 2-72 hours.

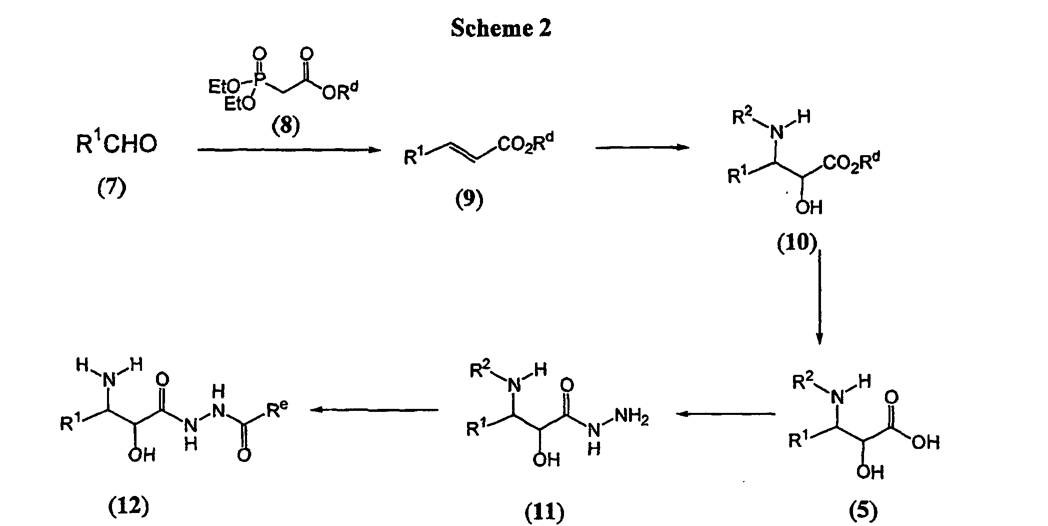

A method for the preparation of compounds of formula (**11**) is shown in Scheme 2. Aldehydes of formula (**7**) can be reacted with compounds of formula (**8**) (R^{d} is alkyl or arylalkyl) in the presence of a base and lithium bromide to provide compounds of formula (**9**). Examples of bases include triethylamine and diisopropylethylamine. Representative solvents include THF, diethyl ether, and methyl tert-butyl ether. The reaction is conducted at about 25 to about 30 °C for about 12 to about 24 hours.

Compounds of formula (**9**) can be treated with an appropriately substituted amine or amide in the presence of tert-butylhypochlorite and base, then treated with potassium osmate dihydrate and hydroquinidine 1,4-phathalazinediyl diether to provide compounds of formula (**10**) (R² is a nitrogen protecting group). Examples of bases include sodium hydroxide, potassium hydroxide, and lithium hydroxide. Representative solvents include water, 1-propanol, isopropanol, acetonitrile, and mixtures thereof. The reaction is typically conducted at about 0 to about 30 °C for about 30 minutes to about 4 hours.

Conversion of compounds of formula (**10**) to compounds of formula (**5**) can be accomplished by treatment with hydrogen peroxide in the presence of hydroxide such as lithium hydroxide. Examples of solvents include THF, water, and mixtures thereof. The reaction is conducted at about 0 to about 25 °C for about 1 to about 6 hours.

Compounds of formula (**5**) can be converted to compounds of formula (**12**) (Re is aryl, arylalkyl, cycloalkyl, (cycloalkyl)alkyl, heterocycle, or (heterocycle)alkyl) either by the methods desribed in Schemes 1 and 3, or by sequentially coupling the carboxylic acid with hydrazine and then coupling with an appropriately substituted carboxylic acid. Conditions for these couplings are similar to those described in Scheme 1 and are known to those of ordinary skill in the art.

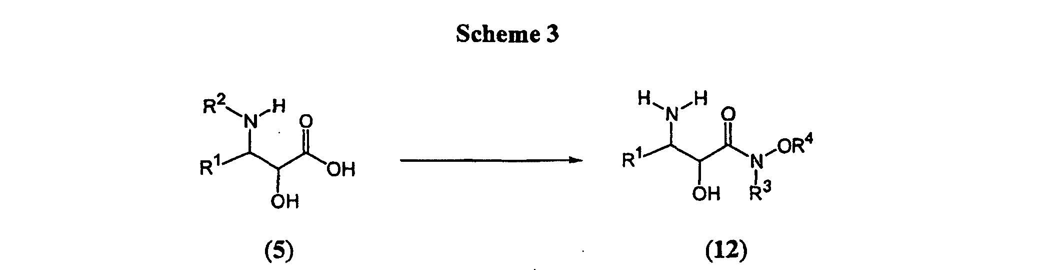

As shown in Scheme 3, compounds of formula (**5**) (made as described in Scheme 1) can be converted to compounds of formula (**12**) by coupling with substituted hydroxylamines (HNR³OR⁴) in the presence of a carbonyl activating group such as DCC, DIC, HOBT, HOAT, EDCI, and PyBOP, and base. Representative bases include NMM, diisopropylethylamine, and DBU. Examples of solvents used in these reactions include dichloromethane, chloroform, DMA, THF, and DMF. The reaction temperature is about -10 °C to 60 °C and depends on the method chosen. Reaction times are typically about 2-72 hours. In a preferred embodiment, compounds of formula (**5**) are treated with HOBT, NMM, EDCI, and a substituted hydroxylamine in dichloromethane/DMF at room temperature for 16 hours to provide compounds of formula (**12**).

The present invention will now be described in connection with certain preferred embodiments which are not intended to limit its scope. On the contrary, the present invention covers all alternatives, modifications, and equivalents as can be included within the scope of the claims. Thus, the following examples, which include preferred embodiments, will illustrate the preferred practice of the present invention, it being understood that the examples are for the purposes of illustration of certain preferred embodiments and are presented to provide what is believed to be the most useful and readily understood description of its procedures and conceptual aspects.

Compounds of the invention were named by ACD/ChemSketch version 4.01 (developed by Advanced Chemistry Development, Inc., Toronto, ON, Canada) or were given names which appeared to be consistent with ACD nomenclature.

### Example 1

### (2RS,3R)-3-amino-N'-benzyl-4-cyclohexyl-2-hydroxybutanohydrazide

### Example 1A

### tert-butyl (1R)-2-cyclohexyl-1-(hydroxymethyl)ethylcarbamate

A solution of (2R)-2-((tert-butoxycarbonyl)amino)-3-cyclohexylpropanoic acid (30.4 g,112 mmol) in toluene (300 mL) at 0 °C was treated with sodium bis(2-methoxyethoxy)aluminum hydride (Red-Al®) (115 mmol) over 45 minutes. The mixture was stirred for 30 minutes, warmed to room temperature, stirred for 1 hour, treated with aqueous Rochelle salt, and extracted with diethyl ether. The extract was washed with brine and aqueous NaHCO₃, dried (MgSO₄), filtered, and concentrated to provide the desired product.

### Example 1B

### tert-butyl (1R)-2-cyclohexyl-1-formylethylcarbamate

A solution of Example 1A (25.8 g,100 mmol), sulfur trioxide pyridine complex (79.6 g, 500 mmol), and triethylamine (69.7 mL, 500 mmol) in DMSO (70 mL) at room temperature was stirred for 30 minutes, cooled to 0 °C, treated with water and saturated aqueous KHSO₄, and extracted with ethyl acetate. The extract was washed with saturated aqueous KHSO₄ and brine, dried (MgSO₄), filtered, and concentrated to provide the desired product.

### Example 1 C

### (2RS,3R)-3-((tert-butoxycarbonyl)amino)-4-cyclohexyl-2-hydroxybutanoic acid

A solution of Example 1B (19.7 g, 77.1 mmol) and sodium bisulfite (8.0 g, 77.1 mmol) in water (500 mL) at 5 °C was stirred for 24 hours, warmed to room temperature, treated with a solution of potassium cyanide (5.1 g, 78.8 mmol) in ethyl acetate (350 mL), and stirred for 5 hours. The aqueous phase was separated and extracted with ethyl acetate. The combined extracts were washed with water and brine, dried (MgSO₄), filtered, and concentrated to provide tert-butyl (1R)-2-cyano-1-(cyclohexylmethyl)-2-hydroxyethylcarbamate.
The concentrate was dissolved in dioxane (150 mL), treated with 12N HCl (150 mL), heated to reflux, stirred for 21 hours, and cooled to room temperature. The mixture was concentrated, dissolved in a mixture of water (30 mL) and acetone (200 mL), adjusted to pH 5.5 with 1N NaOH, treated with acetone (3.5 L), and cooled to 0 °C for 4 hours. The resulting precipitate was collected by filtration and dried to provide (3R)-3-amino-4-cyclohexyl-2-hydroxybutanoic acid.
A solution of the acid, BOC-ON (1.2 eq.), and triethylamine (2 eq.) in 1:1 water/dioxane at 45 °C was stirred for 15 hours, treated with 10% aqueous KHSO₄, and extracted with ethyl acetate. The extract was washed with water and brine, dried (MgSO₄), filtered, and concentrated to provide the desired product.

### Example 1D

### (2RS,3R)-3-amino-N'-benzyl-4-cyclohexyl-2-hydroxybutanohydrazide

A solution of Example 1C (50 mg, 0.17 mmol), 1-hydroxybenzotriazole hydrate (30 mg, 0.22 mmol), and N-methylmorpholine (0.07 mL, 0.63 mmol) in 5:1/dichloromethane:N,N-dimethylacetamide (2 mL) at 0 °C was treated with 1,3-diisopropylcarbodiimide (0.03 mL, 0.21 mmol), and stirred for 5 minutes. The solution was treated with 1-benzylhydrazine dihydrochloride ( 0.05 g, 0.25 mmol), stirred for 2 hours, and warmed to room temperature over 44 hours. The reaction was washed with 2N HCl and saturated NaHCO₃, and concentrated. The concentrate was purified by silica gel chromatography with 3:1/hexanes:ethyl acetate then 1:1/hexanes:ethyl acetate, then dissolved in 4N HCl in dioxane (1 mL), stirred for 1 hour, and concentrated, then purified by HPLC to provide the desired product.
MS (APCI) m/e 306 (M+H)⁺;
¹H NMR (300 MHz, CD₃OD) δ 7.43-7.26 (m, 5H), 4.13 (d, 1H), 4.01 (s, 2H), 3.53 (m, 1H), 3.39 (s, 1H), 1.80-1.67 (m, 5H), 1.57-1.51 (m, 1H), 1.46-1.17 (m, 5H), 1.00-0.90 (m, 2H).

### Example 2

### (2RS,3R)-3-amino-N'-benzyl-4-cyclohexyl-2-hydroxybutanohydrazide

The desired product was prepared by substituting diphenylhydrazine for 1-benzylhydrazine dihydrochloride in Example 1.
MS (APCI) m/e 368 (M+H)⁺;
¹H NMR (300 MHz, CD₃OD) δ 7.32-7.26 (m, 4H), 7.16-7.13 (m, 4H), 7.06-7.01 (m, 2H), 4.26 (d, 1H), 3.52 (m, 1H), 1.72-1.12 (m, 13H).

### Example 3

### (2RS,3R)-3-amino-N'-(7-chloro-4-quinolinyl)-4-cyclohexyl-2-hydroxybutanohydrazide

The desired product was prepared by substituting 7-chloro-4-hydrazinoquinoline for 1-benzylhydrazine dihydrochloride in Example 1.
MS (APCI) m/e 377 (M+H)⁺;
¹H NMR (300 MHz, CD₃OD) δ 8.51 (d, 1H), 8.35 (d, 1H), 7.96 (d, 1H), 7.76 (dd, 1H), 6.96 (d, 1H), 4.47 (d, 1H), 3.70 (m, 1H), 1.86-0.98 (m, 13H).

### Example 4

### (2RS,3R)-3-amino-4-cyclohexyl-2-hydroxy-N'-(2-phenylethyl)butanohydrazide

The desired product was prepared by substituting 1-(2-phenethyl)hydrazine for 1-benzylhydrazine dihydrochloride in Example 1.
MS (APCI) m/e 320 (M+H)⁺;
¹H NMR (300 MHz, CD₃OD) δ 7.30-7.21 (m, 5H), 4.16 (d, 1H), 3.59 (m, 1H), 3.09 (t, 2H), 2.82 (t, 2H), 1.81-0.91 (m, 13H).

### Example 5

### (2RS,3R)-3-amino-4-cyclohexyl-2-hydroxy-N'-methyl-N'-phenylbutanohydrazide

The desired product was prepared by substituting 1-methyl-1-phenylhydrazine for 1-benzylhydrazine dihydrochloride in Example 1.
MS (APCI) m/e 306 (M+H)⁺;
¹H NMR (300 MHz, CD₃OD) δ 7.21-7.26 (m, 2H), 6.82-6.91 (m, 3H), 4.28 (d, 1H), 3.63 (m, 1H), 3.16 (s, 3H), 1.68-1.88 (m, 6H), 1.48-1.58 (m, 2H), 1.19-1.38 (m, 3H), 0.92-1.08 (m, 2H).

### Example 6

### N'-((2RS,3R)-3-amino-4-cyclohexyl-2-hydroxybutanoyl)-1-naphthohydrazide

The desired product was prepared by substituting 1-naphthohydrazide for 1-benzylhydrazine dihydrochloride in Example 1.
MS (APCI) m/e 370 (M+H)⁺;
¹H NMR (300 MHz, CD₃OD) δ 8.37 (m, 1H), 8.05 (d, 1H), 7.95 (m, 1H), 7.77 (dd, 1H), 7.57 (m, 3H), 4.42 (d, 1H), 3.77 (m, 1H), 1.90-0.97 (m, 13H).

### Example 7

### (2RS,3R)-3-amino-4-cyclohexyl-2-hydroxy-N'-(4-methylphenyl)butanohydrazide

The desired product was prepared by substituting 1-(4-methylphenyl)hydrazine for 1-benzylhydrazine dihydrochloride in Example 1.
MS (APCI) m/e 306 (M+H)⁺;
¹H NMR (300 MHz, CD₃OD) δ 7.01 (d, 2H), 6.76 (d, 2H), 4.27 (d, 1H), 3.61 (m, 1H), 2.23 (s, 3H), 1.85-1.64 (m, 6H), 1.54-1.42 (m, 2H), 1.36-1.15 (m, 3H), 1.06-0.89 (m, 2H).

### Example 8

### 2-((2RS,3R)-3-amino-4-cyclohexyl-2-hydroxybutanoyl)-N-(4-iodophenyl)hydrazinecarboxamide

The desired product was prepared by substituting N-(4-iodophenyl)hydrazinecarboxamide for 1-benzylhydrazine dihydrochloride in Example 1.
MS (APCI) m/e 461 (M+H)⁺;
¹H NMR (300 MHz, CD₃OD δ 7.59 (d, 2H), 7.23 (d, 2H), 4.33 (d, 1H), 3.68 (m, 1H), 1.85-1.65 (m, 6H), 1.59-1.19 (m, 5H), 1.09-0.93 (m, 2H).

### Example 9

### N'-((2RS,3R)-3-amino-4-cyclohexyl-2-hydroxybutanoyl)-2,4,6-trimethylbenzenesulfonohydrazide

The desired product was prepared by substituting 2,4,6-trimethylbenzenesulfonohydrazide for 1-benzylhydrazine dihydrochloride in Example 1.
MS (APCI) m/e 398 (M+H)⁺;
¹H NMR (300 MHz, CD₃OD) δ 7.01 (s, 2H), 4.01 (d, 1H), 3.36 (m, 1H), 2.67 (s, 6H), 2.29 (s, 3H), 1.78-1.65 (m, 4H), 1.47-1.20 (m, 7H), 1.00-0.78 (m, 2H).

### Example 10

### ethyl (2-((2RS,3R)-3-amino-4-cyclohexyl-2-hydroxybutanoyl)hydrazino)acetate

The desired product was prepared by substituting ethyl hydrazinoacetate for 1-benzylhydrazine dihydrochloride in Example 1.
MS (APCI) m/e 302 (M+H)⁺;
¹H NMR (300 MHz, CD₃OD δ 4.20 (q, 2H), 4.18 (d, 1H), 3.62 (d, 2H), 3.60 (m, 1H), 1.82-1.57 (m, 7H), 1.56-1.20 (m, 4H), 1.28 (t, 3H), 1.05-0.90 (m, 2H).

### Example 11

### (2RS,3R)-3-amino-4-cyclohexyl-2-hydroxy-N'-(4-methoxyphenyl)butanohydrazide

The desired product was prepared by substituting 1-(4-methoxyphenyl)hydrazine for 1-benzylhydrazine dihydrochloride in Example 1.
MS (APCI) m/e 322 (M+H)⁺;
¹H NMR (300 MHz, CD₃OD) δ 6.82 (m, 4H), 4.27 (d, 1H), 3.72 (s, 3H), 3.61 (m, 1H), 1.85-1.62 (m, 6H), 1.55-1.18 (m, 5H), 1.08-0.90 (m, 2H).

### Example 12

### (2RS,3R)-3-amino-4-cyclohexyl-2-hydroxy-N'-(1-naphthyl)butanohydrazide

The desired product was prepared by substituting 1-(1-naphthyl)hydrazine for 1-benzylhydrazine dihydrochloride in Example 1.
MS (APCI) m/e 342 (M+H)⁺;
¹H NMR (300 MHz, CD₃OD δ 8.06 (m, 1H), 7.82 (m, 1H), 7.48 (m, 2H), 7.41-7.29 (m, 2H), 6.91 (dd, 1H), 4.38 (d, 1H), 3.68 (m, 1H), 1.89-1.66 (m, 6H), 1.60-1.47 (m, 2H), 1.19-1.31 (m, 3H), 1.09-0.92 (m, 2H).

### Example 13

### benzyl 2-((2RS,3R)-3-amino-4-cyclohexyl-2-hydroxybutanoyl)hydrazinecarboxylate

### Example 13A

### benzyl 2-((2RS,3R)-3-((tert-butoxycarbonyl)amino)-4-cyclohexyl-2-hydroxybutanoyl)hydrazinecarboxylate

A solution of Example 1C (1.10 g, 3.65 mmol) in dichloromethane at 0 °C was treated with dicyclohexylcarbodiimide (0.83 g, 4.02 mmol), stirred for 30 minutes, treated with benzyl carbazate (0.69 g, 4.02 mmol), warmed to room temperature, and stirred for 32 hours. The mixture was filtered, and concentrated, and the concentrate was purified by flash column chromatography on silica gel with ethyl acetate in hexanes to provide the desired product.

### Example 13B

### benzyl 2-((2RS,3R)-3-amino-4-cyclohexyl-2-hydroxybutanoyl)hydrazinecarboxylate

Example 13A (0.09 g, 0.2 mmol) was treated with 4N HCl in dioxane, stirred for 4 hours, concentrated, and precipitated from diethyl ether to provide the desired product.
MS (APCI) m/e 350 (M+H)⁺;
¹H NMR (300 MHz, DMSO-d₆) δ 10.23 (m, 1H), 10.08 (m, 1H), 9.32 (m, 1H), 7.85 (m, 2H), 7.38 (m, 5H), 6.51 (m, 1H), 5.18 (s, 1H), 5.10 (s, 1H), 4.07 (m, 1H), 3.69 (m, 1H), 3.49 (m 1H), 3.23 (m, 1H), 1.63 (m, 5H), 1.47 (m, 3H), 1.19 (m, 2H).

### Example 14

### (2RS,3R)-3-amino-N'-((E)-(4-chlorophenyl)methylidene)-4-cyclohexyl-2-hydroxybutanohydrazide

### Example 14A

### tert-butyl (1R,2RS)-1-(cyclohexylmethyl)-3-hydrazino-2-hydroxy-3-oxopropylcarbamate

A solution of Example 13A (0.37 g, 0.82 mmol) in methanol (15mL) was treated with Pd-carbon (0.05 g), stirred under a hydrogen atmosphere for 16 hours, filtered, and concentrated to provide the desired product.

### Example 14B

### (2RS,3R)-3-amino-N'-((E)-(4-chlorophenyl)methylidene)-4-cyclohexyl-2-hydroxybutanohydrazide

A solution of Example 14A (0.064 g, 0.20 mmol) in ethanol (3mL) was treated with pyridine (2mL) and 4-chlorobenzaldehyde (0.033 mL, 0.23 mmol), heated to 85 °C, stirred for 16 hours, and concentrated. The concentrate was purified by flash column chormatography on silica gel with ethyl acetate in hexanes, and the purified concntrate was treated with 4N HCl in dioxane, stirred for 4 hours, concentrated, precipitated from diethyl ether, and collected by filtration to provide the desired product.
MS (APCI) m/e 336 (M-H)⁻;
¹H NMR (300 MHz, DMSO-d₆) δ 11.60 (m, 1H), 8.42 (m, 1H), 7.83 (m, 2H), 7.71 (m, 2H), 7.53 (m, 2H), 6.61 (m, 1H), 4.18 (m, 1H), 4.12 (m, 1H), 3.70 (m, 1H), 1.66 (m, 5H), 1.43 (m, 3H), 1.19 (m, 2H).

### Example 15

### ethyl 3-(2-((2RS,3R)-3-amino-4-cyclohexyl-2-hydroxybutanoyl)hydrazino)-3-oxopropanoate

A solution of Example 14A (0.096 g, 0.30 mmol) in dichloromethane (8 mL) at 0 °C was treated with N-methylmorpholine (0.094 mL, 0.67 mmol) and ethyl-3-chloro-3-oxo propionate (0.049 mL, 0.38 mmol), stirred for 30 minutes, warmed to room temperature and stirred for 16 hours. The mixture was treated with dichloromethane, washed sequentially with 0.5M HCl, water, and brine, dried (MgSO₄), filtered, and concentrated. The concentrate was purified by flash column chromatography on silica gel with ethyl acetate in hexanes, and the purified concentrate was treated with 4N HCl in dioxane, stirred for 4 hours, concentrated, precipitated from diethyl ether, and collected by filtration to provide the desired product.
MS (APCI) m/e 330 (M+H)⁺;
¹H NMR (300 MHz, DMSO-d₆) δ 10.24 (m, 1H), 7.71 (m, 2H), 6.53 (m, 1H), 4.10 (m, 5H), 3.70 (m, 1H), 3.49 (m, 2H), 3.37 (m, 1H), 1.65 (m, 5H), 1.43 (m, 3H), 1.20 (m, 5H).

### Example 16

### benzyl 2-((2RS, 3S)-3-amino-4-((cyclohexylmethyl)sulfanyl)-2-hydroxybutanoyl)hydrazinecarboxylate

### Example 16A

### (2S)-2-((tert-butoxycarbonyl)amino)-3-((cyclohexylmethyl)sulfanyl)propanoic acid

A solution of D-cysteine hydrochloride (4.78 g, 39.4 mmol) in liquid ammonia (250mL) at -70 °C was slowly treated with sodium (3.78 g, 161 mmol), stirred for 30 minutes, treated with (bromomethyl)cyclohexane (6.33 mL, 45.4 mmol), warmed to room temperature, and stirred until the ammonia evaporated. The residue was treated with water(75 mL), isopropanol (50 mL) and di-tert-butyl dicarbonate (9.97 mL, 43.3 mmol), stirred for 24 hours, and concentrated. The concentrate was dissolved in water (150 mL), cooled to 0 °C, adjusted to pH <7 with 3N HCI, and extracted with diethyl ether. The combined extracts were washed with brine, dried (MgSO₄), filtered, and concentrated to provide the desired product.
MS (APCI) m/e 318 (M+H)⁺.

### Example 16B

### (2RS,3R) 3-(tert-butoxycarbonylamino)-2-hydroxy-4-(cyclohexylmethylthio)butanoic acid

The desired product was prepared by substituting Example 16A for (2R)-2-((tert-butoxycarbonyl)amino)-3-cyclohexylpropanoic acid in Examples 1A-1C.
MS (APCI) m/e 346 (M+H)⁺.

### Example 16C

### benzyl 2-((2RS, 3S)-3-amino-4-((cyclohexylmethyl)sulfanyl)-2-hydroxybutanoyl)hydrazinecarboxylate

The desired product was prepared by substituting Example 16B for Example 1 C in Example 13.
MS (APCI) m/e 396 (M+H)⁺;
¹H NMR (300 MHz, DMSO-d₆) δ 9.32 (m, 1H), 7.97 (m, 2H), 7.38 (m, 5H), 6.63 (m, 1H), 5.10 (m, 2H), 4.23 (m, 1H), 3.69 (m, 3H), 2.43 (m, 2H), 1.83-1.58 (m, 6H), 1.18 (m, 5H).

### Example 17

### benzyl 2-((2RS, 3S)-3-amino-2-hydroxy-4-(propylsulfanyl)butanoyl)hydrazinecarboxylate

### Example 17A

### N-(tert-butoxycarbonyl)-S-propyl-D-cysteine

The desired product was prepared by substituting 1-bromopropane for (bromomethyl)cyclohexane in Example 16.
MS (APCI) m/e 264 (M+H)⁺.

### Example 17B

### (2RS,3R) 3-(tert-butoxycarbonylamino)-2-hydroxy-4-(propylthio)butanoic acid

The desired product was prepared by substituting Example 17A for (2R)-2-((tert-butoxycarbonyl)amino)-3-cyclohexylpropanoic acid in Examples 1A-1C.
MS (APCI) m/e 294 (M+H)⁺.

### Example 17C

### benzyl 2-((2RS, 3S)-3-amino-2-hydroxy-4-(propylsulfanyl)butanoyl)hydrazinecarboxylate

The desired product was prepared by substituting Example 17B for Example 1C in Example 13.
MS (APCI) m/e 342 (M+H)⁺;
¹H NMR (300 MHz, DMSO-d₆) δ 9.30 (m, 1H), 8.12 (m, 2H), 7.38 (m, 5H), 6.63 (m, 1H), 5.10 (m, 2H), 4.28 (m, 1H), 3.70 (m, 2H), 3.49(m, 1H), 2.84 (m, 1H), 2.48 (m, 1H), 1.52 (m, 2H), 0.95 (m, 3H).

### Example 18

### (2RS,3R)-3-amino-4-cyclohexyl-2-hydroxy-N'-(2,2,2-trifluoroethyl)butanohydrazide

The desired product was prepared by substituting 1-(2,2,2-trifluoroethyl)hydrazine for 1-benzylhydrazine dihydrochloride in Example 1.
MS (APCI) m/e 298 (M+H)⁺;
¹H NMR (300 MHz, CD₃OD) δ 4.17 (d, 1H), 3.48 (t, 2H), 3.68 (m, 1H), 1.84-0.90 (m, 13H).

### Example 19

### (2RS,3R)-3-amino-4-cyclohexyl-2-hydroxybutanohydrazide

The desired product was prepared by substituting tert-butyl hydrazinecarboxylate for 1-benzylhydrazine dihydrochloride in Example 1.
MS (APCI) m/e 216 (M+H)⁺;
¹H NMR (300 MHz, CD₃OD δ 4.39 (d, 1H), 3.68 (m, 1H), 1.84-0.92 (m, 13H).

### Example 20

### (2RS,3R)-3-amino-4-cyclohexyl-2-hydroxy-N'-methyl-N'-(3-nitro-2-pyridinyl)butanohydrazide

The desired product was prepared by substituting 2-(1-methylhydrazino)-3-nitropyridine for 1-benzylhydrazine dihydrochloride in Example 1.
MS (APCI) m/e 352 (M+H)⁺;
¹H NMR (300 MHz, CD₃OD) δ 8.39 (dd, 1H), 8.01 (d, 1H), 6.99 (dd, 1H), 4.14 (d, 1H), 3.66 (s, 3H), 3.53 (m, 1H), 1.84-0.92 (m, 13H).

### Example 21

### (2RS,3R)-3-amino-4-cyclohexyl-2-hydroxy-N-((2S)-2-(methoxymethyl)pyrrolidinyl)butanamide

The desired product was prepared by substituting (2S)-2-(methoxymethyl)-1-pyrrolidinamine for 1-benzylhydrazine dihydrochloride in Example 1.
MS (APCI) m/e 314 (M+H)⁺;
¹H NMR (300 MHz, CD₃OD) δ 4.33 (d, 1H), 4.21 (m, 1H), 3.68 (m, 2H), 3.60-3.42 (m, 2H), 3.34 (m, 5H), 1.98 (m, 2H), 1.88-1.65 (m, 7H), 1.52 (t, 2H), 1.37-1.20 (m, 4H), 1.60-0.89 (m, 2H).

### Example 22

### (2RS,3R)-3-amino-4-cyclohexyl-2-hydroxy-N-(1-pyrrolidinyl)butanamide

The desired product was prepared by substituting 1-pyrrolidinamine for 1-benzylhydrazine dihydrochloride in Example 1.
MS (APCI) m/e 270 (M+H)⁺;
¹H NMR (300 MHz, CD₃OD) δ 4.43 (d, 1H), 3.78-3.63 (m, 5H), 2.19 (m, 4H), 1.84-0.92 (m, 13H).

### Example 23

### (2RS,3R)-3-amino-4-cyclohexyl-N'-(2,4-difluorophenyl)-2-hydroxybutanohydrazide

The desired product was prepared by substituting 1-(2,4-difluorophenyl)hydrazine for 1-benzylhydrazine dihydrochloride in Example 1.
MS (APCI) m/e 328 (M+H)⁺;
¹H NMR (300 MHz, CD₃OD) δ 6.98-6.88 (m, 2H), 6.81 (m, 1H), 4.30 (d, 1H), 3.60 (m, 1H), 1.83-1.65 (m, 6H), 1.55-1.41 (m, 2H), 1.38-1.20 (m, 3H), 1.08-0.92 (m, 2H).

### Example 24

### (2RS,3R)-3-amino-N',4-dicyclohexyl-2-hydroxybutanohydrazide

The desired product was prepared by substituting 1-cyclohexylhydrazine for 1-benzylhydrazine dihydrochloride in Example 1.
MS (APCI) m/e 298 (M+H)⁺;
¹H NMR (300 MHz, CD₃OD) δ 4.42 (d, 1H), 3.79 (m, 1H), 3.67 (m, 1H), 2.14-2.05 (m, 2H), 1.95-1.86 (m, 2H), 1.81-1.68 (m, 6H), 1.55-1.25 (m, 11H), 1.08-0.92 (m, 2H).

### Example 25

### 4-(2-((2RS,3R)-3-amino-4-cyclohexyl-2-hydroxybutanoyl)hydrazino)benzenesulfonamide

The desired product was prepared by substituting 4-hydrazinobenzenesulfonamide for 1-benzylhydrazine dihydrochloride in Example 1.
MS (APCI) m/e 371 (M+H)⁺;
¹H NMR (300 MHz, CD₃OD) δ 7.72 (d, 2H), 6.90 (d, 2H), 4.34 (d, 1H), 3.67 (m, 1H), 1.88-1.65 (m, 6H), 1.60-1.47 (m, 2H), 1.37-1.21 (m, 3H), 1.02-0.92 (m, 2H).

### Example 26

### (2RS,3R)-3-amino-4-cyclohexyl-2-hydroxy-N'-phenylbutanohydrazide

The desired product was prepared by substituting 1-phenylhydrazine for 1-benzylhydrazine dihydrochloride in Example 1.
MS (APCI) m/e 292 (M+H)⁺;
¹H NMR (300 MHz, CD₃OD δ 7.19 (m, 2H), 6.83 (m, 3H), 4.29 (d, 1H), 3.61 (m, 1H), 1.84-1.64 (m, 6H), 1.56-1.42 (m, 2H), 1.38-1.17 (m, 3H), 1.18-0.90 (m, 2H).

### Example 27

### (2RS,3R)-3-amino-4-cyclohexyl-2-hydroxy-N'-(2-pyridinyl)butanohydrazide

The desired product was prepared by substituting 2-hydrazinopyridine for 1-benzylhydrazine dihydrochloride in Example 1.
MS (APCI) m/e 293 (M+H)⁺;
¹H NMR (300 MHz, CD₃OD) δ 8.01 (m, 1H), 7.78 (m, 1H), 6.93 (m, 2H), 4.39 (d, 1H), 3.68 (m, 1H), 1.85-0.98 (m, 13H).

### Example 28

### N'-((2RS,3R)-3-amino-4-cyclohexyl-2-hydroxybutanoyl)-4-ethoxybenzohydrazide

The desired product was prepared by substituting 4-ethoxybenzohydrazide for 1-benzylhydrazine dihydrochloride in Example 1.
MS (APCI) m/e 364 (M+H)⁺;
¹H NMR (300 MHz, CD₃OD δ 7.86 (d, 2H), 7.00 (d, 2H), 4.35 (d, 1H), 4.11 (q, 2H), 3.69 (m, 1H), 1.88-1.70 (m, 6H), 1.66-1.52 (m, 2H), 1.41 (t, 3H), 1.37-1.20 (m, 3H), 1.10-0.96 (m, 2H).

### Example 29

### (2RS,3R)-3-amino-4-cyclohexyl-2-hydroxy-N-(4-oxo-2-thioxo-1,3-thiazolidin-3-yl)butanamide

The desired product was prepared by substituting 3-amino-2-thioxo-1,3-thiazolidin-4-one for 1-benzylhydrazine dihydrochloride in Example 1.
MS (APCI) m/e 332 (M+H)⁺;
¹H NMR (300 MHz, CD₃OD) δ 4.38 (d, 1H), 4.25 (br s, 1H), 3.74 (m, 1H), 1.86-0.96 (m, 13H).

### Example 30

### (2RS,3R)-3-amino-N'-(6-chloro-3-pyridazinyl)-4-cyclohexyl-2-hydroxy-N'-methylbutanohydrazide

The desired product was prepared by substituting 3-chloro-6-(1-methylhydrazino)pyridazine for 1-benzylhydrazine dihydrochloride in Example 1.
MS (APCI) m/e 342 (M+H)⁺;
¹H NMR (300 MHz, CD₃OD) δ 7.57 (d, 1H), 7.33 (d, 1H), 4.40 (d, 1H), 3.64 (m, 1H), 3.40 (s, 3H), 1.86-1.67 (m, 6H), 1.64-1.16 (m, 5H), 1.08-0.92 (m, 2H).

### Example 31

### (2RS,3R)-3-amino-N'-(5-chloro-1-methyl-6-oxo-1,6-dihydro-4-pyridazinyl)-4-cyclohexyl-2-hydroxy-N'-methylbutanohydrazide

The desired product was prepared by substituting 4-chloro-2-methyl-5-(1-methylhydrazino)-3(2H)-pyridazinone for 1-benzylhydrazine dihydrochloride in Example 1. MS (APCI) m/e 372 (M+H)⁺.

### Example 32

### (2RS,3R)-3-amino-4-cyclohexyl-2-hydroxy-N-(1-piperidinyl)butanamide

The desired product was prepared by substituting 1-aminopiperidine for 1-benzylhydrazine dihydrochloride in Example 1.
MS (APCI) m/e 284 (M+H)⁺;
¹H NMR (300 MHz, CD₃OD) δ 4.47 (d, 1H), 3.75-3.58 (m, 2H), 3.54-3.40 (m, 3H), 1.86-1.50 (m, 13H), 1.47-1.37 (m, 1H), 1.37-1.16 (m, 3H), 1.06-0.89 (m, 2H).

### Example 33

### N'-((2RS,3R)-3-amino-4-cyclohexyl-2-hydroxybutanoyl)-3,5-dimethoxybenzohydrazide

The desired product was prepared by substituting 3,5-dimethoxybenzohydrazide for 1-benzylhydrazine dihydrochloride in Example 1.
MS (APCI) m/e 380 (M+H)⁺;
¹H NMR (300 MHz, CD₃OD) δ 7.03 (m, 2H), 6.70 (m, 1H), 4.36 (d, 1H), 3.81 (m, 6H), 3.68 (m, 1H), 1.87-1.68 (m, 6H), 1.63-1.41 (m, 2H), 1.38-1.19 (m, 3H), 1.10-0.93 (m, 2H).

### Example 34

### N'-((2RS,3R)-3-amino-4-cyclohexyl-2-hydroxybutanoyl)-1,3-benzodioxole-5-carbohydrazide

The desired product was prepared by substituting 1,3-benzodioxole-5-carbohydrazide for 1-benzylhydrazine dihydrochloride in Example 1.
MS (APCI) m/e 364 (M+H)⁺;
¹H NMR (300 MHz, CD₃OD δ 7.50 (dd, 1H), 7.35 (d, 1H), 6.92 (d, 1H), 4.35 (d, 1H), 4.27 (d, 1H), 3.80 (s, 2H), 3.61 (m, 1H), 1.86-1.18 (m, 11H), 1.10-0.92 (m, 2H).

### Example 35

### (2RS,3R)-3-amino-N'-(4-bromophenyl)-4-cyclohexyl-2-hydroxybutanohydrazide

The desired product was prepared by substituting 1-(4-bromophenyl)hydrazine for 1-benzylhydrazine in Example 1.
MS (APCI) m/e 371(M+H)⁺;
¹H NMR (300 MHz, CD₃OD) δ 7.30 (d. 2H), 6.77 (d, 2H), 4.29 (d, 1H), 3.62 (m, 1H), 1.86-1.64 (m, 6H), 1.56-1.16 (m, 5H), 1.07-0.98 (m, 2H).

### Example 36

### (2RS,3R)-3-amino-5-ethylsulfanyl-2-hydroxy-N'-(4-methylphenyl)pentanohydrazide

The desired product was prepared by substituting (2R)-2-((tert-butoxycarbonyl)amino)-4-(ethylsulfanyl)butanoic acid and 1-(4-methylphenyl)hydrazine for (2R)-2-((tert-butoxycarbonyl)amino)-3-cyclohexylpropanoic acid and 1-benzylhydrazine dihydrochloride, respectively, in Example 1.
MS (ESI) m/e 298 (M+H)⁺;
¹H NMR (300 MHz, DMSO-d₆) δ 10.04 (bs, 1H), 7.87 (bs, 1H), 6.95 (d, 2H), 6.68 (d, 2H), 4.13 (t, 1H), 3.43 (m, 1H), 2.73-2.60 (m, 2H), 2.56 (q, 2H), 2.18 (s, 3H), 1.98-1.78 (m, 2H), 1.21 (t, 3H).

### Example 37

### (2RS,3R)-3-amino-5-ethylsulfanyl-2-hydroxy-N'-(4-methoxyphenyl)pentanohydrazide

The desired product was prepared by substituting 1-(4-methoxyphenyl)hydrazine for 1-(4-methylphenyl)hydrazine in Example 36.
MS (ESI) m/e 314 (M+H)⁺;
¹H NMR (300 MHz, DMSO-d₆) δ 10.03 (bs, 1H), 7.86 (bs, 1H), 6.74 (m, 4H), 4.13 (m, 1H), 3.37 (s, 3H), 3.40 (m, 1H), 2.73-2.60 (m, 2H), 2.56 (q, 2H), 1.98-1.78 (m, 2H), 1.21 (t, 3H).

### Example 38

### (2RS,3R)-3-amino-5-ethylsulfanyl-2-hydroxy-N'-(1-naphthyl)pentanohydrazide

The desired product was prepared by substituting 1-(1-naphthyl)hydrazine for 1-(4-methylphenyl)hydrazine in Example 36.
MS (ESI) m/e 334 (M+H)⁺;
¹H NMR (300 MHz, DMSO-d₆) δ 10.27 (bs, 1H), 8.21 (m, 1H), 7.92 (bs, 1H), 7.83 (m, 1H), 7.48 (m, 2H), 7.32 (m, 1H), 6.80-6.72 (m, 2H), 4.25 (t, 1H), 3.48 (m, 1H), 2.73-2.60 (m, 2H), 2.56 (q, 2H), 1.98-1.78 (m, 2H), 1.21 (t, 3H).

### Example 39

### methyl 2-(2-((2RS,3R)-3-amino-4-cyclohexyl-2-hydroxybutanoyl)hydrazino)-4-(trifluoromethyl)-5-pyrimidinecarboxylate

The desired product was prepared by substituting methyl 2-hydrazino-4-(trifluoromethyl)-5-pyrimidinecarboxylate for 1-benzylhydrazine dihydrochloride in Example 1.
MS (APCI) m/e 420 (M+H)⁺.

### Example 40

### (2RS,3R)-3-amino-4-cyclohexyl-2-hydroxy-N'-(2-methylphenyl)butanohydrazide

The desired product was prepared by substituting 1-(2-methylphenyl)hydrazine for 1-benzylhydrazine dihydrochloride in Example 1.
MS (APCI) m/e 306 (M+H)⁺;
¹H NMR (300 MHz, CD₃OD) δ 7.10-7.02 (m, 2H), 6.85-6.73 (m, 2H), 4.32 (d, 1H), 3.75 (m, 1H), 2.25 (s, 3H), 1.88-1.65 (m, 6H), 1.58-1.46 (m, 2H), 1.38-1.18 (m, 3H), 1.09-0.92 (m, 2H).

### Example 41

### (2RS,3R)-3-amino-N'-(2-chlorophenyl)-4-cyclohexyl-2-hydroxybutanohydrazide

The desired product was prepared by substituting 1-(2-chlorophenyl)hydrazine for 1-benzylhydrazine dihydrochloride in Example 1.
MS (APCI) m/e 326 (M+H)⁺;
¹H NMR (300 MHz, CD₃OD) δ 7.28 (dd, 1H), 7.18 (m, 1H), 7.93 (dd, 1H), 6.82 (m, 1H), 4.34 (d, 1H), 3.66 (m, 1H), 1.86-1.66 (m, 6H), 1.58-1.18 (m, 5H), 1.06-0.89 (m, 2H).

### Example 42

### (2RS,3R)-3-amino-4-cyclohexyl-2-hydroxy-N'-(3-(trifluoromethyl)phenyl)butanohydrazide

The desired product was prepared by substituting 1-(2-(trifluoromethyl)phenyl)hydrazine for 1-benzylhydrazine dihydrochloride in Example 1.
MS (APCI) m/e 360 (M+H)⁺;
¹H NMR (300 MHz, CD₃OD) δ 7.36 (m, 1H), 7.08 (m, 3H), 4.32 (d, 1H), 3.62 (m, 1H), 1.88-1.62 (m, 6H), 1.58-1.16 (m, 5H), 1.08-0.87 (m, 2H).

### Example 43

### (2RS,3R)-3-amino-4-cyclohexyl-2-hydroxy-N'-(2-hydroxy-3-(3-(trifluoromethyl)phenoxy)propyl)-N'-methylbutanohydrazide

The desired product was prepared by substituting 1-(1-methylhydrazino)-3-(3-(trifluoromethyl)phenoxy)-2-propanol for 1-benzylhydrazine dihydrochloride in Example 1.
MS (APCI) m/e 448 (M+H)⁺;
¹H NMR (300 MHz, CD₃OD) δ 7.49 (m, 1H), 7.28-7.18 (m, 3H), 4.40 (dd, 1H), 4.33 (m, 1H), 4.10 (m, 2H), 3.58 (m, 1H), 3.34 (d, 3H), 3.12 (d, 2H), 1.84-1.62 (m, 6H), 1.58-1.20 (m, 5H), 1.06-0.87 (m, 2H).

### Example 44

### methyl 3-(2-((2RS,3R)-3-amino-4-cyclohexyl-2-hydroxybutanoyl)hydrazino)-2-thiophenecarboxylate

The desired product was prepared by substituting methyl 3-hydrazino-2-thiophenecarboxylate for 1-benzylhydrazine dihydrochloride in Example 1.
MS (APCI) m/e 356 (M+H)⁺;
¹H NMR (300 MHz, CD₃OD) δ 7.52 (d, 1H), 6.78 (d, 1H), 4.32 (d, 1H), 3.83 (s, 3H), 3.68 (m, 1H), 1.86-1.62 (m, 6H), 1.55-1.12 (m, 5H), 1.05-0.91 (m, 2H).

### Example 45

### (2RS,3R)-3-amino-4-cyclohexyl-2-hydroxy-N'-(2-pyridinylcarbonyl)butanohydrazide

The desired product was prepared by substituting 2-pyridinecarbohydrazide for 1-benzylhydrazine dihydrochloride in Example 1.
MS (APCI) m/e 321 (M+H)⁺;
¹H NMR (300 MHz, CD₃OD δ 8.81 (m, 1H), 8.33 (m, 2H), 7.88 (m, 1H), 4.40 (d, 1H), 3.71 (m, 1H), 1.88-1.64 (m, 6H), 1.64-1.42 (m, 2H), 1.42-1.22 (m, 3H), 1.10-0.94 (m, 2H).

### Example 46

### N'-((2RS,3R)-3-amino-4-cyclohexyl-2-hydroxybutanoyl)-2-chlorobenzohydrazide

The desired product was prepared by substituting 2-chlorobenzohydrazide for 1-benzylhydrazine dihydrochloride in Example 1.
MS (APCI) m/e 354 (M+H)⁺;
¹H NMR (300 MHz, CD₃OD) δ 7.62 (m, 1H), 7.50 (m, 2H), 7.42 (m, 1H), 4.37 (d, 1H), 3.72 (m, 1H), 1.86-1.63 (m, 6H), 1.63-1.40 (m, 2H), 1.40-1.16 (m, 3H), 1.10-0.92 (m, 2H).

### Example 47

### N'-((2RS,3R)-3-amino-4-cyclohexyl-2-hydroxybutanoyl)-3-bromobenzohydrazide

The desired product was prepared by substituting 3-bromobenzohydrazide for 1-benzylhydrazine dihydrochloride in Example 1.
MS (APCI) m/e 399 (M+H)⁺;
¹H NMR (300 MHz, CD₃OD δ 8.06 (t, 1H), 7.87 (m, 1H), 7.78 (m, 1H), 7.45 (t, 1H), 4.38 (d, 1H), 3.69 (m, 1H), 1.88-1.66 (m, 6H), 1.62-1.42 (m, 2H), 1.38-1.18 (m, 3H), 1.10-0.95 (m, 2H).

### Example 48

### N'-((2RS,3R)-3-amino-4-cyclohexyl-2-hydroxybutanoyl)-3-methoxybenzohydrazide

The desired product was prepared by substituting 3-methoxybenzohydrazide for 1-benzylhydrazine dihydrochloride in Example 1.
MS (APCI) m/e 350 (M+H)⁺;
¹H NMR (300 MHz, CD₃OD δ 7.48 (d, 1H), 7.42 (q, 2H), 7.17 (d, 1H), 4.37 (d, 1H), 3.86 (s, 3H), 3.70 (m, 1H), 1.88-1.67 (m, 6H), 1.63-1.40 (m, 2H), 1.40-1.18 (m, 3H), 1.10-0.95 (m, 2H).

### Example 49

### N-((2RS,3R)-3-amino-4-cyclohexyl-2-hydroxybutanoyl)-2,5-dichlorobenzohydrazide

The desired product was prepared by substituting 2,5-dichlorobenzohydrazide for 1-benzylhydrazine dihydrochloride in Example 1.
MS (APCI) m/e 389 (M+H)⁺;
¹H NMR (300 MHz, CD₃OD) δ 7.67 (m, 1H), 7.52 (d, 2H), 4.36 (d, 1H), 3.68 (m, 1H), 1.87-1.67 (m, 6H), 1.62-1.50 (m, 1H), 1.50-1.40 (m, 1H), 1.40-1.20 (m, 3H), 1.10-0.95 (m, 2H).

### Example 50

### N'-((2RS,3R)-3-amino-4-cyclohexyl-2-hydroxybutanoyl)-2-methoxybenzohydrazide

The desired product was prepared by substituting 2-methoxybenzohydrazide for 1-benzylhydrazine dihydrochloride in Example 1.
MS (APCI) m/e 350 (M+H)⁺;
¹H NMR (300 MHz, CD₃OD) δ 7.99 (dd, 1H), 7.57 (dt, 1H), 7.20 (d, 1H), 7.10 (t, 1H), 4.38 (d, 1H), 4.01 (s, 3H), 3.71 (m, 1H), 1.88-1.68 (m, 6H), 1.62-1.50 (m, 1H), 1.50-1.40 (m, 1H), 1.38-1.17 (m, 3H), 1.10-0.96 (m, 2H).

### Example 51

### N-((2RS,3R)-3-amino-4-cyclohexyl-2-hydroxybutanoyl)-3-chlorobenzohydrazide

The desired product was prepared by substituting 3-chlorobenzohydrazide for 1-benzylhydrazine dihydrochloride in Example 1.
MS (APCI) m/e 354 (M+H)⁺;
¹H NMR (300 MHz, CD₃OD) δ 7.91 (m, 1H), 7.82 (m, 1H), 7.62 (m, 1H), 7.51 (t, 1H), 4.38 (d, 1H), 3.69 (m, 1H), 1.88-1.68 (m, 6H), 1.62-1.52 (m, 1H), 1.52-1.40 (m, 1H), 1.38-1.20 (m, 3H), 1.10-0.94 (m, 2H).

### Example 52

### N'-((2RS,3R)-3-amino-4-cyclohexyl-2-hydroxybutanoyl)-3-methylbenzohydrazide

The desired product was prepared by substituting 3-methylbenzohydrazide for 1-benzylhydrazide dihydrochloride in Example 1.
MS (APCI) m/e 334 (M+H)⁺;
¹H NMR (300 MHz, CD₃OD) δ 7.75-7.66 (m, 2H), 7.48-7.34 (m, 2H), 4.37 (d, 1H), 3.69 (m, 1H), 2.41 (s, 3H), 1.88-1.67 (m, 6H), 1.62-1.50 (m, 1H), 1.50-1.40 (m, 1H), 1.40-1.18 (m, 3H), 1.10-0.94 (m, 2H).

### Example 53

### N'-((2RS,3R)-3-amino-4-cyclohexyl-2-hydroxybutanoyl)-2,4-dihydroxybenzohydrazide

The desired product was prepared by substituting 2,4-dihydroxybenzohydrazide for 1-benzylhydrazine dihydrochloride in Example 1.
MS (APCI) m/e 352 (M+H)⁺;
¹H NMR (300 MHz, CD₃OD) δ 7.72 (d,1H), 6.38 (dt, 2H), 4.37 (d, 1H), 3.67 (m, 1H), 1.87-1.65 (m, 6H), 1.62-1.52 (m, 1M, 1.52-1.49 (m, 1H), 1.39-1.18 (m, 3H), 1.10-0.97 (m, 2H).

### Example 54

### ethyl 3-(2-((2RS,3R)-3-amino-4-cyclohexyl-2-hydroxybutanoly)hydrazino)-6,6-dimethyl-4-oxo-4,5,6,7-tctrahydro-2-benzothiophene-1-carboxylate

The desired product was prepared by substituting ethyl 3-hydrazino-6,6-dimethyl-4-oxo-4,5,6,7-tetrahydro-2-benzothiophene-1-carboxylate for 1-benzylhydrazine dihydrochloride in Example 1.
MS (APCI) m/e 466 (M+H)⁺;
¹H NMR (300 MHz, CD₃OD) δ 4.38 (d, 1H), 4.28 (q, 2H), 3.67 (m, 1H), 2.54 (d, 2H), 2.39 (s, 2H), 1.90-1.71 (m, 6H), 1.71-1.43 (m, 2H), 1.40-1.16 (m, 6H), 1.08 (s, 3H), 1.06 (s, 3H), 1.10-0.94 (m, 2H).

### Example 55

### (2RS,3R)-3-amino-4-cyclohexyl-2-hydroxy-N'-(4-iodophenyl)butanohydrazide

The desired product was prepared by substituting 1-(4-iodophenyl)hydrazine for 1-benzylhydrazine dihydrochloride in Example 1.
MS (APCI) m/e 418 (M+H)⁺;
¹H NMR (300 MHz, CD₃OD) δ 7.47 (d, 2H), 6.66 (d, 2H), 4.28 (d, 1H), 3.62 (m, 1H), 1.85-1.62 (m, 6H), 1.55-1.42 (m, 2H), 1.37-1.17 (m, 3H), 1.08-0.88 (m, 2H).

### Example 56

### (2RS,3R)-3-amino-N'-( 1,3-benzothiazol-2-yl)-4-cyclohexyl-2-hydroxybutanohydrazide

The desired product was prepared by substituting 2-hydrazino-1,3-benzothiazole for 1-benzylhydrazine dihydrochloride in Example 1.
MS (APCI) m/e 349 (M+H)⁺;
¹H NMR (300 MHz, CD₃OD) δ 7.76 (dd, 1H), 7.51 (m, 2H), 7.33 (m, 1H), 3.87 (d, 1H), 3.78 (m, 1H), 1.82-1.60 (m, 6H), 1.48-1.16 (m, 5H), 1.16-0.91 (m, 2H).

### Example 57

### N'-((2RS,3R)-3-amino-4-cyclohexyl-2-hydroxybutanoyl)-2,5-dimethoxybenzohydrazide

The desired product was prepared by substituting 2,5-dimethoxybenzohydrazide for 1-benzylhydrazine dihydrochloride in Example 1.
MS (APCI) m/e 380 (M+H)⁺;
¹H NMR (300 MHz, CD₃OD) δ 7.54 (m, 1H), 7.14 (m, 2H), 4.38 (d, 1H), 3.96 (s, 3H), 3.79 (s, 3H), 3.71 (m, 1H), 1.88-1.67 (m, 6H), 1.62-1.50 (m, 1H), 1.50-1.40 (m, 1H), 1.38-1.18 (m, 3H), 1.10-0.93 (m, 2H).

### Example 58

### (2RS,3R)-3-amino-N'-(3-chlorophenyl)-4-cyclohexyl-2-hydroxybutanohydrazide

The desired product was prepared by substituting 1-(3-chlorophenyl)hydrazine for 1-benzylhydrazine dihydrochloride in Example 1.
MS (APCI) m/e 326 (M+H)⁺;
¹H NMR (300 MHz, CD₃OD δ 7.15 (t, 1H), 6.84-6.73 (m, 2H), 4.30 (d, 1H), 3.62 (m, 1H), 1.88-1.64 (m, 6H), 1.58-1.42 (m, 2H), 1.37-1.17 (m, 3H), 1.08-0.87 (m, 2H).

### Example 59

### (2RS,3R)-3-amino-4-cyclohexyl-2-hydroxy-N'-(3-methoxyphenyl)butanohydrazide

The desired product was prepared by substituting 1-(3-methoxyphenyl)hydrazine for 1-benzylhydrazine dihydrochloride in Example 1.
MS (APCI) m/e 322 (M+H)⁺;
¹H NMR (300 MHz, CD₃OD) δ 7.12-7.05 (m, 1H), 6.46-6.39 (m, 2H), 4.27 (d, 1H), 3.73 (s, 3H), 3.61 (m, 1H), 1.84-1.63 (m, 6H), 1.56-1.40 (m, 2H), 1.40-1.25 (m, 3H), 1.08-0.96 (m, 2H).

### Example 60

### (2RS,3R)-3-amino-4-cyclohexyl-N'-(3,5-dichlorophenyl)-2-hydroxybutanohydrazide

The desired product was prepared by substituting 1-(3,5-dichlorophenyl)hydrazine for 1-benzylhydrazine dihydrochloride in Example 1.
MS (APCI) m/e 361 (M+H)⁺;
¹H NMR (300 MHz, CD₃OD) δ 6.82 (m, 1H), 6.77 (m, 2H), 4.30 (d, 1H), 3.61 (m, 1H), 1.88-1.63 (m, 6H), 1.57-1.43 (m, 2H), 1.28-1.17 (m, 3H), 1.09-0.98 (m, 2H).

### Example 61

### (2RS,3R)-3-amino-N'-(3-bromophenyl)-4-cyclohexyl-2-hydroxybutanohydrazide

The desired product was prepared by substituting 1-(3-bromophenyl)hydrazine for 1-benzylhydrazine dihydrochloride in Example 1.
MS (APCI) m/e 371 (M+H)⁺;
¹H NMR (300 MHz, CD₃OD) δ 7.09 (t, 1H), 7.00-6.91 (m, 2H), 6.81 (m, 1H), 4.29 (d, 1H), 3.62 (m, 1H), 1.87-1.63 (m, 6H), 1.56-1.43 (m, 2H), 1.37-1.18 (m, 3H), 1.09-0.90 (m, 2H).

### Example 62

### (2RS,3R)-3-amino-4-cyclohexyl-2-hydroxy-N'-(trifluoroacetyl)butanohydrazide

The desired product was prepared by substituting 2,2,2-trifluoroacetohydrazide for 1-benzylhydrazine dihydrochloride in Example 1.
MS (APCI) m/e 312 (M+H)⁺;
¹H NMR (300 MHz, CD₃OD) δ 4.36 (d, 1H), 3.68 (m, 1H), 1.85-1.67 (m, 6H), 1.59-1.18 (m, 5H), 1.08-0.93 (m, 2H).

### Example 63

### (2RS,3R)-3-amino-4-cyclohexyl-2-hydroxy-N'-(4-isopropylphenyl)butanohydrazide

The desired product was prepared by substituting 1-(4-isopropylphenyl)hydrazine for 1-benzylhydrazine dihydrochloride in Example 1.
MS (APCI) m/e 334 (M+H)⁺;
¹H NMR (300 MHz, CD₃OD δ 7.12 (d, 2H), 6.78 (d, 2H), 4.29 (d, 1H), 3.61 (m, 1H), 2.83 (m, 1H), 1.82-0.95 (m, 19H).

### Example 64

### (2RS,3R)-3-amino-N'-(3-chloro-4-methylphenyl)-4-cyclohexyl-2-hydroxybutanohydrazide

The desired product was prepared by substituting 1-(3-chloro-4-methylphenyl)hydrazine for 1-benzylhydrazine dihydrochloride in Example 1.
MS (APCI) m/e 340 (M+H)⁺;
¹H NMR (300 MHz, CD₃OD) δ 7.91 (d, 1H), 7.85 (s, 1H), 6.73 (d, 1H), 4.28 (d, 1H), 3.62 (m, 1H), 2.25 (s, 3H), 1.83-1.00 (m, 13H).

### Example 65

### (2RS,3R)-3-amino-4-cyclohexyl-2-hydroxy-N'-(4-(trifluoromethoxy)phenyl)butanohydrazide

The desired product was prepared by substituting 1-(4-(trifluoromethoxy)phenyl)hydrazine for 1-benzylhydrazine dihydrochloride in Example 1. MS (APCI) m/e 376 (M+H)⁺;
¹H NMR (300 MHz, CD₃OD) δ 7.11 (d, 2H), 6.93 (m, 2H), 4.28 (d, 1H), 3.62 (m, 1H), 1.80-1.02 (m,13H).

### Example 66

### (2RS,3R)-3-amino-4-cyclohexyl-N'-(4-fluorophenyl)-2-hydroxybutanohydrazide

The desired product was prepared by substituting 1-(4-fluorophenyl)hydrazine for 1-benzylhydrazine dihydrochloride in Example 1.
MS (APCI) m/e 310 (M+H)⁺;
¹H NMR (300 MHz, CD₃OD) δ 6.94 (m, 4H), 4.31 (d, 1H), 3.58 (m, 1H), 1.83-0.97 (m,13H).

### Example 67

### (2RS,3R)-3-amino-N'-(4-chlorophenyl)-4-cyclohexyl-2-hydroxybutanohydrazide

The desired product was prepared by substituting 1-(4-chlorophenyl)hydrazine for 1-benzylhydrazine dihydrochloride in Example 1.
MS (APCI) m/e 326 (M+H)⁺;
¹H NMR (300 MHz, CD₃OD) δ 7.20 (d, 2H), 6.83 (d, 2H), 4.29 (d, 1H), 3.61 (m, 1H), 1.80-1.00 (m, 13H).

### Example 68

### (2RS,3R)-3-amino-4-cyclohexyl-N'-(2-ethylphenyl)-2-hydroxybutanohydrazide

The desired product was prepared by substituting 1-(2-ethylphenyl)hydrazine for 1-benzylhydrazine dihydrochloride in Example 1.
MS (APCI) m/e 320 (M+H)⁺;
¹H NMR (300 MHz, CD₃OD) δ 7.10 (m, 2H), 6.82 (m, 2H), 4.31 (d, 1H), 3.58 (m, 1H), 2.61 (q, 2H), 1.80-1.00 (m, 16H).

### Example 69

### (2RS,3R)-3-amino-4-cyclohexyl-N'-(3-fluorophenyl)-2-hydroxybutanohydrazide

The desired product was prepared by substituting 1-(3-fluorophenyl)hydrazine for 1-benzylhydrazine dihydrochloride in Example 1.
MS (APCI) m/e 310 (M+H)⁺;
¹H NMR (300 MHz, CD₃OD) δ 7.17 (m, 1H), 6.62 (m, 3H), 4.30 (d, 1H), 3.61 (m, 1H), 2.63 (q, 2H), 1.80-1.00 (m, 13H).

### Example 70

### N'-((2RS,3R)-3-amino-4-cyclohexyl-2-hydroxybutanoyl)-3-chloro-1-benzothiophene-2-carbohydrazide

The desired product was prepared by substituting 3-chloro-1-benzothiophene-2-carbohydrazide for 1-benzylhydrazine dihydrochloride in Example 1.
MS (APCI) m/e 410 (M+H)⁺;
¹H NMR (300 MHz, CD₃OD) δ 8.01 (d, 2H), 7.60 (d, 2H), 4.38 (d, 1H), 3.71 (m, 1H), 1.80-1.00 (m, 13H).

### Example 71

### N'-((2RS,3R)-3-amino-4-cyclohexyl-2-hydroxybutanoyl)-4-methylbenzohydrazide

The desired product was prepared by substituting 4-methylbenzohydrazide for 1-benzylhydrazine dihydrochloride in Example 1.
MS (APCI) m/e 334 (M+H)⁺;
¹H NMR (300 MHz, CD₃OD) δ 7.80 (d, 2H), 7.32 (d, 2H), 4.39 (d, 1H), 3.70 (m, 1H), 1.80-1.00 (m, 13H).

### Example 72

### N'-((2RS,3R)-3-amino-4-cyclohexyl-2-hydroxybutanoyl)-4-nitrobenzohydrazide

The desired product was prepared by substituting 4-nitrobenzohydrazide for 1-benzylhydrazine dihydrochloride in Example 1.
MS (APCI) m/e 365 (M+H)⁺;
¹H NMR (300 MHz, CD₃OD) δ 8.42 (d, 2H), 8.08 (d, 2H), 4.42 (d, 1H), 3.73 (m, 1H), 1.80-1.00.(m, 13H).

### Example 73

### N'-((2RS,3R)-3-amino-4-cyclohexyl-2-hydroxybutanoyl)-2-naphthohydrazide

The desired product was prepared by substituting 2-naphthohydrazide for 1-benzylhydrazine dihydrochloride in Example 1.
MS (APCI) m/e 370 (M+H)⁺;
¹H NMR (300 MHz, CD₃OD) δ 8.53 (s, 1H), 8.01 (m, 5H), 7.59 (m, 2H), 4.38 (d, 1H), 3.72 (m, 1H),1.80-1.00 (m, 13H).

### Example 74

### (2RS,3R)-3-amino-N'-(4-chloro-2-methylphenyl)-4-cyclohexyl-2-hydroxybutanohydrazide

The desired product was prepared by substituting 1-(4-chloro-2-methylphenyl)hydrazine for 1-benzylhydrazine dihydrochloride in Example 1.
MS (APCI) m/e 340 (M+H)⁺;
¹H NMR (300 MHz, CD₃OD) δ 7.08-7.01 (m, 2H), 6.77 (d, 1H), 4.33 (d, 1H), 3.66 (m, 1H), 2.23 (s, 3H), 1.86-1.64 (m, 6H), 1.57-1.44 (m, 2H), 1.37-1.22 (m, 3H), 1.05-0.92 (m, 2H).

### Example 75

### (2RS,3R)-3-amino-4-cyclohexyl-2-hydroxy-N'-mesitylbutanohydrazide

The desired product was prepared by substituting 1-mesitylhydrazine for 1-benzylhydrazine dihydrochloride in Example 1.
MS (APCI) m/e 334 (M+H)⁺;
¹H NMR (300 MHz, CD₃OD) δ 6.77 (bs, 1H), 6.74 (bs, 1H), 4.14 (d, 1H), 3.52 (m, 1H), 2.32 (d, 6H), 2.18 (d, 3H), 1.78-1.61 (m, 6H), 1.48-1.14 (m, 5H), 1.02-0.75 (m, 2H).

### Example 76

### (2RS,3R)-3-amino-4-cyclohexyl-2-hydroxy-N'-(4-((E)-2-(2-pyridinyl)ethenyl)phenyl)butanohydrazide

The desired product was prepared by substituting 2-((1E)-3-(4-hydrazinophenyl)-1-propenyl)pyridine for 1-benzylhydrazine dihydrochloride in Example 1.
MS (APCI) m/e 395 (M+H)⁺.

### Example 77

### (2RS,3R)-3-amino-4-cyclohexyl-N'-(2-fluorophenyl)-2-hydroxybutanohydrazide

The desired product was prepared by substituting 1-(2-fluorophenyl)hydrazine for 1-benzylhydrazine dihydrochloride in Example 1.
MS (APCI) m/e 310 (M+H)⁺;
¹H NMR (300 MHz, CD₃OD) δ 7.04 (dt, 1H), 7.00 (dd, 1H), 6.94 (dt, 1H), 6.82 (m, 1H), 4.31 (d, 1H), 3.64 (m, 1H), 1.86-1.64 (m, 6H), 1.57-1.42 (m, 2H), 1.42-1.17 (m, 3H), 1.08-0.92 (m, 2H).

### Example 78

### (2RS,3R)-3-amino-4-cyclohexyl-2-hydroxy-N'-(2-quinoxalinyl)butanohydrazide

The desired product was prepared by substituting 2-hydrazinoquinoxaline for 1-benzylhydrazine dihydrochloride in Example 1.
MS (APCI) m/e 344 (M+H)⁺;
¹H NMR (300 MHz, CD₃OD) δ 8.71 (s, 1H), 8.03 (d, 1H), 7.93 (d, 1H), 7.78 (t, 1H), 7.66 (t, 1H), 4.55 (d, 1H), 3.77 (m, 1H), 1.87-1.65 (m, 6H), 1.65-1.44 (m, 2H), 1.44-1.16 (m, 3H), 1.10-0.94 (m, 2H).

### Example 79

### (2RS,3R)-3-amino-4-cyclohexyl-2-hydroxy-N'-(4-(trifluoromethyl)phenyl)butanohydrazide

The desired product was prepared by substituting 1-(4-(trifluoromethyl)phenyl)hydrazine for 1-benzylhydrazine dihydrochloride in Example 1.
MS (APCI) m/e 360 (M+H)⁺;
¹H NMR (300 MHz, CD₃OD) δ 8.07 (d, 2H), 7.84 (d, 2H), 4.39 (d, 1H), 3.70 (m, 1H), 1.87-1.67 (m, 6H), 1.62-1.52 (m, 1H), 1.52-1.42 (m, 1H), 1.42-1.16 (m, 3H), 1.10-0.94 (m, 2H).

### Example 80

### (2RS,3R)-3-amino-N'-(2-chloro-6-fluorophenyl)-4-cyclohexyl-2-hydroxybutanohydrazide

The desired product was prepared by substituting 1-(2-chloro-6-fluorophenyl)hydrazine for 1-benzylhydrazine dihydrochloride in Example 1.
MS (APCI) m/e 344 (M+H)⁺;
¹H NMR (300 MHz, CD₃OD) δ 7.16 (dt, 1H), 7.07-6.99 (m, 1H), 6.92 (dt, 1H), 4.20 (d, 1H), 3.55 (m, 1H), 1.80-1.66 (m, 6H), 1.50-1.35 (m, 2H), 1.35-1.18 (m, 3H), 1.03-0.87 (m, 2H).

### Example 81

### (2RS,3R)-3-amino-4-cyclohexyl-2-hydroxy-N'-methyl-N'-(5-(trifluoromethyl)-2-pyridinyl)butanohydrazide

The desired product was prepared by substituting 2-(1-methylhydrazino)-5-(trifluoromethyl)pyridine for 1-benzylhydrazine dihydrochloride in Example 1.
MS (APCI) m/e 375 (M+H)⁺;
¹H NMR (300 MHz, CD₃OD) δ 8.42 (bs, 1H), 8.11 (dd, 1H), 7.25 (d, 1H), 4.50 (d, 1H), 3.72 (m, 1H), 3.48 (s, 3H), 1.87-1.66 (m, 6H), 1.62-1.52 (m, 1H), 1.52-1.45 (m, 1H), 1.38-1.21 (m, 3H), 1.08-0.93 (m,2H).

### Example 82

### (2RS,3R)-3-amino-4-cyclohexyl-N'-(2,5-difluorophenyl)-2-hydroxybutanohydrazide

The desired product was prepared by substituting 1-(2,5-difluorophenyl)hydrazine for 1-benzylhydrazine dihydrochloride in Example 1.
MS (APCI) m/e 328 (M+H)⁺;
¹H NMR (300 MHz, CD₃OD) δ 7.01 (m, 1H), 6.67 (m, 1H), 6.50 (m, 1H), 4.33 (d, 1H), 3.65 (m, 1H), 1.86-1.65 (m, 6H), 1.58-1.44 (m, 2H), 1.38-1.18 (m, 3H), 1.06-0.92 (m, 2H).

### Example 83

### (2RS,3R)-3-amino-4-cyclohexyl-N'-(1,3-dimethyl-4-nitro-1H-pyrazol-5-yl)-2-hydroxybutanohydrazide

The desired product was prepared by substituting 5-hydrazino-1,3-dimethyl-4-nitro-1H-pyrazole for 1-benzylhydrazine dihydrochloride in Example 1.
MS (APCI) m/e 355 (M+H)⁺;
¹H NMR (300 MHz, CD₃OD) δ 4.39 (d, 1H), 3.74 (s, 3H), 3.67 (m, 1H), 2.40 (s, 3H), 1.83-1.64 (m, 6H), 1.55-1.17 (m, 5H), 1.08-0.90 (m, 2H).

### Example 84

### 2-((2RS,3R)-3-amino-4-cyclohexyl-2-hydroxybutanoyl)-N-phenylhydrazinecarboxamide

The desired product was prepared by substituting N-phenylhydrazinecarboxamide for 1-benzylhydrazine dihydrochloride in Example 1.
MS (APCI) m/e 335 (M+H)⁺;
¹H NMR (300 MHz, CD₃OD) δ 7.41 (d, 2H), 7.28 (t, 2H), 7.03 (t, 1H), 4.33 (d, 1H), 3.68 (m, 1H), 1.87-1.67 (m, 6H), 1.60-1.50 (m, 1H), 1.50-1.38 (m, 1H), 1.37-1.18 (m, 3H), 1.08-0.97 (m, 2H).

### Example 85

### 2-((2RS,3R)-3-amino-4-cyclohexyl-2-hydroxybutanoyl)-N-(4-chloro-2-methoxyphenyl)hydrazinecarboxamide

The desired product was prepared by substituting N-(4-chloro-2-methoxyphenyl)hydrazinecarboxamide for 1-benzylhydrazine dihydrochloride in Example 1.
MS (APCI) m/e 399 (M+H)⁺;
¹H NMR (300 MHz, CD₃OD) δ 8.10 (d, 1H), 6.96 (d, 2H), 4.34 (d, 1H), 3.90 (s, 3H), 3.68 (m, 1H), 1.86-1.66 (m, 6H), 1.62-1.50 (m, 1H), 1.50-1.38 (m, 1H), 1.38-1.17 (m, 3H), 1.11-0.93 (m, 2H).

### Example 86

### 2-((2RS,3R)-3-amino-4-cyclohexyl-2-hydroxybutanoyl)-N-(3-fluorophenyl)hydrazinecarboxamide

The desired product was prepared by substituting N-(3-fluorophenyl)hydrazinecarboxamide for 1-benzylhydrazine dihydrochloride in Example 1.
MS (APCI) m/e 353 (M+H)⁺;
¹H NMR (300 MHz, CD₃OD δ 7.41 (d, 1H), 7.26 (q, 1H), 7.09 (d, 1H), 6.75 (t, 1H), 4.34 (d, 1H), 3.68 (m, 1H), 1.87-1.66 (m, 6H), 1.62-1.50 (m, 1H), 1.50-1.48 (m, 1H), 1.48-1.18 (m, 3H), 1.00-0.93 (m, 2H).

### Example 87

### N-((1R)-1-((2-((2RS,3R)-3-amino-4-cyclohexyl-2-hydroxybutanoyl)hydrazino)carbonyl)-3-(methylsulfanyl)propyl)-4-(trifluoromethyl)benzamide

The desired product was prepared by substituting N-((1R)-1-(hydrazinocarbonyl)-3-(methylsulfanyl)propyl)-4-(trifluoromethyl)benzamide for 1-benzylhydrazine dihydrochloride in Example 1.
MS (APCI) m/e 519 (M+H)⁺;
¹H NMR (300 MHz, CD₃OD δ 8.05 (d, 2H), 7.79 (d, 2H), 4.31 (d, 1H), 3.81 (q, 1H), 3.65 (m, 1H), 2.68 (m, 2H), 2.30-2.15 (m, 2H), 2.12 (s, 3H), 1.84-1.63 (m, 6H), 1.58-1.48 (m, 1H), 1.48-1.36 (m, 1H), 1.36-1.20 (m, 3H), 1.06-0.95 (m, 2H).

### Example 88

### (2RS,3R)-3-amino-4-cyclohexyl-2-hydroxy-N'-(2-thienylcarbonyl)butanohydrazide

The desired product was prepared by substituting 2-thiophenecarbohydrazide for 1-benzylhydrazine dihydrochloride in Example 1.
MS (APCI) m/e 326 (M+H)⁺;
¹H NMR (300 MHz, CD₃OD) δ 7.81 (dd, 2H), 7.18 (d, 1H), 4.42 (d, 1H), 3.73 (m, 1H), 1.80-1.00 (m, 13H).

### Example 89

### N'-((2RS,3R)-3-amino-4-cyclohexyl-2-hydroxybutanoyl)-4-chlorobenzohydrazide

The desired product was prepared by substituting 4-chlorobenzohydrazide for 1-benzylhydrazine dihydrochloride in Example 1.
MS (APCI) m/e 354 (M+H)⁺;
¹H NMR (300 MHz, CD₃OD) δ 7.91 (d, 2H), 7.55 (d, 2H), 4.43 (d, 1H), 3.68 (m, 1H), 1.80-1.00 (m, 13H).

### Example 90

### (2RS,3R)-3-amino-N'-benzoyl-4-cyclohexyl-2-hydroxybutanohydrazide

The desired product was prepared by substituting benzohydrazide for 1-benzylhydrazine dihydrochloride in Example 1.
MS (APCI) m/e 320 (M+H)⁺;
¹H NMR (300 MHz, CD₃OD) δ 7.91 (d, 2H), 7.58 (m, 3H), 4.42 (d, 1H), 3.68 (m, 1H), 1.80-1.00 (m, 13H).

### Example 91

### N'-((2RS,3R)-3-amino-4-cyclohexyl-2-hydroxybutanoyl)-4-bromobenzohydrazide

The desired product was prepared by substituting 3-bromobenzohydrazide for 1-benzylhydrazine dihydrochloride in Example 1.
MS (APCI) m/e 400 (M+H)⁺;
¹H NMR (300 MHz, CD₃OD) δ 7.81 (d, 2H), 7.72 (d, 2H), 4.37 (d, 1H), 3.69 (m, 1H), 1.80-1.00 (m, 13H).

### Example 92

### N'-((2RS,3R)-3-amino-4-cyclohexyl-2-hydroxybutanoyl)-4-tert-butylbenzohydrazide

The desired product was prepared by substituting for 1-benzylhydrazine dihydrochloride in Example 1.
MS (APCI) m/e 376 (M+H)⁺;
¹H NMR (300 MHz, CD₃OD) δ 7.83 (d, 2H), 7.55 (d, 2H), 4.41 (d, 1H), 3.73 (m, 1H), 1.80-1.00 (m, 22H).

### Example 93

### 4-chlorobenzyl 2-(2-((2RS,3R)-3-amino-4-cyclohexyl-2-hydroxybutanoyl)hydrazino)-4-(trifluoromethyl)-5-pyrimidinecarboxylate

The desired product was prepared by substituting 4-chlorobenzyl 2-hydrazino-4-(trifluoromethyl)-5-pyrimidinecarboxylate for 1-benzylhydrazine dihydrochloride in Example 1.
MS (APCI) m/e 530 (M+H)⁺;
¹H NMR (300 MHz, CD₃OD) δ 8.96 (bs, 1H), 7.41 (m, 4H), 4.34 (d, 1H), 3.62 (m, 1H), 1.85-1.67 (m, 6H), 1.62-1.40 (m, 2H), 1.40-1.17 (m, 3H), 1.08-0.93 (m, 2H).

### Example 94

### (2RS,3R)-3-amino-N'-(3-chloro-4-fluorophenyl)-4-cyclohexyl-2-hydroxybutanohydrazide

The desired product was prepared by substituting 3-chloro-4-fluorophenylhydrazine for 1-benzylhydrazine dihydrochloride in Example 1.
MS (APCI) m/e 344 (M+H)⁺;
¹H NMR (300 MHz, CD₃OD) δ 7.06 (t, 1H), 6.91 (q, 1H), 6.78 (m, 1H), 4.31 (d, 1H), 3.62 (m, 1H), 1.88-1.63 (m, 6H), 1.57-1.41 (m, 2H), 1.41-1.12 (m, 2H), 1.12-0.90 (m, 2H).

### Example 95

### (2RS,3R)-3-amino-N'-(6-chloro-3-pyridazinyl)-4-cyclohexyl-2-hydroxybutanohydrazide

The desired product was prepared by substituting 3-chloro-6-hydrazinopyridazine for 1-benzylhydrazine dihydrochloride in Example 1.
MS (APCI) m/e 328 (M+H)⁺;
¹H NMR (300 MHz, CD₃OD) δ 7.55 (d, 1H), 7.17 (d, I H), 4.38 (d, 1H), 3.64 (m, 1H), 1.87-1.68 (m, 6H), 1.56-1.49 (m, 1H), 1.49-1.37 (m, 1H), 1.37-1.17 (m, 3H), 1.07-0.95 (m, 2H).

### Example 96

### (2RS,3R)-3-amino-N'-(2-chlorobenzyl)-4-cyclohexyl-2-hydroxybutanohydrazide

The desired product was prepared by substituting 1-(2-chlorobenzyl)hydrazine for 1-benzylhydrazine dihydrochloride in Example 1.
MS (APCI) m/e 340 (M+H)⁺;
¹H NMR (300 MHz, CD₃OD) δ 7.54 (m, 1H), 7.49 (m, 1H), 7.28 (m, 2H), 4.13 (q, 2H), 4.11 (d, 1H), 3.52 (m, 1H), 1.80-1.66 (m, 6H), 1.60-1.50 (m, 1H), 1.45-1.16 (m, 4H), 1.00-0.96 (m, 2H).

### Example 97

### (2RS,3R)-3-amino-4-cyclohexyl-2-hydroxy-N-((2R)-2-(methoxymethyl)pyrrolidinyl)butanamide

The desired product was prepared by substituting (2R)-2-(methoxymethyl)-1-pyrrolidinamine for 1-benzylhydrazine dihydrochloride in Example 1.
MS (APCI) m/e 314 (M+H)⁺;
¹H NMR (300 MHz, CD₃OD) δ 4.11 (d, 1H), 3.58 (m, 1H), 3.45 (q, 1H), 3.35 (t, 1H), 3.32 (s, 3H), 3.21 (m, 1H), 3.07 (m, 1H), 2.80 (q, 2H), 2.08-1.93 (m, 1H), 1.88-1.68 (m, 6H), 1.67-1.53 (ml 1H), 1.53-1.48 (m, 2H), 1.48-1.18 (m, 3H), 1.04-0.98 (m, 2H).

### Example 98

### (2RS,3R)-3-amino-5-ethylsulfanyl-2-hydroxy-N-phenoxypentamide

### Example 98A

### 3-amino-3,4-dideoxy-5-S-ethyl-5-thio-D-glycero-pentonic acid

The desired product was prepared by substituting (2R)-2-((tert-butoxycarbonyl)amino)-4-(ethylsulfanyl)butanoic acid for (2R)-2-((tert-butoxycarbonyl)amino)-3-cyclohexylpropanoic acid in Examples 1A-C.

### Example 98B

### (2RS,3R)-3-amino-5-ethylsulfanyl-2-hydroxy-N-phenoxypentamide

A solution of Example 98A (0.39 g, 1.3 mmol), O-phenyl hydroxylamine hydrochloride (0.27 g, 1.9 mmol), 1-(3-dimethylaminopropyl)-3-ethylcarbodiimide hydrochloride (0.29 g, 1.5 mmol), 1-hydroxybenzotriazole hydrate (0.20 g, 1.5 mmol), and N-methylmorpholine (0.40 mL, 3.6 mmol) in 3:1 dichloromethane/DMF (8 mL) at room temperature was stirred for 16 hours, diluted with dichloromethane, washed sequentially with aqueous NaHCO₃, brine, 10% KHSO₄, and brine, dried (MgSO₄), filtered, and concentrated. The concentrate was purified by flash column chromatography on silica gel with 95:5/dichloromethane:methanol, and the purified concentrate was dissolved in saturated HCl/dioxane (2 mL), stirred for 1 hour, concentrated, treated with diethyl ether, then concentrated to provide the desired product.
MS (ESI) m/e 285 (M+H)⁺;
¹H NMR (300 MHz, DMSO-d₆) δ 12.31 (bs, 1H), 8.08 (bs, 0.4H), 7.94 (bs, 0.6H), 7.34 (m, 2H), 7.03 (m, 3H), 6.73 (d, 0.6H), 6.62 (d, 0.4H), 4.41 (m, 0.4H), 4.28 (t, 0.6H), 3.48 (m, 1H), 2.73-2.60 (m, 2H), 2.56 (q, 2H), 1.98-1.78 (m, 2H), 1.21 (t, 3H).

### Example 99

### (2RS,3R)-3-amino-4-cyclohexyl-2-hydroxy-N-phenoxybutanamide

The desired product was prepared by substituting Example 1C for Example 98A in Example 98B.
MS (ESI) m/e 293 (M+H)⁺;
¹H NMR (300 MHz, DMSO-d₆) δ 7.92 (bs, 1H), 7.34 (m, 2H), 7.06 (m, 3H), 6.73 (d, 0.4H), 6.70 (d, 0.6H), 4.21 (m, 1H), 3.36 (m, 1H),), 1.74-1.60 (m, 6H), 1.50-1.40 (m, 1H), 1.33-1.15 (m, 4H), 0.96-0.80 (m, 2H).

### Example 100

### (2RS,3R)-3-amino-N-(benzyloxy)-4-cyclohexyl-2-hydroxybutanamide

A solution of Example 1C (0.20 g, 0.66 mmol), O-benzyl hydroxylamine hydrochloride (0.22 g, 1.4 mmol), 1-(3-dimethylaminopropyl)-3-ethylcarbodiimide hydrochloride (0.17 g, 0.89 mmol), 1-hydroxybenzotriazole (0.14 g, 1.0 mmol), and N-methylmorpholine (0.40 mL, 3.6 mmol) in 5:1 dichloromethane/DMF (6 mL) at room temperature was stirred for 16 hours, diluted with dichloromethane, washed sequentially with aqueous NaHCO₃, brine, 10% KHSO₄, and brine, dried (MgSO₄) filtered, and concentrated. The concentrate was purified by flash column chromatography on silica gel with 98:2/dichloromethane:methanol, and the purified concentrate was dissolved in saturated HCl/dioxane (8 mL), stirred for 1 hour, concentrated, treated with diethyl ether, then concentrated to provide the desired product.
MS (ESI) m/e 307 (M+H)⁺;
¹H NMR (300 MHz, DMSO-d₆) δ 7.78 (br s, 1H), 7.40 (m, 5H), 6.42 (d, 1H), 4.84 (s, 2H), 3.95 (m, 1H), 1.74-1.60 (m, 6H), 1.50-1.40 (m, 1H), 1.33-1.15 (m, 4H), 0.96-0.80 (m, 2H).

### Example 101

### (2RS,3R)-3-amino-N-(methoxy)-4-cyclohexyl-2-hydroxybutanamide

The desired product was prepared by substituting O-methyl hydroxylamine hydrochloride for O-benzyl hydroxylamine hydrochloride in Example 100.
MS (APCI) m/e 231 (M+H)⁺;
¹H NMR (300 MHz, DMSO-d₆) δ 7.74 (br s, 1H), 6.42 (d, 1H) 3.95 (m, 1H), 3.63 (s, 3H), 1.74-1.60 (m, 6H), 1.50-1.40 (m, 1H), 1.33-1.15 (m, 4H), 0.96-0.80 (m, 2H).

### Example 102

### (2RS,3R)-3-amino-N-(tert-butoxy)-4-cyclohexyl-2-hydroxybutanamide

The desired product was prepared by substituting O-*tert*-butyl hydroxylamine hydrochloride for O-benzyl hydroxylamine hydrochloride in Example 100.
MS (APCI) m/e 273 (M+H)⁺;
¹H NMR (300 MHz, DMSO-d₆) δ 7.74 (br s, 1H), 6.44 (d, 1H) 3.92 (m, 1H), 1.74-1.60 (m, 6H), 1.50-1.40 (m, 1H), 1.33-1.15 (m, 4H), 1.19 (s, 9H), 0.96-0.80 (m, 2H).

### Example 103

### (2RS,3R)-3-amino-4-cyclohexyl-N-ethoxy-2-hydroxybutanamide

The desired product was prepared by substituting O-ethyl hydroxylamine hydrochloride for O-benzyl hydroxylamine hydrochloride in Example 100.
MS (APCI) m/e 245 (M+H)⁺;
¹H NMR (300 MHz, DMSO-d₆) δ 7.73 (br s, 1H), 6.41 (d, 1H), 3.92 (m, 1H), 3.84 (q, 2H), 1.74-1.60 (m, 6H), 1.50-1.40 (m, 1H), 1.33-1.15 (m, 4H), 1.14 (t, 3H), 0.96-0.80 (m, 2H).

### Example 104

### (2RS,3R)-N-(allyloxy,-3-amino-4-cyclohexyl-2-hydroxybutanamide

The desired product was prepared by substituting O-allyl hydroxylamine hydrochloride for O-benzyl hydroxylamine hydrochloride in Example 100.
MS (APCI) m/e 257 (M+H)⁺;
¹H NMR (300 MHz, DMSO-d₆) δ 7.74 (br s, 1H), 6.43 (d, 1H), 5.95 (m, 1H), 5.32 (dd, 1H), 5.27 (m, 1H), 4.32 (d, 2H), 3.92 (m, 1H), 1.74-1.60 (m, 6H), 1.50-1.40 (m, 1H), 1.33-1.15 (m, 4H), 0.96-0.80 (m, 2H).

### Example 105

### (2RS,3R)-3-amino-4-cyclohexyl-2-hydroxy-N-isobutoxybutanamide

The desired product was prepared by substituting O-*iso*butyl hydroxylamine hydrochloride for O-benzyl hydroxylamine hydrochloride in Example 100.
MS (APCI) m/e 273 (M+H)⁺;
¹H NMR (300 MHz, DMSO-d₆) δ 7.73 (br s, 1H), 6.39 (d, 1H), 3.92 (m, 1H), 3.58 (d, 2H), 1.88 (sept, 1H), 1.74-1.60 (m, 6H), 1.50-1.40 (m, 1H), 1.33-1.15 (m, 4H), 0.91 (d, 6H), 0.90-0.75 (m, 2H).

### Example 106

### (2RS,3R)-3-amino-4-cyclohexyl-N,2-dihydroxybutanamide

### Example 106A

### (2RS,3R)-3-(tert-butoxycarbonyl)amino-N-(benzyloxy)-4-cyclohexyl-2-hydroxybutanamide

A solution of Example 1C (0.20 g, 0.66 mmol), O-benzyl hydroxylamine hydrochloride (0.22 g, 1.4 mmol), 1-(3-dimethylaminopropyl)-3-ethylcarbodiimide hydrochloride (0.17 g, 0.89 mmol), 1-hydroxybenzotriazole (0.14 g, 1.0 mmol), and N-methylmorpholine (0.40 mL, 3.6 mmol) in 5:1 dichloromethane/DMF (6 mL) at room temperature was stirred for 16 hours, diluted with dichloromethane, washed sequentially with aqueous NaHCO₃, brine, 10% KHSO₄, and brine, dried (MgSO₄), filtered, and concentrated. The concentrate was purified by flash column chromatography on silica gel with 98:2/dichloromethane:methanol to provide the desired product.
MS (APCI) m/e 407 (M+H)⁺.

### Example 106B

### (2RS,3R)-3-amino-4-cyclohexyl-N,2-dihydroxybutanamide

A solution of Example 106A (0.33 g, 0.82 mmol), and 10% palladium on charcoal (0.13 g) in THF (8 mL) at room temperature was stirred for 16 hours under an atmosphere of hydrogen gas, filtered, and concentrated. The concentrate was dissolved in saturated HCl/dioxane (5 mL), stirred for 1 hour, concentrated, treated with diethyl ether, then concentrated to provide the desired product.
MS (APCI) m/e 217 (M+H)⁺;
¹H NMR (300 MHz, DMSO-d₆) δ 10.89 (br s, 1H), 8.89 (br s, 1H), 7.80 (br s, 2H), 6.31 (d, 1H), 3.92 (m, 1H), 1.74-1.60 (m, 6H), 1.50-1.40 (m, 1H), 1.33-1.15 (m, 4H), 0.90-0.75 (m, 2H).

### Example 107

### (2RS,3S)-3-amino-4-(ethylsulfanyl)-2-hydroxy-N-phenoxybutanamide

The desired product was prepared by substituting O-phenylhydroxylamine for 4-methylphenylhydrazine in Example 193.
MS (ESI) m/e 271 (M+H)⁺;
¹H NMR (500 MHz, CD₃OD) δ 7.32 (t, 2H), 7.10 (d, 2H), 7.06 (m, 1H), 4.56 (d, 0.65H), 4.54 (d, 0.35H), 3.66 (m, 1H), 2.95 (dd, 1H), 2.78 (dd, 1H), 2.64 (dd, 1.3H), 2.59 (dd, 0.7H), 1.29 (t, 1.95H), 1.25 (t, 1.05H).

### Example 108

### (2RS,3R)-3-amino-3-cyclohexyl-2-hydroxy-N-phenoxypropanamide

The desired product was prepared by substituting O-phenylhydroxylamine for 4-methylphenylhydrazine and Example 122A for Example 97A in Example 193.
MS (ESI) m/e 279 (M+H)⁺;
¹H NMR (500 MHz, CD₃OD) δ 7.33 (t, 2H), 7.08 (m, 3H), 4.51 (d, 0.65H), 4.48 (d, 0.35H), 3.37 (dd, 1H), 1.87-1.72 (m, 6H), 1.35-1.11 (m, 5H).

### Example 109

### (2RS,3 S)-3-amino-2-hydroxy-N-phenoxy-4-(propylsulfanyl)butanamide

The desired product was prepared by substituting O-phenylhydroxylamine for 4-methylphenylhydrazine and Example 195B for Example 97A in Example 193.
MS (ESI) m/e 285 (M+H)⁺;
¹H NMR (500 MHz, CD₃OD) δ 7.33 (t, 2H), 7.08 (m, 3H), 4.58 (d, 0.65H), 4.56 (d, 0.35H), 3.68 (m, 1H), 2.94 (dd, 0.65H), 2.87 (dd, 0.35H), 2.78 (m, 1H), 2.60 (dd, 1H), 2.54 (m, 1H), 1.65 (dd, 1H), 1.60 (dd, 1H), 1.02 (t, 1.95 H), 0.99 (t, 1.05H).

### Example 110

### (2RS,3R)-3-amino-2-hydroxy-5-(isopropylsulfanyl)-N-phenoxypentanamide

The desired product was prepared by substituting O-phenylhydroxylamine for 4-methylphenylhydrazine and Example 124B for Example 97A in Example 193.
MS (ESI) m/e 299 (M+H)⁺;
¹H NMR (500 MHz, CD₃OD) δ 7.33 (dd, 2H), 7.08 (m, 3H), 4.48 (d, 0.35H), 4.31 (d, 0.65H), 3.75 (m, 1H), 2.97 (dd, 0.65H), 2.93 (dd, 0.35H), 2.71 (m, 1.65H), 2.67 (m, 0.35H), 2.10 (dd, 0.65H), 1.99 (dd, 0.35H), 1.92 (dd, 1H), 1.28 (d, 3 H), 1.27 (d, 3H).

### Example 111

### (2RS,3S)-3-amino-2-hydroxy-4-(isobutylsulfanyl)-N-phenoxybutanamide

The desired product was prepared by substituting O-phenylhydroxylamine for 4-methylphenylhydrazine and Example 124B for Example 97A in Example 193.
MS (ESI) m/e 299 (M+H)⁺;
¹H NMR (500 MHz, CD₃OD) δ 7.33 (t, 2H), 7.08 (m, 3H), 4.60 (d, 1H), 3.69 (ddd, 1H), 2.93 (dd, 1H), 2.78 (dd, 1H), 2.50 (m, 2H), 1.83 (ddd, 111), 1.03 (d, 6H).

### Example 112

### (2RS,3R)-3-amino-5-phenyl-2-hydroxy-N-phenoxypentanamide

The desired product was prepared by substituting O-phenylhydroxylamine for 4-methylphenylhydrazine and Example 125A for Example 97A in Example 193.
MS (ESI) m/e 301 (M+H)⁺;
¹H NMR (500 MHz, CD₃OD) δ 7.30 (m, 3H), 7.26-7.20 (m, 4H), 7.06 (m, 3H), 4.48 (d, 0.5H), 4.45 (d, 0.5H), 3.59 (m, 1H), 2.79 (m, 1.5H), 2.71 (m, 0.5H), 2.15 (m, 0.5H), 2.02 (m, 1H), 1.94 (m, 0.5H).

### Example 113

### (2RS,3R)-3-amino-3-cyclooctyl-2-hydroxy-N-phenoxypropanamide

The desired product was prepared by substituting O-phenylhydroxylamine for 4-methylphenylhydrazine and Example 199A for Example 97A in Example 193.
MS (ESI) m/e 307 (M+H)⁺;
¹H NMR (500 MHz, CD₃OD) δ 7.33 (m, 2H), 7.10 (m, 3H), 3.86 (m, 1H), 3.79 (m, 1H), 1.80-1.52 (m, 15H).

### Example 114

### (2RS,3R)-3-amino-5-cyclohexyl-2-hydroxy-N-phenoxypentanamide

The desired product was prepared by substituting O-phenylhydroxylamine for 4-methylphenylhydrazine and Example 238A for Example 97A in Example 193.
MS (ESI) m/e 307 (M+H)⁺;
¹H NMR (500 MHz, CD₃OD) δ 7.33 (m, 2H), 7.10 (m, 3H), 3.89 (m, 1H), 3.71 (d, 1H), 1.74 (m, 3H), 1.65 (m, 2H), 1.53 (m, 2H), 1.29 (m, 4H), 1.20 (m, 2H), 0.95 (m, 2H).

### Example 115

### (2RS,3S)-3-amino-4-((cyclohexylmethyl)sulfanyl)-2-hydroxy-N-phenoxybutanamide

The desired product was prepared by substituting O-phenylhydroxylamine for 4-methylphenylhydrazine and Example 200B for Example 97A in Example 193.
MS (ESI) m/e 339 (M+H)⁺;
¹H NMR (500 MHz, CD₃OD) δ 7.32 (dd, 2H), 7.08 (dd, 3H), 4.60 (d, 0.65H), 4.56 (d, 0.35H), 3.70 (m, 1H), 2.91 (dd, 1H), 2.82 (dd, 0.35H), 2.77 (dd, 0.65H), 2.51 (m, 1.5H), 2.43 (m, 0.5H), 1.88 (m, 2H), 1.75-1.66 (m, 3H), 1.32-1.16 (m, 4H), 0.98 (m, 2H).

### Example 116

### ethyl ((((2RS,3R)-3-amino-4-cyclohexyl-2-hydroxybutanoyl)amino)oxy)acetate

The desired product was prepared by substituting O-(carboethoxy)methyl hydroxylamine hydrochloride for O-benzyl hydroxylamine hydrochloride in Example 100. MS (APCI) m/e 303 (M+H)⁺;
¹H NMR (300 MHz, DMSO-d₆) δ 7.95 (br s, 1H), 6.39 (d, 1H), 4.50 (d, 2H), 4.18 (m, 2H), 3.92 (m, 1H), 1.74-1.60 (m, 6H), 1.50-1.40 (m, 1H), 1.33-1.15 (m, 4H), 1.23 (t, 3H), 0.90-0.75 (m, 2H).

### Example 117

### benzyl ((((2RS,3R)-3-amino-4-cyclohexyl-2-hydroxybutanoyl)amino)oxy)acetate

### Example 117A

### benzyl ((((2RS,3R)-3-(tert-butoxycarbonyl)amino-4-cyclohexyl-2-hydroxybutanoyl)amino)oxy)acetate

The desired product was prepared by substituting O-(carbobenzyloxy)methyl hydroxylamine for O-(carboethoxy)methyl hydroxylamine hydrochloride in Example 106A. MS (APCI) m/e 465 (M+H)⁺.

### Example 117B

### benzyl ((((2RS,3R)-3-amino-4-cyclohexyl-2-hydroxybutanoyl)amino)oxy)acetate

Example 117A was dissolved in saturated HCl/dioxane (3 mL), stirred for 1 hour, concentrated, treated with diethyl ether, then concentrated to provide the desired product.
MS (APCI) m/e 365 (M+H)⁺;
¹H NMR (300 MHz, DMSO-d₆) δ 7.96 (br s, 1H), 7.39 (m, 5H), 6.39 (d, 1H), 5.20 (s, 2H), 4.49 (s, 2H), 3.92 (m, 1H), 1.74-1.60 (m, 6H), 1.50-1.40 (m, 1H), 1.33-1.15 (m, 4H), 0.90-0.75 (m, 2H).

### Example 118

### ((((2RS,3R)-3-amino-4-cyclohexyl-2-hydroxybutanoyl)amino)oxy)acetic acid

### Example 118A

### (((2RS,3R)-3-(tert-butoxycarbonyl)amino-4-cyclohexyl-2-hydroxybutanoyl)amino)oxy)acetic acid

A solution of Example 117A (0.99 g, 2.1 mmol), and 10% Palladium on charcoal (0.21 g) in THF (10 mL) at room temperature was stirred for 4 hours under an atmosphere of hydrogen gas, filtered, and concentrated to provide the desired product.
MS (APCI) m/e 375 (M+H)⁺;

### Example 118B

### ((((2RS,3R)-3-amino-4-cyclohexyl-2-hydroxybutanoyl)amino)oxy)acetic acid

Example 118A (0.17 g, 0.45 mmol) was dissolved in saturated HCl/dioxane (4 mL), stirred for 1 hour, concentrated, treated with diethyl ether, then concentrated to provide the desired product..
MS (APCI) m/e 275 (M+H)⁺;
¹H NMR (300 MHz, DMSO-d₆) δ 7.96 (br s, 1H), 6.39 (d, 1H), 5.20 (s, 2H), 3.92 (m, 1H), 1.74-1.60 (m, 6H), 1.50-1.40 (m, 1H), 1.33-1.15 (m, 4H), 0.90-0.75 (m, 2H).

### Example 119

### ethyl (2S)-2-((((((2RS,3R)-3-amino-4-cyclohexyl-2-hydroxybutanoyl)amino)oxy)acetyl)amino)propanoate

A solution of Example 118A (0.17 g, 0.45 mmol), L-alanine ethyl ester hydrochloride (0.098 g, 0.64 mmol), 1-(3-dimethylaminopropyl)-3-ethylcarbodiimide hydrochloride (0.098 g, 0.51 mmol), 1-hydroxybenzotriazole (0.091 g, 0.67 mmol), and N-methylmorpholine (0.11 mL, 1.0 mmol) in dichloromethane (5 mL) at room temperature was stirred for 16 hours, diluted with dichloromethane, washed sequentially with 1 M HCl, aqueous NaHCO₃, dried (MgSO₄), filtered, and concentrated. The concentrate was dissolved in saturated HCl/dioxane (4 mL), stirred for 1 hour, concentrated, then purified by HPLC to provide the desired product.
MS (ESI) m/e 374 (M+H)⁺;
¹H NMR (300 MHz, DMSO-d₆) δ 11.71 (br s, 1H), 8.56 (br s, 1), 7.74 (br s, 2H), 6.55 (d, 1H), 4.35 (d, 2H), 4.29 (m, 1H), 4.09 (m, 2H), 4.03 (m, 1H), 1.74-1.60 (m, 6H), 1.40-1.35 (m, 1H), 1.31 (d, 3H), 1.33-1.15 (m, 4H), 1.19 (t, 3H), 0.96-0.80 (m, 2H).

### Example 120

### (2RS,3R)-3-amino-4-cyclohexyl-2-hydroxy-N-(2-oxo-2-((2-phenylethyl)amino)ethoxy)butanamide

The desired product was prepared by substituting 2-phenylethylamine for L-alanine ethyl ester hydrochloride in Example 119.
MS (APCI) m/e 378 (M+H)⁺;
¹H NMR (300 MHz, DMSO-d₆) δ 11.69 (br s, 1H), 8.29 (br s, 1H), 7.75 (br s, 2H), 7.30 (m, 3H), 7.22 (m, 2H), 6.56 (d, 1H), 4.28 (d, 2H), 4.01 (m, 1H), 2.75 (m, 2H), 1.74-1.60 (m, 6H), 1.50-1.40 (m, 1H), 1.36 (m, 2H), 1.33-1.15 (m, 4H), 0.90-0.75 (m, 2H).

### Example 121

### (2RS,3R)-3-amino-5-(ethylsulfanyl)-2-hydroxy-N-benzyloxypentanamide

The desired product was prepared by substituting O-benzyl hydroxylamine hydrochloride for O-phenyl hydroxylamine hydrochloride in Example 98B.
MS (ESI) m/e 299 (M+H)⁺;
¹H NMR (300 MHz, DMSO-d₆) δ 11.51 (br s, 1H), 7.92 (br s, 0.3H), 7.84 (br s, 0.7H), 7.39 (m, 5H), 6.49 (d, 0.7H), 6.40 (d, 0.3H), 4.84 (s, 1.4H), 4.82 (s, 0.6H), 4.17 (m, 0.3H), 4.03 (m, 0.7H), 3.48 (m, 1H), 2.60 (m, 2H), 2.48 (q, 2H), 1.73 (m, 2H), 1.18 (t, 3H).

### Example 122

### (2RS,3R)-3-amino-N-(benzyloxy)-3-cyclohexyl-2-hydroxypropanamide

### Example 122A

### (2RS,3R)-3-amino-3-cyclohexyl-2-hydroxypropanoic acid

The desired product was prepared by substituting (2R)-2-((tert-butoxycarbonyl)amino)-2-(cyclohexyl)ethanoic acid for (2R)-2-((tert-butoxycarbonyl)amino)-3-cyclohexylpropanoic acid in Examples 1A-C.
MS (ESI) m/e 288 (M+H)⁺.

### Example 122B

### (2RS,3R)-3-amino-N-(benzyloxy)-3-cyclohexyl-2-hydroxypropanamide

The desired product was prepared by substituting O-benzyl hydroxylamine hydrochloride for O-phenyl hydroxylamine hydrochloride and Example 122A for Example 98A in Example 98B.
MS (ESI) m/e 293 (M+H)⁺;
¹H NMR (300 MHz, DMSO-d₆) δ 11.52 (s, 1H), 7.73 (br s, 2H), 7.41 (m, 5H), 6.40 (m, 1H), 4.85 (s, 2H), 4.08 (m, 1H), 1.77-1.44 (m, 6H), 1.20-0.94 (m, 5H).

### Example 123

### (2RS,3R)-3-amino-N-(benzyloxy)-2-hydroxy-5-(isopropylsulfanyl)pentanamide

### Example 123A

### (2R)-2-((tert-butoxycarbonyl)amino)-4-((isopropyl)sulfanyl)butanoic acid

A solution of D-homocystine (20 g, 75 mmol) in liquid ammonia (600 mL) was treated sequentially with sodium (8.9 g, 390 mmol) and 2-bromopropane (20 mL, 210 mmol). The ammonia was allowed to evaporate under a stream of nitrogen, and the residues take up in 1:1 2-propanol/water (500 mL), then treated with di-tert-butyl dicarbonate (50 g, 230 mmol) at room temperature for 6 hours, then concentrated. The residues were taken up in water and the pH adjusted to 10 with NaOH. The solution was washed twice with ether, then adjusted to pH 2 with HCl, then extracted twice with ethyl acetate. The ethyl acetate extracts were dried (MgSO₄), filtered, then concentrated to provide the desired product.
MS (ESI) m/e 279 (M+H)⁺.

### Example 123B

### (2RS,3R)-3-amino-2-hydroxy-5-(isopropylsulfanyl)pentanoic acid

The desired product was prepared by substituting Example 123A for (2R)-2-((tert-butoxycarbonyl)amino)-3-cyclohexylpropanoic acid in Examples 1A-C.
MS (ESI) m/e 308 (M+H)⁺.

### Example 123C

### (2RS,3R)-3-amino-N-(benzyloxy)-2-hydroxy-5-(isopropylsulfanyl)pentanamide

The desired product was prepared by substituting O-benzyl hydroxylamine hydrochloride for O-phenyl hydroxylamine hydrochloride and Example 123B for Example 98A in Example 98B.
MS (ESI) m/e 313 (M+H)⁺;
¹H NMR (300 MHz, DMSO-d₆) δ 11.50 (br s, 1H), 7.92 (br s, 0.6H), 7.84 (br s, 1.4H), 7.40 (m, 5H), 6.48 (d, 0.7H), 6.38 (d, 0.3H), 4.84 (s, 1.4H), 4.82 (s, 0.6H), 4.17 (m, 0.3H), 4.03 (m, 0.7H), 3.48 (m, 1H), 2.91 (m, 1H), 2.73 (m, 2H), 1.74 (m, 2H), 1.20 (d, 6H).

### Example 124

### (2RS,3S)-3-amino-N-(benzyloxy)-2-hydroxy-4-(isobutylsulfanyl)butanamide

### Example 124A

### (2S)-2-((tert-butoxycarbonyl)amino)-3-(isobutylsulfanyl)propanoic acid

The desired product was prepared by substituting D-cystine for D-homocystine and 1-bromo-2-methylpropane for 2-bromopropane in Example 123A.
MS (ESI) m/e 279 (M+H)⁺.

### Example 124B

### (2RS,3S)-3-((tert-butoxycarbonyl)amino)-2-hydroxy-4-(isobutylsulfanyl)butanoic acid

The desired product was prepared by substituting Example 124A for (2R)-2-((tert-butoxycarbonyl)amino)-3.-cyclohexylpropanoic acid in Examples 1A-C.
MS (ESI) m/e 308 (M+H)⁺.

### Example 124C

### (2RS,3S)-3-amino-N-(benzyloxy)-2-hydroxy-4-(isobutylsulfanyl)butanamide

The desired product was prepared by substituting O-benzyl hydroxylamine hydrochloride for O-phenyl hydroxylamine hydrochloride and Example 124B for Example 98A in Example 98B.
MS (ESI) m/e 360 (M+H)⁺;
¹H NMR (300 MHz, DMSO-d₆) δ 11.55 (br s, 1H), 7.93 (br s, 2H), 7.40 (m, 5H), 6.56 (m, 1H), 4.86 (s, 2H), 4.21 (m, 1H), 2.63 (m, 2H), 2.42 (d, 2H), 1.75 (m, 1H), 0.95 (d, 6H).

### Example 125

### (2RS,3R)-3-amino-N-(benzyloxy)-2-hydroxy-5-phenylpentanamide

### Example 125A

### (2RS,3R)-3-amino-2-hydroxy-5-phenylpentanoic acid

The desired product was prepared by substituting (2R)-2-((tert-butoxycarbonyl)amino)-4-phenylbutanoic acid for (2R)-2-((tert-butoxycarbonyl)amino)-3-cyclohexylpropanoic acid in Examples 1A-C.
MS (ESI) m/e 309 (M+H)⁺.

### Example 125B

### (2RS,3R)-3-amino-N-(benzyloxy)-2-hydroxy-5-phenylpentanamide

The desired product was prepared by substituting O-benzyl hydroxylamine hydrochloride for O-phenyl hydroxylamine hydrochloride and Example 125A for Example 98A in Example 98B.
MS (ESI) m/e 315 (M+H)⁺;
¹H NMR (300 MHz, DMSO-d₆) δ 7.86 (br s, 2H), 7.35 (m, 5H), 7.20 (m, 5H), 6.48 (m, 1H), 4.82 (s, 2H), 4.05 (m, 1H), 2.65 (m, 2H), 1.78 (m, 2H).

### Example 126

### (2S,3S)-3-amino-N-(cyclohexylmethoxy)-2-hydroxy-4-(isobutylsulfanyl)butanamide

### Example 126A

### O-Cyclohexylmethylhydroxylamine

The title compound was prepared by substituting cyclohexylmethyl bromide for Example 128A in Example 128B-128C.
¹H NMR (300 MHz, DMSO-d₆) δ 5.85 (s, 2H), 1.70-1.60 (m, 5H), 1.26-1.08 (m, 4H), 0.94-0.80 (m, 2H).

### Example 126B

### (2S,3S)-3-((tert-butoxycarbonyl)amino)-2-hydroxy-4-(isobutylsulfanyl)butanoic acid pentafluorophenyl ester

A solution of Example 124B (0.921 g, 3.0 mmol), pentafluorophenol (0.61 g, 3.3 mmol), 1-hydroxybenzotriazole (0.61 g, 4.5 mmol) and 1,3-dicyclohexyl-carbodiimide (0.62 g, 3.0 mmol in dichloromethane (10mL) was stirred at 0 °C for 1h and room temperature for 18 h. Dicyclohexyl urea was removed by filtration, and the filtrate was diluted with ether, washed with NaHCO₃ and then brine, dried (MgSO₄), filtered, and concentrated. The crude material was purified by silica gel chromatography (10:90/ethylacetate:hexanes) to give the title compound.
MS (ESI) m/e 472 (M-H)⁺.
¹H NMR (300 MHz, DMSO-d₆) δ 6.64 (d, 1H), 6.10 (d, 1H), 4.85 (dd, 1H), 4.14 (m, 1H), 2.74 (m, 1H), 2.56 (m, 1H), 2.47 (d, 2H), 1.77 (m, 1H), 1.37 (s, 9H), 0.95 (d, 6H).

### Example 126C

### (2S,3S)-3-amino-N-(cyclohexylmethoxy)-2-hydroxy-4-(isobutylsulfanyl)butanamide

A solution of Example 126B (0.24 g, 0.5 mmol) and Example 126A (0.065 g, 0.5 mmol) in DMF (5 mL) was stirred at room temperature for 18h, diluted with ether, washed with brine, dried (Na₂SO₄) and concentrated, purified by silica gel chromatography (10:90/ethylacetate:hexanes),then treated with 4M HCl in dioxane to give the title compound. MS (ESI) m/e 317 (M-H)⁺.
¹H NMR (300 MHz, DMSO-d₆) δ 11.38 (br s, 1H), 7.9 (br s, 2H), 6.5 (d, 1H), 4.18 (dd, 1H), 3.63 (d, 2H), 3.4 (m, 1H), 2.75-2.59 (m, 2H), 2.53 (d, 2H), 1.8-1.6 (m, 7H), 1.24-1.10 (m, 4H), 1.0-0.9 (m, 1H), 0.95 (d, 6H).

### Example 127

### (2S,3S)-3-amino-2-hydroxy-4-(isobutylsulfanyl) N-(mesitylmethoxy)butanamide

### Example 127A

### O-2,4,6-trimethylbenzylhydroxylamine

The title compound was prepared by substituting 2,4,6-trimethylbenzyl bromide for Example 128A in Example 128B-128C.
¹H NMR (300 MHz, DMSO-d₆) δ 6.80 (s, 2H), 5.96 (s, 2H), 4.57 (s, 2H), 2.30 (s, 6H), 2.20 (s, 3H).

### Example 127B

### (2S,3S)-3-amino-2-hydroxy-4-(isobutylsulfanyl)-N-(mesitylmethoxy)butanamide

The title compound was prepared by substituting Example 127A for Example 126B in Example 126C.
MS (ESI) m/e 353 (M-H)⁺.
¹H NMR (300 MHz, DMSO-d₆) δ 11.55 (br s, 1H), 7.78 (br s, 2H), 6.85 (s, 2H), 6.57 (d, 1H), 4.87 (s, 2H), 4.24 (dd, 1H), 3.44(m, 1H), 2.75-2.59 (m, 2H), 2.44 (d, 2H), 2.38 (s, 6H), 2.21 (s, 3H), 1.8-1.7 (m,1H), 0.95 (d, 6H).

### Example 128

### (2RS,3R)-3-amino-2-hydroxy-5-(ethylsulfanyl)-N-((1RS)-1-phenylethoxy)pentanamide

### Example 128A

### 1-bromoethylbenzene

To a solution of DL-sec-phenethyl alcohol (1.81 ml, 15 mmol) in chloroform (20 ml) was added phosphorus tribromide (15.75 ml, 15.75mmol) dropwise at room temperature. The mixture was stirred at room temperature for 18 hours, poured into wet ice, washed with brine (4X), dried (MgSO₄), then evaporated to dryness to yield 2.62 g of the title compound.

### Example 128B

### N-(1-phenylethoxy)phthalimide

N-hydroxyphthalamide (2.31g, 14.2 mmol), Example 128A (2.62g, 14.2mmol) and potassium carbonate (4.91g, 35.5 mmol) in N,N-dimethylformamide (35 mL) were stirred at room temperature for 1 day, and stirred in 50°C oil bath for 5 hours. The mixture was poured into ice-water, the precipitate was collected by filtration, washed with water and dried to yield 2.69g of the title compound.

### Example 128C

### O-(1-(phenyl)ethyl)hydroxylamine

Example 128B (2.18g, 8.2mmol) and hydrazine hydrate (0.306ml, 9.84mmol) in ethanol (35 mL) was stirred at room temperature for 1.5 hours. The solvent was removed, the residue triturated with 30 ml of ether, filtered, and the filtrate was concentrated in vacuo to yield the title compound (1.09g) as an oil.

### Example 128D

### (2RS,3R)-3-amino-2-hydroxy-5-(ethylsulfanyl)-N-((1RS)-1-phenylethoxy)pentanamide

The desired product was prepared by substituting Example 128C for O-phenyl hydroxylamine hydrochloride in Example 98B.
MS (ESI) m/e 313(M+H)⁺;
¹H NMR (300 MHz, DMSO-d₆) δ 11.28 (d, 0.5H), 11.25 (d, 0.5H), 7.89-8.08 (br. 3H), 7.28-7.43 (m, 5H), 6.43 (d, 0.6H), 6.33 (d, 0.4H), 4.92-5.03 (m, 1H), 4.17 (br., 0.4H), 3.98 (br., 0.6H), 2.42-2.67 (m, 4H), 1.60-1.73 (m, 2H), 1.42-1.46 (m, 3H), 1.13-1.21 (m, 3H).

### Example 129

### (2S,3S)-3-amino-N-(benzyloxy)-2-hydroxy-4-(isobutylsulfanyl)-N-methylbutanamide

### Example 129A

### N-methyl-O-benzylhydroxylamine hydrochloride

O-Benzylhydroxylamine hydrochloride (1.59 g, 0.01 mol), di-tert-butyl dicarbonate (2.18 g, 0.01 mol) and N-methyl morpholine (1.1 mL, .01 mol) in dichloromethane (40 mL) were stirred at room temperature for 16 hours. The reaction mixture was diluted with ether, washed with 10% NaHSO₄, then brine, dried over (Na₂SO₄), and concentrated. The residue was treated with methyl iodide (1.6 mL, 0.025 mmol) in the presence of NaH (0.4 g, 0.01 mol) in THF at 0° C, then stirred at room temperature for 16 hours. The reaction was quenched with 1N HCl, diluted with ether, washed with brine, dried (Na₂SO₄), concentrated, purified by silica gel chromatography (20:80/ethyl acetate:hexanes) and treated with 4M HCl in dioxane to give the title compound.

### Example 129B

### (2S,3S)-3-amino-N-(benzyloxy)-2-hydroxy-4-(isobutylsulfanyl)-N-methylbutanamide

The title compound was prepared by substituting Example 129A for Example 126A in Example 126C.
MS (ESI) m/e 327 (M+H)⁺.
¹H NMR (300 MHz, DMSO-d₆) δ 7.9 (br s, 2H), 7.49 (m, 2H), 7.4 (m, 3H), 6.22 (br s, 1H), 4.98 (m, 2H), 4.79 (br s, 1H), 3.24(s, 3H), 2.55 (m, 2H), 2.3 (m, 2H),), 1.68 (m,1H), 0.9 (d, 6H).

### Example 130

### (2RS,3S)-3-amino-N-(benzyloxy)-2-hydroxy-5-(isopropylsulfanyl)-N-methylpentanamide

The desired product was prepared by substituting Example 129A for O-phenyl hydroxylamine hydrochloride and Example 123B for Example 98A in Example 98B.
MS (ESI) m/e 327 (M+H)⁺.
¹H NMR (300 MHz, DMSO-d₆) δ 8.08 & 7.88 (br s, 2H), 7.55-7.38 (m, 5H), 6.25 & 5.97 (br s, 1H), 4.98 (br s, 2H), 4.77 & 4.55 (br s, 1H), 3.7 & 3.48 (m, 1H), 3.2 (br s, 3H), 2.85 (m, 1H), 1.8-1.6 (m, 2H), 1.15 (m,6H).

### Example 131

### (2RS,3R)-3-amino-2-hydroxy-5-(isopropylsulfanyl)-N-((1RS)-1-phenylethoxy)pentanamide

The desired product was prepared by substituting Example 128C for O-phenyl hydroxylamine hydrochloride and Example 123B for Example 98A in Example 98B.
MS (ESI) m/e 327(M+H)⁺;
¹H NMR (300 MHz, DMSO-d₆) δ 11.26 (d, 0.6H), 11.24 (d, 0.4H), 7.88-8.10 (br., 3H), 7.30-7.44 (m, 5H), 6.45 (d, 0.6H), 6.36 (d, 0.4H), 4.90-5.02 (m, 1H), 3.97 (br.m, 1H) 3.64-3.73 (m, 1H), 2.82-2.94 (m, 2H), 2.53-2.73 (m, 2H), 1.58-1.72 (m, 2H), 1.42-1.47 (m, 3H), 1.16-1.23 (m, 6H).

### Example 132

### (2RS,3R)-3-amino-4-cyclohexyl-2-hydroxy-N'-((3RS)-3-(methylsulfanyl)butyl)-N'-phenylbutanohydrazide

The desired product was prepared by substituting 1-(3-(methylsulfanyl)butyl)-1-phenylhydrazine for 1-benzylhydrazine dihydrochloride in Example 1.
MS (ESI) m/e 394 (M+H)⁺;
¹H NMR (300 MHz, CD₃OD) δ 7.20 (m, 2H), 6.88 (m, 2H), 6.80 (m, 1H), 4.27 (dd, 1H), 3.57 (m, 3H), 2.17 (m, 1H), 2.07 (s, 3H), 1.42-1.91 (m, 6H), 1.11-1.42 (m, 4H), 0.76-1.11 (m, 8H).

### Example 133

### (2RS,3R)-3-amino-4-cyclohexyl-2-hydroxy-N'-phenyl-N'-(3-phenylpropyl)butanohydrazide

The desired product was prepared by substituting 1-(3-phenylpropyl)-1-phenylhydrazine for 1-benzylhydrazine dihydrochloride in Example 1.
MS (ESI) m/e 410 (M+H)⁺;
¹H NMR (300 MHz, CD₃OD) δ 7.05-7.41 (m, 8H), 6.83 (m, 2H), 4.26 (d, 1H), 3.57 (m, 1H), 3.48 (m, 2H), 2.74 (t, 2H), 1.86-2.07 (m, 2H), 1.55-1.86 (m, 7H), 1.47 (m, 2H), 1.07-1.39 (m, 2H), 0.75-1.07 (m, 2H).

### Example 134

### (2RS,3R)-3-amino-4-cyclohexyl-2-hydroxy-N'-isobutyl-N'-phenylbutanohydrazide

The desired product was prepared by substituting 1-(2-methylpropyl)-1-phenylhydrazine for 1-benzylhydrazine dihydrochloride in Example 1.
MS (ESI) m/e 348 (M+H)⁺;
¹H NMR (300 MHz, CD₃OD) δ 7.20 (m, 5H), 6.87 (m, 2H), 6.80 (m, 1H), 4.24 (d, 1H), 3.54 (m, 1H), 3.28 (d, 2H), 1.99 (m, 1H), 1.58-1.91 (m, 6H), 1.50 (m, 2H), 1.17-1.42 (m, 3H), 1.02 (s, 3H), 1.00 (s, 3H), 0.91 (m, 2H).

### Example 135

### (2RS,3R)-3-amino-4-cyclohexyl-2-hydroxy-N'-pentyl-N'-phenylbutanohydrazide

The desired product was prepared by substituting 1-pentyl-1-phenylhydrazine for 1-benzylhydrazine dihydrochloride in Example 1.
MS (ESI) m/e 362 (M+H)⁺;
¹H NMR (300 MHz, CD₃OD) δ 7.22 (m, 2H), 6.89 (m, 2H), 6.82 (m, 1H), 4.27 (d, 1H), 3.59 (m, 1H), 3.46 (dd, 2H), 1.58-1.92 (m, 6H), 1.11-1.58 (m, 12H), 0.83-1.11 (m, 4H).

### Example 136

### (2RS,3R)-3-amino-4-cyclohexyl-2-hydroxy-N'-(2-methylbutyl)-N'-phenylbutanohydrazide

The desired product was prepared by substituting 1-(2-methylbutyl)-1-phenylhydrazine for 1-benzylhydrazine dihydrochloride in Example 1.
MS (ESI) m/e 362 (M+H)⁺;
¹H NMR (300 MHz, CD₃OD) δ 7.20 (m, 2H), 6.88 (m, 2H), 6.80 (m, 1H), 4.24 (dd, 1H), 3.53 (m, 1H), 3.40 (m, 1H), 3.24 (m, 1H), 1.42-1.91 (m, 9H), 1.11-1.42 (m, 4H), 0.76-1.11 (m, 8H).

### Example 137

### (2RS,3R)-3-amino-4-cyclohexyl-2-hydroxy-N'-isopentyl-N'-phenylbutanohydrazide

The desired product was prepared by substituting 1-(3-methylbutyl)-1-phenylhydrazine for 1-benzylhydrazine dihydrochloride in Example 1.
MS (ESI) m/e 362 (M+H)⁺;
¹H NMR (300 MHz, CD₃OD) δ 7.22 (m, 2H), 6.89 (m, 2H), 6.83 (m, 1H), 4.28 (d, 1H), 3.58 (m, 1H), 3.49 (m, 2H), 1.61-1.92 (m, 7H), 1.52 (m, 4H), 1.11-1.42 (m, 3H), 0.83-1.11 (m, 8H).

### Example 138

### (2RS,3R)-3-amino-4-cyclohexyl-N'-hexyl-2-hydroxy-N'-phenylbutanohydrazide

The desired product was prepared by substituting 1-hexyl-1-phenylhydrazine for 1-benzylhydrazine dihydrochloride in Example 1.
MS (ESI) m/e 376 (M+H)⁺;
¹H NMR (300 MHz, CD₃OD) δ 7.22 (m, 2H), 6.90 (m, 2H), 6.82 (m, 1H), 4.27 (d, 1H), 3.57 (m, 1H), 3.45 (m, 2H), 1.59-1.91 (m, 7H), 1.11-1.59 (m, 12H), 0.84-1.11 (m, 5H).

### Example 139

### (2RS,3R)-3-amino-4-cyclohexyl-2-hydroxy-N'-(2-methylpentyl)-N'-phenylbutanohydrazide

The desired product was prepared by substituting 1-(2-methylpentyl)-1-phenylhydrazine for 1-benzylhydrazine dihydrochloride in Example 1.
MS (ESI) m/e 376 (M+H)⁺;
¹H NMR (300 MHz, CD₃OD δ 7.20 (m, 2H), 6.87 (m, 2H), 6.80 (m, 1H), 4.19-4.28 (m, 1H), 3.53 (dt, 1H), 3.39 (m, 1H), 3.24 (m, 1H), 1.59-1.99 (m, 7H), 1.50 (m, 4H), 1.04-1.42 (m, 6H), 1.00 (d, 3H), 0.93 (t, 3H), 0.83-1.05 (m, 2H).

### Example 140

### (2RS,3R)-3-amino-4-cyclohexyl-2-hydroxy-N'-(3-methylpentyl)-N'-phenylbutanohydrazide

The desired product was prepared by substituting 1-(3-methylpentyl)-1-phenylhydrazine for 1-benzylhydrazine dihydrochloride in Example 1.
MS (ESI) m/e 376 (M+H)⁺;
¹H NMR (300 MHz, CD₃OD) δ 7.22 (m, 2H), 6.88 (m, 2H), 6.83 (m, 1H), 4.27 (d, 1H), 3.39-3.65 (m, 3H), 1.59-1.93 (m, 7H), 1.37-1.59 (m, 5H), 1.10-1.37 (m, 4H), 0.83-1.10 (m, 8H).

### Example 141

### (2RS,3R)-3-amino-4-cyclohexyl-N'-(3,3-dimethylbutyl)-2-hydroxy-N'-phenylbutanohydrazide

The desired product was prepared by substituting 1-(3,3-dimethylbutyl)-1-phenylhydrazine for 1-benzylhydrazine dihydrochloride in Example 1.
MS (ESI) m/e 376 (M+H)⁺;
¹H NMR (300 MHz, CD₃OD) δ 7.22 (m, 2H), 6.88 (m, 2H), 6.83 (m, 1H), 4.29 (d, 1H), 3.52 (m, 4H), 1.42-1.91 (m, 10H), 1.11-1.42 (m, 3H), 0.76-1.11 (m, 11H).

### Example 142

### (2RS,3R)-3-amino-4-cyclohexyl-N'-(2-ethylbutyl)-2-hydroxy-N'-phenylbutanohydrazide

The desired product was prepared by substituting 1-(2-ethylbutyl)-1-phenylhydrazine for 1-benzylhydrazine dihydrochloride in Example 1.
MS (ESI) m/e 376 (M+H)⁺;
¹H NMR (300 MHz, CD₃OD δ 7.20 (m, 2H), 6.88 (m, 2H), 6.81 (m, 1H), 4.23 (d, 1H), 3.52 (dt, 1H), 3.37 (d, 2H), 1.58-1.92 (m, 7H), 1.38-1.58 (m, 6H), 1.10-1.38 (m, 3H), 0.82-1.10 (m, 8H).

### Example 143

### (2RS,3R)-3-amino-4-cyclohexyl-N'-(cyclopropylmethyl)-2-hydroxy-N'-phenylbutanohydrazide

The desired product was prepared by substituting 1-(cyclopropylmethyl)-1-phenylhydrazine for 1-benzylhydrazine dihydrochloride in Example 1 MS (ESI) m/e 346 (M+H)⁺;
¹H NMR (300 MHz, CD₃OD δ 7.22 (m, 2H), 6.93 (m, 2H), 6.85 (m, 1H), 4.30 (d, 1H), 3.60 (ddd, 1H), 3.43 (m, 1H), 3.28 (m, 1 H), 1.64-1.94 (m, 6H), 1.42-1.64 (m, 2H), 1.16-1.42 (m, 3H), 0.69-1.16 (m, 3H), 0.41-0.69 (m, 2H), 0.15-0.41 (m, 2H).

### Example 144

### (2RS,3R)-3-amino-4-cyclohexyl-N'-dodecyl-2-hydroxy-N'-phenylbutanohydrazide

The desired product was prepared by substituting 1-dodecyl-1-phenylhydrazine for 1-benzylhydrazine dihydrochloride in Example 1.
MS (ESI) m/e 460 (M+H)⁺;
¹H NMR (300 MHz, CD₃OD) δ 7.22 (m, 2H), 6.88 (m, 2H), 6.83 (m, 1H), 4.26 (d, 1H), 3.56 (dt, 1H), 3.46 (m, 2H), 1.57-1.93 (m, 8H), 1.51 (m, 2H), 1.10-1.45 (m, 21H), 0.82-1.10 (m, 5H).

### Example 145

### (2RS,3R)-3-amino-4-cyclohexyl-2-hydroxy-N'-phenyl-N'-(3,5,5-trimethylhexyl)butanohydrazide

The desired product was prepared by substituting 1-(3,5,5-trimethylhexyl)-1-phenylhydrazine for 1-benzylhydrazine dihydrochloride in Example 1.
MS (ESI) m/e 418 (M+H)⁺;
¹H NMR (300 MHz, CD₃OD) δ 7.22 (m, 2H), 6.89 (m, 2H), 6.83 (m, 1H), 4.27 (d, 1H), 3.40-3.63 (m, 3H), 1.59-1.98 (m, 8H), 1.51 (m, 3H), 1.18-1.42 (m, 4H), 1.14 (m, 1H), 1.03 (m, 4H), 0.70-0.97 (m, 10H).

### Example 146

### (2RS,3R)-3-amino-4-cyclohexyl-2-hydroxy-N'-octyl-N'-phenylbutanohydrazide

The desired product was prepared by substituting 1-octyl-1-phenylhydrazine for 1-benzylhydrazine dihydrochloride in Example 1.
MS (ESI) m/e 404 (M+H)⁺;
¹H NMR (300 MHz, CD₃OD) δ 7.22 (m, 2H), 6.88 (m, 2H), 6.82 (m, 1H), 4.27 (d, 1H), 3.56 (dt, 1H), 3.46 (m, 2H), 1.59-1.97 (m, 8H), 1.52 (m, 2H), 1.09-1.45 (m, 13H), 0.76-1.09 (m, 5H).

### Example 147

### (2RS,3R)-3-amino-N'-(2-(benzyloxy)ethyl)-4-cyclohexyl-2-hydroxy-N'-phenylbutanohydrazide

The desired product was prepared by substituting 1-(2-benzyloxymethyl)-1-phenylhydrazine for 1-benzylhydrazine dihydrochloride in Example 1.
MS (ESI) m/e 426 (M+H)⁺;
¹H NMR (300 MHz, CD₃OD) δ 7.35 (m, 10H), 6.92 (m, 2H), 6.83 (m, 1H), 4.53 (m, 4H), 4.32 (d, 1H), 3.60 (m, 3H), 1.58-1.91 (m, 6H), 1.50 (m, 2H), 1.17-1.42 (m, 3H), 0.91 (m, 2H).

### Example 148

### (2RS,3R)-3-amino-4-cyclohexyl-2-hydroxy-N'-phenyl-N'-(2,2,5-trichloropentyl)butanohydrazide

The desired product was prepared by substituting 1-(2,2,5-trichloropentyl)-1-phenylhydrazine for 1-benzylhydrazine dihydrochloride in Example 1.
MS (ESI) m/e 464 (M+H)⁺;
¹H NMR (300 MHz, CD₃OD) δ 7.20 (m, 2H), 6.85 (m, 3H), 4.38 (d, 1H), 3.79 (m, 1H), 3.68 (m, 2H), 2.46 (m, 2H), 2.17 (m, 2H), 1.58-1.91 (m, 8H), 1.50 (m, 2H), 1.17-1.42 (m, 3H), 0.91 (m, 2H).

### Example 149

### (2RS,3R)-3-amino-4-cyclohexyl-2-hydroxy-N'-phenyl-N'-propylbutanohydrazide

The desired product was prepared by substituting 1-propyl-1-phenylhydrazine for 1-benzylhydrazine dihydrochloride in Example 1.
MS (ESI) m/e 334 (M+H)⁺;
¹H NMR (300 MHz, CD₃OD) δ 7.22 (m, 2H), 6.90 (m, 2H), 6.82 (m, 1H), 4.27 (d, 1H), 3.59 (m, 1H), 3.45 (m, 2H), 1.59-1.99 (m, 8H), 1.51 (m, 2H), 1.12-1.43 (m, 3H), 0.78-1.12 (m, 5H).

### Example 150

### (2RS,3R)-3-amino-4-cyclohexyl-N'-heptyl-2-hydroxy-N'-phenylbutanohydrazide

The desired product was prepared by substituting 1-heptyl-1-phenylhydrazine for 1-benzylhydrazine dihydrochloride in Example 1.
MS (ESI) m/e 390 (M+H)⁺;
¹H NMR (300 MHz, CD₃OD) δ 7.22 (m, 2H), 6.88 (m, 2H), 6.82 (m, 1H), 4.27 (d, 1H), 3.56 (dt, 1H), 3.45 (m, 2H), 1.59-1.96 (m, 8H), 1.52 (m, 2H), 1.12-1.45 (m, 11H), 0.83-1.12 (m, 5H).

### Example 151

### (2RS,3R)-3-amino-4-cyclohexyl-N'-ethyl-2-hydroxy-N'-phenlybutanohydrazide

The desired product was prepared by substituting 1-ethyl-1-phenylhydrazine for 1-benzylhydrazine dihydrochloride in Example 1.
MS (ESI) m/e 320 (M+H)⁺;
¹H NMR (300 MHz, CD₃OD) δ 7.23 (m, 2H), 6.91 (m, 2H), 6.83 (m, 1H), 4.30 (d, 1H), 3.61 (m, 1H), 3.54 (dd, 2H), 1.59-1.92 (m, 6H), 1.50 (m, 2H), 1.4-1.12 (m, 3H), 1.22 (t, 3H), 0.85-1.18 (m, 2H).

### Example 152

### (2RS,3R)-3-amino-4-cyclohexyl-2-hydroxy-N'-(3-(methylsutfanyl)propyl)-N'-phenylbutanohydrazide

The desired product was prepared by substituting 1-(3-(methylsulfanyl)propyl)-1-phenylhydrazine for 1-benzylhydrazine dihydrochloride in Example 1.
MS (ESI) m/e 380 (M+H)⁺;
¹H NMR (300 MHz, CD₃OD) δ 7.22 (m, 2H), 6.92 (m, 2H), 6.83 (m, 1H), 4.28 (d, 1H), 3.47-3.72 (m, 3H), 2.50-2.75 (m, 2H), 2.10 (s, 3H), 1.93 (m, 2H), 1.58-1.87 (m, 6H), 1.51 (m, 2H), 1.13-1.42 (m, 3H), 0.82-1.12 (m, 2H).

### Example 153

### (2RS,3R)-3-amino-4-cyclohexyl-N'-(cyclopentylmethyl)-2-hydroxy-N'-phenylbutanohydrazide

The desired product was prepared by substituting 1-(cyclopentylmethyl)-1-phenylhydrazine for 1-benzylhydrazine dihydrochloride in Example 1.
MS (ESI) m/e 374 (M+H)⁺;
¹H NMR (300 MHz, CD₃OD) δ 7.21 (m, 2H), 6.89 (m, 2H), 6.82 (m, 1H), 4.25 (d, 1H), 3.54 (dt, 1H), 3.41 (d, 2H), 2.26 (m, 1H), 1.42-2.00 (m, 15H), 1.10-1.43 (m, 5H), 0.80-1.10 (m, 2H).

### Example 154

### (2RS,3R)-3-amino-4-cyclohexyl-2-hydroxy-N'-(5-hydroxypentyl)-N'-phenylbutanohydrazide

The desired product was prepared by substituting 1-(5-hydroxypentyl)-1-phenylhydrazine for 1-benzylhydrazine dihydrochloride in Example 1.
MS (ESI) m/e 378 (M+H)⁺;
¹H NMR (300 MHz, CD₃OD) δ 7.22 (m, 2H), 6.90 (m, 2H), 6.82 (m, 1H), 4.28 (d, 1H), 3.68 (m, 1H), 3.57 (t, 2H), 3.48 (m, 2H), 1.41-2.11 (m, 14H), 1.12-1.41 (m, 4H), 0.80-1.12 (m, 2H).

### Example 155

### (2RS,3R)-3-amino-4-cyclohexyl-N'-((2R)-2,3-dihydroxypropyl)-2-hydroxy-N'-phenylbutanohydrazide

The desired product was prepared by substituting 1-((2R)-2,3-dihydroxypropyl)-1-phenylhydrazine for 1-benzylhydrazine dihydrochloride in Example 1.
MS (ESI) m/e 366 (M+H)⁺;
¹H NMR (300 MHz, CD₃OD δ 7.20 (m, 2H), 6.86 (m, 3H), 4.27 (d, 1H), 3.62 (m, 3H), 3.28 (d, 4H), 1.58-1.91 (m, 6H), 1.50 (m, 2H), 1.17-1.42 (m, 3H), 0.91 (m, 2H).

### Example 156

### (2RS,3R)-3-amino-4-cyclohexyl-N'-(2,2-dichlorohexyl)-2-hydroxy-N'-phenylbutanohydrazide

The desired product was prepared by substituting 1-(2,2-dichlorohexyl)-1-phenylhydrazine for 1-benzylhydrazine dihydrochloride in Example 1.
MS (ESI) m/e 444 (M+H)⁺;
¹H NMR (300 MHz, CD₃OD) δ 7.20 (m, 2H), 6.87 (m, 3H), 4.29 (d, 1H), 3.57 (m, 3H), 2.27 (m, 2H), 1.58-1.91 (m, 6H), 1.50 (m, 4H), 1.17-1.42 (m, 5H), 1.05 (m, 2H), 0.92 (t, 3H).

### Example 157

### (2RS,3RL3-amino-4-cyclohexyl-2-hydroxy-N'-((3RS)-7-methoxy-3,7-dimethyloctyl)-N'-phenylbutanohydrazide

The desired product was prepared by substituting 1-(7-methoxy-3,7-dimethyloctyl)-1-phenylhydrazine for 1-benzylhydrazine dihydrochloride in Example 1.
MS (ESI) m/e 462 (M+H)⁺;
¹H NMR (300 MHz, CD₃OD) δ 7.23 (m, 2H), 6.89 (m, 2H), 6.83 (m, 1H), 4.27 (s, 1H), 3.38-3.65 (m, 3H), 3.17 (s, 3H), 1.14 (s, 6H), 0.97 (d, 3H), 0.85-1.12 (m, 2H), 0.85-1.12 (m, 20H).

### Example 158

### (2RS,3R)-3-amino-4-cyclohexyl-2-hydroxy-N'-(2-(4-methylphenyl)ethyl)-N'-phenylbutanohydrazide

The desired product was prepared by substituting 1-(2-(4-methylphenyl)ethyl)-1-phenylhydrazine for 1-benzylhydrazine dihydrochloride in Example 1.
MS (ESI) m/e 410 (M+H)⁺;
¹H NMR (300 MHz, CD₃OD) δ 7.24 (m, 2H), 7.13 (m, 4H), 6.91 (m, 2H), 6.83 (m, 1H), 4.29 (d, 1H), 3.69 (m, 2H), 3.57 (m, 1H), 2.90 (t, 2H), 2.30 (s, 3H), 1.59-1.95 (m, 5H), 1.50 (m, 2H), 1.12-1.40 (m, 4H), 0.75-1.12 (m, 2H).

### Example 159

### (2RS,3R)-3-amino-4-cyclohexyl-N'-((2RS)-2-ethylhexyl)-2-hydroxy-N'-phenylbutanohydrazide

The desired product was prepared by substituting 1-(2-ethylhexyl)-1-phenylhydrazine for 1-benzylhydrazine dihydrochloride in Example 1.
MS (ESI) m/e 404 (M+H)⁺;
¹H NMR (300 MHz, CD₃OD) δ 7.21 (m, 2H), 6.88 (m, 2H), 6.81 (m, 1H), 4.23 (m, 1H), 3.52 (dt, 1H), 3.37 (m, 2H), 1.61-1.92 (m, 6H), 1.10-1.61 (m, 14H), 0.75-1.10 (m, 8H).

### Example 160

### (2RS,3R)-3-amino-N'-((2RS)-2-(4-chlorophenyl)-2-cyanoethyl)-4-cyclohexyl-2-hydroxy-N'-phenylbutanohydrazide

The desired product was prepared by substituting 1-(2-(4-chlorophenyl)-2-cyanoethyl)-1-phenylhydrazine for 1-benzylhydrazine dihydrochloride in Example 1.
MS (ESI) m/e 456 (M+H)⁺;
¹H NMR (300 MHz, CD₃OD) δ 7.50-7.10 (m, 9H), 6.84 (m, 1H), 4.57 (d, 1H), 3.82 (m, 1H), 3.41 (m, 2H), 1.58-1.91 (m, 6H), 1.50 (m, 2H), 1.17-1.42 (m, 3H), 0.91 (m, 2H).

### Example 161

### (2RS,3R)-3-amino-4-cyclohexyl-2-hydroxy-N'-phenyl-N'-((2RS)-2-phenylpropyl)butanohydrazide

The desired product was prepared by substituting 1-(2-phenylpropyl)-1-phenylhydrazine for 1-benzylhydrazine dihydrochloride in Example 1.
MS (ESI) m/e 410 (M+H)⁺;
¹H NMR (300 MHz, CD₃OD) δ 7.32 (m, 5H), 7.18 (m, 2H), 6.87 (m, 2H), 6.80 (m, 1H), 4.18 (d, 1H), 3.65 (m, 2H), 3.52 (m, 1H), 3.13 (m, 1H), 1.58-1.91 (m, 6H), 1.50 (m, 2H), 1.17-1.42 (m, 3H), 1.37 (d, 3H), 0.91 (m, 2H).

### Example 162

### (2RS,3R)-3-amino-4-cyclohexyl-N'-(cyclooctylmethyl)-2-hydroxy-N'-phenylbutanohydrazide

The desired product was prepared by substituting 1-(cyclooctylmethyl)-1-phenylhydrazine for 1-benzylhydrazine dihydrochloride in Example 1.
MS (ESI) m/e 416 (M+H)⁺;
¹H NMR (300 MHz, CD₃OD) δ 7.20 (m, 2H), 6.88 (m, 2H), 6.80 (m, 1H), 4.24 (d, 1H), 3.53 (dt, 1H), 3.27 (m, 2H), 1.11-2.08 (m, 26H), 0.82-1.11 (m, 2H).

### Example 163

### (2RS,3R)-3-amino-4-cyclohexyl-N'-((11Z)-11-hexadecenyl)-2-hydroxy-N'-phenylbutanohydrazide

The desired product was prepared by substituting 1-((11Z)-11-hexadecenyl)-1-phenylhydrazine for 1-benzylhydrazine dihydrochloride in Example 1.
MS (ESI) m/e 514 (M+H)⁺;
¹H NMR (300 MHz, CD₃OD) δ 7.22 (m, 2H), 6.88 (m, 2H), 6.82 (m, 1H), 5.34 (m, 2H), 4.27 (d, 1H), 3.56 (dt, 1H), 3.46 (m, 2H), 1.92-2.14 (m, 4H), 1.58-1.92 (m, 7H), 1.51 (m, 2H), 1.12-1.44 (m, 22H), 0.94-1.12 (m, 2H), 0.89 (m, 3H).

### Example 164

### (2RS,3R)-3-amino-4-cyclohexyl-2-hydroxy-N'-phenyl-N'-tridecylbutanohydrazide

The desired product was prepared by substituting 1-tridecyl-1-phenylhydrazine for 1-benzylhydrazine dihydrochloride in Example 1.
MS (ESI) m/e 474 (M+H)⁺;
¹H NMR (300 MHz, CD₃OD) δ 7.22 (m, 2H), 6.90 (m, 2H), 6.83 (m, 1H), 4.27 (d, 1H), 3.56 (m, 1H), 3.46 (m, 2H), 1.57-1.91 (m, 7H), 1.51 (m, 2H), 1.12-1.44 (m, 21H), 1.01 (m, 2H), 0.90 (t, 3H).

### Example 165

### 4-(2-((2RS,3R)-3-amino-4-cyclohexyl-2-hydroxybutanoyl)-1-phenylhydrazino)butanoic acid

The desired product was prepared by substituting 4-(1-phenylhydrazino)butanoic acid for 1-benzylhydrazine dihydrochloride in Example 1.
MS (ESI) m/e 378 (M+H)⁺;
¹H NMR (300 MHz, CD₃OD) δ 7.23 (m, 2H), 6.88 (m, 3H), 4.29 (m, 1H), 3.64 (m, 1H), 3.53 (m, 1H), 3.31 (m, 1H), 2.46 (t, 1H), 1.94 (m, 1H), 1.59-1.88 (m, 7H), 1.50 (m, 3H), 1.12-1.41 (m, 4H), 0.86-1.12 (m, 2H).

### Example 166

### (2RS,3R)-3-amino-4-cyclohexyl-2-hydroxy-N'-((6Z)-6-nonenyl)-N'-phenylbutanohydrazide

The desired product was prepared by substituting 1-((6Z)-6-nonenyl)-1-phenylhydrazine for 1-benzylhydrazine dihydrochloride in Example 1.
MS (ESI) m/e 416 (M+H)⁺;
¹H NMR (300 MHz, CD₃OD) δ 7.22 (m, 2H), 6.89 (m, 2H), 6.83 (m, 1H), 5.35 (m, 2H), 4.27 (d, 1H), 3.57 (dt, 1H), 3.46 (m, 2H), 2.06 (m, 4H), 1.58-1.90 (m, 7H), 1.52 (m, 2H), 1.42 (m, 4H), 1.12-1.38 (m, 4H), 0.95 (t, 3H), 0.86-1.09 (m, 2H).

### Example 167

### (2RS,3R)-3-amino-4-cyclohexyl-N'-((4Z)-4-decenyl)-2-hydroxy-N'-phenylbutanohydrazide

The desired product was prepared by substituting 1-((4Z)-4-decenyl)-1-phenylhydrazine for 1-benzylhydrazine dihydrochloride in Example 1.
MS (ESI) m/e 430 (M+H)⁺;
¹H NMR (300 MHz, CD₃OD) δ 7.22 (m, 2H), 6.89 (m, 2H), 6.83 (m, 1H), 5.41 (m, 2H), 4.26 (d, 1H), 3.57 (m, 1H), 3.47 (m, 2H), 2.05 (m, 4H), 1.59-1.90 (m, 7H), 1.51 (m, 2H), 1.12-1.42 (m, 10H), 0.82-1.08 (m, 5H).

### Example 168

### (2RS,3R)-3-amino-4-cyclohexyl-2-hydroxy-N'-(4-pentenyl)-N'-phenylbutanohydrazide

The desired product was prepared by substituting 1-(4-pentenyl)-1-phenylhydrazine for 1-benzylhydrazine dihydrochloride in Example 1.
MS (ESI) m/e 360 (M+H)⁺;
¹H NMR (300 MHz, CD₃OD) δ 7.20 (m, 2H), 6.87 (m, 2H), 6.78 (m, 1H), 5.87 (m, 1H), 5.05 (m, 2H), 4.27 (d, 1H), 3.58 (m, 1H), 3.48 (m, 2H), 2.20 (m, 2H), 1.58-1.91 (m, 8H), 1.50 (m, 2H), 1.17-1.42 (m, 3H), 0.97 (m, 2H).

### Example 169

### (2RS,3R)-3-amino-4-cyclohexyl-N'-((3RS)-3,7-dimethyl-6-octenyl)-2-hydroxy-N'-phenylbutanohydrazide

The desired product was prepared by substituting 1-(3,7-dimethyl-6-octenyl)-1-phenylhydrazine for 1-benzylhydrazine dihydrochloride in Example 1.
MS (ESI) m/e 430 (M+H)⁺;
¹H NMR (300 MHz, CD₃OD) δ 7.22 (m, 2H), 6.87 (m, 2H), 6.80 (m, 1H), 5.12 (m, 1H), 4.28 (d, 1H), 3.55 (m, 3H), 2.04 (m, 2H), 1.58-1.91 (m, 6H), 1.50 (m, 5H), 1.17-1.42 (m, 5H), 1.12 (s, 6H), 0.97 (d, 3H), 0.91 (m, 2H).

### Example 170

### (2RS,3R)-3-amino-4-cyclohexyl-2-hydroxy-N'-phenyl-N'-(4,4,4-trifluorobutyl)butanohydrazide

The desired product was prepared by substituting 1-(4,4,4-trifluorobutyl)-1-phenylhydrazine for 1-benzylhydrazine dihydrochloride in Example 1.
MS (ESI) m/e 402 (M+H)⁺;
¹H NMR (300 MHz, CD₃OD) δ 7.25 (m, 2H), 6.93 (m, 2H), 6.86 (m, 1H), 3.57 (m, 3H), 2.25-2.46 (m, 2H), 1.91 (m, 3H), 1.62-1.84 (m, 6H), 1.42-1.62 (m, 2H), 1.12-1.42 (m, 3H), 0.84-1.12 (m, 2H).

### Example 171

### (2RS,3R)-3-amino-4-cyclohexyl-2-hydroxy-N'-((3RS)-3-hydroxybutyl)-N'-phenylbutanohydrazide

The desired product was prepared by substituting 1-(3-hydroxybutyl)-1-phenylhydrazine for 1-benzylhydrazine dihydrochloride in Example 1.
MS (ESI) m/e 364 (M+H)⁺;
¹H NMR (300 MHz, CD₃OD) δ 7.23 (m, 2H), 6.92 (m, 2H), 6.84 (m, 1H), 4.30 (d, 1H), 3.44-3.71 (m, 4H), 1.63-1.91 (m, 7H), 1.54 (m, 3H), 1.35 (m, 3H), 1.19 (m, 4H), 0.84-1.12 (m, 2H).

### Example 172

### (2RS,3R)-3-amino-4-cyclohcxyl-N'-(2-(((3RS)-3,7-dimethyl-6-octenyl)oxy)ethyl)-2-hydroxy-N'-phenylbutanohydrazide

The desired product was prepared by substituting 1-(2-((3,7-dimethyl-6-octenyl)oxy)ethyl)-1-phenylhydrazine for 1-benzylhydrazine dihydrochloride in Example 1. MS (ESI) m/e 474 (M+H)⁺;
¹H NMR (300 MHz, CD₃OD) δ 7.24 (m, 2H), 6.93 (m, 2H), 6.85 (m, 1H), 5.09 (m, 1H), 4.33 (d, 1H), 3.72 (m, 2H), 3.67 (m, 2H), 3.50 (m, 3H), 2.04 (m, 2H), 1.58-1.91 (m, 6H), 1.50-1.20 (m, 10H), 1.17 (s, 3H), 1.12 (s, 3H), 0.91 (m, 2H), 0.87 (d, 3H).

### Example 173

### (2RS,3R)-3-amino-4-cyclohexyl-N'-(2-((1RS)-3,3-dimethylcyclohexyl)ethyl)-2-hydroxy-N'-phenylbutanohydrazide

The desired product was prepared by substituting 1-(2-(3,3-dimethylcyclohexyl)ethyl)-1-phenylhydrazine for 1-benzylhydrazine dihydrochloride in Example 1.
MS (ESI) m/e 430 (M+H)⁺;
¹H NMR (300 MHz, CD₃OD) δ 7.22 (m, 2H), 6.89 (m, 2H), 6.82 (m, 1H), 4.26 (d, 1H), 3.53 (m, 3H), 1.65-1.94 (m, 7H), 1.18-1.65 (m, 11H), 0.94-1.18 (m, 4H), 0.92 (s, 3H), 0.91 (s, 3H), 0.65-0.87 (m, 2H).

### Example 174

### (2RS,3R)-3-amino-N'-((4S)-6-bromo-4-methylhexyl)-4-cyclohexyl-2-hydroxy-N'-phenylbutanohydrazide

The desired product was prepared by substituting 1-((4S)-6-bromo-4-methylhexyl)-1-phenylhydrazine for 1-benzylhydrazine dihydrochloride in Example 1.
MS (ESI) m/e 468, 470 (M+H)⁺;
¹H NMR (300 MHz, CD₃OD) δ 7.23 (m, 2H), 6.89 (m, 2H), 6.83 (m, 1H), 4.27 (d, 1H), 3.55 (m, 3H), 1.65-1.89 (m, 6H), 1.45-1.65 (m, 9H), 1.17-1.42 (m, 5H), 0.97 (d, 3H), 0.95 (m, 2H).

### Example 175

### (2RS,3R)-3-amino-4-cyclohexyl-N'-(cyclohexylinethyl)-2-hydroxy-N'-phenylbutanohydrazide

The desired product was prepared by substituting 1-cyclohexylmethyl-1-phenylhydrazine for 1-benzylhydrazine dihydrochloride in Example 1.
MS (ESI) m/e 388 (M+H)⁺;
¹H NMR (300 MHz, CD₃OD) δ 7.21 (m, 2H), 6.86 (m, 2H), 6.80 (m, 1H), 4.25 (d, 1H), 3.54 (dt, 1H), 3.31 (d, 2H), 1.92 (m, 2H), 1.59-1.84 (m, 10H), 1.51 (m, 2H), 1.13-1.42 (m, 6H), 0.81-1.13 (m, 4H).

### Example 176

### N'-((2RS,3R)-3-amino-5-(ethylsulfanyl)-2-hydroxypentanoyl)-2-naphthohydrazide

The desired product was prepared by substituting 2-naphthoylhydrazine for O-phenyl hydroxylamine hydrochloride in Example 98B.
MS (ESI) m/e 362 (M+H)⁺;
¹H NMR (300 MHz, CD₃OD) δ 8.48 (m, 1H), 7.89-8.09 (m, 4H), 7.47-7.75 (m, 2H), 4.54 (d, 0.34H), 4.48 (d, 0.66H), 3.56-3.87 (m, 1H), 2.76 (m, 2H), 2.62 (q, 1.32H), 2.60 (q, 0.68H), 2.10-2.31 (m, 0.68H), 1.84-2.08 (m, 1.32H), 1.28 (t, 1.98H), 1.27 (t, 1.02H).

### Example 177

### (2RS,3R)-3-amino-5-(ethylsulfanyl)-2-hydroxy-N-(1-piperidinyl)pentanamide

The desired product was prepared by substituting 1-aminopiperidine for O-phenyl hydroxylamine hydrochloride in Example 98B.
MS (ESI) m/e 276 (M+H)⁺;
¹H NMR (300 MHz, CD₃OD) δ 4.67 (m, 0.28H), 4.62 (m, 0.72H), 3.37-3.78 (m, 5H), 2.72-3.16 (m, 4H), 2.00-2.30 (m, 2H), 1.70 (m, 4H), 1.57 (m, 2H), 1.35 (t, 2.16H), 1.33 (m, 0.84H).

### Example 178

### N'-((2RS,3R)-3-amino-5-(ethylsulfanyl)-2-hydroxypentanoyl)benzohydrazide

The desired product was prepared by substituting benzoylhydrazine for O-phenyl hydroxylamine hydrochloride in Example 98B.
MS (ESI) m/e 312 (M+H)⁺;
¹H NMR (300 MHz, CD₃OD) δ 7.89 (m, 2H), 7.61 (m, 1H), 7.50 (m, 2H), 4.49 (d, 0.24H), 4.44 (d, 0.76H), 3.79 (m, 1H), 2.71 (t, 2H), 2.60 (q, 2H), 2.17 (m, 1H), 1.99 (m, 1H), 1.27 (t, 3H.)

### Example 179

### (2RS,3R)-3-amino-5-(ethylsulfanyl)-2-hydroxy-N'-(4-iodophenyl)pentanohydrazide

The desired product was prepared by substituting 1-(4-iodophenyl)hydrazine for 1-benzylhydrazine dihydrochloride and Example 98A for Example 1C in Example 1D.
MS (ESI) m/e 410 (M+H)⁺;
¹H NMR (300 MHz, CD₃OD) δ 7.48 (m, 2H), 6.67 (m, 2H), 4.43 (d, 0.38H), 4.36 (d, 0.62H), 3.74 (m, 1H), 2.72 (m, 2H), 2.57 (q, 0.76H), 2.51 (q, 1.24H), 2.11 (m, 0.76H), 1.93 (m, 1.24H), 1.26 (t, 1.86H), 1.22 (t, 1.14H).

### Example 180

### (2RS,3R)-3-amino-5-(ethylsulfanyl)-2-hydroxy-N'-cyclopentylpentanohydrazide

The desired product was prepared by substituting 1-(cyclopentyl)hydrazine for 1-benzylhydrazine dihydrochloride and Example 98A for Example 1C in Example 1D. MS (ESI) m/e 276 (M+H)⁺;
¹H NMR (300 MHz, CD₃OD) δ 4.36 (d, 0.23H), 4.27 (d, 0.77H), 3.67 (td, 1H), 3.53 (m, 1H), 2.68 (m, 2H), 2.57 (q, 1.54H), 2.54 (q, 0.46H), 2.07 (m, 0.46H), 1.91 (m, 1.54H), 1.76 (m, 4H), 1.44-1.67 (m, 4H), 1.26 (t, 2.31H), 1.24 (t, 0.69H).

### Example 181

### (2RS,3R)-3-amino-5-(ethylsulfanyl)-2-hydroxy-N'-(3-chlorophenyl)pentanohydrazide

The desired product was prepared by substituting 1-(3-chlorophenyl)hydrazine for 1-benzylhydrazine dihydrochloride and Example 98A for Example 1C in Example 1D.
MS (ESI) m/e 318 (M+H)⁺;
¹H NMR (300 MHz, CD₃OD δ 7.15 (m, 1H), 6.85 (m, 1H), 6.77 (m, 2H), 5.98 (d, 0.32H), 5.85 (d, 0.68H), 3.71 (m, 1H), 3.43 (q, 1.36H), 3.36 (q, 0.64H), 2.70 (m, 2H), 2.11 (m, 0.64H), 1.95 (m, 1.36H), 1.67 (t, 2.04H), 1.63 (t, 0.96H).

### Example 182

### (2RS,3R)-3-amino-5-(ethylsulfanyl)-2-hydroxy-N'-(3-methoxyphenyl)pentanohydrazide

The desired product was prepared by substituting 1-(3-methoxyphenyl)hydrazine for 1-benzylhydrazine dihydrochloride and Example 98A for Example 1C in Example 1D.
MS (ESI) m/e 314 (M+H)⁺;
¹H NMR (300 MHz, CD₃OD δ 7.09 (m, 1H), 6.44 (m, 3H), 4.43 (d, 0.37H), 4.36 (d, 0.63H), 3.75 (s, 3H), 3.69 (m, 1H), 2.71 (m, 2H), 2.57 (q, 1.26H), 2.50 (q, 0.74H), 2.12 (m, 0.74H), 1.82-2.04 (m, 1.26H), 1.25 (t, 1.89H), 1.21 (t, 1.11H).

### Example 183

### (2RS,3R)-3-amino-5-(ethylsulfanyl)-2-hydroxy-N'-(2-chlorophenyl)pentanohydrazide

The desired product was prepared by substituting 1-(2-chlorophenyl)hydrazine for 1-benzylhydrazine dihydrochloride and Example 98A for Example 1C in Example 1D.
MS (ESI) m/e 318 (M+H)⁺;
¹H NMR (300 MHz, CD₃OD δ 7.29 (m, 1H), 7.17 (m, 1H), 6.93 (m, 1H), 6.83 (m, 1H), 4.47 (d, 0.23H), 4.41 (d, 0.77H), 3.76 (m, 1H), 2.71 (m, 2H), 2.58 (q, 1.54H), 2.52 (q, 0.46H), 2.13 (td, 0.46H), 1.95 (m, 1.54H), 1.26 (t, 2.31H), 1.22 (t, 0.69H).

### Example 184

### (2RS,3R)-3-amino-5-(ethylsulfanyl)-2-hydroxy-N'-(3-trifluoromethylphenyl)pentanohydrazide

The desired product was prepared by substituting 1-(3-(trifluoromethyl)phenyl)hydrazine for 1-benzylhydrazine dihydrochloride and Example 98A for Example 1C in Example 1D.
MS (ESI) m/e 352 (M+H)⁺;
¹H NMR (300 MHz, CD₃OD) δ 7.37 (m, 1H), 7.08 (m, 3H), 4.48 (d, 0.39H), 4.40 (d, 0.61H), 3.69 (m, 1H), 2.71 (m, 2H), 2.57 (q, 1.22H), 2.51 (q, 0.78H), 2.13 (m, 0.78H), 1.84-2.05 (m, 1.22H), 1.25 (t, 1.83H), 1.21 (t, 1.17H).

### Example 185

### N'-((2RS,3R)-3-amino-5-(ethylsulfanyl)-2-hydroxypentanoyl)-3-chlorobenzohydrazide

The desired product was prepared by substituting 3-chlorobenzoylhydrazine for O-phenyl hydroxylamine hydrochloride in Example 98B.
MS (ESI) m/e 346 (M+H)⁺;
¹H NMR (300 MHz, CD₃OD δ 7.89 (m, 1H), 7.81 (m, 1H), 7.61 (m, 1H), 7.50 (m, 1H), 4.49 (d, 0.14H), 4.44 (d, 0.86H), 3.78 (m, 1H), 2.70 (t, 2H), 2.60 (dd, 2H), 2.16 (m, 1H), 1.98 (m, 1H), 1.27 (t, 3H).

### Example 186

### N'-((2RS,3R)-3-amino-5-(ethylsulfanyl)-2-hydroxypentanoyl)-2-chlorobenzohydrazide

The desired product was prepared by substituting 2-chlorobenzoylhydrazine for O-phenyl hydroxylamine hydrochloride in Example 98B.
MS (ESI) m/e 346 (M+H)⁺;
¹H NMR (300 MHz, CD₃OD) δ 7.62 (m, 1H), 7.46 (m, 3H), 4.49 (d, 0.26H), 4.43 (d, 0.74H), 3.80 (m, 1H), 2.71 (t, 2H), 2.60 (q, 0.52H), 2.57 (q, 1.48H), 2.16 (m, 0.52H), 1.99 (m, 1.48H), 1.27 (t, 2.22H), 1.24 (t, 0.78H).

### Example 187

### (2RS,3R)-3-amino-5-(ethylsulfanyl)-2-hydroxy-N'-(4-isopropyl)pentanohydrazide

The desired product was prepared by substituting 1-(4-(2-methylethyl)phenyl)hydrazine for 1-benzylhydrazine dihydrochloride and Example 98A for Example 1C in Example 1D.
MS (ESI) m/e 326 (M+H)⁺;
¹H NMR (300 MHz, CD₃OD) δ 7.02 (d, 2H), 6.77 (d, 2H), 4.42 (d, 0.41H), 4.35 (d, 0.59H), 3.74 (m, 1H), 2.61-2.81 (m, 2H), 2.55 (m, 3H), 2.02-2.24 (m, 0.82H), 1.81-2.02 (m, 1.18H), 1.26 (t, 1.77H), 1.22 (t, 1.23H) 1.17 (d, 6H).

### Example 188

### (2RS,3R)-3-amino-5-(ethylsulfanyl)-2-hydroxy-N'-(3-chloro-4-methylphenyl)pentanohydrazide

The desired product was prepared by substituting 1-(3-chloro-4-methylphenyl)hydrazine for 1-benzylhydrazine dihydrochloride and Example 98A for Example 1C in Example 1D.
MS (ESI) m/e 332 (M+H)⁺;
¹H NMR (300 MHz, CD₃OD) δ 7.09 (m, 1H), 6.86 (m, 1H), 6.71 (m, 1H), 4.43 (d, 0.33H), 4.36 (d, 0.67H), 3.73 (m, 1H), 2.71 (m, 2H), 2.57 (q, 1.34H), 2.50 (q, 0.66H), 2.25 (m, 3H), 2.12 (m, 0.66H), 1.82-2.04 (m, 1.34H), 1.25 (t, 2.01H), 1.21 (t, 1H).

### Example 189

### (2RS,3R)-3-amino-5-(ethylsulfanyl)-2-hydroxy-N'-(3-fluorophenyl)pentanohydrazide

The desired product was prepared by substituting 1-(3-fluorophenyl)hydrazine for 1-benzylhydrazine dihydrochloride and Example 98A for Example 1C in Example 1D.
MS (ESI) m/e 302 (M+H)⁺;
¹H NMR (400 MHz, CD₃OD) δ 7.15 (m, 1H), 6.64 (m, 1H), 6.52 (m, 2H), 4.48 (d, 0.37H), 4.38 (d, 0.63H), 3.80 (m, 0.37H), 3.70 (m, 0.63H), 2.70 (m, 2H), 2.57 (q, 1.23H), 2.52 (q, 0.74H), 2.12 (m, 0.74H), 1.95 (m, 1.23H), 1.25 (t, 1.89H), 1.22 (t, 1.11H).

### Example 190

### (2RS,3R)-3-amino-5-(ethylsulfanyl)-2-hydroxy-N'-(2-ethyhphenyl)pentanohydrazide

The desired product was prepared by substituting 1-(2-ethylphenyl)hydrazine for 1-benzylhydrazine dihydrochloride and Example 98A for Example 1C in Example 1D.
MS (ESI) m/e 312 (M+H)⁺;
¹H NMR (300 MHz, CD₃OD) δ 7.07 (m, 1H), 6.90 (m, 1H), 6.86 (m, 1H), 6.73 (m, 1H), 4.44 (d, 0.26H), 4.37 (d, 0.74H), 3.80 (m, 1H), 2.45-2.71 (m, 6H), 2.02 (m, 2H), 1.15-1.27 (m, 6H).

### Example 191

### (2RS,3R)-3-amino-5-(ethylsulfanyl)-2-hydroxy-N'-(4-fluorophenyl)pentanohydrazide

The desired product was prepared by substituting 1-(4-fluorophenyl)hydrazine for 1-benzylhydrazine dihydrochloride and Example 98A for Example 1C in Example 1D.
MS (ESI) m/e 302 (M+H)⁺;
¹H NMR (300 MHz, CD₃OD) δ 6.96 (m, 2H), 6.84 (m, 2H), 4.42 (d, 0.41H), 4.35 (d, 0.59H), 3.74 (m, 1H), 2.61-2.81 (m, 2H), 2.57 (q, 1.18H), 2.51 (q, 0.82H), 2.02-2.24 (m, 0.82H), 1.81-2.02 (m, 1.18H), 1.26 (t, 1.77H), 1.22 (t, 1.23H).

### Example 192

### (2RS,3R)-3-amino-5-(ethylsulfanyl)-2-hydroxy-N'-(4-trifluoromethoxyphenyl)pentanohydrazide

The desired product was prepared by substituting 1-(4-(trifluoromethoxy)phenyl)hydrazine for 1-benzylhydrazine dihydrochloride and Example 98A for Example 1 C in Example 1D.
MS (ESI) m/e 368 (M+H)⁺;
¹H NMR (300 MHz, CD₃OD δ 7.00-7.20 (m, 2H), 6.87 (m, 2H), 4.44 (d, 0.42H), 4.37 (d, 0.58H), 3.63-3.83 (m, 1H), 2.71 (m, 2H), 2.57 (q, 1.16H), 2.51 (q, 0.84H), 2.11 (m, 0.84H), 1.82-2.03 (m, 1.16H), 1.25 (t, 1.74H), 1.22 (t, 1.26H).

### Example 193

### (2RS,3S)-3-amino-4-(ethylsulfanyl)-2-hydroxy-N'-(4-methylphenyl)butanohydrazide

To DCC resin (148 mg, 0.225 mmol) in 1.0 mL of dichloromethane was added 0.5 mL of a 0.45 M solution of HOBt (0.225 mmol) in dimethylacetamide/dichloromethane (1:6), and 0.5 mL of a 0.3M solution of Example 97A (0.15 mmol) in dimethylacetamide. After 5 minutes, 1.0 mL of a 0.225 M solution of 4-methylphenylhydrazine (0.225 mmol) in dimethylacetamide/dichloromethane (1:1) was added. The mixture was agitated for 18 hours and quenched with 0.19 g of trisamine resin (0.75 mmol) followed by 0.13 g of isocyanate resin (0.225 mmol) and agitated for 4 hours. The mixture was filtered and the resins washed with 1x3 mL of dichloromethane, the solvent was removed in vacuo, and the crude material purified by reverse phase preparative HPLC. The resulting material was treated with 1 mL of 50% trifluoroacetic acid/dichloromethane and agitated at ambient temperature for 18 hours. The solvent was removed in vacuo to give the desired product.
MS (ESI) m/e 284 (M+H)⁺;
¹H NMR (500 MHz, CD₃OD) δ 7.02 (m, 2H), 6.77 (m, 2H), 4.51 (d, 0.65H), 4.39 (d, 0.35H), 3.77 (m, 1H), 2.96 (dd, 1H), 2.76 (dd, 1H), 2.63 (dd, 1.3H), 2.57 (dd, 0.7H), 2.23 (s, 3H), 1.28 (t, 1.95H), 1.23 (t, 1.05H).

### Example 194

### (2RS,3R)-3-amino-3-cyclohexyl-2-hydroxy-N'-(4-methylphenyl)propanohydrazide

The desired product was prepared by substituting Example 122A for Example 97A in Example 193.
MS (ESI) m/e 292 (M+H)⁺;
¹H NMR (500 MHz, CD₃OD) δ 7.02 (m, 2H), 6.77 (m, 2H), 4.45 (d, 0.65H), 4.43 (d, 0.35H), 3.27 (m, 1H), 2.23 (s, 3H), 1.87-1.72 (m, 6H), 1.36-1.12 (m, 5H).

### Example 195

### (2RS,3S)-3-amino-2-hydroxy-N'-(4-methylphenyl)-4-(propylsulfanyl)butanohydrazide

### Example 195A

### (2S)-2-((tert-butoxycarbonyl)amino)-3-(propylsulfanyl)propanoic acid

The desired product was prepared by substituting 1-bromopropane for 2-brompropane and D-cystine for D-homocystine in Example 123A.

### Example 195B

### (2RS,3S)-2-hydroxy-3-((tert-butoxycarbonyl)amino)-3-(propylsulfanyl)propanoic acid

The desired product was prepared by substituting Example 195A for (2R)-2-((tert-butoxycarbonyl)amino)-3-cyclohexylpropanoic acid in Examples 1A-C.
MS (ESI) m/e 294 (M+H)⁺.

### Example 195C

### (2RS,3S)-3-amino-2-hydroxy-N'-(4-methylphenyl)-4-(propylsulfanyl)butanohydrazide

The desired product was prepared by substituting Example 195B for Example 97A in Example 193.
MS (ESI) m/e 298 (M+H)⁺;
¹H NMR (500 MHz, CD₃OD) δ 7.02 (dd, 2H), 6.77 (dd, 2H), 4.50 (d, 0.65H), 4.48 (d, 0.35H), 3.76 (m, 0.65H), 3.67-3.58 (m, 0.35H), 2.93 (dd, 0.65H), 2.84 (dd, 0.35H), 2.75 (m, 1H), 2.58 (dd, 1H), 2.51 (m, 1H), 2.23 (s, 3H), 1.65 (dd, 1H), 1.57 (dd, 1H), 1.02 (t, 1.95 H), 0.98 (t, 1.05H).

### Example 196

### (2RS,3R)-3-amino-2-hydroxy-5-(isopropylsulfanyl)-N'-(4-methylphenyl)pentanohydrazide

The desired product was prepared by substituting Example 123B for Example 97A in Example 193.
MS (ESI) m/e 312 (M+H)⁺;
¹H NMR (500 MHz, CD₃OD) δ 7.01 (dd, 2H), 6.77 (d, 2H), 4.40 (d, 0.35H), 4.33 (d, 0.65H), 3.66 (m, 1H), 2.96 (dd, 0.65H), 2.89 (dd, 0.35H), 2.69 (m, 1.65H), 2.58 (m, 0.35H), 2.23 (s, 3H), 2.09 (dd, 0.65H), 1.96 (dd, 0.35H), 1.89 (dd, 1H), 1.27 (d, 3 H), 1.26 (d, 3H).

### Example 197

### (2RS,3S)-3-amino-2-hydxoay-4-(isobutylsulfanyl)-N'-(4-methylphenyl)butanohydrazide

The desired product was prepared by substituting Example 124B for Example 97A in Example 193.
MS (ESI) m/e 312 (M+H)⁺;
¹H NMR (500 MHz, CD₃OD) δ 7.02 (d, 2H), 6.77 (d, 2H), 4.52 (d,1H 3.65 (ddd, 1H), 2.94 (dd, 1H), 2.75 (dd, 1H), 2.51 (d, 2H), 2.23 (s, 3H), 1.82 (ddd, 1H), 1.02 (d, 6H).

### Example 198

### (2RS,3R)-3-amino-2-hydroxy-5-phenyl-N'-(4-methylphenyl)pentanohydrazide

The desired product was prepared by substituting Example 125A for Example 97A in Example 193.
MS (ESI) m/e 314 (M+H)⁺;
¹H NMR (500 MHz, CD₃OD) δ 7.30 (m, 2H), 7.25-7.18 (m, 3H), 6.97 (m, 2H), 6.74 (m, 2H), 4.43 (d, 0.5H), 4.40 (d, 0.5H), 3.57 (m, 1H), 2.78 (m, 1.5H), 2.67 (m, 0.5H), 2.27 (m, 0.5H), 2.23 (s, 1.5H), 2.21 (s, 1.5H), 2.14 (m, 0.5H), 2.00 (m, 0.5H), 1.93 (m, 0.5H).

### Example 199

### (2S,3R)-3-amino-3-cyclooctyl-2-hydroxy-N'-(4-methylphenyl)propanohydrazide

### Example 199A

### (2S,3R)-3-(tertbutoxycarbonyl)amino-3-cyclooctyl-2-hydroxy-propanoic acid

The desired product was prepared by substituting cyclooctyl aldehyde for 2-ethylhexanal in Examples 236A-236C.

### Example 199B

### (2S,3R)-3-amino-3-cyclooctyl-2-hydroxy-N'-(4-methylphenyl)propanohydrazide

The desired product was prepared by substituting Example 199A for Example 97A in Example 193.
MS (ESI) m/e 320 (M+H)⁺;
¹H NMR (500 MHz, CD₃OD) δ 7.02 (d, 2H), 6.77 (m, 2H), 4.46 (d, 0.35H), 4.38 (d, 0.65H), 3.85 (m, 1H), 3.78 (m, 1H), 2.25 (s, 1.05H), 2.23 (s, 2.95H), 1.80-1.50 (m, 14H).

### Example 200

### (2RS,3S)-3-amino-4-((cyclohexylmethyl)sulfanyl)-2-hydroxy-N'-(4-methylphenyl)butanohydrazide

### Example 200A

### (2S)-2-((tert-butoxycarbonyl)amino)-3-(cyclohexylmethylsulfanyl)propanoic acid

The desired product was prepared by substituting cyclohexylmethyl bromide for 2-brompropane and D-cystine for D-homocystine in Example 123A.

### Example 200B

### (2RS,3S)-2-((tert-butoxycarbonyl)amino)-3-(cyclohexylmethylsulfanyl)propanoic acid

The desired product was prepared by substituting Example 200A for (2R)-2-((tert-butoxycarbonyl)amino)-3-cyclohexylpropanoic acid in Examples 1A-C.

### Example 200C

### (2RS,3S)-3-amino-4-((cyclohexylmethyl)sulfanyl)-2-hydroxy-N'-(4-methylphenyl)butanohydrazide

The desired product was prepared by substituting Example 200B for Example 97A in Example 193.
MS (ESI) m/e 298 (M+H)⁺;
¹H NMR (500 MHz, CD₃OD) δ 7.02 (dd, 2H), 6.77 (d, 2H), 4.52 (d, 0.65H), 4.50 (d, 0.35H), 3.76 (m, 0.35H), 3.67-3.58 (m, 0.65H), 2.93 (dd, 0.65H), 2.82 (dd, 0.35H), 2.75 (m, 1H), 2.48 (m, 1H), 2.41 (m, 1H), 2.23 (s, 3H), 1.86 (m, 2H), 1.75-1.66 (m, 3H), 1.47 (m, 1H), 1.32-1.15 (m, 3H), 1.00 (m, 2H).

### Example 201

### (2RS,3S)-3-amino-4-(ethylsulfanyl)-2-hydroxy-N'-(1-naphthyl)butanohydrazide

The desired product was prepared by substituting 1-napthylhydrazine for 4-methylphenylhydrazine in Example 193.
MS (ESI) m/e 320 (M+H)⁺;
¹H NMR (500 MHz, CD₃OD) δ 8.07 (m, 1H), 7.81 (ddd, 1H), 7.47 (ddd, 2H), 7.39 (dd, 1H), 7.33 (ddd, 1H), 6.91 (dd, 1H), 4.62 (d, 0.65H), 4.60 (d, 0.35H), 4.51 (m, 0.5H), 3.71 (m, 1H), 3.00 (dd, 0.5H), 2.81 (m, 1H), 2.65 (dd, 1.3H), 2.60 (dd, 0.7H), 1.30 (t, 1.95H); 1.25 (t, 1.05H).

### Example 202

### (2RS,3R)-3-amino-3-cyclohexyl-2-hydroxy-N'-(1-naphthyl)propanohydrazide

The desired product was prepared by substituting 1-napthylhydrazine for 4-methylphenylhydrazine and Example 122A for Example 97A in Example 193.
MS (ESI) m/e 328 (M+H)⁺;
¹H NMR (500 MHz, CD₃OD) δ 8.08 (m, 1H), 7.82 (ddd, 1H), 7.47 (ddd, 2H), 7.39 (d, 1H), 7.33 (m, 1H), 6.91 (dd, 1H), 4.56 (d, 0.65H), 4.53 (d, 0.35H), 3.37 (dd, 1H), 1.99-1.72 (m, 6H), 1.39-1.12 (m, 5H).

### Example 203

### (2RS,3R)-3-amino-2-hydroxy-5-(isopropylsulfanyl)-N'-(1-naphthyl)pentanohydrazide

The desired product was prepared by substituting 1-napthylhydrazine for 4-methylphenylhydrazine and Example 123B for Example 97A in Example 193.
MS (ESI) m/e 348 (M+H)⁺;
¹H NMR (500 MHz, CD₃OD) δ 8.07 (m, 1H), 7.81 (ddd, 1H), 7.47 (ddd, 2H), 7.39 (m, 1H), 7.34 (m, 1H), 6.90 (dd, 1H), 4.51 (d, 0.35H), 4.46 (d, 0.65H), 3.76 (m, 1H), 2.99 (dd, 0.65H), 2.93 (dd, 0.35H), 2.74 (m, 1.65H), 2.69 (m, 0.35H), 2.17 (dd, 0.65H), 2.05 (dd, 0.35H), 1.95 (dd, 1H), 1.29 (d, 3 H), 1.27 (d, 3H).

### Example 204

### (2RS,3S)-3-amino-2-hydroxy-4-(isobutylsulfanyl)-N'-(1-naphthyl)butanohydrazide

The desired product was prepared by substituting 1-napthylhydrazine for 4-methylphenylhydrazine and Example 124B for Example 97A in Example 193.
MS (ESI) m/e 347 (M+H)⁺;
¹H NMR (500 MHz, CD₃OD) δ 8.06 (m, 1H), 7.82 (ddd, 1H), 7.48 (ddd, 2H), 7.41 (m, 1H), 7.33 (t, 1H), 6.92 (d, 1H), 4.63 (d, 1H), 3.70 (ddd, 1H), 2.99 (dd, 1H), 2.80 (dd, 1H), 2.53 (d, 2H), 1.84 (ddd, 1H), 1.03 (d, 6H).

### Example 205

### (2RS,3R)-3-amino-2-hydroxy-5-phenyl-N'-(1-naphthyl)pentanohydrazide

The desired product was prepared by substituting 1-napthylhydrazine for 4-methylphenylhydrazine and Example 125A for Example 97A in Example 193.
MS (ESI) m/e 350 (M+H)⁺;
¹H NMR (500 MHz, CD₃OD) δ 8.06 (m, 1H), 7.80 (m, 1H), 7.46 (m, 2H), 7.37 (m, 1H), 7.31 (dd, 1H), 7.25 (m, 5H), 6.86 (dd, 1H), 4.53 (d, 0.5H), 4.51 (d, 0.5H), 3.65 (m, 0.5H), 3.60 (m, 0.5H), 2.82 (m, 1.5H), 2.72 (m, 0.5H), 2.20 (m, 0.5H), 2.08 (m, 1H), 1.98 (m, 0.5H).

### Example 206

### (2RS,3R)-3-amino-3-cyclooctyl-2-hydroxy-N'-(1-naphthyl)propanohydrazide

The desired product was prepared by substituting 1-napthylhydrazine for 4-methylphenylhydrazine and Example 199A for Example 97A in Example 193.
MS (ESI) m/e 356 (M+H)⁺;
¹H NMR (500 MHz, CD₃OD) δ 8.08 (m, 1H), 7.84 (m, 1H), 7.49 (m, 2H), 7.41 (m, 1H), 7.32 (t, 1H), 6.97 (ddd, 1H), 4.49 (d, 0.5H), 3.99 (d, 0.5H), 3.85 (m, 1H), 1.80-1.52 (m, 15H).

### Example 207

### N'-((2RS,3S)-3-amino-4-(ethylsulfanyl)-2-hydroxybutanoyl)-3-chlorobenzohydrazide

The desired product was prepared by substituting 3-chlorobenzoylhydrazide for 4-methylphenylhydrazine in Example 193.
MS (ESI) m/e 332 (M+H)⁺;
¹H NMR (500 MHz, CD₃OD) δ 7.91 (m, 1H), 7.81 (m, 1H), 7.62 (m, 1H), 7.51 (m, 1H), 4.63 (d, 0.65H), 4.58 (d, 0.35H), 3.70 (m, 1H), 3.00 (dd, 1H), 2.84 (m, 1H), 2.66 (dd, 1.3H), 2.62 (dd, 0.7H), 1.31 (t, 1.95H), 1.29 (t, 1.05H).

### Example 208

### N'-((2RS,3R)-3-amino-3-cyclohexyl-2-hydroxypropanoyl)-3-chlorobenzohydrazide

The desired product was prepared by substituting 3-chlorobenzoylhydrazide for 4-methylphenylhydrazine and Example 122A for Example 97A in Example 193.
MS (ESI) m/e 340 (M+H)⁺;
¹H NMR (500 MHz, CD₃OD) δ 7.91 (m, 1H), 7.82 (m, 1H), 7.62 (m, 1H), 7.50 (m, 1H), 4.56 (d, 0.65H), 4.52 (d, 0.35H), 3.33 (m, 1H), 1.95 (m, 1H), 1.86 (m, 4H), 1.74 (m, 1H), 1.35 (m, 2H), 1.24 (m, 1H), 1.16 (m, 2H).

### Example 209

### N'-((2RS,3S)-3-amino-2-hydroxy-4-(propylsulfanyl)butanoyl)-3-chlorobenzohydrazide

The desired product was prepared by substituting 3-chlorobenzoylhydrazide for 4-methylphenylhydrazine and Example 195B for Example 97A in Example 193.
MS (ESI) m/e 346 (M+H)⁺;
¹H NMR (500 MHz, CD₃OD) δ 7.91 (ddd, 1H), 7.81 (ddd, 1H), 7.62 (ddd, 1H), 7.50 (m, 1H), 4.63 (d, 0.65H), 4.58 (d, 0.35H), 3.70 (m, 1H), 3.17 (dd, 0.35H), 2.98 (dd, 0.65H), 2.84 (dd, 0.65H), 2.80 (dd, 0.35H), 2.60 (m, 2H), 1.66 (m, 2H), 1.03 (t, 1.95 H), 1.01 (t, 1.05H).

### Example 210

### N'-((2RS,3R)-3-amino-2-hydroxy-5-(isopropylsulfanyl)pentanoyl)-3-chlorobenzohydrazide

The desired product was prepared by substituting 3-chlorobenzoylhydrazide for 4-methylphenylhydrazine and Example 123B for Example 97A in Example 193.
MS (ESI) m/e 360 (M+H)⁺;
¹H NMR (500 MHz, CD₃OD) δ 7.90 (dt, 1H), 7.81 (m, 1H), 7.62 (m, 1H), 7.50 (m, 1H), 4.49 (d, 0.3H), 4.45 (d, 0.7H), 3.78 (m, 1H), 3.00 (m, 1H), 2.72 (t, 2H), 2.15 (m, 1H), 1.98 (m, 1H), 1.29-1.24 (m, 6H).

### Example 211

### N'-((2RS,3S)-3-amino-2-hydroxy-4-(isobutylsulfanyl)butanoyl)-3-chlorobenzohydrazide

The desired product was prepared by substituting 3-chlorobenzoylhydrazide for 4-methylphenylhydrazine and Example 124B for Example 97A in Example 193.
MS (ESI) m/e 360 (M+H)⁺;
¹H NMR (500 MHz, CD₃OD) δ 7.91 (dd, 1H), 7.82 (ddd, 1H), 7.63 (ddd, 1H), 7.51 (t, 1H), 4.64 (d, 1H), 3.70 (ddd, 1H), 2.97 (dd, 1H), 2.83 (dd, 1H), 2.53 (d, 2H), 1.85 (ddd, 1H), 1.04 (d, 6H).

### Example 212

### N'-((2RS,3R)-3-amino-2-hydroxy-5-phenylpentanoyl)-3-chlorobenzohydrazide

The desired product was prepared by substituting 3-chlorobenzoylhydrazide for 4-methylphenylhydrazine and Example 125A for Example 97A in Example 193.
MS (ESI) m/e 362 (M+H)⁺;
¹H NMR (500 MHz, CD₃OD) δ 7.91 (m, 1H), 7.81 (ddd, 1H), 7.61 (ddd, 1H), 7.50 (m, 1H), 7.29 (m, 4H), 7.20 (m, 1H), 4.50 (m, 1H), 3.62 (m, 1H), 2.80 (m, 2H), 2.20 (m, 1H), 2.02 (m, 1H).

### Example 213

### N'-((2RS,3R)-3-amino-3-cyclooctyl-2-hydroxypropanoyl)-3-chlorobenzohydrazide

The desired product was prepared by substituting 3-chlorobenzoylhydrazide for 4-methylphenylhydrazine and Example 199A for Example 97A in Example 193.
MS (ESI) m/e 368 (M+H)⁺;
¹H NMR (500 MHz, CD₃OD) δ 7.90 (m, 1H), 7.82 (m, 1H), 7.62 (ddd, 1H), 7.50 (t, 1H), 3.95 (d, 1H), 3.82 (t, 1H), 1.81 (m, 4H), 1.68 (m, 4H), 1.55 (m, 7H).

### Example 214

### N-((2RS,3R)-3-amino-5-cyclohexyl-2-hydroxypentanoyl)-3-chlorobenzohydrazide

The desired product was prepared by substituting 3-chlorobenzoylhydrazide for 4-methylphenylhydrazine and Example 238A for Example 97A in Example 193.
MS (ESI) m/e 367 (M+H)⁺;
¹H NMR (500 MHz, CD₃OD) δ 7.89 (dd, 1H), 7.80 (d, 1H), 7.62 (ddd, 1H), 7.50 (t, 1H), 3.90 (m, 1H), 3.78 (d, 1H), 1.81-1.72 (m, 5H), 1.68 (m, 1H), 1.54 (m, 2H), 1.29 (m, 4H), 1.21 (m, 1H), 0.96 (m, 2H).

### Example 215

### (2RS,3R)-3-amino-5-cyclohexyl-2-hydroxy-N'-(4-methylphenyl)pentanohydrazide

The desired product was prepared by substituting Example 238A for Example 97A in Example 193.
MS (ESI) m/e 320 (M+H)⁺;
¹H NMR (500 MHz, CD₃OD) δ 7.07 (d, 0.5H), 7.00 (d, 1.5H), 6.77 (m, 2H), 3.81 (m, 1H), 3.62 (d, 1H), 2.22 (s, 3H), 1.77-1.60 (m, 6H), 1.50 (m, 2H), 1.29-1.17 (m, 6H), 0.97-0.91 (m, 1H).

### Example 216

### (2RS,3S)-3-amino-2-hydroxy-N'-(1-naphthyl)-4-(propylsulfanyl)butanohydrazide

The desired product was prepared by substituting 1-naphthylhydrazine for 4-methylphenylhydrazine and Example 195B for Example 97A in Example 193.
MS (ESI) m/e 334 (M+H)⁺;
¹H NMR (500 MHz, CD₃OD) δ 8.06 (m, 1H), 7.81 (ddd, 1H), 7.47 (ddd, 2H), 7.39 (d, 1H), 7.34 (m, 1H), 6.90 (dd, 1H), 4.62 (d, 0.65H), 4.59 (d, 0.35H), 3.71 (m, 1H), 2.99 (dd, 0.65H), 2.92 (dd, 0.35H), 2.81 (m, 1H), 2.60 (dd, 1H), 2.54 (m, 1H), 1.66 (dd, 1H), 1.59 (dd, 1H), 1.02 (t, 1.95 H), 0.98 (t, 1.05H).

### Example 217

### (2RS,3R)-3-amino-5-cyclohexyl-2-hydroxy-N'-(1-naphthyl)pentanohydrazide

The desired product was prepared by substituting 1-naphthylhydrazine for 4-methylphenylhydrazine and Example 238A for Example 97A in Example 193.
MS (ESI) m/e 356 (M+H)⁺;
¹H NMR (500 MHz, CD₃OD) δ 8.06 (m, 1H), 7.81 (m, 1H), 7.47 (m, 2H), 7.41 (m, 1H), 7.31 (t, 1H), 6.95 (dd, 1H), 3.91 (m, 1H), 3.78 (d, 1H), 1.74 (m, 5H), 1.55 (m, 2H), 1.28 (m, 4H), 1.20 (m, 2H), 0.97 (m, 2H).

### Example 218

### (2RS,3S)-3-amino-4-((cyclohexylmethyl)sulfanyl)-2-hydroxy-N'-(1-naphthyl)butanohydrazide

The desired product was prepared by substituting 1-naphthylhydrazine for 4-methylphenylhydrazine and Example 200B for Example 97A in Example 193.
MS (ESI) m/e 388 (M+H)⁺;
¹H NMR (500 MHz, CD₃OD) δ 8.06 (m, 1H), 7.81 (ddd, 1H), 7.47 (ddd, 2H), 7.39 (dd, 1H), 7.32 (m, 1H), 6.90 (dd, 1H), 4.62 (d, 0.65H), 4.58 (d, 0.35H), 3.68 (m, 1H), 2.96 (dd, 1H), 2.83 (dd, 0.35H), 2.77 (dd, 0.65H), 2.51 (d, 1.5H), 2.44 (d, 0.5H), 1.88 (m, 2H), 1.75-1.66 (m, 3H), 1.50 (m, 1H), 1.22, (m, 3H), 0.98 (m, 2H).

### Example 219

### N'-((2RS,3S)-3-amino-4-((cyclohexylmethyl)sulfanyl)-2-hydroxybutanoyl)-3-chlorobenzohydrazide

The desired product was prepared by substituting 3-chlorobenzoylhydrazide for 4-methylphenylhydrazine and Example 200B for Example 97A in Example 193.
MS (ESI) m/e 401 (M+H)⁺;
¹H NMR (500 MHz, CD₃OD) δ 7.91 (m, 1H), 7.81 (dd, 1H), 7.61 (ddd, 1H), 7.50 (t, 1H), 4.63 (d, 0.65H), 4.56 (d, 0.35H), 3.68 (m, 1H), 2.95 (dd, 1H), 2.81 (dd, 1H), 2.78 (dd, 0.35H), 2.52 (d, 1H), 2.46 (m, 0.65H), 1.88 (m, 2H), 1.76-1.64 (m, 3H), 1.51 (m, 1H), 1.32-1.16 (m, 3H), 0.99 (m, 2H).

### Example 220

### (2RS,3R)-3-amino-2-hydroxy-5-phenyl-N'-(1-naphthyl)pentanohydrazide

The desired product was prepared by substituting 2-naphthoylhydrazine for O-phenyl hydroxylamine hydrochloride and Example 125A for Example 98A in Example 98B.
MS (ESI) m/e 378 (M+H)⁺;
¹H NMR (300 MHz, DMSO-d₆) δ 10.73 (br s, 1H), 10.32 (br s, 1H), 8.52 (s, 1H), 8.04 (m, 6H), 7.65 (m, 2H), 7.30 (m, 5H), 6.69 (m, 1H), 4.32 (m, 1H), 2.74 (m, 2H), 2.06 (m, 1H), 1.89 (m, 1H).

### Example 221

### N'-((2RS,3R)-3-amino-3-cyclohexyl-2-hydroxypropanoyl)-2-naphthohydrazide

The desired product was prepared by substituting 2-naphthoylhydrazine for O-phenyl hydroxylamine hydrochloride and Example 122A for Example 98A in Example 98B.
MS (ESI) m/e 356 (M+H)⁺;
¹H NMR (300 MHz, DMSO-d₆) δ 10.60 (s, 1H), 10.29 (s, 1H), 8.51 (s, 1H), 8.07 (m, 4H), 7.80 (br s, 2H), 7.64 (m, 2H), 6.54 (m, 1H), 4.41 (m, 1H), 1.77-1.44 (m, 6H), 1.20-0.94 (m, 5H).

### Example 222

### N'-((2RS,3R)-3-amino-2-hydroxy-5-isopropylsulfanylpentanoyl)-2-naphthohydrazide

The desired product was prepared by substituting 2-naphthoylhydrazine for O-phenyl hydroxylamine hydrochloride and Example 123B for Example 98A in Example 98B.
MS (ESI) m/e 376 (M+H)⁺;
¹H NMR (300 MHz, DMSO-d₆) δ 10.56 (br s, 0.7H), 10.47 (br s, 0.3H), 9.90 (br s, 0.3H), 9.83 (br s, 0.7H), 8.75 (s, 0.3H), 8.51 (s, 0.7H), 8.00 (m, 3H), 7.63 (m, 2H), 7.38 (m, 1H), 7.22 (m, 1H), 6.89 (m, 1H), 6.18 (m, 1H), 4.21 (m, 0.7H), 4.13 (m, 0.3H), 2.96 (m, 1H), 2.70 (m, 1H), 2.62 (m, 1H), 1.91 (m, 1H), 1.73 (m, 1H), 1.21 (d, 6H).

### Example 223

### N'-((2RS,3S)-3-amino-2-hydroxy-4-(isobutylsulfanyl)butanoyl)-2-naphthohydrazide

The desired product was prepared by substituting 2-naphthoylhydrazine for O-phenyl hydroxylamine hydrochloride and Example 124B for Example 98A in Example 98B.
MS (ESI) m/e 376 (M+H)⁺;
¹H NMR (300 MHz, DMSO-d₆) δ 8.52 (s, 1H), 8.04 (m, 6H), 7.65 (m, 2H), 6.74 (m, 1H), 4.40 (m, 1H), 2.94 (dd. 1H), 2.72 (dd, 1H), 2.48 (d, 2H), 1.80 (m, 1H), 0.98 (d, 6H).

### Example 224

### N'-((2S,3R)-3-amino-2-hydroxy-5-(isopropylsulfanyl)pentanoyl)-3-chlorobenzohydrazide

### Example 224A

### N'-((2S,3R)-3-(tert-butoxycarbonyl)amino-2-hydroxy-5-(isopropylsulfanyl)pentanoyl)-3-chlorobenzohydrazide

A solution of Example 123B (0.20 g, 0.65 mmol), 3-chlorobenzoyl hydrazine (0.17 g, 1.0 mmol), 1-(3-dimethylaminopropyl)-3-ethylcarbodiimide hydrochloride (0.13 g, 0.68 mmol), 1-hydroxybenzotriazole (0.11 g, 0.81 mmol), and N-methylmorpholine (0.070 mL, 0.64 mmol) in dichloromethane (6 mL) at room temperature was stirred for 16 hours, diluted with dichloromethane, washed sequentially with aqueous NaHCO₃, brine, 10% KHSO₄, and brine, dried (MgSO₄), filtered, and concentrated. The concentrate was purified by HPLC on silica gel with 4:1/hexanes:acetone to provide the desired product.
MS (ESI) m/e 460 (M+H)⁺.

### Example 224B

### N'-((2S,3R)-3-amino-2-hydroxy-5-(isopropylsulfanyl)pentanoyl)-3-chlorobenzohydrazide

The desired product was prepared by substituting Example 224A for Example 118A in Example 118B.
MS (ESI) m/e 360 (M+H)⁺;
¹H NMR (300 MHz, DMSO-d₆) δ 11.60 (br s, 1H), 10.30 (br s, 1H), 8.01 (br s, 2H), 7.92 (m, 1H), 7.85 (d, 1H), 7.69 (m, 1H), 7.57 (t, 1H), 6.67 (d, 1H), 4.26 (m, 1H), 2.97 (m, 1H), 2.70 (m, 1H), 2.62 (m, 1H), 1.97 (m, 1H), 1.85 (m, 1H), 1.21 (d, 6H).

### Example 225

### N'-((2S,3R)-3-anuno-2-hydroxy-5-(ethylsulfanyl)pentanoyl)-3-chlorobenzohydrazide

### Example 225A

### N'-((2S,3R)-3-(tert-butoxycarbonyl)amino-2-hydroxy-5-(isopropylsulfanyl)pentanoyl)-3-chlorobenzohydrazide

The desired product was prepared by substituting Example 98A for Example 123B in Example 224A.
MS (ESI) m/e 446 (M+H)⁺.

### Example 225B

### N'-((2S,3R)-3-amino-2-hydroxy-5-(ethylsulfanyl)pentanoyl)-3-chlorobenzohydrazide

The desired product was prepared by substituting Example 225A for Example 118A in Example 118B.
MS (ESI) m/e 346 (M+H)⁺;
¹H NMR (300 MHz, DMSO-d₆) δ 10.68 (br s, 1H), 10.29 (br s, 1H), 7.99 (br s, 2H), 7.91 (m, 1H), 7.84 (m, 1H), 7.69 (m, 1H), 7.57 (t, 1H), 6.67 (d, 1H), 4.25 (m, 1H), 3.40 (m, 1H), 2.67 (m, 2H), 2.50 (m, 2H), 1.99 (m, 1H), 1.85 (m, 1H), 1.19 (t, 3H).

### Example 226

### N'-((2RS,3R)-3-amino-4-cyclohexyl-2-hydroxybutanoyl)-1-naphthohydrazide

The desired product was prepared by substituting 1-naphthoylhydrazine for O-phenyl hydroxylamine hydrochloride and Example 1C for Example 98A in Example 98B.
MS (ESI) m/e 370 (M+H)⁺;
¹H NMR (300 MHz, DMSO-d₆) δ 10.53 (br s, 1H), 10.34 (br s, 1H), 8.34 (dd, 1H), 8.08 (d, 1H), 8.01 (m, 1H), 7.85 (br s, 2H), 7.66 (m, 1H), 7.59 (m, 3H), 6.61 (d, 1H), 4.20 (m, 1H), 1.74-1.60 (m, 6H), 1.50-1.40 (m, 1H), 1.33-1.15 (m, 4H), 0.96-0.80 (m, 2H).

### Example 227

### N'-((2RS,3R)-3-amino-4-cyclohexyl-2-hydroxybutanoyl)-3-hydroxy-2-naphthohydrazide

The desired product was prepared by substituting 3-hydroxy-2-naphthoylhydrazine for O-phenyl hydroxylamine hydrochloride and Example 1C for Example 98A in Example 98B.
MS (ESI) m/e 386 (M+H)⁺;
¹H NMR (300 MHz, DMSO-d₆) δ 11.44 (br s, 1H), 10.70 (br s, 1H), 10.58 (br s, 1H), 8.52 (s, 1H), 7.93 (d, 1H), 7.79 (m, 3H), 7.53 (t, 1H), 7.38 (d, 1H), 7.43 (s, 1H), 6.61 (d, 1H), 4.22 (m, 1H), 1.74-1.60 (m, 6H), 1.50-1.40 (m, 1H), 1.33-1.15 (m, 4H), 0.96-0.80 (m, 2H).

### Example 228

### N'-((2RS,3R)-3-amino-5-(ethylsulfanyl)-2-hydroxypentanoyl)-1-naphthohydrazide

The desired product was prepared by substituting 1-naphthoylhydrazine for O-phenyl hydroxylamine hydrochloride in Example 98B.
MS (ESI) m/e 362 (M+H)⁺;
¹H NMR (300 MHz, DMSO-d₆) δ 10.54 (br s, 1H), 10.37 (br s, 1H), 8.34 (br s, 1H), 8.01 (m, 3H), 7.59 (m, 4H), 6.68 (d, 0.7H), 6.60 (d, 0.3H), 4.40 (m, 0.3H), 4.26 (m, 0.7H), 3.39 (m, 1H), 2.70 (m, 2H), 2.54 (m, 2H), 2.06 (m, 1H), 1.87 (m, 1H), 1.19 (t, 3H).

### Example 229

### N'-((2RS,3R)-3-amino-5-(ethylsulfanyl)-2-hydroxypentanoyl)-3-hydroxy-2-naphthohydrazide

The desired product was prepared by substituting 3-hydroxy-2-naphthoylhydrazine for O-phenyl hydroxylamine hydrochloride in Example 98B.
MS (ESI) m/e 378 (M+H)⁺;
¹H NMR (300 MHz, DMSO-d₆) δ 11.45 (br s, 1H), 10.69 (br s, 1H), 10.58 (br s, 1H), 8.52 (br s, 1H), 8.03 (br s, 2H), 7.90 (m, 2H), 7.77 (m, 1H), 7.53 (t, 1H), 7.38 (d, 1H), 7.33 (m, 1H), 6.68 (d, 0.6H), 6.62 (d, 0.4H), 4.40 (m, 0.4H), 4.29 (m, 0.6H), 3.39 (m, 1H), 2.66 (m, 2H), 2.54 (m, 2H), 1.97 (m, 1H), 1.87 (m, 1H), 1.20 (t, 3H).

### Example 23 0

### N'-((2S,3R)-3-amino-2-hydroxy-5-phenylpentanoyl)-1-naphthohydrazide

The desired product was prepared by substituting 1-naphthoylhydrazine for O-phenyl hydroxylamine hydrochloride and Example 125A for Example 98A in Example 98B.
MS (ESI) m/e 378 (M+H)⁺;
¹H NMR (300 MHz, DMSO-d₆) δ 10.54 (br s, 1H), 10.38 (br s, 1H), 8.35 (m, 1H), 8.08 (m, 1H), 8.00 (br s, 2H), 7.59 (m, 4H), 7.29 (m, 5H), 7.18 (m, 1H), 6.68 (m, 1H), 4.31 (m, 1H), 2.74 (m, 2H), 2.06 (m, 1H), 1.89 (m, 1H).

### Example 231

### N'-((2S,3R)-3-amino-2-hydroxy-5-phenylpentanoyl)-3-hydroxy-2-naphthohydrazide

The desired product was prepared by substituting 3-hydroxy-2-naphthoylhydrazine for O-phenyl hydroxylamine hydrochloride and Example 125A for Example 98A in Example 98B.
MS (ESI) m/e 394 (M+H)⁺;
¹H NMR (300 MHz, DMSO-d₆) δ 11.43 (br s, 1H), 10.70 (br s, 1H), 10.58 (br s, 1H), 8.52 (s, 1H), 7.94 (m, 3H), 7.77 (d, 1H), 7.53 (t, 1H), 7.30 (m, 7H), 6.69 (d, 1H), 4.35 (m, 1H), 2.72 (m, 2H), 1.99 (m, 1H), 1.91 (m, 1H).

### Example 232

### (2S,3R)-3-amino-4-cyclohexyl-2-hydroxybutanohydrazide

### Example 232A

### (2S,3R)-3-((tert-butoxycarbonyl)amino)-4-cyclohexyl-2-hydroxybutanoic acid pentafluorophenyl ester

The title compound was prepared by substituting Example 1C for Example 124A in Example 126B.
MS (ESI) m/e 466 (M-H)⁺.

### Example 232B

### (2S,3R)-3-amino-4-cyclohexyl-2-hydroxybutanohydrazide

The title compound was prepared by substituting Example 232A for Example 126B and anhydrous hydrazine for Example 126A in Example 126C.
MS (ESI) m/e 216 (M+H)⁺.
¹H NMR (300 MHz, DMSO-d₆) δ 11.05 (br s, 1H), 7.97 (br s, 2H), 6.75 (br s, 1H), 4.22 (d, 2H), 1.74-1.60 (m 6H), 1.42 (d, 2H), 1.28-1.10 (m, 3H), 0.92-0.74 (m, 2H).

### Example 233

### N'-((2R,3R)-3-amino-2-hydroxy-5-(ethylsulfanyl)pentanoyl)-3-chlorobenzoyl hydrazide

### Example 233A

### N'-((2R,3R)-3-(tert-butoxycarbonyl)amino-2-hydroxy-5-(ethylsulfanyl)pentanoyl)-3-chlorobenzoyl hydrazide

The desired product was prepared by substituting Example 98A for Example 123B in Example 224A.
MS (ESI) m/e 446 (M+H)⁺.

### Example 233B

### N'-((2R,3R)-3-amino-2-hydroxy-5-(ethylsulfanyl)pentanoyl)-3-chlorobenzoyl hydrazide

The desired product was prepared by substituting Example 233A for Example 118A in Example 118B.
MS (ESI) m/e 346 (M+H)⁺;
¹H NMR (300 MHz, DMSO-d₆) δ 10.52 (br s, 1H), 10.20 (br s, 1H), 8.07 (br s, 2H), 7.90 (m, 1H), 7.83 (m, 1H), 7.67 (m, 1H), 7.56 (t, 1H), 6.60 (d, 1H), 4.39 (m, 1H), 3.57 (m, 1H), 2.69 (m, 2H), 2.53 (m, 2H), 1.95 (m, 1H), 1.86 (m, 1H), 1.18 (t, 3H).

### Example 234

### N'-((2R,3R)-3-amino-2-hydroxy-5-(isopropylsulfanyl)pentanoyl)-3-chlorobenzoyl hydrazide

### Example 234A

### N'-((2R,3R)-3-amino-2-hydroxy-5-(isopropylsulfanyl)pentanoyl)-3-chlorobenzoyl hydrazide

The desired product was prepared as described in Example 224A.
MS (ESI) m/e 460 (M+H)⁺.

### Example 234B

### N'-((2R,3R)-3-amino-2-hydroxy-5-(isopropylsulfanyl)pentanoyl)-3-chlorobenzoyl hydrazide

The desired product was prepared by substituting Example 234A for Example 118A in Example 118B.
MS (ESI) m/e 360 (M+H)⁺;
¹H NMR (300 MHz, DMSO-d₆) δ 10.52 (br s, 1H), 10.18 (br s, 1H), 8.11 (br s, 2H), 7.90 (m, 1H), 7.84 (d, 1H), 7.64 (m, 1H), 7.56 (t, 1H), 6.59 (d, 1H), 4.42 (m, 1H), 2.97 (m, 1H), 2.69 (m, 1H), 2.62 (m, 1H), 1.89 (m, 2H), 1.20 (d, 6H).

### Example 235

### 3-(2-aminoethyl)-N'-((2S,3S)-3-amino-2-hydroxy-4-(isobutylsulfanyl)butanoyl)benzohydrazide

### Example 235A

### methyl 3-(cyanomethyl)benzoate

A solution of methyl 3-(bromomethyl)benzoate (2.29 g, 0.01 mol) and potassium cyanide (3.26 g, 0.05 mol) in DMSO (20 mL) was heated to 100°C for 1h. The reaction mixture was cooled, partitioned between ether and water, washed with brine, dried (Na₂SO₄) and concentrated to give the title compound.
MS (ESI) m/e 193 (M+NH4)⁺.

### Example 235B

### 3-(2-(tertbutoxycarbonylamino)ethyl)benzoyl hydrazide

A solution of Example 23 5A ( 1.23 g, 7 mmol) in methanol (15 mL) was hydrogenated in the presence of RaNi (2.5 g) for 18h. After filtration and evaporation of the solvent, the resulting oil (0.1 g, 0.6 mmol) was dissolved in dichloromethane (5 mL) , treated with di-tert-butyl dicarbonate (0.13 g, 0.6 mmol) for 16 h at room temperature. The solvent was evaporated and the residue treated with hydrazine hydrate (0.2 mL, 6.0 mmol) in ethanol at reflux for 48h. After the solvent was evaporated, and the residue purified by silica gel chromatography (10:90/methanol:dichloromethane) to give the title compound.
MS (ESI) m/e 280 (M+H)⁺.

### Example 235C

### 3-(2-aminoethyl)-N'-((2S,3S)-3-amino-2-hydroxy-4-(isobutylsulfanyl)butanoyl)benzohydrazide

The title compound was prepared by substituting Example 235B for Example 126A in Example 126C.
MS (ESI) m/e 367 (M-H)⁺.
¹H NMR (300 MHz, DMSO-d₆) δ 10.62 (s, 1H), 10.27 (s, 1H), 8.12 (br s, 2H), 8.0 (br s, 2H), 7.78 (m, 2H), 7.5 (m, 2H), 6.74 (d, 1H), 4.4 (t, 1H), 3.7 (m, 2H), 2.92 (m, 3H), 2.75 (m, 1H), 2.48 (d, 2H), 1.84-1.7 (m, 1H), 0.97 (d, 6H).

### Example 236

### N'-((2S,3R,4RS)-3-amino-4-ethyl-2-hydroxyoctanoyl)-3-chlorobenzohydrazide

### Example 236A

### Ethyl 4-ethyl-oct-2-enoate

A solution of 2-ethyl-hexanal (5.0 g, 39 mmol), triethyl phosphonoacetate (8.0 mL, 40 mmol), lithium bromide (3.65 g, 42 mmol), and triethylamine (5.6 mL, 40 mmol) in tetrahydrofuran (160 mL) at room temperature was stirred for 16 hours, quenched with water, stirred for 15 minutes, diluted with ethyl acetate, washed sequentially with pH 7 buffer and brine, dried (MgSO₄), filtered, and concentrated. The concentrate was purified by flash column chromatography on silica gel with 1:1/dichloromethane:hexanes to provide the desired product.
MS (ESI) m/e 199 (M+H)⁺.

### Example 236B

### ethyl (2S,3R,4RS)-3-(tert-butoxycarbonyl)amino-4-ethyl-2-hydroxyoctanoate

A solution of tert-butylcarbamate (2.85 g, 24 mmol), tert-butylhypochlorite (2.7 mL, 24 mmol), and 0.5 M NaOH (50 mL, 25 mmol) in 1-propanol (75 mL) at room temperature was stirred for 15 minutes. Example 236A (1.70 g, 8.6 mmol), potassium osmate dihydrate (0.27 g, 0.73 mmol) and hydroquinidine 1,4-phthalazinediyl diether (0.62 g, 0.80 mmol) were added, and the mixture stirred in an ice bath for 2 hours, diluted with ethyl acetate, washed sequentially with water, 1 M HCl, aqueous NaHCO₃, and brine, dried (MgSO₄), filtered, and concentrated. The concentrate was purified by flash column chromatography on silica gel with 1:4/ethyl acetate:hexanes to provide the desired product.
MS (ESI) m/e 331 (M+H)⁺.

### Example 236C

### (2S,3R,4RS)-3-(tert-butoxycarbonyl)amino-4-ethyl-2-hydroxyoctanoic acid

A solution of Example 236B (0.168 g, 0.51 mmol), 30% hydrogen peroxide (0.25 mL, 2.2 mmol), and lithium hydroxide monohydrate (0.042 g, 1.0 mmol) in 3:1 tetrahydrofuran/water (7 mL) was stirred in an ice bath for 3 hours, then concentrated. The residues were taken up in water and the pH adjusted to 10 with NaOH. The solution was washed twice with ether, adjusted to pH 2 with HCl, then extracted twice with ethyl acetate. The ethyl acetate extracts were dried (MgSO₄) filtered, then concentrated to provide the desired product.
MS (ESI) m/e 304 (M+H)⁺.

### Example 236D

### N'-((2S,3R,4RS)-3-amino-4-ethyl-2-hydroxyoctanoyl)-3-chlorobenzohydrazide

The desired product was prepared by substituting 3-chlorobenzoylhydrazine for O-phenyl hydroxylamine hydrochloride and Example 236C for Example 98A in Example 98B.
MS (ESI) m/e 356 (M+H)⁺;
¹H NMR (300 MHz, DMSO-d₆) δ 8.17 (br s, 2H), 7.96 (m, 1H), 7.87 (d, 1H), 7.58 (m, 1H), 7.52 (t, 1H), 5.79 (m, 1H), 3.70 (m, 1H), 1.58 (m, 2H), 1.40-0.80 (m, 5H), 0.70 (t, 3H), 0.64 (m, 2H), 0.42 (t, 3H).

### Exzample 237

### N'-((2S,3S)-3-amino-2-hydroxy-4-(isobutylsulfanyl)butanoyl)-3-propoxybenzohydrazide

### Example 237A

### methyl 3-propoxybenzoate

The title compound was prepared by substituting 1-bromopropane for Example 364A in Example 364B.
¹H NMR (300 MHz, DMSO-d₆) δ 7.54 (m, 1H), 7.43 (m, 2H), 7.23 (m, 1H), 3.98 (t, 2H), 3.84 (s, 3H), 1.8-1.66 (m, 2H), 0.99 (t, 3H).

### Example 237B

### N'-((2S,3S)-3-amino-2-hydroxy-4-(isobutylsulfanyl)butanoyl)-3-propoxybenzohydrazide

The title compound was prepared by substituting Example 237B for Example 126A in Example 126C.
MS (ESI) m/e 382 (M-H)⁺.
¹H NMR (300 MHz, DMSO-d₆) δ 10.58 (s, 1H), 10.24 (s, 1H), 8.07 (br s, 2H), 7.44 (m, 3H), 7.16 (m, 1H), 6.24 (d, 1H), 4.4 (t, 1H), 3.98 (t, 2H), 3.4 (m, 1H), 2.92 (dd, 1H), 2.73 (dd, 1H), 2.46 (d, 2H), 1.84-1.7 (m, 3H), 1.0 (t, 3H), 0.97 (d, 6H).

### Example 238

### N'-((2S,3R)-3-amino-5-cyclohexyl-2-hydroxypentanoyl)-3-chlorobenzohydrazide

### Example 238A

### (2S,3R)-3-(tert-butoxycarbonyl)amino-5-cyclohexyl-2-hydroxypentanoic acid

The desired product was prepared by substituting 3-cyclohexylpropanal for 2-ethylhexanal in Examples 236A-236C.
MS (ESI) m/e 316 (M+H)⁺.

### Example 238B

### N'-(L2S,3R)-3-amino-5-cyclohexyl-2-hydroxypentanoyl)-3-chlorobenzohydrazide

The desired product was prepared by substituting 3-chlorobenzoylhydrazine for O-phenyl hydroxylamine hydrochloride and Example 238A for Example 98A in Example 98B.
MS (ESI) m/e 368 (M+H)⁺;
¹H NMR (300 MHz, DMSO-d₆) δ 10.67 (br s, 1H), 10.27 (br s, 1H), 7.91 (m, 1H), 7.85 (m, 3H), 7.69 (d, 1H), 7.57 (t, 1H), 6.58 (m, 1H), 4.18 (m, 1H), 1.80-1.50 (m, 7H), 1.35-1.10 (m, 6H), 0.89 (m, 2H).

### Example 239

### N'-((2RS,3R)-3-amino-5-(ethylsulfanyl)-2-hydroxypentanoyl)-3-trifluoromethylsulfanylbenzohydrazide

### Example 239A

### N'-((2RS,3R)-3-(tert-butoxycarbonyl)amino-5-(ethylsulfanyl)-2-hydroxypentanoyl)hydrazide

To a solution of Example 97A (2.00 g, 6.5 mmol) in acetonitrile (120 mL) was added HOAt (0.443 g, 3.3 mmol) and the mixture stirred for 5 min until homogeneous. To this solution was added DCC (2.00 g, 9.75 mmol) in acetonitrile (20 mL) and the mixture was stirred for 2 minutes. Hydrazine monohydrate (0.306 mL, 9.76 mmol) was added and the reaction stirred fo 16 hours. The solvent was removed *in vacuo* and the crude material filtered, the solid washed with dichloromethane, and the resulting oil was purified by column chromatography using ethyl acetate to give the desired product 1.2 g (60%).

### Example 239B

### N'-((2RS,3R)-3-amino-5-(ethylsulfanyl)-2-hydroxypentanoyl)-3-trifluoromethylsulfanylbenzohydrazide

To DCC resin (148 mg, 0.225 mmol) in 1.0 mL of dichloromethane was added 0.5 mL of a 0.45 M solution of HOBt (0.225 mmol) in dimethylacetamide/dichloromethane (1:6), and 0.5 mL of a 0.3M solution of 3-(ethylsulfanyl)benzoic acid (0.15 mmol) in dimethylacetamide. After 5 minutes, 1.0 mL of a 0.225 M solution of Example 239A (0.225 mmol) in dimethylacetamide/dichloromethane (1:1) was added. The mixture was agitated for 18 hours and quenched with 0.19 g oftrisamine resin (0.75 mmol) followed by 0.13 g of isocyanate resin (0.225 mmol) and agitated for 4 hours. The mixture was filtered and the resins washed with 1x3 mL of dichloromethane, the solvent was removed in vacuo, and the crude material purified by reverse phase preparative HPLC. The resulting material was treated with 1 mL of 50% trifluoroacetic acid/dichloromethane and agitated at ambient temperature for 18 hours. The solvent was removed in vacuo to give the desired product.
MS (ESI) m/e 412 (M+H)⁺;
¹H NMR (500 MHz, CD₃OD) δ 8.22 (d, 1H), 8.07 (dd, 1H), 7.93 (t, 1H), 7.65 (m, 1H), 4.49 (d, 0.35), 4.45 (d, 0.65), 3.78 (m, 1H), 2.71 (t, 2H), 2.61 (dd, 2H), 2.17 (dd, 1H), 1.99 (dt, 1H), 1.28 (t, 1.95H), 1.25 (t, 1.05H).

### Example 240

### N'-((2RS,3R)-3-amino-5-(ethylsulfanyl)-2-hydroxypentanoyl)-2-methylbenzohydrazide

The desired product was prepared by substituting 2-methylbenzoic acid for 3-(ethylsulfanyl)benzoic acid in Example 239B.
MS (ESI) m/e 326 (M+H)⁺;
¹H NMR (500 MHz, CD₃OD) δ 7.49 (m, 1H), 7.40 (dd, 1H), 7.28 (m, 2H), 4.48 (d, 0.35), 4.44 (d, 0.65), 3.80 (m, 1H), 2.71 (t, 2H), 2.61 (dd, 2H), 2.47 (s, 1.95H), 2.46 (s, 1.05H), 2.17 (dd, 1H), 1.99 (dt, 1H), 1.27 (t, 1.95H), 1.245 (t, 1.05H).

### Example 241

### N'-((2RS,3R)-3-amino-5-(ethylsulfanyl)-2-hydroxypentanoyl)-3-methylbenzohydrazide

The desired product was prepared by substituting 3-methylbenzoic acid for 3-(ethylsulfanyl)benzoic acid in Example 239B.
MS (ESI) m/e 326 (M+H)⁺;
¹H NMR (500 MHz, CD₃OD) δ 7.72 (d, 1H), 7.67 (dd, 1H), 7.42 (t, 1H), 7.38 (m, 1H), 4.48 (d, 0.35), 4.43 (d, 0.65), 3.77 (m, 1H), 2.71 (t, 2H), 2.61 (dd, 2H), 2.41 (s, 3H), 2.16 (dd, 1H), 1.99 (dt, 1H), 1.28 (t, 1.95H), 1.25 (t, 1.05H).

### Example 242

### N'-((2RS,3R)-3-amino-5-(ethylsulfanyl)-2-hydroxypentanoyl)-4-methylbenzohydrazide

The desired product was prepared by substituting 4-methylbenzoic acid for 3-(ethylsulfanyl)benzoic acid in Example 239B.
MS (ESI) m/e 326 (M+H)⁺;
¹H NMR (500 MHz, CD₃OD) δ 7.79 (m, 2H), 7.32 (m, 2H), 4.48 (d, 0.35), 4.44 (d, 0.65), 3.78 (m, 1H), 2.71 (t, 2H), 2.61 (dd, 2H), 2.41 (s, 3H), 2.16 (dd, 1H), 2.00 (dt, 1H), 1.27 (t, 1.95H), 1.26 (t, 1.05H).

### Example 243

### N'-((2RS,3R)-3-amino-5-(ethylsulfanyl)-2-hydroxypentanoyl)-2-aminobenzohydrazide

The desired product was prepared by substituting 2-aminobenzoic acid for 3-(ethylsulfanyl)benzoic acid in Example 239B.
MS (ESI) m/e 327 (M+H)⁺;
¹H NMR (500 MHz, CD₃OD) δ 7.56 (dd, 1H), 7.24 (m, 1H), 6.78 (d, 1H), 6.65 (ddd, 1H), 4.48 (d, 0.35), 4.44 (d, 0.65), 3.78 (m, 1H), 2.71 (t, 2H), 2.61 (dd, 2H), 2.17 (dd, 1H), 2.00 (dt, 1H), 1.28 (t, 1.95H), 1.25 (t, 1.05H).

### Example 244

### N-((2RS,3R)-3-amino-5-(ethylsulfanyl)-2-hydroxypentanoyl)-3-aminobenzohydrazide

The desired product was prepared by substituting 3-aminobenzoic acid for 3-(ethylsulfanyl)benzoic acid in Example 239B.
MS (ESI) m/e 327 (M+H)⁺;
¹H NMR (500 MHz, CD₃OD) δ 7.26 (m, 3H), 6.99 (m, 1H), 4.48 (d, 0.35), 4.43 (d, 0.65), 3.78 (m, 1H), 2.71 (t, 2H), 2.61 (dd, 2H), 2.16 (dd, 1H), 1.99 (dt, 1H), 1.27 (t, 1.95H), 1.25 (t, 1.05H).

### Example 245

### N'-((2RS,3R)-3-amino-5-(ethylsulfanyl)-2-hydroxypentanoyl)-4-aminobenzohydrazide

The desired product was prepared by substituting 4-aminobenzoic acid for 3-(ethylsulfanyl)benzoic acid in Example 239B.
MS (ESI) m/e 327 (M+H)⁺;
¹H NMR (500 MHz, CD₃OD) δ 7.66 (dd, 2H), 6.67 (dd, 2H), 4.46 (d, 0.35), 4.42 (d, 0.65), 3.76 (m, 1H), 2.70 (t, 2H), 2.61 (dd, 2H), 2.15 (dd, 1H), 1.99 (dt, 1H), 1.27 (t, 1.95H), 1.25 (t, 1.05H).

### Example 246

### N-((2RS,3R)-3-amino-5-(ethylsulfanyl)-2-hydroxypentanoyl)-2-hydroxybenzohydrazide

The desired product was prepared by substituting 2-hydroxybenzoic acid for 3-(ethylsulfanyl)benzoic acid in Example 239B.
MS (ESI) m/e 328 (M+H)⁺;
¹H NMR (500 MHz, CD₃OD) δ 7.86 (ddd, 1H), 7.44 (m, 1H), 6.96 (m, 2H), 4.49 (d, 0.35), 4.44 (d, 0.65), 3.78 (m, 1H), 2.71 (t, 2H), 2.61 (dd, 2H), 2.17 (dd, 1H), 1.99 (dt, 1H), 1.27 (t, 1.95H), 1.25 (t, 1.05H).

### Example 247

### N'-((2RS,3R)-3-amino-5-(ethylsulfanyl)-2-hydroxypentanoyl)-3-hydroxybenzohydrazide

The desired product was prepared by substituting 3-hydroxybenzoic acid for 3-(ethylsulfanyl)benzoic acid in Example 239B.
MS (ESI) m/e 328 (M+H)⁺;
¹H NMR (500 MHz, CD₃OD) δ 7.30 (m, 3H), 7.00 (ddd, 1H), 4.46 (d, 0.35), 4.42 (d, 0.65), 3.75 (m, 1H), 2.71 (t, 2H), 2.61 (dd, 2H), 2.15 (dd, 1H), 1.98 (dt, 1H), 1.27 (t, 1.95H), 1.25 (t, 1.05H).

### Example 248

### N'-((2RS,3R)-3-amino-5-(ethylsulfanyl)-2-hydroxypentanoyl)-4-hydroxybenzohydrazide

The desired product was prepared by substituting 4-hydroxybenzoic acid for 3-(ethylsulfanyl)benzoic acid in Example 239B.
MS (ESI) m/e 328 (M+H)⁺;
¹H NMR (500 MHz, CD₃OD) δ 7.77 (m, 2H), 6.85 (m, 2H), 4.47 (d, 0.35), 4.43 (d, 0.65), 3.77 (m, 1H), 2.71 (t, 2H), 2.61 (dd, 2H), 2.16 (dd, 1H), 1.99 (dt, 1H), 1.27 (t, 1.95H), 1.25 (t, 1.05H).

### Example 249

### N'-((2RS,3R)-3-amino-5-(ethylsulfanyl)-2-hydroxypentanoyl)-3-methoxybenzohydrazide

The desired product was prepared by substituting 3-methoxybenzoic acid for 3-(ethylsulfanyl)benzoic acid in Example 239B.
MS (ESI) m/e 342 (M+H)⁺;
¹H NMR (500 MHz, CD₃OD) δ 7.44 (m, 3H), 7.16 (ddd, 1H), 4.49 (d, 0.35), 4.44 (d, 0.65), 3.85 (s, 3H), 3.78 (m, 1H), 2.71 (t, 2H), 2.61 (dd, 2H), 2.17 (dd, 1H), 2.00 (dt, 1H), 1.28 (t, 1.95H), 1.25 (t, 1.05H).

### Example 250

### N'-((2RS,3P,)-3-amino-5-(ethylsulfanyl)-2-hydroxypentanoyl)-4-methoxybenzohydrazide

The desired product was prepared by substituting 4-hydroxybenzoic acid for 3-(ethylsulfanyl)benzoic acid in Example 239B.
MS (ESI) m/e 342 (M+H)⁺;
¹H NMR (500 MHz, CD₃OD) δ 7.87 (m, 2H), 7.02 (m, 2H), 4.47 (d, 0.35), 4.43 (d, 0.65), 3.87 (s, 3H), 3.76 (m, 1H), 2.71 (t, 2H), 2.61 (dd, 2H), 2.15 (dd, 1H), 1.99 (dt, 1H), 1.27 (t, 1.95H), 1.25 (t, 1.05H).

### Example 251

### N'-((2RS,3R)-3-amino-5-(ethylsulfanyl)-2-hydroxypentanoyl)-2-fluorobenzohydrazide

The desired product was prepared by substituting 2-fluorobenzoic acid for 3-(ethylsulfanyl)benzoic acid in Example 239B.
MS (ESI) m/e 330 (M+H)⁺;
¹H NMR (500 MHz, CD₃OD) δ 7.82 (m, 1H), 7.60 (m, 1H), 7.73 (t, 1H), 7.26 (m, 1H), 4.48 (d, 0.35), 4.44 (d, 0.65), 3.79 (m, 1H), 2.71 (t, 2H), 2.61 (dd, 2H), 2.16 (dd, 1H), 1.99 (dt, 1H), 1.27 (t, 1.95H), 1.25 (t, 1.05H).

### Example 252

### N'-((2RS,3R)-3-amino-5-(ethylsulfanyl)-2-hydroxypentanoyl)-3-fluorobenzohydrazide

The desired product was prepared by substituting 3-fluorobenzoic acid for 3-(ethylsulfanyl)benzoic acid in Example 239B.
MS (ESI) m/e 330 (M+H)⁺;
¹H NMR (500 MHz, CD₃OD) δ 7.71 (dd, 1H), 7.62 (m, 1H), 7.53 (t, 1H), 7.36 (m, 1H), 4.49 (d, 0.35), 4.45 (d, 0.65), 3.78 (m, 1H), 2.71 (t, 2H), 2.61 (dd, 2H), 2.17 (dd, 1H), 2.00 (dt, 1H), 1.28 (t, 1.95H), 1.25 (t, 1.05H).

### Example 253

### N'-((2RS,3R)-3-amino-5-(ethylsulfanyl)-2-hydroxmentanoyl)-4-fluorobenzohydrazide

The desired product was prepared by substituting 4-fluorobenzoic acid for 3-(ethylsulfanyl)benzoic acid in Example 239B.
MS (ESI) m/e 330 (M+H)⁺;
¹H NMR (500 MHz, CD₃OD) δ 7.95 (m, 2H), 7.24 (m, 2H), 4.49 (d, 0.35), 4.44 (d, 0.65), 3.78 (m, 1H), 2.71 (t, 2H), 2.61 (dd, 2H), 2.17 (dd, 1H), 2.00 (dt, 1H), 1.27 (t, 1.95H), 1.26 (t, 1.05H).

### Example 254

### (2RS,3R)-N'-acetyl-3-amino-5-(ethylsulfanyl)-2-hydroxypentanohydrazide

The desired product was prepared by substituting acetic acid for 3-(ethylsulfanyl)benzoic acid in Example 239B.
MS (ESI) m/e 250 (M+H)⁺;
¹H NMR (500 MHz, CD₃OD) δ 4.41 (d, 0.35), 4.37 (d, 0.65), 3.72 (m, 1H), 2.68 (ddd, 2H), 2.58 (dd, 1.3H), 2.55 (dd, 0.7H), 2.10 (m, 1H), 2.03 (s, 1.95H), 2.01 (s, 1.05H),1.95 (m, 1H), 1.23-1.28 (m, 3H).

### Example 255

### (2RS,3R)-3-amino-5-(ethylsulfanyl)-2-hydroxy-N'-isobutyrylpentanohydrazide

The desired product was prepared by substituting 2-methylpropionic acid for 3-(ethylsulfanyl)benzoic acid in Example 239B.
MS (ESI) m/e 278 (M+H)⁺;
¹H NMR (500 MHz, CD₃OD) δ 4.41 (d, 0.35), 4.37 (d, 0.65), 3.73 (m, 1H), 2.73-2.51 (m, 5H), 2.10 (m, 1H), 1.95 (dt, 1H), 1.26 (t, 1.95H), 1.24 (t, 1.05H), 1.17 (m, 6H).

### Example 256

### (2RS,3R)-3-amino-5-(ethylsulfanyl)-2-hydroxy-N'-(3-methylbutanoyl)pentanohydrazide

The desired product was prepared by substituting 3-methylbutyric acid for 3-(ethylsulfanyl)benzoic acid in Example 239B.
MS (ESI) m/e 292 (M+H)⁺;
¹H NMR (500 MHz, CD₃OD) δ 4.41 (d, 0.35), 4.37 (d, 0.65), 3.73 (m, 1H), 2.67 (dd, 2H), 2.59 (dd, 1.3H), 2.55 (dd, 0.7H), 2.07-2.16 (m, 4H), 1.95 (dt, 1H), 1.26 (t, 1.95H), 1.24 (t, 1.05H), 1.10 (t, 6H).

### Example 257

### (2RS,3R)-3-amino-5-(ethylsulfanyl)-N'-heptanoyl-2-hydroxypentanohydrazide

The desired product was prepared by substituting heptanoic acid for 3-(ethylsulfanyl)benzoic acid in Example 239B.
MS (ESI) m/e 320 (M+H)⁺;
¹H NMR (500 MHz, CD₃OD δ 4.41 (d, 0.35), 4.37 (d, 0.65), 3.73 (m, 1H), 2.67 (dd, 2H), 2.59 (dd, 1.3H), 2.55 (dd, 0.7H), 2.28 (dd, 2H), 2.12 (ddd, 1H), (1.95 (dt, 1H), 1.64 (dd, 2H), 1.31-1.40 (m, 6H), 1.26 (t, 1.95H), 1.24 (t, 1.05H), 0.91 (t, 3H).

### Example 258

### (2RS,3R)-3-amino-5-(ethylsulfanyl)-2-hydroxy-N'-(tetrahydro-2-furanylcarbonyl)pentanohydrazide

The desired product was prepared by substituting 2-tetrahydrofuroic acid for 3-(ethylsulfanyl)benzoic acid in Example 239B.
MS (ESI) m/e 306 (M+H)⁺;
¹H NMR (500 MHz, CD₃OD) δ 4.48-4.41 (m, 1.35), 4.37 (d, 0.65), 4.01 (m, 1H), 3.88 (m, 1H), 3.73 (m, 1H), 2.67 (dd, 2H), 2.59 (dd, 1.3H), 2.55 (dd, 0.7H), 2.29 (m. 1H), 2.08 (m, 2H), 1.96 (m, 3H), 1.26 (t, 1.95H), 1.24 (t, 1.05H).

### Example 259

### (2RS,3R)-3-amino-N'-(cyclohexylacetyl)-5-(ethylsulfanyl)-2-hydroxyheptanohydrazide

The desired product was prepared by substituting cyclohexylacetic acid for 3-(ethylsulfanyl)benzoic acid in Example 239B.
MS (ESI) m/e 332 (M+H)⁺;
¹H NMR (500 MHz, CD₃OD) δ 4.41 (d, 0.35), 4.36 (d, 0.65), 3.73 (m, 1H), 2.67 (dd, 2H), 2.59 (dd, 1.3H), 2.55 (dd, 0.7H), 2.16-2.07 (m, 3H), 1.95 (dt, 1H), 1.65-1.81 (m, 9H), 1.26 (t, 1.95H), 1.24 (t, 1.05H) 1.02 (dd, 2H).

### Example 261

### N'-((2RS,3R)-3-amino-5-(etlaylsulfanyl)-2-hydroxypentanoyl)-4-bromobenzohydrazide

The desired product was prepared by substituting 4-bromobenzoic acid for 3-(ethylsulfanyl)benzoic acid in Example 239B.
MS (ESI) m/e 391 (M+H)⁺;
¹H NMR (500 MHz, CD₃OD) δ 7.80 (m, 2H), 7.68 (m, 2H), 4.49 (d, 0.35), 4.44 (d, 0.65), 3.78 (m, 1H), 2.71 (t, 2H), 2.61 (dd, 2H), 2.16 (dd, 1H), 1.99 (dt, 1H), 1.27 (t, 1.95H), 1.25 (t, 1.05H).

### Example 262

### (2RS,3R)-3-amino-5-(ethylsulfanyl)-2-hydroxy-N'-(phenylacetyl)pentanohydrazide

The desired product was prepared by substituting phenylacetic acid for 3-(ethylsulfanyl)benzoic acid in Example 239B.
MS (ESI) m/e 326 (M+H)⁺;
¹H NMR (500 MHz, CD₃OD δ 7.35-7.23 (m, 5H), 4.41 (d, 0.35), 4.37 (d, 0.65), 3.72 (m, 1H), 3.61 (d, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 263

### (2RS,3R)-3-amino-5-(ethylsulfanyl)-2-hydroxy-N'-((2-methoxyphenyl)acetyl)pentanohydrazide

The desired product was prepared by substituting 2-methoxyphenylacetic acid for 3-(ethylsulfanyl)benzoic acid in Example 239B.
MS (ESI) m/e 356 (M+H)⁺;
¹H NMR (500 MHz, CD₃OD δ 7.21 (dt, 1H), 6.94 (d, 1H), 6.90 (d, 1H), 6.81 (m, 1H), 4.41 (d, 0.35), 4.37 (d, 0.65), 3.78 (s, 3H), 3.72 (m, 1H), 3.58 (d, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 264

### (2RS,3R)-3-amino-5-(ethylsulfanyl)-2-hydroxy-N'-((3-methoxyphenyl)acetyl)pentanohydrazide

The desired product was prepared by substituting 3-methoxyphenylacetic acid for 3-(ethylsulfanyl)benzoic acid in Example 239B.
MS (ESI) m/e 356 (M+H)⁺;
¹H NMR (500 MHz, CD₃OD) δ 7.25 (m, 2H), 6.95 (d, 1H), 6.90 (d, 1H), 4.41 (d, 0.35), 4.37 (d, 0.65), 3.83 (s, 3H), 3.72 (m, 1H), 3.61 (dd, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 265

### (2RS,3R)-3-amino-5-(ethylsulfanyl)-2-hydroxy-N'-((4-methoxyphenyl)acetyl)pentanohydrazide

The desired product was prepared by substituting 4-methoxyphenylacetic acid for 3-(ethylsulfanyl)benzoic acid in Example 239B.
MS (ESI) m/e 356 (M+H)⁺;
¹H NMR (500 MHz, CD₃OD) δ 7.25 (d, 2H), 6.87 (dd, 2H), 4.41 (d, 0.35), 4.37 (d, 0.65), 3.77 (s, 3H), 3.72 (m, 1H), 3.53 (d, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 266

### (2RS,3R)-3-amino-5-(ethylsulfanyl)-2-hydroxy-N'-((2-chlorophenyl)acetyl)pentanohydrazide

The desired product was prepared by substituting 2-chlorophenylacetic acid for 3-(ethylsulfanyl)benzoic acid in Example 239B.
MS (ESI) m/e 360 (M+H)⁺;
¹H NMR (500 MHz, CD₃OD) δ 7.43 (m, 2H), 7.27 (m, 2H), 4.42 (d, 0.35), 4.38 (d, 0.65), 3.80 (d, 1H), 3.78 (s, 1H), 3.73 (m, 1H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 267

### (2RS,3R)-3-amino-5-(ethylsulfanyl)-2-hydroxy-N'-((3-chlorophenyl)acetyl)pentanohydrazide

The desired product was prepared by substituting 3-chlorophenylacetic acid for 3-(ethylsulfanyl)benzoic acid in Example 239B.
MS (ESI) m/e 360 (M+H)⁺;
¹H NMR (500 MHz, CD₃OD) δ 7.39 (m, 1H), 7.25-7.31 (m, 3H), 4.42 (d, 0.35), 4.38 (d, 0.65), 3.72 (m, 1H), 3.61 (d, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 268

### (2RS,3R)-3-amino-5-(ethylsulfanyl)-2-hydroxy-N'-((4-chlorophenyl)acetyl)pentanohydrazide

The desired product was prepared by substituting 4-chlorophenylacetic acid for 3-(ethylsulfanyl)benzoic acid in Example 239B.
MS (ESI) m/e 360 (M+H)⁺;
¹H NMR (500 MHz, CD₃OD) δ 7.32 (m, 4H), 4.42 (d, 0.35), 4.37 (d, 0.65), 3.72 (m, 1H), 3.61 (d, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 269

### N'-((2RS,3R)-3-amino-5-(ethylsulfanyl)-2-hydroxypentanoyl)-2-(1,1'-biphenyl)-4-ylacetohydrazide

The desired product was prepared by substituting 4-phenylphenylacetic acid for 3-(ethylsulfanyl)benzoic acid in Example 239B.
MS (ESI) m/e 402 (M+H)⁺;
¹H NMR (500 MHz, CD₃OD) δ 7.58 (m, 4H), 7.42 (m, 4H), 7.32 (m, 1H), 4.42 (d, 0.35), 4.38 (d; 0.65), 3.72 (m, 1H), 3.65 (d, 2H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 270

### N'-((2RS,3R)-3-amino-5-(ethylsulfanyl)-2-hydroxypentanoyl)-2-(4-dimethylaminophenyl)acetohydrazide

The desired product was prepared by substituting 4-dimethylaminophenylacetic acid for 3-(ethylsulfanyl)benzoic acid in Example 239B..
MS (ESI) m/e 369 (M+H)⁺;
¹H NMR (500 MHz, CD₃OD) δ 7.36 (m, 2H), 7.13 (m, 2H), 4.42 (d, 0.35), 4.37 (d, 0.65), 3.72 (m, 1H), 3.59 (d, 2H), 3.09 (s, 3H), 3.07 (s, 3H), 2.67 (t, 2H), 2.58 (dd, 1.3H), 2.53 (dd, 0.7H), 2.11 (dd, 1H), 1.94 (dt, 1H), 1.25 (t, 1.95H), 1.21 (t, 1.05H).

### Example 271

### N'-((2RS,3R)-3-amino-5-(ethylsulfanyl)-2-hydroxypentanoyl)-2-(1-naphthyl)acetohydrazide

The desired product was prepared by substituting 1-naphthylacetic acid for 3-(ethylsulfanyl)benzoic acid in Example 239B.
MS (ESI) m/e 369 (M+H)⁺;
¹H NMR (500 MHz, CD₃OD) δ 8.10 (d, 1H), 7.88 (d, 1H), 7.81 (d, 1H), 7.51 (m, 3H) 7.45 (t, 1H), 4.42 (d, 0.35), 4.37 (d, 0.65), 4.10 (d, 2H), 3.70 (m, 1H), 2.65 (t, 2H), 2.56 (dd, 1.3H), 2.47 (dd, 0.7H), 2.09 (dd, 1H), 1.92 (dt, 1H), 1.24 (t, 1.95H), 1.15 (t, 1.05H).

### Example 272

### N'-((2RS,3R)-3-amino-5-(ethylsulfanyl)-2-hydroxypentanoyl)-2-(2-naphthyl)acetohydrazide

The desired product was prepared by substituting 2-naphthylacetic acid for 3-(ethylsulfanyl)benzoic acid in Example 239B.
MS (ESI) m/e 376 (M+H)⁺;
¹H NMR (500 MHz, CD₃OD) δ 7.82 (m, 4H), 7.46 (m, 3H), 4.42 (d, 0.35), 4.38 (d, 0.65), 3.78 (d, 2H), 3.73 (m, 1H), 2.66 (t, 2H), 2.57 (dd, 1.3H), 2.52 (dd, 0.7H), 2.09 (dd, 1H), 1.94 (dt, 1H), 1.25 (t, 1.95H), 1.19 (t, 1.05H).

### Example 273

### N'-((2RS,3R)-3-amino-5-(ethylsulfanyl)-2-hydroxypentanoyl)-2-furohydrazide

The desired product was prepared by substituting 2-furoic acid for 3-(ethylsulfanyl)benzoic acid in Example 239B.
MS (ESI) m/e 302 (M+H)⁺;
¹H NMR (500 MHz, CD₃OD) δ 7.73 (dd, 0.65H), 7.72 (dd, 0.35H), 7.24 (dd, 0.65H), 7.22 (dd, 0.35H), 6.64 (dd, 0.65H), 6.63 (dd, 0.35H), 4.47 (d, 0.35), 4.43 (d, 0.65), 3.77 (m, 1H), 2.70 (t, 2H), 2.60 (dd, 1.3H), 2.58 (dd, 0.7H), 2.15 (dd, 1H), 1.98 (dt, 1H), 1.27 (t, 1.95H), 1.25 (t, 1.05H).

### Example 274

### N'-((2RS,3R)-3-amino-5-(ethylsulfanyl)-2-hydroxypentanoyl)-3-furohydrazide

The desired product was prepared by substituting 3-furoic acid for 3-(ethylsulfanyl)benzoic acid in Example 239B.
MS (ESI) m/e 302 (M+H)⁺;
¹H NMR (500 MHz, CD₃OD) δ 8.16 (m, 0.65H), 8.14 (m, 0.35H), 7.62 (dd, 0.65H), 7.61 (dd, 0.35H), 6.83 (m, 1H), 4.47 (d, 0.35), 4.42 (d, 0.65), 3.76 (m, 1H), 2.70 (t, 2H), 2.60 (dd, 1.3H), 2.58 (dd, 0.7H), 2.15 (dd, 1H), 1.98 (dt, 1H), 1.27 (t, 1.95H), 1.25 (t, 1.05H).

### Example 275

### N'-((2RS,3R)-3-amino-5-(ethylsulfanyl)-2-hydroxypentanoyl)-2-thiophenecarbohydrazide

The desired product was prepared by substituting 2-thiophene carboxylic acid for 3-(ethylsulfanyl)benzoic acid in Example 239B.
MS (ESI) m/e 318 (M+H)⁺;
¹H NMR (500 MHz, CD₃OD) δ 7.79 (dd, 0.65H), 7.78 (dd, 0.35H), 7.77 (dd, 0.65H), 7.75 (dd, 0.35H), 7.17 (m, 1H), 4.47 (d, 0.35), 4.43 (d, 0.65), 3.76 (m, 1H), 2.70 (t, 2H), 2.60 (dd, 1.3H), 2.58 (dd, 0.7H), 2.16 (dd, 1H), 1.98 (dt, 1H), 1.27 (t, 1.95H), 1.25 (t, 1.05H).

### Example 276

### N'-((2RS,3R)-3-amino-5-(ethylsulfanyl)-2-hydroxypentanoyl)-3-thiophenecarbohydrazide

The desired product was prepared by substituting 3-thiophene carboxylic acid for 3-(ethylsulfanyl)benzoic acid in Example 239B.
MS (ESI) m/e 318 (M+H)⁺;
¹H NMR (500 MHz, CD₃OD) δ 8.18 (dd, 0.65H), 8.16 (dd, 0.35H), 7.53 (m, 2H), 4.48 (d, 0.35), 4.43 (d, 0.65), 3.77 (m, 1H), 2.71 (t, 2H), 2.60 (dd, 1.3H), 2.58 (dd, 0.7H), 2.16 (dd, 1H), 1.99 (dt, 1H), 1.27 (t, 1.95H), 1.25 (t, 1.05H).

### Example 277

### N'-((2RS,3R)-3-amino-5-(ethylsulfanyl)-2-hydroxypentanoyl)-1H-pyrrole-2-carbohydrazide

The desired product was prepared by substituting 2-pyrrole carboxylic acid for 3-(ethylsulfanyl)benzoic acid in Example 239B.
MS (ESI) m/e 301 (M+H)⁺;
¹H NMR (500 MHz, CD₃OD δ 6.99 (dd, 0.65H), 6.98 (dd, 0.35H), 6.91 (dd, 0.65H, 6.89 (dd, 0.35H), 6.21 (m, 1H), 4.46 (d, 0.35), 4.42 (d, 0.65), 3.77 (m, 1H), 2.71 (m, 2H), 2.60 (dd, 1.3H), 2.58 (dd, 0.7H), 2.15 (dd, 1H), 1.99 (dt, 1H), 1.27 (t, 1.95H), 1.25 (t, 1.05H).

### Example 278

### N'-((2RS,3R)-3-amino-5-(ethylsulfanyl)-2-hydroxypentanoyl)-1,3-thiazole-2-carbohydrazide

The desired product was prepared by substituting 2-thiazole carboxylic acid for 3-(ethylsulfanyl)benzoic acid in Example 239B.
MS (ESI) m/e 319 (M+H)⁺;
¹H NMR (500 MHz, CD₃OD) δ 8.02 (d, 0.65H), 8.00 (d, 0.35H), 7.94 (d, 0.65H, 7.91 (d, 0.35H), 4.49 (d, 0.35), 4.44 (d, 0.65), 3.79 (m, 1H), 2.71 (m, 2H), 2.60 (dd, 1.3H), 2.58 (dd, 0.7H), 2.16 (dd, 1H), 1.98 (dt, 1H), 1.27 (t, 1.95H), 1.25 (t, 1.05H).

### Example 279

### N'-((2RS,3R)-3-amino-5-(ethylsulfanyl)-2-hydroxypentanoyl)-1,3-thiazole-4-carbohydrazide

The desired product was prepared by substituting 4-thiazole carboxylic acid for 3-(ethylsulfanyl)benzoic acid in Example 239B.
MS (ESI) m/e 319 (M+H)⁺;
¹H NMR (500 MHz, CD₃OD) δ 9.06 (d, 0.65H), 9.04 (d, 0.35H), 8.40 (d, 0.65H, 8.37 (d, 0.35H), 4.48 (d, 0.35), 4.45 (d, 0.65), 3.79 (m, 1H), 2.71 (t, 2H), 2.60 (dd, 1.3H), 2.58 (dd, 0.7H), 2.16 (dd, 1H), 1.98 (dt, 1H), 1.27 (t, 1.95H), 1.25 (t, 1.05H).

### Example 280

### N'-((2RS,3R)-3-amino-5-(ethylsulfanyl)-2-hydroxypentanoyl)-1,3-thiazole-5-carbohydrazide

The desired product was prepared by substituting 5-thiazole carboxyl acid for 3-(ethylsulfanyl)benzoic acid in Example 239B.
MS (ESI) m/e 319 (M+H)⁺;
¹H NMR (500 MHz, CD₃OD) δ 9.21 (s, 0.65H), 9.19 (s, 0.35H), 8.49 (s, 0.65H, 8.47 (s, 0.35H), 4.49 (d, 0.35), 4.44 (d, 0.65); 3.78 (m, 1H), 2.70 (t, 2H), 2.60 (dd, 1.3H), 2.58 (dd, 0.7H), 2.16 (dd, 1H), 1.98 (dt, 1H), 1.27 (t, 1.95H), 1.25 (t, 1.05H).

### Example 281

### N'-((2RS,3R)-3-amino-5-(ethylsulfanyl)-2-hydroxypentanoyl)-1H-pyrazole-5-carbohydrazide

The desired product was prepared by substituting 1H-pyrazole-5-carboxylic acid for 3-(ethylsulfanyl)benzoic acid in Example 239B.
MS (ESI) m/e 302 (M+H)⁺;
¹H NMR (500 MHz, CD₃OD) δ 7.74 (d, 0.65H), 7.73 (d, 0.35H), 8.83 (m, 1H), 4.47 (d, 0.35), 4.43 (d, 0.65), 3.78 (m, 1H), 2.70 (t, 2H), 2.60 (dd, 1.3H), 2.58 (dd, 0.7H), 2.15 (dd, 1H), 1.98 (dt, 1H), 1.27 (t, 1.95H), 1.25 (t, 1.05H).

### Example 282

### N'-((2RS,3R)-3-amino-5-(ethylsulfanyl)-2-hydroxypentanoyl)-1H-pyrazole-4-carbohydrazide

The desired product was prepared by substituting 1-H-pyrazole-4-carboxylic acid for 3-(ethylsulfanyl)benzoic acid in Example 239B.
MS (ESI) m/e 302 (M+H)⁺;
¹H NMR (500 MHz, CD₃OD) δ 8.11 (s, 1H), 8.10 (s, 1H), 4.47 (d, 0.35), 4.43 (d, 0.65), 3.76 (m, 1H), 2.70 (t, 2H), 2.60 (dd, 1.3H), 2.58 (dd, 0.7H), 2.15 (dd, 1H), 1.99 (dt, 1H), 1.27 (t, 1.95H), 1.25 (t, 1.05H).

### Example 283

### N'-((2RS,3R)-3-amino-5-(ethylsulfanyl)-2-hydroxypentanoyl)-5-isoxazolecarbohydrazide

The desired product was prepared by substituting 5-isoxazole caboxylic acid for 3-(ethylsulfanyl)benzoic acid in Example 239B.
MS (ESI) m/e 303 (M+H)⁺;
¹H NMR (500 MHz, CD₃OD) δ 8.58 (d, 0.65H), 8.56 (d, 0.35H), 7.09 (d, 0.65H), 7.06 (d, 0.35H), 4.49 (d, 0.35), 4.44 (d, 0.65), 3.78 (m, 1H), 2.70 (t, 2H), 2.60 (dd, 1.3H), 2.58 (dd, 0.7H), 2.16 (dd, 1H), 1.97 (dt, 1H), 1.27 (t, 1.95H), 1.25 (t, 1.05H).

### Example 284

### N'-((2RS,3R)-3-amino-5-(ethylsulfanyl)-2-hydroxypentanoyl)-2-pyridinecarbohydrazide

The desired product was prepared by substituting 2-pyridine caboxylic acid for 3-(ethylsulfanyl)benzoic acid in Example 239B.
MS (ESI) m/e 313 (M+H)⁺;
¹H NMR (500 MHz, CD₃OD) δ 8.67 (t, 1H), 8.11 (t, 1H), 7.99 (m, 1H), 7.60 (m, 1H), 4.49 (d, 0.35), 4.45 (d, 0.65), 3.78 (m, 1H), 2.71 (t, 2H), 2.61 (dd, 1.3H), 2.58 (dd, 0.7H), 2.16 (dd, 1H), 1.99 (dt, 1H), 1.28 (t, 1.95H), 1.26 (t, 1.05H).

### Example 285

### N'-((2RS,3R)-3-amino-5-(ethylsulfanyl)-2-hydroxypentanoyl)-2-(2-pyridinyl)acetohydrazide

The desired product was prepared by substituting 2-pyridylacetic acid for 3-(ethylsulfanyl)benzoic acid in Example 239B.
MS (ESI) m/e 327 (M+H)⁺;
¹H NMR (500 MHz, CD₃OD) δ 8.63 (d, 0.35H), 8.59 (d, 0.65H), 8.11 (dt, 0.35H), 8.04 (dt, 0.65H), 7.71 (d, 0.35H), 7.66 (d, 0.65H), 7.59 (ddd, 0.35H), 7.53 (ddd, 0.65H), 4.44 (d, 0.35), 4.38 (d, 0.65), 3.73 (m, 1H), 2.67 (t, 2H), 2.58 (dd, 1.3H), 2.52 (dd, 0.7H), 2.10 (dd, 1H), 1.93 (dt, 1H), 1.25 (t, 1.95H), 1.20 (t, 1.05H).

### Example 286

### N'-((2RS,3R)-3-amino-5-(ethylsulfanyl)-2-hydroxypentanoyl)-3-pyridinecarbohydrazide

The desired product was prepared by substituting 3-pyridylacetic acid for 3-(ethylsulfanyl)benzoic acid in Example 239B.
MS (ESI) m/e 313 (M+H)⁺;
¹H NMR (500 MHz, CD₃OD) δ 9:04 (m, 1H), 8.77 (d, 1H), 8.32 (dd, 0.35H), 8.30 (dd, 0.65H), 7.61 (d, 0.35H), 7.60 (d, 0.65H), 4.51 (d, 0.35), 4.46 (d, 0.65), 3.79 (m, 1H), 2.71 (t, 2H), 2.61 (dd, 1.3H), 2.58 (dd, 0.7H), 2.17 (dd, 1H), 2.00 (dt, 1H), 1.28 (t, 1.95H), 1.25 (t, 1.05H).

### Example 287

### N'-((2RS,3R)-3-amino-5-(ethylsulfanyl)-2-hydroxypentanoyl)-2-(3-pyridinyl)acetohydrazide

The desired product was prepared by substituting 3-pyridylacetic acid for 3-(ethylsulfanyl)benzoic acid in Example 239B.
MS (ESI) m/e 327 (M+H)⁺;
¹H NMR (500 MHz, CD₃OD) δ 8.69 (d, 1H), 8.61 (m, 1H), 8.24 (d, 0.35H), 8.19 (dd, 0.65H), 7.75 (dd, 0.35H), 7.71 (dd, 0.65H), 4.44 (d, 0.35), 4.38 (d, 0.65), 3.81 (m, 2H), 3.73 (m, 1H), 2.67 (t, 2H), 2.57 (dd, 1.3H), 2.52 (dd, 0.7H), 2.10 (dd, 1H), 1.93 (dt, 1H), 1.25 (t, 1.95H), 1.20 (t, 1.05H).

### Example 288

### N'-((2RS,3R)-3-amino-5-(ethylsulfanyl)-2-hydroxypentanoyl)-4-pyridinecarbohydrazide

The desired product was prepared by substituting 4-pyridine caboxylic acid for 3-(ethylsulfanyl)benzoic acid in Example 239B.
MS (ESI) m/e 313 (M+H)⁺;
¹H NMR (500 MHz, CD₃OD) δ 8.76 (m, 2H), 7.86 (m, 2H), 4.51 (d, 0.35), 4.45 (d, 0.65), 3.79 (m, 1H), 2.71 (t, 2H), 2.60 (dd, 1.3H), 2.58 (dd, 0.7H), 2.17 (dd, 1H), 2.00 (dt, 1H), 1.27 (t, 1.95H), 1.25 (t, 1.05H).

### Example 289

### N'-((2RS,3R)-3-amino-5-(ethylsulfanyl)-2-hydroxypentanoyl)-2-(4-pyridinyl)acetohydrazide

The desired product was prepared by substituting 4-pyridylacetic acid for 3-(ethylsulfanyl)benzoic acid in Example 239B.
MS (ESI) m/e 327 (M+H)⁺;
¹H NMR (500 MHz, CD₃OD δ 8.68 (t, 2H), 7.87 (d, 0.7H), 7.83 (d, 1.3H), 4.45 (d, 0.35), 4.38 (d, 0.65), 3.73 (m, 1H), 2.67 (t, 2H), 2.58 (dd, 1.3H), 2.52 (dd, 0.7H), 2.10 (dd, 1H), 1.93 (dt, 1H), 1.25 (t, 1.95H), 1.20 (t, 1.05H).

### Example 290

### N'-((2RS,3R)-3-amino-5-(ethylsulfanyl)-2-hydroxypentanoyl)-3-pyridazinecarbohydrazide

The desired product was prepared by substituting 3-pyridazine carboxylic acid for 3-(ethylsulfanyl)benzoic acid in Example 239B.
MS (ESI) m/e 314 (M+H)⁺;
¹H NMR (500 MHz, CD₃OD) δ 9.37 (ddd, 1H), 8.31 (ddd; 1H), 7.92 (dd, 1H), 4.51 (d, 0.35), 4.47 (d, 0.65), 3.81 (m, 1H), 2.72 (t, 2H), 2.61 (dd, 2H), 2.18 (dd, 1H), 2.00 (dt, 1H), 1.28 (t, 1.95H), 1.26 (t, 1.05H).

### Example 291

### N'-((2RS,3R)-3-amino-5-(ethylsulfanyl)-2-hydroxypentanoyl)-4-pyrimidinecarbohydrazide

The desired product was prepared by substituting 4-pyrimidine carboxylic acid for 3-(ethylsulfanyl)benzoic acid in Example 239B.
MS (ESI) m/e 314 (M+H)⁺;
¹H NMR (500 MHz, CD₃OD) δ 9.31 (d, 0.65H), 9.30 (d, 0.35H), 9.07 (d, 0.65H), 9.06 (0.35H), 8.11 (dd, 0.65H), 8.09 (dd, 0.35H), 4.50 (d, 0.35), 4.45 (d, 0.65), 3.79 (m, 1H), 2.71 (t, 2H), 2.61 (dd, 2H), 2.17 (dd, 1H), 1.99 (dt, 1H), 1.27 (t, 1.95H), 1.26 (t, 1.05H).

### Example 292

### N'-((2RS,3R)-3-amino-5-(ethylsulfanyl)-2-hydroxypentanoyl)-2-pyrazinecarbohydrazide

The desired product was prepared by substituting 2-pyrazine carboxylic acid for 3-(ethylsulfanyl)benzoic acid in Example 239B.
MS (ESI) m/e 314 (M+H)⁺;
¹H NMR (500 MHz, CD₃OD) δ 9.25 (d, 0.65H), 9.24 (d, 0.35H), 8.85 (d, 0.65H), 8.83 (0.35H), 8.72 (dd, 0.65H), 8.71 (dd, 0.35H), 4.50 (d, 0.35), 4.46 (d, 0.65), 3.80 (m, 1H), 2.71 (t, 2H), 2.60 (t, 2H), 2.18 (dd, 1H), 1.99 (dt, 1H), 1.28 (t, 1.95H), 1.26 (t, 1.05H).

### Example 293

### N'-((2RS,3R)-3-amino-5-(ethylsulfanyl)-2-hydroxypentanoyl)4-isopropylbenzohydrazide

The desired product was prepared by substituting 4-(2-methylethyl)phenylbenzoic acid for 3-(ethylsulfanyl)benzoic acid in Example 239B.
MS (ESI) m/e 355 (M+H)⁺;
¹H NMR (500 MHz, CD₃OD) δ 7.82 (m, 2H), 7.37 (m, 2H), 4.48 (d, 0.35), 4.44 (d, 0.65), 3.78 (m, 1H), 2.71 (t, 2H), 2.60 (dd, 2H), 2.16 (dd, 1H), 1.99 (dt, 1H), 1.24-1.29 (m, 10H)

### Example 294

### N'-((2RS,3R)-3-amino-5-(ethylsulfanyl)-2-hydroxypentanoyl)-4-propoxybenzohydrazide

The desired product was prepared by substituting 4-propoxybenzoic acid for 3-(ethylsulfanyl)benzoic acid in Example 239B.
MS (ESI) m/e 370 (M+H)⁺;
¹H NMR (500 MHz, CD₃OD) δ 7.86 (m, 2H), 7.01 (m, 2H), 4.48 (d, 0.35), 4.44 (d, 0.65), 4.01 (dt, 2H), 3.77 (m, 1H), 2.71 (t, 2H), 2.60 (dd, 2H), 2.16 (dd, 1H), 1.99 (dt, 1H), 1.82 (ddd, 2H), 1.28 (t, 1.95H), 1.25 (t, 1.05H) 1.05 (t, 3H).

### Example 295

### N'-((2RS,3R)-3-amino-5-(ethylsulfanyl)-2-hydroxypentanoyl)-4-(methylsulfanyl)benzohydrazide

The desired product was prepared by substituting 4-methylsulfanylbenzoic acid for 3-(ethylsulfanyl)benzoic acid in Example 239B.
MS (ESI) m/e 358 (M+H)⁺;
¹H NMR (500 MHz, CD₃OD) δ 7.81 (m, 2H), 7.35(m, 2H), 4.48 (d, 0.35), 4.44 (d, 0.65), 3.78 (m, 1H), 2.71 (t, 2H), 2.61 (dd, 2H), 2.53 (s, 3H), 2.16 (dd, 1H), 1.99 (dt, 1H), 1.28 (t, 1.95H), 1.25 (t, 1.05H).

### Example 296

### N'-((2RS,3R)-3-amino-5-(ethylsulfanyl)-2-hydroxypentanoyl)-4-isopropoxybenzohydrazide

The desired product was prepared by substituting 4-(2-methylethoxy)benzoic acid for 3-(ethylsulfanyl)benzoic acid in Example 239B.
MS (ESI) m/e 370 (M+H)⁺;
¹H NMR (500 MHz, CD₃OD) δ 7.84 (m, 2H), 6.99 (m, 2H), 4.71 (ddd, 1H), 4.48 (d, 0.35), 4.44 (d, 0.65), 3.78 (m, 1H), 2.71 (t, 2H), 2.61 (dd, 2H), 2.16 (dd, 1H), 2.00 (dt, 1H), 1.34 (d, 6H), 1.28 (t, 1.95H), 1.25 (t, 1.05H).

### Example 297

### N'-((2RS,3R)-3-amino-5-(ethylsulfanyl)-2-hydroxypentanoyl)-4-(diethylamino)benzohydrazide

The desired product was prepared by substituting 4-(diethylamino)benzoic acid for 3-(ethylsulfanyl)benzoic acid in Example 239B.
MS (ESI) m/e 383 (M+H)⁺;
¹H NMR (500 MHz, CD₃OD) δ 7.76 (dd, 2H), 6.73 (m, 2H), 4.47 (d, 0.35), 4.43 (d, 0.65), 3.77 (m, 1H), 3.46 (q, 4H), 2.70 (t, 2H), 2.61 (dd, 2H), 2.16 (dd, 1H), 2.00 (dt, 1H), 1.28 (t, 1.95H), 1.26 (t, 1.05H), 1.19 (t, 6H).

### Example 298

### N'-((2RS,3R)-3-amino-5-(ethylsulfanyl)-2-hydroxypentanoyl)-4-butoxybenzohydrazide

The desired product was prepared by substituting 4-butoxybenzoic acid for 3-(ethylsulfanyl)benzoic acid in Example 239B.
MS (ESI) m/e 384 (M+H)⁺;
¹H NMR-(500 MHz, CD₃OD) δ 7.85 (m, 2H), 6.99 (m, 2H), 4.48 (d, 0.35), 4.44 (d, 0.65), 4.06 (dt, 2H), 3.77 (m, 1H), 2.71 (t, 2H), 2.61 (dd, 2H), 2.16 (dd, 1H), 2.00 (dt, 1H), 1.78 (ddd, 2H), 1.53 (ddd, 2H), 1.28 (t, 1.95H), 1.26 (t, 1.05H), 0.99 (t, 3H).

### Example 299

### N'-((2RS,3R)-3-amino-5-(ethylsulfanyl)-2-hydroxypentanoyl)-3,4-diethoxybenzohydrazide

The desired product was prepared by substituting 2,3-diethoxybenzoic acid for 3-(ethylsulfanyl)benzoic acid in Example 239B.
MS (ESI) m/e 400 (M+H)⁺;
¹H NMR (500 MHz, CD₃OD) δ 7.52 (dd, 1H), 7.48 (d, 1H), 7.03 (m, 1H), 4.48 (d, 0.35), 4.44 (d, 0.65), 4.13 (m, 4H), 3.77 (m, 1H), 2.71 (t, 2H), 2.61 (dd, 2H), 2.16 (dd, 1H), 2.00 (dt, 1H), 1.43 (m, 6H), 1.28 (t, 1.95H), 1.25 (t, 1.05H).

### Example 300

### N'-((2RS,3R)-3-amino-5-(ethylsulfanyl)-2-hydroxypentanoyl)-4-chlorobenzohydrazide

The desired product was prepared by substituting 4-chlorobenzoic acid for 3-(ethylsulfanyl)benzoic acid in Example 239B.
MS (ESI) m/e 346 (M+H)⁺;
¹H NMR (500 MHz, CD₃OD) δ 7.87 (dd, 2H), 7.52 (dd, 2H), 4.49 (d, 0.35), 4.44 (d, 0.65), 3.78 (m, 1H), 2.71 (t, 2H), 2.61 (dd, 2H), 2.17 (dd, 1H), 1.99 (dt, 1H), 1.27 (t, 1.95H), 1.25 (t, 1.05H).

### Example 301

### N'-((2RS,3R)-3-amino-5-(ethylsulfanyl)-2-hydroxypentanoyl)-2-bromobenzohydrazide

The desired product was prepared by substituting 2-bromobenzoic acid for 3-(ethylsulfanyl)benzoic acid in Example 239B.
MS (ESI) m/e 391 (M+H)⁺;
¹H NMR (500 MHz, CD₃OD) δ 7.69 (dd, 1H), 7.59 (ddd, 1H), 7.47 (m, 1H), 7.41 (m, 1H), 4.48 (d, 0.35), 4.43 (d, 0.65), 3.79 (m, 1H), 2.71 (t, 2H), 2.61 (dd, 2H), 2.16 (dd, 1H), 1.99 (dt, 1H), 1.27 (t, 1.95H), 1.24 (t, 1.05H).

### Example 302

### (2RS,3R)-3-amino-N'-((2RS,3S)-3-amino-2-hydroxy-5-(isopropylsulfanyl)pentanoyl)-2-hydroxy-5-(isopropylsulfanyl)pentanohydrazide

The title compound is obtained by substituting hydrazine hydrate for O-phenylhydroxylamine hydrochloride and Example 123B for Example 98A in Example 98B.
MS (ESI) m/e 409 (M-H)⁺.
¹H NMR (300 MHz, DMSO-d₆) δ 10.94 (br s, 1H), 10.11 & 9.93 (s, 1H), 8.2 & 8.02 (m, 4H), 6.8 & 6.65(br s, 2H), 4.44 & 4.28 (m, 1H), 4.2 (m, 1H), 3.0-2.9 (m, 2H), 2.75-2.55 (m, 4H), 1.95-1.75 (m, 4H), 1.2 (d, 12H).

### Example 304

### N'-((2RS,3R)-3-amino-5-(ethylsulfanyl)-2-hydroxypentanoyl)-3-(dimethylamino)benzohydrazide

The desired product was prepared by substituting 3-dimethylaminobenzoic acid for 3-(ethylsulfanyl)benzoic acid in Example 239B.
MS (ESI) m/e 355 (M+H)⁺;
¹H NMR (500 MHz, CD₃OD) δ 7.32 (dd, 1H), 7.28 (m, 1H), 7.21 (d, 1H), 7.01 (dd, 1H), 4.48 (d, 0.35), 4.44 (d, 0.65), 3.79 (m, 1H), 3.00 (s, 6H), 2.71 (t, 2H), 2.61 (dd, 2H), 2.17 (dd, 1H), 2.00 (dt, 1H), 1.28 (t, 1.95H), 1.26 (t, 1.05H).

### Example 305

### N'-((2RS,3R)-3-amino-5-(ethylsulfanyl)-2-hydroxypentanoyl)-4-(dimethylamino)benzohydrazide

The desired product was prepared by substituting 4-dimethylaminobenzoic acid for 3-(ethylsulfanyl)benzoic acid in Example 239B.
MS (ESI) m/e 355 (M+H)⁺;
¹H NMR (500 MHz, CD₃OD) δ 7.78 (dd, 2H), 6.75 (d, 2H), 4.46 (d, 0.35), 4.43 (d, 0.65), 3.77 (m, 1H), 3.04 (s, 6H), 2.70 (t, 2H), 2.61 (dd, 2H), 2.16 (dd, 1H), 2.00 (dt, 1H), 1.28 (t, 1.95H), 1.26 (t, 1.05H).

### Example 306

### N'-((2RS,3R)-3-amino-5-(ethylsulfanyl)-2-hydroxypentanoyl)-3-(trifluoromethyl)benzohydrazide

The desired product was prepared by substituting 3-(trifluoromethyl)benzoic acid for 3-(ethylsulfanyl)benzoic acid in Example 239B.
MS (ESI) m/e 380 (M+H)⁺;
¹H NMR (500 MHz, CD₃OD) δ 8.21 (d, 1H), 8.14 (t, 1H), 7.92 (t, 1H), 7.73 (dd, 1H), 4.50 (d, 0.35), 4.46 (d, 0.65), 3.79 (m, 1H), 2.72 (t, 2H), 2.61 (dd, 2H), 2.18 (dd, 1H), 2.00 (dt, 1H), 1.28 (t, 1.95H), 1.26 (t, 1.05H).

### Example 307

### N'-((2RS,3R)-3-amino-5-(ethylsulfanyl)-2-hydroxypentanoyl)-4-(trifluoromethyl)benzohydrazide

The desired product was prepared by substituting 4-(trifluoromethyl)benzoic acid for 3-(ethylsulfanyl)benzoic acid in Example 239B.
MS (ESI) m/e 380 (M+H)⁺;
¹H NMR (500 MHz, CD₃OD) δ 8.05 (dd, 2H), 7.82 (dd, 2H), 4.50 (d, 0.35), 4.45 (d, 0.65), 3.79 (m, 1H), 2.71 (t, 2H), 2.61 (dd, 2H), 2.17 (dd, 1H), 2.00 (dt, 1H), 1.28 (t, 1.95H), 1.25 (t, 1.05H).

### Example 308

### N'-((2RS,3R)-3-amino-5-(ethylsulfanyl)-2-hydroxypentanoyl)-3-(trifluoromethoxy)benzohydrazide

The desired product was prepared by substituting 3-(trifluoromethoxy)benzoic acid for 3-(ethylsulfanyl)benzoic acid in Example 239B.
MS (ESI) m/e 396 (M+H)⁺;
¹H NMR (500 MHz, CD₃OD) δ 7.89 (dd, 1H), 7.80 (d, 1H), 7.62 (dd, 1H), 7.53 (dd, 1H), 4.50 (d, 0.35), 4.45 (d, 0.65), 3.78 (m, 1H), 2.71 (t, 2H), 2.61 (dd, 2H), 2.17 (dd, 1H), 2.00 (dt, 1H), 1.28 (t, 1.95H), 1.25 (t, 1.05H).

### Example 309

### N'-((2RS,3R)-3-amino-5-(ethylsulfanyl)-2-hydroxyuentanoyl)-4-phenoxybenzohydrazide

The desired product was prepared by substituting 4-phenoxybenzoic acid for 3-(ethylsulfanyl)benzoic acid in Example 239B.
MS (ESI) m/e 404 (M+H)⁺;
¹H NMR (500 MHz, CD₃OD) δ 7.89 (m, 2H), 7.42 (m, 2H), 7.22 (m, 1H), 7.08 (m, 2H), 7.04 (m, 2H), 4.48 (d, 0.35), 4.44 (d, 0.65), 3.78 (m, 1H), 2.71 (t, 2H), 2.61 (dd, 2H), 2.16 (dd, 1H), 2.00 (dt, 1H), 1.27 (t, 1.95H), 1.26 (t, 1.05H).

### Example 310

### N'-((2RS,3R)-3-amino-5-(ethylsulfanyl)-2-hydroxypentanoyl)-4-(phenoxymethyl)benzohydrazide

The desired product was prepared by substituting 4-(phenoxymethyl)benzoic acid for 3-(ethylsulfanyl)benzoic acid in Example 239B.
MS (ESI) m/e 418 (M+H)⁺;
¹H NMR (500 MHz, CD₃OD) δ 7.87 (m, 2H), 7.44 (d, 2H), 7.38 (t, 2H), 7.32 (t, 1H), 7.11 (m, 2H), 5.17 (s, 2H), 4.48 (d, 0.35), 4.44 (d, 0.65), 3.77 (m, 1H), 2.71 (t, 2H), 2.61 (dd, 2H), 2.16 (dd, 1H), 2.00 (dt, 1H), 1.27 (t, 1.95H), 1.25 (t, 1.05H).

### Example 311

### N'-((2RS,3R)-3-amino-5-(ethylsulfanyl)-2-hydroxypentanoyl)-2,3-dimethylbenzohydrazide

The desired product was prepared by substituting 2,3-dimethylbenzoic acid for 3-(ethylsulfanyl)benzoic acid in Example 239B.
MS (ESI) m/e 340 (M+H)⁺;
¹H NMR (500 MHz, CD₃OD) δ 7.29 (m, 2H), 7.16 (m, 1H), 4.48 (d, 0.35), 4.43 (d, 0.65), 3.80 (m, 1H), 2.71 (t, 2H), 2.61 (dd, 2H), 2.36 (s, 3H), 2.32 (s, 3H), 2.17 (dd, 1H), 1.99 (dt, 1H), 1.28 (t, 1.95H), 1.24 (t, 1.05H).

### Example 312

### N'-((2RS,3R)-3-amino-5-(ethylsulfanyl)-2-hydroxypentanoyl)-2,4-dimethylbenzohydrazide

The desired product was prepared by substituting 2,4-dimethylbenzoic acid for 3-(ethylsulfanyl)benzoic acid in Example 239B.
MS (ESI) m/e 340 (M+H)⁺;
¹H NMR (500 MHz, CD₃OD) δ 7.40 (m, 1H), 7.12 (d, 1H), 7.08 (dd, 1H), 4.48 (d, 0.35), 4.43 (d, 0.65), 3.79 (m, 1H), 2.71 (t, 2H), 2.61 (dd, 2H), 2.43 (s, 3H), 2.34 (s, 3H), 2.16 (dd, 1H), 1.99 (dt, 1H), 1.27 (t, 1.95H), 1.24 (t, 1.05H).

### Example 313

### N'-((2RS,3R)-3-amino-5-(ethylsulfanyl)-2-hydroxypentanoyl)-2,5-dimethylbenzohydrazide

The desired product was prepared by substituting 2,5-dimethylbenzoic acid for 3-(ethylsulfanyl)benzoic acid in Example 239B.
MS (ESI) m/e 340 (M+H)⁺;
¹H NMR (500 MHz, CD₃OD) δ 7.33 (m, 1H), 7.21 (dd, 1H), 7.17 (m, 1H), 4.48 (d, 0.35), 4.43 (d, 0.65), 3.79 (m, 1H), 2.71 (t, 2H), 2.61 (dd, 2H), 2.41 (s, 3H), 2.34 (s, 3H), 2.16 (dd, 1H), 1.99 (dt, 1H), 1.27 (t, 1.95H), 1.24 (t, 1.05H).

### Example 314

### N'-((2RS,3R)-3-amino-5-(ethylsulfanyl)-2-hydroxypentanoyl)-4,5-dimethylbenzohydrazide

The desired product was prepared by substituting 3,4-dimethylbenzoic acid for 3-(ethylsulfanyl)benzoic acid in Example 239B.
MS (ESI) m/e 340 (M+H)⁺;
¹H NMR (500 MHz, CD₃OD) δ 7.68 (d, 1H), 7.61 (dd, 1H), 7.25 (m, 1H), 4.48 (d, 0.35), 4.44 (d, 0.65), 3.78 (m, 1H), 2.71 (t, 2H), 2.61 (dd, 2H), 2.33 (s, 6H), 2.16 (dd, 1H), 2.00 (dt, 1H), 1.28 (t, 1.95H), 1.25 (t, 1.05H).

### Example 315

### N'-((2RS,3R)-3-amino-5-(ethylsulfanyl)-2-hydroxypentanoyl)-3,5-dimethylbenzohydrazide

The desired product was prepared by substituting 3,5-dimethylbenzoic acid for 3-(ethylsulfanyl)benzoic acid in Example 239B.
MS (ESI) m/e 340 (M+H)⁺;
¹H NMR (500 MHz, CD₃OD) δ 7.50 (d, 2H), 7.25 (d, 1H), 4.48 (d, 0.35), 4.44 (d, 0.65), 3.78 (m, 1H), 2.71 (t, 2H), 2.61 (dd, 2H), 2.37 (s, 6H), 2.16 (dd, 1H), 2.00 (dt, 1H), 1.28 (t, 1.95H), 1.26 (t, 1.0511).

### Example 316

### N'-((2RS,3R)-3-amino-5-(ethylsulfanyl)-2-hydroxypentanoyl)-2,3-dimethoxybenzohydrazide

The desired product was prepared by substituting 2,3-dimethoxybenzoic acid for 3-(ethylsulfanyl)benzoic acid in Example 239B.
MS (ESI) m/e 372 (M+H)⁺;
¹H NMR (500 MHz, CD₃OD) δ 7.42 (dd, 0.65H), 7.40 (dd, 0.35H), 7.24 (ddd, 1H), 7.19 (m, 1H), 4.48 (d, 0.35), 4.44 (d, 0.65), 3.95 (s, 1.95H), 3.94 (s, 1.05H), 3.91 (s, 1.95H), 3.90 (s, 1.05H), 3.79 (m, 1H), 2.71 (t, 2H), 2.61 (dd, 2H), 2.16 (dd, 1H), 1.99 (dt, 1H), 1.27 (t, 1.95H), 1.25 (t, 1.05H).

### Example 317

### N'-((2RS,3R)-3-amino-5-(ethylsulfanyl)-2-hydroxypentanoyl)-2,4-dimethoxybenzohydrazide

The desired product was prepared by substituting 2,4-dimethoxybenzoic acid for 3-(ethylsulfanyl)benzoic acid in Example 239B.
MS (ESI) m/e 372 (M+H)⁺;
¹H NMR (500 MHz, CD₃OD) δ 7.98 (m, 1H), 6.68 (m, 2H), 4.47 (d, 0.35), 4.44 (d, 0.65), 4.00 (s, 1.95H), 3.99 (s, 1.05H), 3.88 (s, 3H), 3.78 (m, 1H), 2.70 (t, 2H), 2.61 (dd, 2H), 2.16 (dd, 1H), 1.99 (dt, 1H), 1.28 (t, 1.95H), 1.25 (t, 1.05H).

### Example 318

### N'-((2RS,3R)-3-amino-5-(ethylsulfanyl)-27-hydroxypentanoyl)-2,5-dimethoxybenzohydrazide

The desired product was prepared by substituting 2,5-dimethoxybenzoic acid for 3-(ethylsulfanyl)benzoic acid in Example 239B.
MS (ESI) m/e 372 (M+H)⁺;
¹H NMR (500 MHz, CD₃OD) δ 7.54 (dd, 0.65H), 7.53 (dd, 0.35H), 7.14 (m, 2H), 4.48 (d, 0.35), 4.45 (d, 0.65), 3.96 (s, 1.95H), 3.95 (s, 1.05H), 3.79 (s, 3H), 3.78 (m, 1H), 2.71 (t, 2H), 2.61 (dd, 2H), 2.16 (dd, 1H), 1.99 (dt, 1H), 1.28 (t, 1.95H), 1.25 (t, 1.05H).

### Example 319

### N'-((2RS,3R)-3-amino-5-(ethylsulfanyl)-2-hydroxypentanoyl)-3,4-dimethoxybenzohydrazide

The desired product was prepared by substituting 3,4-dimethoxybenzoic acid for 3-(ethylsulfanyl)benzoic acid in Example 239B.
MS (ESI) m/e 372 (M+H)⁺;
¹H NMR (500 MHz, CD₃OD) δ 7.55 (ddd, 1H), 7.49 (dd, 1H), 7.05 (m, 1H), 4.48 (d, 0.35), 4.44 (d, 0.65), 3.90 (s, 1.95H), 3.89 (s, 1.05H), 3.88 (s, 3H), 3.78 (m, 1H), 2.71 (t, 2H), 2.61 (dd, 2H), 2.17 (dd, 1H), 2.00 (dt, 1H), 1.28 (t, 1.95H), 1.26 (t, 1.05H).

### Example 320

### N'-((2RS,3R)-3-amino-5-(ethylsulfanyl)-2-hydroxypentanoyl)-3,5-dimethoxybenzohydrazide

The desired product was prepared by substituting 3,5-dimethoxybenzoic acid for 3-(ethylsulfanyl)benzoic acid in Example 239B.
MS (ESI) m/e 372 (M+H)⁺;
¹H NMR (500 MHz, CD₃OD) δ 7.04 (m, 2H), 6.71 (dd, 0.65H), 6.99 (dd, 0.35H), 4.48 (d, 0.35), 4.44 (d, 0.65), 3.83 (s, 6H), 3.78 (m, 1H), 2.71 (t, 2H), 2.61 (dd, 2H), 2.17 (dd, 1H), 1.99 (dt, 1H), 1.28 (t, 1.95H), 1.25 (t, 1.05H).

### Example 321

### N'-((2RS,3R)-3-amino-5-(ethylsulfanyl)-2-hydroxypentanoyl)-1,3-benzodioxole-5-carbohydrazide

The desired product was prepared by substituting 1,3-benzodioxole carboxylic acid for 3-(ethylsulfanyl)benzoic acid in Example 239B.
MS (ESI) m/e 356 (M+H)⁺;
¹H NMR (500 MHz, CD₃OD) δ 7.49 (m, 1H), 7.35 (d, 0.65H), 7.34 (d, 0.35H), 6.92 (m, 1H), 6.06 (s, 1.3H), 6.05 (s, 0.7H), 4.47 (d, 0.35), 4.43 (d, 0.65), 3.77 (m, 1H), 2.70 (t, 2H), 2.60 (dd, 2H), 2.16 (dd, 1H), 1.99 (dt, 1H), 1.27 (t, 1.95H), 1.25 (t, 1.05H).

### Example 322

### N'-((2RS,3R)-3-amino-5-(ethylsulfanyl)-2-hydroxypentanoyl)-3,4,5-trimethoxybenzohydrazide

The desired product was prepared by substituting 3,4,5-trimethoxybenzoic acid for 3-(ethylsulfanyl)benzoic acid in Example 239B.
MS (ESI) m/e 402 (M+H)⁺;
¹H NMR (500 MHz, CD₃OD) δ 7.25 (s, 1.3H), 7.24 (s, 0.7H), 4.50 (d, 0.35), 4.45 (d, 0.65), 3.89 (s, 6H), 3.83 (s, 1.95H), 3.82 (s, 1.05H), 3.78 (m, 1H), 2.71 (t, 2H), 2.61 (dd, 2H), 2.17 (dd, 1H), 2.00 (dt, 1H), 1.28 (t, 1.95H), 1.26 (t, 1.05H).

### Example 323

### N'-((2RS,3R)-3-amino-5-(ethylsulfanyl)-2-hydroxypentanoyl)-2,3-dichlorobenzohydrazide

The desired product was prepared by substituting 2,3-dichlorobenzoic acid for 3-(ethylsulfanyl)benzoic acid in Example 239B.
MS (ESI) m/e 381 (M+H)⁺;
¹H NMR (500 MHz, CD₃OD) δ 7.68 (ddd, 1H), 7.54 (m, 1H), 7.41 m, 1H), 4.48 (d, 0.35), 4.43 (d, 0.65), 3.80 (m, 1H), 2.71 (t, 2H), 2.61 (dd, 2H), 2.17 (dd, 1H), 1.99 (dt, 1H), 1.27 (t, 1.95H), 1.24 (t, 1.05H).

### Example 324

### N'-((2RS,3R)-3-amino-5-(ethylsulfanyl)-2-hydroxypentanoyl)-2,4-dichlorobenzohydrazide

The desired product was prepared by substituting 2,4-dichlorobenzoic acid for 3-(ethylsulfanyl)benzoic acid in Example 239B.
MS (ESI) m/e 381 (M+H)⁺;
¹H NMR (500 MHz, CD₃OD) δ 7.60 (m, 2H), 7.46 (m, 1H), 4.48 (d, 0.35), 4.43 (d, 0.65), 3.79 (m, 1H), 2.71 (t, 2H), 2.60 (dd, 2H), 2.16 (dd, 1H), 1.99 (dt, 1H), 1.27 (t, 1.95H), 1.24 (t, 1.05H).

### Example 325

### N'-((2RS,3R)-3-amino-5-(ethylsulfanyl)-2-hydroxypentanoyl)-2,5-dichlorobenzohydrazide

The desired product was prepared by substituting 2,5-dichlorobenzoic acid for 3-(ethylsulfanyl)benzoic acid in Example 239B.
MS (ESI) m/e 381 (M+H)⁺;
¹H NMR (500 MHz, CD₃OD) δ 7.65 (m, 1H), 7.52 (m, 2H), 4.48 (d, 0.35), 4.43 (d, 0.65), 3.79 (m, 1H), 2.71 (t, 2H), 2.60 (dd, 2H), 2.16 (dd, 1H), 1.99 (dt, 1H), 1.27 (t, 1.95H), 1.24 (t, 1.05H).

### Example 326

### N'-((2RS,3R)-3-amino-5-(ethylsulfanyl)-2-hydroxypentanoyl)-3,4-dichlorobenzohydrazide

The desired product was prepared by substituting 3,4-dichlorobenzoic acid for 3-(ethylsulfanyl)benzoic acid in Example 239B.
MS (ESI) m/e 381 (M+H)⁺;
¹H NMR (500 MHz, CD₃OD) δ 8.05 (d, 0.65H), 8.04 (d, 0.35H), 7.80 (m, 1H), 7.68 (m, 1H), 4.49 (d, 0.35), 4.44 (d, 0.65), 3.79 (m, 1H), 2.71 (t, 2H), 2.61 (dd, 2H), 2.16 (dd, 1H), 2.00 (dt, 1H), 1.27 (t, 1.95H), 1.25 (t, 1.05H).

### Example 327

### N'-((2RS,3R)-3-amino-5-(ethylsulfanyl)-2-hydroxypentanoyl)-3,5-dichlorobenzohydrazide

The desired product was prepared by substituting 3,5-dichlorobenzoic acid for 3-(ethylsulfanyl)benzoic acid in Example 239B.
MS (ESI) m/e 3 81 (M+H)⁺;
¹H NMR (500 MHz, CD₃OD) δ 7.85 (d, 1.3H), 7.83 (d, 0.7H), 7.72 (t, 0.65H), 7.70 (t, 0.35H), 4.49 (d, 0.35), 4.44 (d, 0.65), 3.79 (m, 1H), 2.71 (t, 2H), 2.61 (dd, 2H), 2.16 (dd, 1H), 1.99 (dt, 1H), 1.27 (t, 1.95H), 1.25 (t, 1.05H).

### Example 328

### N'-((2RS,3R)-3-amino-4-cyclohexyl-2-hydroxybutanoyl)-2-hydroxybenzohydrazide

The desired compound was prepared by substituting salicylic acid for o-toluic acid in Example 329B.
MS (ESI) m/e 336 (M+H)⁺;
¹H NMR (500 MHz, CD₃OD) δ 7.87 (m, 1H), 7.44 (m, 1H), 6.95 (m, 2H), 4.45 (d, 0.3H), 4.38 (d, 0.7H), 3.69 (m, 1H), 1.76 (m, 6H), 1.58 (m, 1H), 1.47 (m, 1H), 1.32 (m, 1H), 1.24 (m, 1H), 1.03 (m, 1.4H), 0.93 (m, 0.6H).

### Example 329

### N'-((2RS,3R)-3-amino-4-cyclohexyl-2-hydroxybutanoyl)-2-methylbenzohydrazide

### Example 329A

### N'-((2RS,3R)-3-(tert-butoxycarbonyl)amino-4-cyclohexyl-2-hydroxybutanoyl)hydrazide

The desired compound was prepared by substituting Example 1 C for Example 97A acid in Example 239A.

### Example 329B

### N'-((2RS,3R)-3-amino-4-cyclohexyl-2-hydroxybutanoyl)-2-methylbenzohydrazide

To DCC resin (148 mg, 0.225 mmol) in 1.0 mL of dichloromethane was added 0.5 mL of a 0.45 M solution of HOBt (0.225 mmol) in dimethylacetamide/dichloromethane (1:6), and 0.5 mL of a 0.3M solution of o-toluic acid (0.15 mmol) in dimethylacetamide. After 5 minutes, 1.0 mL of a 0.225 M solution of Example 329A (0.225 mmol) in dimethylacetamide/dichloromethane (1:1) was added. The mixture was agitated for 18 hours and quenched with 0.19 g of trisamine resin (0.75 mmol) followed by 0.13 g of isocyanate resin (0.225 mmol) and agitated for 4 hours. The mixture was filtered and the resins washed with 1x3 mL of dichloromethane, the solvent was removed in vacuo, and the crude material purified by reverse phase preparative HPLC. The resulting material was treated with 1 mL of 50% trifluoroacetic acid/dichloromethane and agitated at ambient temperature for 18 hours. The solvent was removed in vacuo to give the desired product.
MS (ESI) m/e 334 (M+H)⁺;
¹H NMR (500 MHz, CD₃OD δ 7.50 (m, 1H), 7.39 (ddd, 1H), 7.28 (m, 2H), 4.44 (d, 0.3H), 4.36 (d, 0.7H), 3.71 (m, 0.7H), 3.67 (m 0.3H), 2.46 (s, 3H), 1.73 (m, 6H), 1.56 (m, 1H), 1.47 (m, 1H), 1.28 (m, 3H), 1.02 (m, 1.4H), 0.93 (m, 0.6H).

### Example 330

### 2-amino-N'-((2RS,3R)-3-amino-4-cyclohexyl-2-hydroxybutanoyl)benzohydrazide

The desired compound was prepared by substituting o-aminobenzoic acid for o-toluic acid in Example 329B.
MS (ESI) m/e 335 (M+H)⁺;
¹H NMR (500 MHz, CD₃OD δ 7.57 (m, 1H), 7.25 (m, 1H), 6.80 (m, 1H), 6.67 (m, 1H), 4.44 (d, 0.3H), 4.36 (d, 0.7H), 3.69 (m, 0.7H), 3.65 (m 0.3H), 1.76 (m, 6H), 1.57 (m, 1H), 1.47 (m, 1H), 1.37-1.20 (m, 3H), 1.03 (m, 1.4H), 0.93 (m, 0.6H).

### Example 331

### 4-amino-N'-((2RS,3R)-3-amino-4-cyclohexyl-2-hydroxybutanoyl)benzohydrazide

The desired compound was prepared by substituting p-aminobenzoic acid for o-toluic acid in Example 329B.
MS (ESI) m/e 335 (M+H)⁺;
¹H NMR (500 MHz, CD₃OD δ 7.67 (m, 2H), 6.69 (m, 2H), 4.42 (d, 0.4H), 4.35 (d, 0.6H), 3.69-3.62 (m, 1H), 1.85-1.61 (m, 6H), 1.56 (m, 1H), 1.46 (m, 1H), 1.37-1.20 (m, 3H), 1.03 (m, 1.2H), 0.91 (m, 0.8H).

### Example 332

### 3-amino-N'-((2RS,3R)-3-amino-4-cyclohexyl-2-hydroxybutanoyl)benzohydrazkde

The desired compound was prepared by substituting m-aminobenzoic acid for o-toluic acid in Example 329B.
MS (ESI) m/e 335 (M+H)⁺;
¹H NMR (500 MHz, CD₃OD) δ 7.36 (m, 3H), 7.09 (m, 1H), 4.45 (d, 0.25H), 4.36 (d, 0.75H), 3.69 (m, 1H), 1.85-1.64 (m, 6H), 1.56 (m, 1H), 1.47 (m, 1H), 1.33 (m, 2H), 1.22 (m, 1H), 1.02 (m, 1.5 H), 0.92 (m, 0.5H).

### Example 333

### N'-((2RS,3R)-3-amino-4-cyclohexyl-2-hydroxybutanoyl)-3-hydroxybenzohydrazide

The desired compound was prepared by substituting 3-hydroxybenzoic acid for o-toluic acid in Example 329B.
MS (ESI) m/e 336 (M+H)⁺;
¹H NMR (500 MHz, CD₃OD) δ 7.31 (m, 3H), 7.01 (m, 1H), 6.80 (m, 1H), 4.44 (d, 0.3H), 4.36 (d, 0.7H), 3.69 (m, 0.7H), 3.65 (m 0.3H), 1.85-1.64 (m, 6H), 1.57 (m, 1H), 1.47 (m, 1H), 1.33 (m, 2H), 1.24 (m, 1H), 1.03 (m, 1.4H), 0.93 (m, 0.6H).

### Example 334

### N'-((2RS,3R)-3-amino-4-cyclohexyl-2-hydroxybutanoyl)-4-hydroxybenzohydrazide

The desired compound was prepared by substituting 4-hydroxybenzoic acid for o-toluic acid in Example 329B.
MS (ESI) m/e 336 (M+H)⁺;
¹H NMR (500 MHz, CD₃OD) δ 7.78 (m, 2H), 6.85 (m, 2H), 4.43 (d, 0.3H), 4.36 (d, 0.7H), 3.71-3.62 (m, 1H), 1.75 (m, 6H), 1.57 (m, 1H), 1.47 (m, 1H), 1.38-1.20 (m, 3H), 1.03 (m, 1.4H), 0.92 (m, 0.6H).

### Example 335

### N'-((2RS,3R)-3-amino-4-cyclohexyl-2-hydroxybutanoyl)-4-methoxybenzohydrazide

The desired compound was prepared by substituting p-anisic acid for o-toluic acid in Example 329B.
MS (ESI) m/e 350 (M+H)⁺;
¹H NMR (500 MHz, CD₃OD) δ 7.87 (m, 2H), 7.02 (m, 2H), 6.80 (m, 1H), 6.67 (m, 1H), 4.44 (d, 0.3H), 4.36 (d, 0.7H), 3.70-3.64 (m, 1H), 1.84-1.62 (m, 6H), 1.57 (m, 1H), 1.47 (m, 1H), 1.39-1.20 (m, 3H), 1.03 (m, 1.4H), 0.93 (m, 0.6H).

### Example 336

### N-((2RS,3R)-3-amino-4-cyclohexyl-2-hydroxybutanoyl)-2-fluorobenzohydrazide

The desired compound was prepared by substituting 2-fluorobenzoic acid for o-toluic acid in Example 329B.
MS (ESI) m/e 338 (M+H)⁺;
¹H NMR (500 MHz, CD₃OD) δ 7.81 (m, 1H), 7.61 (m, 1H), 7.34-7.23 (m, 2H), 4.44 (d, 0.3H), 4.37 (d, 0.7H), 3.69 (m, 1H), 1.75 (m, 6H), 1.56 (m, 1H), 1.47 (m, 1H), 1.38-1.20 (m, 3H), 1.03 (m, 1.4H), 0.93 (m, 0.6H).

### Example 337

### N'-((2RS,3R)-3-amino-4-cyclohexyl-2-hydroxybutanoyl)-3-fluorobenzohydrazide

The desired compound was prepared by substituting 3-fluorobenzoic acid for o-toluic acid in Example 329B.
MS (ESI) m/e 338 (M+H)⁺;
¹H NMR (500 MHz, CD₃OD) δ 7.72 (m, 1H), 7.63 (m, 1H), 6.53 (m, 1H), 7.35 (m, 1H), 4.45 (d, 0.25H), 4.37 (d, 0.75H), 3.68 (m, 1H), 1.83-1.64 (m, 6H), 1.57 (m, 1H), 1.47 (m, 1H), 1.33 (m, 2H), 1.24 (m, 1H), 1.02 (m, 1.5H), 0.92 (m, 0.5H).

### Example 338

### N'-((2RS,3R)-3-amino-4-cyclohexyl-2-hydroxybutanoyl)-4-fluorobenzohydrazide

The desired compound was prepared by substituting 4-fluorobenzoic acid for o-toluic acid in Example 329B.
MS (ESI) m/e 338 (M+H)⁺;
¹H NMR (500 MHz, CD₃OD δ 7.95 (m, 2H), 7.24 (m, 2H), 6.53 (m, 1H), 7.35 (m, 1H), 4.45 (d, 0.3H), 4.37 (d, 0.7H), 3.68 (m, 1H), 1.85-1.64 (m, 6H), 1.57 (m, 1H), 1.47 (m, 1H), 1.33 (m, 2H), 1.24 (m, 1H), 1.02 (m, 1.4H), 0.92 (m, 0.6H).

### Example 339

### N'-((2RS,3R)-3-amino-4-cyclohexyl-2-hydroxybutanoyl)-2-bromobenzohydrazide

The desired compound was prepared by substituting 2-bromobenzoic acid for o-toluic acid in Example 329B.
MS (ESI) m/e 399 (M+H)⁺;
¹H NMR (500 MHz, CD₃OD) δ 7.72 (m, 1H), 7.69 (m, 1H), 7.46 (m, 1H), 7.41 (m, 1H), 4.44 (d, 0.3H), 4.36 (d, 0.7H), 3.68 (m, 1H), 1.85-1.65 (m, 6H), 1.56 (m, 1H), 1.47 (m, 1H), 1.38-1.18 (m, 3H), 1.03 (m, 1.4H), 0.92 (m, 0.6H).

### Example 340

### N'-((2RS,3R)-3-amino-4-cyclohexyl-2-hydroxybutanoyl)-3-cyanobenzohydrazide

The desired compound was prepared by substituting 3-cyanobenzoic acid for o-toluic acid in Example 329B.
MS (ESI) m/e 345 (M+H)⁺;
¹H NMR (500 MHz, CD₃OD) δ 8.23 (m, 1H), 8.17 (m, 1H), 7.96 (m, 1H), 7.71 (m, 1H), 4.46 (d, 0.3H), 4.38 (d, 0.7H), 3.69 (m, 1H), 1.85-1.65 (m, 6H), 1.58 (m, 1H), 1.47 (m, 1H), 1.33 (m, 2H), 1.24 (m, 1H), 1.02 (m, 1.5H), 0.92 (m, 0.5H).

### Example 341

### N'-((2RS,3R)-3-amino-4-cyclohexyl-2-hydroxybutanoyl)-4-cyanobenzohydrazide

The desired compound was prepared by substituting 4-cyanobenzoic acid for o-toluic acid in Example 329B.
MS (ESI) m/e 345 (M+H)⁺;
¹H NMR (500 MHz, CD₃OD δ 8.02 (m, 2H), 7.88 (m, 2H), 4.46 (d, 0.3H), 4.37 (d, 0.7H), 3.69 (m, 1H), 1.85-1.65 (m, 6H), 1.56 (m, 1H), 1.46 (m, 1H), 1.33 (m, 2H), 1.24 (m, 1H), 1.03 (m, 1.4H), 0.92 (m, 0.6H).

### Example 342

### N'-((2RS,3R)-3-amino-4-cyclohexyl-2-hydroxybutanoyl)-3-(dimethylamino)benzohydrazide

The desired compound was prepared by substituting 3-dimethylaminobenzoic acid for o-toluic acid in Example 329B.
MS (ESI) m/e 363 (M+H)⁺;
¹H NMR (500 MHz, CD₃OD) δ 7.32 (t, 1H), 7.28 (m, 1H), 7.21 (m, 1H), 7.01 (dd, 1H), 4.44 (d, 0.3H), 4.37 (d, 0.7H), 3.68 (m, 1H), 3.01-3.00 (2S, 6H), 1.85-1.65 (m, 6H), 1.57 (m, 1H), 1.47 (m, 1H), 1.32 (m, 2H), 1.24 (m, 1H), 1.03 (m, 1.4H), 0.92 (m, 0.6H).

### Example 343

### N'-((2RS,3R)-3-amino-4-cyclohexyl-2-hydroxybutanoyl)-4-(dimethylamino)benzohydrazide

The desired compound was prepared by substituting 4-dimethylaminobenzoic acid for o-toluic acid in Example 329B.
MS (ESI) m/e 363 (M+H)⁺;
¹H NMR (500 MHz, CD₃OD) δ 7.78 (d, 2H), 7.65 (d, 2H), 6.53 (m, 1H), 4.42 (d, 0.3H), 4.35 (d, 0.7H), 3.68 (m, 1H), 3.04 (2S, 6H), 1.85-1.63 (m, 6H), 1.57 (m, 1H), 1.46 (m, 1H), 1.33 (m, 2H), 1.24 (m, 1H), 1.02 (m, 1.4H), 0.92 (m, 0.6H).

### Example 344

### N'-((2RS,3R)-3-amino-4-cyclohexyl-2-hydroxybutanoyl)-3-(trifluoromethyl)benzohydrazide

The desired compound was prepared by substituting 3-(trifluoromethyl)benzoic acid for o-toluic acid in Example 329B.
MS (ESI) m/e 388 (M+H)⁺;
¹H NMR (500 MHz, CD₃OD) δ 8.21 (s, 1H), 8.14 (m, 1H), 7.93 (m, 1H), 7.73 (m, 1H), 4.45 (d, 0.3H), 4.38 (d, 0.7H), 3.69 (m, 1H), 1.85-1.65 (m, 6H), 1.57 (m, 1H), 1.47 (m, 1H), 1.33 (m, 2H), 1.24 (m, 1H), 1.03 (m, 1.4H), 0.92 (m, 0.6H).

### Example 345

### N'-((2RS,3R)-3-amino-4-cyclohexyl-2-hydroxybutanoyl)-4-(trifluoromethyl)benzohydrazide

The desired compound was prepared by substituting 4-(trifluoromethyl)benzoic acid for o-toluic acid in Example 329B.
MS (ESI) m/e 388 (M+H)⁺;
¹H NMR (500 MHz, CD₃OD) δ 8.06 (m, 2H), 7.82 (m, 2H), 4.46 (d, 0.3H), 4.38 (d, 0.7H), 3.69 (m, 1H), 1.85-1.65 (m, 6H), 1.57 (m, 1H), 1.47 (m, 1H), 1.33 (m, 2H), 1.24 (m, 1H), 1.02 (m, 1.4H), 0.92 (m, 0.6H).

### Example 346

### N'-((2RS,3R)-3-amino-4-cyclohexyl-2-hydroxybutanoyl)-3-(trifluoromethoxy)benzohydrazide

The desired compound was prepared by substituting 3-(trifluoromethoxy)benzoic acid for o-toluic acid in Example 329B.
MS (ESI) m/e 404 (M+H)⁺;
¹H NMR (500 MHz, CD₃OD) δ 7.89 (m, 1H), 7.80 (s, 1H), 7,62 (m, 1H), 7.53 (m, 1H), 4.46 (d, 0.3H), 4.38 (d, 0.7H), 3.69 (m, 1H), 1.85-1.65 (m, 6H), 1.57 (m, 1H), 1.47 (m, 1H), 1.33 (m, 2H), 1.24 (m, 1H), 1.02 (m, 1.4H), 0.92 (m, 0.6H).

### Example 347

### N'-((2RS,3R)-3-amino-4-cyclohexyl-2-hydroxybutanoyl)-4-phenoxybenzohydrazide

The desired compound was prepared by substituting 4-phenoxybenzoic acid for o-toluic acid in Example 329B.
MS (ESI) m/e 412 (M+H)⁺;
¹H NMR (500 MHz, CD₃OD) δ 7.89 (m, 2H), 7.42 (m, 2H), 7.21 (m, 1H), 7.07 (m, 2H), 7.03 (m, 2H), 4.44 (d, 0.3H), 4.37 (d, 0.7H), 3.69 (m, 1H), 1.85-1.64 (m, 6H), 1.56 (m, 1H), 1.46 (m, 1H), 1.33 (m, 2H), 1.24 (m, 1H), 1.02 (m; 1.4H), 0.92 (m, 0.6H).

### Example 348

### N'-((2RS,3R)-3-amino-4-cyclohexyl-2-hydroxybutanoyl)-2,4-dimethylbenzohydrazide

The desired compound was prepared by substituting 2, 4-dimethylbenzoic acid for o-toluic acid in Example 329B.
MS (ESI) m/e 348 (M+H)⁺;
¹H NMR (5 00 MHz, CD₃OD) δ 7.41 (dd, 1 H), 7.12 (s, 1H), 7.09 (m, 1H), 4.43 (d, 0.25H), 4.36 (d, 0.75H), 3.70 (m, 0.75H), 3.66 (m, 0.25H), 2.43 (s, 3H), 2.34 (s, 3H), 1.85-1.65 (m, 6H), 1.57 (m, 1H), 1.46 (m, 1H), 1.32 (m, 2H), 1.24 (m, 1H), 1.02 (m, 1.5H), 0.92 (m, 0.5H).

### Example 349

### N'-((2RS,3R)-3-amino-4-cyclohexyl-2-hydroxybutanoyl)-2,5-dimethylbenzohydrazide

The desired compound was prepared by substituting 2, 5-dimethylbenzoic acid for o-toluic acid in Example 329B.
MS (ESI) m/e 348 (M+H)⁺;
¹H NMR (500 MHz, CD₃OD) δ 7.33 (s, 1H), 7.21 (m, 1H), 7.17 (m, 1H), 4.44 (d, 0.3H), 4.36 (d, 0.7H), 3.70 (m, 0.7H), 3.66 (m, 0.3H), 2.41 (s, 3H), 2.34 (s, 3H), 1.85-1.65 (m, 6H), 1.57 (m, 1H), 1.47 (m, 1H), 1.33 (m, 2H), 1.24 (m, 1H), 1.02 (m, 1.4H), 0.92 (m, 0.6H).

### Example 350

### N'-((2RS,3R)-3-amino-4-cyclohexyl-2-hydroxybutanoyl)-3,4-dimethylbenzohydrazide

The desired compound was prepared by substituting 3,4-dimethylbenzoic acid for o-toluic acid in Example 329B.
MS (ESI) m/e 348 (M+H)⁺;
¹H NMR (500 MHz, CD₃OD) δ 7.66 (s, 1H), 7.62 (dd, 1H), 7.26 (dd, 1H), 4.44 (d, 0.3H), 4.36 (d, 0.7H), 3.68 (m, 1H), 2.07 (s, 6H), 1.85-1.65 (m, 6H), 1.56 (m, 1H), 1.47 (m, 1H), 1.33 (m, 2H), 1.24 (m, 1H), 1.02 (m, 1.4H), 0.92 (m, 0.6H).

### Example 351

### N'-((2RS,3R)-3-amino-4-cyclohexyl-2-hydroxybutanoyl)-3,5-dimethylbenzohydrazide

The desired compound was prepared by substituting 3,5-dimethylbenzoic acid for o-toluic acid in Example 329B.
MS (ESI) m/e 348 (M+H)⁺;
¹H NMR (500 MHz, CD₃OD) δ 7.50 (s, 2H), 7.26 (s, 0.75H), 7.24 (s, 0.25H), 4.44 (d, 0.25H), 4.36 (d, 0.75H), 3.68 (m, 1H), 2.37 (s, 6H), 1.85-1.65 (m, 6H), 1.55 (m, 1H), 1.47 (m, 1H), 1.33 (m, 2H), 1.24 (m, 1H), 1.03 (m, 1.5H), 0.92 (m, 0.5H).

### Example 352

### N'-((2RS,3R)-3-amino-4-cyclohexyl-2-hydroxybutanoyl)-2,3-dimethoxybenzohydrazide

The desired compound was prepared by substituting 2, 3-dimethoxybenzoic acid for o-toluic acid in Example 329B.
MS (ESI) m/e 380 (M+H)⁺;
¹H NMR (500 MHz, CD₃OD) δ 7.41 (m, 1H), 7.24 (td, 2H), 7.19 (td, 1H), 4.43 (d, 0.3H), 4.37 (d, 0.7H), 3.95 (s, 2.1H), 3.95 (s, 0.9H), 3.91 (s, 2.1H), 3.90 (s, 0.9H), 3.69 (m, 1H), 1.85-1.65 (m, 6H), 1.57 (m, 1H), 1.46 (m, 1H), 1.33 (m, 2H), 1.24 (m, 1H), 1.02 (m, 1.4H), 0.92 (m, 0.6H).

### Example 353

### N'-((2RS,3R)-3-amino-4-cyclohexyl-2-hydroxybutanoyl)-3,4-dimethoxybenzohydrazide

The desired compound was prepared by substituting 3,4-dimethoxybenzoic acid for o-toluic acid in Example 329B.
MS (ESI) m/e 380 (M+H)⁺;
¹H NMR (500 MHz, CD₃OD) δ 7.55 (m, 1H), 7.50 (d, 1H), 7.05 (dd, 1H), 4.44 (d, 0.3H), 4.37 (d, 0.7H), 3.90 (s, 3H), 3.88 (s, 3H), 3.69 (m, 1H), 1.85-1.65 (m, 6H), 1.57 (m, 1H), 1.47 (m, 1H), 1.33 (m, 2H), 1.24 (m, 1H), 1.02 (m, 1.4H), 0.92 (m, 0.6H).

### Example 354

### N'-((2RS,3R)-3-amino-4-cyclohexyl-2-hydroxybutanoyl)-3,4,5-trimethoxybenzohydrazide

The desired compound was prepared by substituting 3,4,5-trimethoxybenzoic acid for o-toluic acid in Example 329B.
MS (ESI) m/e 410 (M+H)⁺;
¹H NMR (500 MHz, CD₃OD) δ 7.25 (s, 2H), 4.46 (d, 0.3H), 4.37 (d, 0.7H), 3.89 (s, 6H), 3.83 (s, 3H), 3.69 (m, 1H), 1.85-1.65 (m, 6H), 1.57 (m, 1H), 1.47 (m, 1H), 1.33 (m, 2H), 1.24 (m, 1H), 1.02 (m, 1.4H), 0.92 (m, 0.6H).

### Example 355

### N'-((2RS,3R)-3-amino-4-cyclohexyl-2-hydroxybutanoyl)-2,3-dichlorobenzohydrazide

The desired compound was prepared by substituting 2, 3-dichlorobenzoic acid for o-toluic acid in Example 329B.
MS (ESI) m/e 389 (M+H)⁺;
¹H NMR (500 MHz, CD₃OD) δ 7.68 (td, 1H), 7.54 (dt, 1H), 6.42 (td, 1H), 4.44 (d, 0.3H), 4.36 (d, 0.7H), 3.69 (m, 1H), 1.85-1.65 (m, 6H), 1.55 (m, 1H), 1.47 (m, 1H), 1.33 (m, 2H), 1.24 (m, 1H), 1.02 (m, 1.4H), 0.92 (m, 0.6H).

### Example 356

### N'-((2RS,3R)-3-amino-4-cyclohexyl-2-hydroxybutanoyl)-2,4-dichlorobenzohydrazide

The desired compound was prepared by substituting 2, 4-dichlorobenzoic acid for o-toluic acid in Example 329B.
MS (ESI) m/e 389 (M+H)⁺;
¹H NMR (500 MHz, CD₃OD) δ 7.60 (m, 2H), 7.46 (m, 1H), 4.44 (d, 0.3H), 4.35 (d, 0.7H), 3.68 (m, 1H), 1.85-1.65 (m, 6H), 1.55 (m, 1H), 1.46 (m, 1H), 1.33 (m, 2H), 1.23 (m, 1H), 1.02 (m, 1.4H), 0.92 (m, 0.6H).

### Example 357

### N'-((2RS,3R)-3-amino-4-cyclohexyl-2-hydroxybutanoyl)-3,4-dichlorobenzohydrazide

The desired compound was prepared by substituting 3,4-dichlorobenzoic acid for o-toluic acid in Example 329B.
MS (ESI) m/e 389 (M+H)⁺;
¹H NMR (500 MHz, CD₃OD δ 8.05 (m, 1H), 7.80 (m, 1H), 7.68 (m, 1H), 4.45 (d, 0.3H), 4.37 (d, 0.7H), 3.67 (m, 1H), 1.85-1.65 (m, 6H), 1.56 (m, 1H), 1.47 (m, 1H), 1.33 (m, 2H), 1.24 (m, 1H), 1.02 (m, 1.4H), 0.92 (m, 0.6H).

### Example 358

### N'-((2RS,3R)-3-amino-4-cyclohexyl-2-hydroxybutanoyl)-3,5-dichlorobenzohydrazide

The desired compound was prepared by substituting 3,5-dichlorobenzoic acid for o-toluic acid in Example 329B.
MS (ESI) m/e 389 (M+H)⁺;
¹H NMR (500 MHz, CD₃OD) δ 7.97-7.69 (m, 3H), 4.45 (d, 0.3H), 4.37 (d, 0.7H), 3.68 (m, 1H), 1.85-1.65 (m, 6H), 1.56 (m, 1H), 1.47 (m, 1H), 1.33 (m, 2H), 1.24 (m, 1H), 1.02 (m, 1.4H), 0.92 (m, 0.6H).

### Example 359

### N'-((2RS,3R)-3-amino-4-cyclohexyl-2-hydroxybutanoyl)-2,3-dimethylbenzohydrazide

The desired compound was prepared by substituting 2, 3-dimethylbenzoic acid for o-toluic acid in Example 329B.
MS (ESI) m/e 348 (M+H)⁺;
¹H NMR (500 MHz, CD₃OD) δ 7.29 (m, 2H), 7.16 (m, 1H), 4.44 (d, 0.3H), 4.36 (d, 0.7H), 3.68 (m, 1H), 1.85-1.64 (m, 6H), 1.57 (m, 1H), 1.47 (m, 1H), 1.33 (m, 2H), 1.24 (m, 1H), 1.02 (m, 1.4H), 0.92 (m, 0.6H).

### Example 360

### N'-((2RS,3R)-3-amino-5-(ethylsulfanyl)-2-hydroxypentanoyl)benzenesulfonohydrazide

The desired product was prepared by substituting benzenesulfonylhydrazine for O-phenyl hydroxylamine hydrochloride in Example 98B.
MS (ESI) m/e 348 (M+H)⁺;
¹H NMR (300 MHz, DMSO-d₆) δ 7.38 (m, 5H), 6.68 (d, 0.6H), 6.62 (d, 0.4H), 4.30 (m, 0.4H), 4.14 (m, 0.6H), 3.87 (m, 1H), 2.60 (m, 2H), 2.45 (m, 2H), 1.64 (m, 1H), 1.36 (m, 1H), 1.16 (t, 3H).

### Example 361

### N'-((2RS,3R)-3-amino-5-(ethylsulfanyl)-2-hydroxypentanoyl)-3-chlorobenzenesulfonohydrazide

The desired product was prepared by substituting 3-chlorophenylsulfonylhydrazine for O-phenyl hydroxylamine hydrochloride in Example 98B.
MS (ESI) m/e 383 (M+H)⁺;
¹H NMR (300 MHz, DMSO-d₆) δ 10.25 (m, 1H), 10.14 (m, 1H), 8.55 (br s, 2H), 7.85 (m, 1H), 7.78 (m, 2H), 7.65 (m, 1H), 6.68 (d, 0.6H), 6.62 (d, 0.4H), 4.30 (m, 0.4H), 4.14 (m, 0.6H), 3.87 (m, 1H), 2.60 (m, 2H), 2.45 (m, 2H), 1.64 (m, 1H), 1.36 (m, 1H), 1.16 (t, 3H).

### Example 362

### N'-(2RS,3R)-3-amino-5-(ethylsulfanyl)-2-hydroxypentanoyl)-1-naphthalenesulfonohydrazide

The desired product was prepared by substituting 1-naphthylsulfonylhydrazine for O-phenyl hydroxylamine hydrochloride in Example 98B.
MS (ESI) m/e 398 (M+H)⁺;
¹H NMR (300 MHz, DMSO-d₆) δ 10.25 (m, 1H), 10.14 (m, 1H), 8.70 (br s, 2H), 8.27 (m, 1H), 8.17 (m, 1H), 8.13 (m, 1H), 7.68 (m, 4H), 6.68 (d, 0.6H), 6.62 (d, 0.4H), 4.30 (m, 0.4H), 4.14 (m, 0.6H), 3.87 (m, 1H), 2.60 (m, 2H), 2.45 (m, 2H), 1.64 (m, 1H), 1.36 (m, 1H), 1.16 (t, 3H).

### Example 363

### N'-((2RS,3R)-3-amino-5-(ethylsulfanyl)-2-hydroxypentanoyl)-4-methylbenzenesulfonohydrazide

The desired product was prepared by substituting 4-methylphenylsulfonylhydrazine for O-phenyl hydroxylamine hydrochloride in Example 98B.
MS (ESI) m/e 362 (M+H)⁺;
¹H NMR (300 MHz, DMSO-d₆) δ 7.72 (m, 2H), 7.38 (m, 2H), 6.68 (d, 0.6H), 6.62 (d, 0.4H), 4.30 (m, 0.4H), 4.14 (m, 0.6H), 3.87 (m, 1H), 2.60 (m, 2H), 2.45 (m, 2H), 2.40 (s, 3H), 1.64 (m, 1H), 1.36 (m, 1H), 1.16 (t, 3H).

### Example 364

### (2RS,3R)-3-amino-N'-(3-(2-aminoethoxy)phenyl)- 2-hydroxy-5-(isopropylsulfanyl)pentanohydrazide

### Example 364A

### N-(tert-butoxycarbonyl)-2-bromoethylamine

A solution of 2-bromoethylamine hydrobromide (1.0 g, 4.9 mmol), di-tert-butyl dicarbonate (1.06 g, 4.9 mmol) and triethylamine (0.7 mL, 4.9 mmol) in dichloromethane (40 mL) was stirred at room temperature for 18h. The reaction mixture was diluted with ether, washed with brine, dried (Na₂SO₄), and concentrated to give the title compound.

### Example 364B

### 3-(2-(tertbutoxycarbonylamino)ethoxy)benzoyl hydrazide

Example 364A, methyl-3-hydroxy-benzoate (1.09 g, 4.9 mmol) and potassium tert-butoxide (6.5 g, 5.8 mmol) in DMSO were stirred at room temperature for 16 hours. The reaction was poured into ice water and extracted with ether, washed with brine, dried over Na2SO4, evaporated, and treated with hydrazine hydrate in ethanol at reflux for 48h. The reaction mixture was evaporated to dryness to give the title compound.

### Example 364C

### (2RS,3R)-3-amino-N'-(3-(2-aminoethoxy)phenyl)- 2-hydroxy-5-(isopropylsulfanyl)pentanohydrazide

The desired product was prepared by substituting Example 364A for O-phenyl hydroxylamine hydrochloride and Example 123B for Example 98A in Example 98B.
MS (ESI) m/e 383 (M-H)⁺.
¹H NMR (300 MHz, DMSO-d₆) δ 10.6 & 10.42 (s, 1H), 10.25 & 10.12 (s, 1H), 8.15 & 8.04 (br s, 2H), 7.56-7.33 (m, 3H), 7.2 (m, 1H), 6.67 & 6.59 (br s, 1H), 4.45 & 4.24 (m, 3H), 3.25 (m, 2H), 3.0-2.9 (m, 1H), 2.75-2.55 (m, 2H), 2.0-1.80 (m, 2H), 1.2 (m,6H).

### Example 365

### N'-((2RS,3R)-3-amino-5-(ethylsulfanyl)-2-hydroxypentanoyl)-3-bromobenzohydrazide

The desired product was prepared by substituting 3-bromobenzoic acid for 3-(ethylsulfanyl)benzoic acid in Example 239B.
MS (ESI) m/e 391 (M+H)⁺;
¹H NMR (500 MHz, CD₃OD) δ 8.06 (ddd, 1H), 7.85 (ddd, 1H), 7.77 (ddd, 1H), 7.44 (m, 1H), 4.49 (d, 0.35), 4.44 (d, 0.65), 3.78 (m, 1H), 2.71 (t, 2H), 2.61 (dd, 2H), 2.17 (dd, 1H), 1.99 (dt, 1H), 1.28 (t, 1.95H), 1.25 (t, 1.05H).

### Example 366

### N'-((2RS,3R)-3-amino-5-(ethylsulfanyl)-2-hydroxypentanoyl)-3-cyanobenzohydrazide

The desired product was prepared by substituting 2-cyanobenzoic acid for 3-(ethylsulfanyl)benzoic acid in Example 239B.
MS (ESI) m/e 337 (M+H)⁺;
¹H NMR (500 MHz, CD₃OD) δ 8.23 (ddd, 1H), 8.16 (ddd, 1H), 7.96 (ddd, 1H), 7.71 (m, 1H), 4.50 (d, 0.35), 4.45 (d, 0.65), 3.79 (m, 1H), 2.71 (t, 2H), 2.61 (dd, 2H), 2.17 (dd, 1H), 2.00 (dt, 1H), 1.28 (t, 1.95H), 1.25 (t, 1.05H).

### Example 367

### N'-((2RS,3R)-3-amino-5-(ethylsulfanyl)-2-hydroxypentanoyl)-4-cyanobenzohydrazide

The desired product was prepared by substituting 4-cyanobenzoic acid for 3-(ethylsulfanyl)benzoic acid in Example 239B.
MS (ESI) m/e 337 (M+H)⁺;
¹H NMR (500 MHz, CD₃OD) δ 8.02 (m, 2H), 7.88 (m, 2H), 4.50 (d, 0.35), 4.45 (d, 0.65), 3.79 (m, 1H), 2.71 (t, 2H), 2.61 (dd, 2H), 2.17 (dd, 1H), 2.00 (dt, 1H), 1.27 (t, 1.95H), 1.25 (t, 1.05H).

### Example 368

### N'-((2RS,3R)-3-amino-2-hydroxy-5-(isopropylsulfanyl)pentanoyl)-3-hydroxybenzohydrazide

The title compound is obtained by substituting 3-hydroxybenzoyl hydrazide for O-phenylhydroxylamine hydrochloride and Example 123B for Example 98A in Example 98B. MS (ESI) m/e 340 (M-H)⁺.
¹H NMR (300 MHz, DMSO-d₆) δ 10.44 & 10.26 (s, 1H), 10.17 & 10.06 (s, 1H), 9.78 & 9.74 (s, 1H), 8.05 & 8.7.97 (br s, 2H), 7.26 (m, 3H), 6.97 (m, 1H), 6.63 & 6.595(bd, 1H), 4.38 & 4.22 (m, 1H), 3.7 & 3.6 (m, 1H), 3.0-2.9 (m, 1H), 2.75-2.55 (m, 2H), 2.05-1.77 (m, 2H), 1.2 (m, 6H).

### Example 369

### N'-((2RS,3R)-3-amino-2-hydroxy-5-(isopropylsulfanyl)pentanoyl)-2-methylbenzohydrazide

### Example 369A

### N'-((2RS,3R)-3-(tert-butoxycarbonyl)amino-5-(isopropylsulfanyl)-2-hydroxypentanoyl)hydrazide

The desired compound was prepared by substituting Example 123B for Example 97A acid in Example 239A.

### Example 369B

### N'-((2RS,3R)-3-amino-2-hydroxy-5-(isopropylsulfanyl)pentanoyl)-2-methylbenzohydrazide

To DCC resin (148 mg, 0.225 mmol) in 1.0 mL of dichloromethane was added 0.5 mL of a 0.45 M solution of HOBt (0.225 mmol) in dimethylacetamide/dichloromethane (1:6), and 0.5 mL of a 0.3M solution of o-toluic acid (0.15 mmol) in dimethylacetamide. After 5 minutes, 1.0 mL of a 0.225 M solution of Example 369A (0.225 mmol) in dimethylacetamide/dichloromethane (1:1) was added. The mixture was agitated for 18 hours and quenched with 0.19 g of trisamine resin (0.75 mmol) followed by 0.13 g of isocyanate resin (0.225 mmol) and agitated for 4 hours. The mixture was filtered and the resins washed with 1 x3 mL of dichloromethane, the solvent was removed in vacuo, and the crude material purified by reverse phase preparative HPLC. The resulting material was treated with 1 mL of 50% trifluoroacetic acid/dichloromethane and agitated at ambient temperature for 18 hours. The solvent was removed in vacuo to give the desired product.
MS (ESI) m/e 340 (M+H)⁺;
¹H NMR (500 MHz, CD₃OD) δ 7.50 (m, 1H), 7.40 (ddd, 1H), 7.28 (m, 2H), 4.49 (d, 0.3H), 4.44 (d, 0.7H), 3.79 (m, 1H), 3.00 (m, 1H), 2.72 (t, 2H), 2.47 (2s, 3H), 2.16 (m, 1H), 1.98 (m, 1H), 1.29-1.25 (m, 6H).

### Example 370

### N'-((2RS,3R)-3-amino-2-hydroxy-5-(isopropylsulfanyl)pentanoyl)-3-methylbenzohydrazide

The desired compound was prepared by substituting m-toluic acid for o-toluic acid in Example 369.
MS (ESI) m/e 340 (M+H)⁺;
¹H NMR (500 MHz, CD₃OD) δ 7.69 (m, 2H), 7.40 (m, 2H), 4.49 (d, 0.3H), 4.44 (d, 0.7H), 3.78 (m, 1H), 3.00 (m, 1H), 2.72 (t, 2H), 2.41 (s, 3H), 2.16 (m, 1H), 1.98 (m, 1H), 1.29-1.25 (m, 6H).

### Example 371

### N'-((2RS,3R)-3-amino-2-hydroxy-5-(isopropylsulfanyl)pentanoyl)-4-methylbenzohydrazide

The desired compound was prepared by substituting p-toluic acid for o-toluic acid in Example 369.
MS (ESI) m/e 340 (M+H)⁺;
¹H NMR (500 MHz, CD₃OD) δ 7.78 (m, 2H), 7.31 (m, 2H), 4.49 (d, 0.3H), 4.44 (d, 0.7H), 3.77 (m, 1H), 3.00 (m, 1H), 2.72 (t, 2H), 2.41 (s, 3H), 2.15 (m, 1H), 1.99 (m, 1H), 1.29-1.24 (m, 6H).

### Example 372

### N'-((2RS,3R)-3-amino-2-hydroxy-5-(isopropylsulfanyl)pentanoyl)-2-methoxybenzohydrazide

The desired compound was prepared by substituting o-anisic acid for o-toluic acid in Example 369.
MS (ESI) m/e 356 (M+H)⁺;
¹H NMR (500 MHz, CD₃OD) δ 7.99 (dd, 1H), 7.57 (m, 1H), 7.19 (m, 1H), 7.09 (m, 1H), 4.48 (d, 0.3H), 4.45 (d, 0.7H), 4.00 (s, 2.1H), 3.99 (s, 0.9H), 3.78 (m, 1H), 3.00 (m, 1H), 2.72 (t, 2H), 2.14 (m, 1H), 1.98 (m, 1H), 1.29-1.24 (m, 6H).

### Example 373

### N'-((2RS,3R)-3-amino-2-hydroxy-5-(isopropyisulfanyl)pentanoyl)-3-methoxybenzohydrazide

The desired compound was prepared by substituting m-anisic acid for o-toluic acid in Example 369.
MS (ESI) m/e 356 (M+H)⁺;
¹H NMR (500 MHz, CD₃OD) δ 7.43 (m, 3H), 7.16 (m, 1H), 4.49 (d, 0.3H), 4.44 (d, 0.7H), 3.85 (s, 3H), 3.78 (m, 1H), 3.00 (m, 1H), 2.72 (t, 2H), 2.16 (m, 1H), 1.98 (m, 1H), 1.29-1.24 (m, 6H).

### Example 374

### N'-((2RS,3R)-3-amino-2-hydroxy-5-(isopropylsulfanyl)pentanoyl)-4-methoxybenzohydrazide

The desired compound was prepared by substituting p-anisic acid for o-toluic acid in Example 369.
MS (ESI) m/e 356 (M+H)⁺;
¹H NMR (500 MHz, CD₃OD) δ 7.87 (m, 2H), 7.02 (m, 2H), 4.48 (d, 0.3H), 4.44 (d, 0.7H), 3.87 (s, 2,1H), 3.86 (s, 0.9H), 3.77 (m, 1H), 3.00 (m, 1H), 2.71 (t, 2H), 2.14 (m, 1H), 1.98 (m, 1H), 1.29-1.25 (m, 6H).

### Example 375

### N'-((2RS,3R)-3-amino-2-hydroxy-5-(isopropylsulfanyl)pentanoyl)-2-fluorobenzohydrazide

The desired compound was prepared by substituting o-fluorobenzoic acid for o-toluic acid in Example 369.
MS (ESI) m/e 344 (M+H)⁺;
¹H NMR (500 MHz, CD₃OD) δ 7.81 (m, 1H), 7.60 (m, 1H), 7.33 (m, 1H), 7.27 (m, 1H), 4.48 (d, 0.3H), 4.44 (d, 0.7H), 3.78 (m, 1H), 3.00 (m, 1H), 2.72 (t, 2H), 2.15 (m, 1H), 1.98 (m, 1H), 1.29-1.24 (m, 6H).

### Example 376

### N'-((2RS,3R)-3-amino-2-hydroxy-5-(isopropylsulfanyl)pentanoyl)-3-fluorobenzohydrazide

The desired compound was prepared by substituting m-fluorobenzoic acid for o-toluic acid in Example 369.
MS (ESI) m/e 344 (M+H)⁺;
¹H NMR (500 MHz, CD₃OD δ 7.72 (t, 1H), 7.62 (m, 1H), 7.53 (ddd, 1H), 7.36 (ddd, 1H), 4.49 (d, 0.3H), 4.44 (d, 0.7H), 3.78 (m, 1H), 3.00 (m, 1H), 2.72 (t, 2H), 2.16 (m, 1H), 1.98 (m, 1H), 1.29-1.24 (m, 6H).

### Example 377

### N'-((2RS,3R)-3-amino-2-hydroxy-5-(isopropylsulfanyl)pentanoyl)-4-fluorobenzohydrazide

The desired compound was prepared by substituting p-fluorobenzoic acid for o-toluic acid in Example 369.
MS (ESI) m/e 344 (M+H)⁺;
¹H NMR (500 MHz, CD₃OD) δ 7.95 (m, 2H), 7.24 (m, 2H), 4.48 (d, 0.3H), 4.44 (d, 0.7H), 3.77 (m, 1H), 3.00 (m, 1H), 2.72 (t, 2H), 2.15 (m, 1H), 1.98 (m, 1H), 1.29-1.25 (m, 6H).

### Example 378

### N'-((2RS,3R)-3-amino-2-hydroxy-5-(isopropylsulfanyl)pentanoyl)-2-chlorobenzohydrazide

The desired compound was prepared by substituting o-chlorobenzoic acid for o-toluic acid in Example 369.
MS (ESI) m/e 360 (M+H)⁺;
¹H NMR (500 MHz, CD₃OD) δ 7.61 (m, 1H), 7.50 (m, 2H), 7.42 (m, 1H), 4.48 (d, 0.3H), 4.43 (d, 0.7H), 3.78 (m, 1H), 3.00 (m, 1H), 2.72 (t, 2H), 2.15 (m, 1H), 1.98 (m, 1H), 1.29-1.24 (m, 6H).

### (399005) Example 379

### N'-((2RS,3R)-3-amino-2-hydroxy-5-(isopropylsulfanyl)pentanoyl)-4-chlorobenzohydrazide

The desired compound was prepared by substituting p-chlorobenzoic acid for o-toluic acid in Example 369.
MS (ESI) m/e 360 (M+H)⁺;
¹H NMR (500 MHz, CD₃OD) δ 7.87 (m, 2H), 7.52 (m, 2H), 4.49 (d, 0.3H), 4.44 (d, 0.7H), 3.77 (m, 1H), 3.00 (m, 1H), 2.72 (t, 2H), 2.15 (m, 1H), 1.98 (m, 1H), 1.29-1.24 (m, 6H).

### Example 380

### N'-((2RS,3R)-3-amino-2-hydroxy-5-(isopropylsulfanyl)pentanoyl)-2-bromobenzohydrazide

The desired compound was prepared by substituting o-bromobenzoic acid for o-toluic acid in Example 369.
MS (ESI) m/e 405 (M+H)⁺;
¹H NMR (500 MHz, CD₃OD) δ 7.69 (m, 1H), 7.59 (m, 1H), 7.46 (m, 1H), 7.42 (m, 1H), 4.48 (d, 0.3H), 4.43 (d, 0.7H), 3.78 (m, 1H), 3.00 (m, 1H), 2.72 (t, 2H), 2.15 (m, 1H), 1.98 (m, 1H), 1.29-1.24 (m, 6H).

### Example 3 81

### N'-((2RS,3R)-3-amino-2-hydroxy-5-(isopropylsulfanyl)pentanoyl)-3-bromobenzohydrazide

The desired compound was prepared by substituting 3-bromobenzoic acid for o-toluic acid in Example 369. MS (ESI) m/e 405 (M+H)⁺;
¹H NMR (500 MHz, CD₃OD) δ 8.06 (dt, 1H), 7.85 (m, 1H), 7.77 (m, 1H), 7.44 (m, 1H), 4.49 (d, 0.3H), 4.44 (d, 0.7H), 3.78 (m, 1H), 3.00 (m, 1H), 2.72 (t, 2H), 2.15 (m, 1H), 1.98 (m, 1H), 1.29-1.24 (m, 6H).

### Example 382

### N'-((2RS,3R)-3-amino-2-hydroxy-5-(isopropylsulfanyl)pentanoyl)-4-bromobenzohydrazide

The desired compound was prepared by substituting 4-bromobenzoic acid for o-toluic acid in Example 369.
MS (ESI) m/e 405 (M+H)⁺;
¹H NMR (500 MHz, CD₃OD) δ 7.80 (m, 2H), 7.68 (m, 2H), 4.49 (d, 0.3H), 4.44 (d, 0.7H), 3.77 (m, 1H), 3.00 (m, 1H), 2.72 (t, 2H), 2.15 (m, 1H), 1.98 (m, 1H), 1.29-1.24 (m, 6H).

### Example 383

### N'-((2RS,3R)-3-amino-2-hydroxy-5-(isopropylsulfanyl)pentanoyl)-2,3-dichlorobenzohydrazide

The desired compound was prepared by substituting 2, 3-dichlorobenzoic acid for o-toluic acid in Example 369.
MS (ESI) m/e 395 (M+H)⁺;
¹H NMR (500 MHz, CD₃OD) δ 7.69 (td, 1H), 7.53 (m, 1H), 7.42 (m, 1H), 4.49 (d, 0.3H), 4.43 (d, 0.7H), 3.78 (m, 1H), 3.00 (m, 1H), 2.72 (t, 2H), 2.15 (m, 1H), 1.98 (m, 1H), 1.29-1.24 (m, 6H).

### Example 384

### (2RS,3R)-3-amino-2-hydroxy-5-(isopropylsulfanyl)-N'-(tetrahydro-2-furanylcarbonyl)pentanohydrazide

The desired compound was prepared by substituting tetrahydrofuran-2-carboxylic acid for o-toluic acid in Example 369.
MS (ESI) m/e 320 (M+H)⁺;
¹H NMR (500 MHz, CD₃OD) δ 4.45 (m, 1.3H), 4.38 (d, 0.7H), 4.02 (m, 1H), 3.87 (m, 1H), 3.73 (m, 1H), 2.98 (m, 1H), 2.69 (t, 2H), 2.29 (m, 1H), 2.10 (m, 2H), 1.94 (m, 3H), 1.28-1.23 (m, 6H).

### Example 385

### (2RS,3R)-3-amino-2-hydroxy-5-(isopropylsulfanyl)-N'-(tetrahydro-3-furanylcarbonyl)pentanohydrazide

The desired compound was prepared by substituting tetrahydro-3-furic acid for o-toluic acid in Example 369.
MS (ESI) m/e 320 (M+H)⁺;
¹H NMR (500 MHz, CD₃OD) δ 4.42 (d, 0.3H), 4.38 (d, 0.7H), 3.96 (m, 1H), 3.88 (m, 2H), 3.79 (m, 1H), 3.72 (m, 1H), 3.10 (m, 1H), 2.98 (m, 1H), 2.69 (t, 2H), 2.13 (m, 3H), 1.94 (m, 1H), 1.28-1.23 (m,6H).

### Example 386

### (2RS,3R)-3-amino-2-hydroxy-5-(isopropylsulfanyl)-N'-cyclopentylpentanohydrazide

The desired compound was prepared by substituting cyclopentanecarboxylic acid for o-toluic acid in Example 369.
MS (ESI) m/e 318 (M+H)⁺;
¹H NMR (500 MHz, CD₃OD δ 4.41 (d, 0.3H), 4.37 (d, 0.7H), 3.72 (m, 1H), 2.98 (m, 1H), 2.70 (m, 2H), 2.11(m, 1H), 2.10 (m, 1H), 1.91 (m, 3H), 1.77 (m, 5H), 1.62 (m, 1H), 1.28-1.23 (m, 6H).

### Example 3 87

### N'-((2RS,3R)-3-amino-2-hydroxy-5-(isopropylsulfanyl)pentanoyl)-2-cyclopentylacetohydrazide

The desired compound was prepared by substituting cyclopentylacetic acid for o-toluic acid in Example 369.
MS (ESI) m/e 332 (M+H)⁺;
¹H NMR (500 MHz, CD₃OD) δ 4.41 (d, 0.3H), 4.37 (d, 0.7H), 3.72 (m, 1H), 2.98 (m, 1H), 2.69 (t, 2H), 2.27 (m, 3H), 2.10 (m, 1H), 1.94 (m, 1H), 1.85 (m, 2H), 1.67 (m, 2H), 1.58 (m, 2H), 1.28-1.23 (m, 8H).

### Example 388

### (2RS,3R)-3-amino-2-hydroxy-5-(isopropylsulfanyl)-N'-cyclohexylpentanohydrazide

The desired compound was prepared by substituting cyclohexanecarboxylic acid for o-toluic acid in Example 369.
MS (ESI) m/e 332 (M+H)⁺;
¹H NMR (500 MHz, CD₃OD δ 4.41 (d, 0.3H), 4.37 (d, 0.7H), 3.72 (m, 1H), 2.98 (m, 1H), 2.69 (t, 2H), 2.30 (m, 1H), 2.10 (m, 1H), 1.94 (m, 1H), 1.82 (m, 4H), 1.71 (m, 1H), 1.48 (m, 2H), 1.37-1.23 (m, 9H).

### Example 389

### N'-((2RS,3R)-3-amino-2-hydroxy-5-(isopropylsulfanyl)pentanoyl)-2-cyclohexylacetohydrazide

The desired compound was prepared by substituting cyclohexylacetic acid for o-toluic acid in Example 369.
MS (ESI) m/e 346 (M+H)⁺;
¹H NMR (500 MHz, CD₃OD) δ 4.41 (d, 0.3H), 4.36 (d, 0.7H), 3.72 (m, 1H), 2.98 (m, 1H), 2.69 (t, 2H), 2.12 (m, 3H), 1.94 (m, 1H), 1.74 (m, 6H), 1.26 (m, 9H), 1.02 (m, 2H).

### Example 390

### N'-((2RS,3R)-3-amino-2-hydroxy-5-(isopropylsulfanyl)pentanoyl)-2-furohydrazide

The desired compound was prepared by substituting furan-2-carboxylic acid for o-toluic acid in Example 369.
MS (ESI) m/e 316(M+H)⁺;
¹H NMR (500 MHz, CD₃OD) δ 7.74 (d, 0.7H), 7.72 (d, 0.3H), 7.24 (d, 0.7H), 7.21 (d, 0.3H), 6.64 (m, 1H), 4.47 (d, 0.3H), 4.43 (d, 0.7H), 3.77 (m, 1H), 3.00 (m, 1H), 2.71 (t, 2H), 2.14 (m, 1H), 1.97 (m, 1H), 1.28-1.23 (m, 6H).

### Example 391

### N'-((2RS,3R)-3-amino-2-hydroxy-5-(isopropylsulfanyl)pentanoyl)-3-furohydrazide

The desired compound was prepared by substituting furan-3-carboxylic acid for o-toluic acid in Example 369.
MS (ESI) m/e 316 (M+H)⁺;
¹H NMR (500 MHz, CD₃OD δ 8.17 (d, 0.7H), 8.14 (d, 0.3H), 7.62 (m, 1H), 6.83 (m, 1H), 4.47 (d, 0.3H), 4.43 (d, 0.7H), 3.75 (m, 1H), 3.00 (m, 1H), 2.71 (t, 2H), 2.14 (m, 1H), 1.97 (m, 1H), 1.28-1.23 (m, 6H).

### Example 392

### N'-((2RS,3R)-3-amino-2-hydroxy-5-(isopropylsulfanyl)pentanoyl)-2,5-dimethyl-3-furohydrazide

The desired compound was prepared by substituting 2,5-dimethyl-3-furoic acid for o-toluic acid in Example 369.
MS (ESI) m/e 344 (M+H)⁺;
¹H NMR (500 MHz, CD₃OD) δ 6.32 (s, 0.7H), 6.31 (d, 0.3H), 4.46 (d, 0.3H), 4.41 (d, 0.7H), 3.75 (m, 1H), 3.00 (m, 1H), 2.70 (t, 2H), 2.50 (s, 3H), 2.25 (s, 3H), 2.14 (m, 1H), 1.97 (m, 1H), 1.28-1.23 (m, 6H).

### Example 393

### N'-((2RS,3R)-3-amino-2-hydroxy-5-(isopropylsulfanyl)pentanoyl)-2-thiophenecarbohydrazide

The desired compound was prepared by substituting thiophene-2-carboxylic acid for o-toluic acid in Example 369.
MS (ESI) m/e 332 (M+H)⁺;
¹H NMR (500 MHz, CD₃OD δ 7.77 (m, 2H), 7.17 (m, 1H), 4.48 (d, 0.3H), 4.43 (d, 0.7H), 3.78 (m, 1H), 3.00 (m, 1H), 2.71 (t, 2H), 2.14 (m, 1H), 1.97 (m, 1H), 1.29-1.25 (m, 6H).

### Example 394

### N'-((2RS,3R)-3-amino-2-hydroxy-5-(isopropylsulfanyl)pentanoyl)-3-thiophenecarbohydrazide

The desired compound was prepared by substituting thiophene-3-carboxylic acid for o-toluic acid in Example 369.
MS (ESI) m/e 332 (M+H)⁺;
¹H NMR (500 MHz, CD₃OD) δ 8.19 (m, 0.7H), 8.16 (m, 0.3H), 7.54 (m, 2H), 4.48 (d, 0.3H), 4.44 (d, 0.7H), 3.77 (m, 1H), 3.00 (m, 1H), 2.71 (t, 2H), 2.14 (m, 1H), 1.98 (m, 1H), 1.29-1.24 (m, 6H).

### Example 395

### N'-((2RS,3R)-3-amino-2-hydroxy-5-(isopropylsulfanyl)pentanoyl)-3-methyl-2-thiophenecarbohydrazide

The desired compound was prepared by substituting 3-methylthiophene-2-carboxylic acid for o-toluic acid in Example 369.
MS (ESI) m/e 346 (M+H)⁺;
¹H NMR (500 MHz, CD₃OD) δ 7.56 (d, 0.7H), 7.53 (d, 0.3H), 6.99 (m, 1H), 4.47 (d, 0.3H), 4.43 (d, 0.7H), 3.77 (m, 1H), 3.00 (m, 1H), 2.71 (t, 2H), 2.51 (s, 2.1H), 2.50 (s, 0.9H), 2.14 (m, 1H), 1.97 (m, 1H), 1.29-1.24 (m, 6H).

### Example 396

### N'-((2RS,3R)-3-amino-2-hydroxy-5-(isopropylsulfanyl)pentanoyl)-5-methyl-2-thiophenecarbohydrazide

The desired compound was prepared by substituting 5-methylthiophene-2-carboxylic acid for o-toluic acid in Example 369.
MS (ESI) m/e 346 (M+H)⁺;
¹H NMR (500 MHz, CD₃OD) δ 7.61 (d, 0.7H), 7.58 (d, 0.3H), 6.86 (m, 1H), 4.46 (d, 0.3H), 4.42 (d, 0.7H), 3.76 (m, 1H), 3.00 (m, 1H), 2.71 (t, 2H), 2.53(s, 3H), 2.14 (m, 1H), 1.97 (m, 1H), 1.28-1.24 (m, 6H).

### Example 397

### N'-((2RS,3R)-3-amino-2-hydroxy-5-(isopropylsulfanyl)pentanoyl)-1H-pyrrole-2-carbohydrazide

The desired compound was prepared by substituting pyrrole-2-carboxylic acid for o-toluic acid in Example 369.
MS (ESI) m/e 315 (M+H)⁺;
¹H NMR (500 MHz, CD₃OD) δ 6.99 (m, 1H), 6.90 (m, 1H), 6.21 (m, 1H), 4.45 (d, 0.3H), 4.42 (d, 0.7H), 3.76 (m, 1H), 3.00 (m, 1H), 2.71 (t, 2H), 2.13 (m, 1H), 1.97 (m, 1H), 1.29-1.25 (m, 6H).

### Example 398

### N'-((2RS,3R)-3-amino-2-hydroxy-5-(isopropylsulfanyl)pentanoyl)-1-inethy]-1H-pyrrole-2-carbohydrazide

The desired compound was prepared by substituting 1-methylpyrrole-2-carboxylic acid for o-toluic acid in Example 369.
MS (ESI) m/e 329 (M+H)⁺;
¹H NMR (500 MHz, CD₃OD) δ 6.90 (m, 2H), 6.10 (m, 1H), 4.45 (d, 0.3H), 4.42(d, 0.7H), 3.89 (s, 3H), 3.76 (m, 1H), 3.00 (m, 1H), 2.71 (t, 2H), 2.14 (m, 1H), 1.98 (m, 1H), 1.29-1.24 (m, 6H).

### Example 399

### N'-((2RS,3R)-3-amino-2-hydroxy-5-(isopropylsulfanyl)pentanoyl)-1,3-thiazole-2-carbohydrazide

The desired compound was prepared by substituting thiazole-2-carboxylic acid for o-toluic acid in Example 369.
MS (ESI) m/e 333 (M+H)⁺;
¹H NMR (500 MHz, CD₃OD) δ 8.02 (d, 0.6H), 8.00 (d, 0.4H), 7.94 (d, 0.6H), 7.92 (d, 0.4H), 4.49 (d, 0.4H), 4.44 (d, 0.6H), 3.78 (m, 1H), 3.00 (m, 1H), 2.71 (t, 2H), 2.15 (m, 1H), 1.97 (m, 1H), 1.29-1.26 (m, 6H).

### Example 400

### N'-((2RS,3R)-3-amino-2-hydroxy-5-(isopropylsulfanyl)pentanoyl)-1,3-thiazole-4-carbohydrazide

The desired compound was prepared by substituting thiazole-4-carboxylic acid for o-toluic acid in Example 369.
MS (ESI) m/e 333 (M+H)⁺;
¹H NMR (500 MHz, CD₃OD) δ 90.6 (d, 0.7H), 9.04 (d, 0.3H), 8.41 (d, 0.7H), 8.37 (d, 0.3H), 4.48 (d, 0.3H), 4.45 (d, 0.7H), 3.78 (m, 1H), 3.00 (m, 1H), 2.71 (t, 2H), 2.15 (m, 1H), 1.98 (m, 1H), 1.29-1.24 (m, 6H).

### Example 401

### N'-((2RS,3R)-3-amino-2-hydroxy-5-isopropylpentanoyl)-2-pyridinecarbohydrazide

The desired compound was prepared by substituting pyridine-2-carboxylic acid for o-toluic acid in Example 369.
MS (ESI) m/e 327 (M+H)⁺;
¹H NMR (500 MHz, CD₃OD) δ 8.67 (t, 1H), 8.11 (t, 1H), 7.99 (m, 1H), 7.61 (m, 1H), 4.49 (d, 0.3H), 4.45 (d, 0.7H), 3.79 (m, 1H), 3.00 (m, 1H), 2.72 (t, 2H), 2.15 (m, 1H), 1.98 (m, 1H), 1.29-1.24 (m, 6H).

### Example 402

### N'-((2RS,3R)-3-amino-2-hydroxy-5-(isopropylsulfanyl)pentanoyl)-6-chloro-2H-chromene-3-carbohydrazide

The desired compound was prepared by substituting 6-chloro(2H)-1-benzopyran-3-carboxylic acid for o-toluic acid in Example 369.
MS (ESI) m/e 414 (M+H)⁺;
¹H NMR (500 MHz, CD₃OD) δ 7.28 (s, 1H), 7.23 (m, 2H), 6.84 (m, 1H), 4.97 (s, 2H), 4.47 (d, 0.3H), 4.42 (d, 0.7H), 3.75 (m, 1H), 3.00 (m, 1H), 2.71 (t, 2H), 2.13 (m, 1H), 1.96 (m, 1H), 1.28-1.24 (m, 6H).

### Example 403

### N'-((2RS,3R)-3-amino-2-hydroxy-7-(isopropylsulfanyl)pentanoyl)-2-(4-morpholinyl)acetohydrazide

The desired compound was prepared by substituting 1-morpholineacetic acid for o-toluic acid in Example 369.
MS (ESI) m/e 349 (M+H)⁺;
¹H NMR (500 MHz, CD₃OD) δ 4.48 (d, 0.3H), 4.39 (d, 0.7H), 3.91 (m, 6H), 3.73 (m, 1H), 3.27 (m, 4H), 2.98 (m, 1H), 2.70 (t, 2H), 2.11 (m, 1H), 1.94 (m, 1H), 1.27-1.23 (m, 6H).

### Example 404

### N'-((2RS,3R)-3-amino-2-hydroxy-7-(isopropylsulfanyl)pentanoyl)-2-(4-methyl-1-piperazinyl)acetohydrazide

The desired compound was prepared by substituting 4-N-methylpiperazine-1-acetic acid for o-toluic acid in Example 369.
MS (ESI) m/e 362 (M+H)⁺;
¹H NMR (500 MHz, CD₃OD) δ 4.47-4.30 (m, 1H), 3.73 (m, 2H), 3.48 (m, 2H), 3.31 (m, 3H), 3.16 (m, 2H), 3.00 (m, 1H), 2.90 (s, 2H), 2.69 (m, 4H), 2.09 (m, 1H), 1.93 (m, 2H), 1.28-1.22 (m, 6H).

### Example 405

### 1-acetyl-N'-((2RS,3R)-3-amino-2-hydroxy-7-(isopropylsulfanyl)pentanoyl)-4-piperidinecarbohydrazide

The desired compound was prepared by substituting 1-acetylpiperidine-4-carboxylic acid for o-toluic acid in Example 369.
MS (ESI) m/e 375 (M+H)⁺;
¹H NMR (500 MHz, CD₃OD) δ 4.49 (m, 1H), 4.42(d, 0.3H), 4.37 (d, 0.7H), 3.96 (m, 1H), 3.72 (m, 1H), 3.19 (m, 1H), 2.98 (m, 1H), 2.70 (m, 2H), 2.59 (m, 1H), 2.10 (m, 4H), 1.91 (m, 3H), 1.66 (m, 2H), 1.28-1.23 (m, 6H).

### Example 406

### N'-((2RS,3R)-3-amino-2-hydroxy-5-(isopropylsulfanyl)pentanoyl)-4-ethylbenzohydrazide

The desired compound was prepared by substituting 4-ethylbenzoic acid for o-toluic acid in Example 369.
MS (ESI) m/e 354 (M+H)⁺;
¹H NMR (500 MHz, CD₃OD) δ 7.82 (m, 2H), 7.34 (m, 2H), 4.48 (d, 0.3H), 4.44 (d, 0.7H), 3.77 (m, 1H), 3.00 (m, 1H), 2.72 (t, 4H), 2.15 (m, 1H), 1.98 (m, 1H), 1.29-1.24 (m, 9H).

### Example 407

### N-((2RS,3R)-3-amino-2-hydroxy-5-(isopropylsulfanyl)pentanoyl)-3-fluoro-2-methylbenzohydrazide

The desired compound was prepared by substituting 3-fluoro-2-methyl-benzoic acid for o-toluic acid in Example 369.
MS (ESI) m/e 358 (M+H)⁺;
¹H NMR (500 MHz, CD₃OD) δ 7.32 (m, 2H), 7.21 (m, 1H), 4.48 (d, 0.3H), 4.44 (d, 0.7H), 3.79 (m, 1H), 3.00 (m, 1H), 2.72 (t, 2H), 2.36 (m, 3H), 2.16 (m, 1H), 1.98 (m, 1H), 1.29-1.24 (m, 6H).

### Example 408

### N'-((2RS,3R)-3-amino-2-hydroxy-5-(isopropylsulfanyl)pentanoyl)-2,3-difluorobenzohydrazide

The desired compound was prepared by substituting 2, 3-difluorobenzoic acid for o-toluic acid in Example 369.
MS (ESI) m/e 362 (M+H)⁺;
¹H NMR (500 MHz, CD₃OD) δ 7.55 (m, 1H), 7.49 (m, 1H), 7.31 (m, 1H), 4.48 (d, 0.3H), 4.44 (d, 0.7H), 3.78 (m, 1H), 3.00 (m, 1H), 2.71 (t, 2H), 2.15 (m, 1H), 1.97 (m, 1H), 1.29-1.24 (m, 6H).

### Example 409

### N'-((2RS,3R)-3-amino-2-hydroxy-5-(isopropylsulfanyl)pentanoyl)-4-propylbenzotydrazide

The desired compound was prepared by substituting 4-N-propylbenzoic acid for o-toluic acid in Example 369.
MS (ESI) m/e 368 (M+H)⁺;
¹H NMR (500 MHz, CD₃OD) δ 7.81 (m, 1H), 7.32 (m, 1H), 4.48 (d, 0.3H), 4.44 (d, 0.7H), 3.78 (m, 1H), 3.00 (m, 1H), 2.70 (t, 4H), 2.15 (m, 1H), 1.98 (m, 1H), 1.68 (m, 2H), 1.29-1.24 (m, 6H), 0.95 (t, 1H).

### Example 410

### N'-((2RS,3R)-3-amino-2-hydroxy-5-(isopropylsulfanyl)pentanoyl)-4-isopropylbenzohydrazide

The desired compound was prepared by substituting 4-isopropylbenzoic acid for o-toluic acid in Example 369.
MS (ESI) m/e 368 (M+H)⁺;
¹H NMR (500 MHz, CD₃OD) δ 7.82 (m, 2H), 7.38 (m, 2H), 4.48 (d, 0.3H), 4.44 (d, 0.7H), 3.78 (m, 1H), 3.00 (m, 2H), 2.72 (t, 2H), 2.15 (m, 1H), 1.98 (m, 1H), 1.29-1.24 (m, 12H).

### Example 411

### N'-((2RS,3R)-3-amino-2-hydroxy-5-(isopropylsulfanyl)pentanoyl)-2-ethoxybenzohydrazide

The desired compound was prepared by substituting 2-ethoxybenzoic acid for o-toluic acid in Example 369.
MS (ESI) m/e 370 (M+H)⁺;
¹H NMR (500 MHz, CD₃OD) δ 7.97 (m, 1H), 7.54 (m, 1H), 7.18 (m, 1H), 7.09 (t, 1H), 4.49 (d, 0.3H), 4.44 (d, 0.7H), 4.29 (q, 2H), 3.78 (m, 1H), 3.00 (m, 1H), 2.71 (t, 2H), 2.15 (m, 1H), 1.97 (m, 1H), 1.51 (t, 3H), 1.29-1.24 (m, 6H).

### Example 412

### N'-((2RS,3R)-3-amino-2-hydroxy-5-(isopropylsulfanyl)pentanoyl)-4-ethoxybenzohydrazide

The desired compound was prepared by substituting 4-ethoxybenzoic acid for o-toluic acid in Example 369.
MS (ESI) m/e 370 (M+H)⁺;
¹H NMR (500 MHz, CD₃OD) δ 7.85 (m, 1H), 6.99 (m, 1H), 7.31 (m, 1H), 4.48 (d, 0.3H), 4.44 (d, 0.7H), 4.12 (q, 2H), 3.77 (m, 1H), 3.00 (m, 1H), 2.71 (t, 2H), 2.15 (m, 1H), 1.98 (m, 1H), 1.41 (t, 3H), 1.29-1.24 (m, 6H).

### Example 413

### N'-(2RS,3R)-3-amino-2-hydroxy-5-(isopropylsulfanyl)pentanoyl)-1-naphthohydrazide

The desired compound was prepared by substituting 1-naphthoic acid for o-toluic acid in Example 369.
MS (ESI) m/e 376 (M+H)⁺;
¹H NMR (500 MHz, CD₃OD) δ 7.38 (m, 1H), 8.04 (t, 1H), 7.95 (m, 1H), 7.77 (t, 1H), 7.56 (m, 3H), 4.53 (d, 0.3H), 4.49 (d, 0.7H), 3.84 (m, 1H), 3.02 (m, 1H), 2.74 (t, 2H), 2.19 (m, 1H), 2.01 (m, 1H), 1.31-1.24 (m, 6H).

### Example 414

### N'-((2RS,3R)-3-amino-2-hydroxy-5-(isopropylsulfanyl)pentanoyl)-4-tert-butylbenzohydrazide

The desired compound was prepared by substituting 4-tert-butylbenzoic acid for o-toluic acid in Example 369.
MS (ESI) m/e 382 (M+H)⁺;
¹H NMR (500 MHz, CD₃OD) δ 7.83 (m, 2H), 7.55 (m, 2H), 4.48 (d, 0.3H), 4.44 (d, 0.7H), 3.78 (m, 1H), 3.00 (m, 1H), 2.72 (t, 2H), 2.15 (m, 1H), 1.99 (m, 1H), 1.35 (s, 9H), 1.29-1.24 (m, 6H).

### Example 415

### N-(4-((2-((2RS,3R)-3-amino-2-hydroxy-5-(isopropylsulfanyl)pentanoyl)hydrazino)carbonyl)phenyl)acetamide

The desired compound was prepared by substituting 4-acetamidobenzoic acid for o-toluic acid in Example 369.
MS (ESI) m/e 383 (M+H)⁺;
¹H NMR (500 MHz, CD₃OD) δ 7.86 (m, 2H), 7.71 (m, 2H), 4.48 (d, 0.3H), 4.44 (d, 0.7H), 3.78 (m, 1H), 3.00 (m, 1H), 2.72 (t, 2H), 2.15 (m, 4H), 1.97 (m, 1H), 1.29-1.24 (m, 6H).

### Example 416

### N'-((2RS,3R)-3-amino-2-hydroxy-5-(isopropylsulfanyl)pentanoyl)-4-propoxybenzohydrazide

The desired compound was prepared by substituting p-propoxybenzoic acid for o-toluic acid in Example 369.
MS (ESI) m/e 384 (M+H)⁺;
¹H NMR (500 MHz, CD₃OD) δ 7.85 (m, 2H), 7.00 (m, 2H), 7.31 (m, 1H), 4.48 (d, 0.3H), 4.44 (d, 0.7H), 4.01 (m,2H), 3.78 (m, 1H), 3.00 (m, 1H), 2.71 (t, 2H), 2.15 (m, 1H), 1.97 (m, 1H), 1.82 (m, 2H), 1.29-1.24 (m, 6H), 1.05 (t, 3H).

### Example 417

### N'-((2RS,3R)-3-amino-2-hydroxy-5-(isopropylsulfanyl)pentanoyl)-4 isopropoxybenzohydrazide

The desired compound was prepared by substituting 4-isopropoxybenzoic acid for o-toluic acid in Example 369.
MS (ESI) m/e 384 (M+H)⁺;
¹H NMR (500 MHz, CD₃OD) δ 7.84 (m, 2H), 6.98 (m, 2H), 4.71 (m, 1H), 4.48 (d, 0.3H), 4.44 (d, 0.7H), 3.78 (m, 1H), 3.00 (m, 1H), 2.71 (t, 2H), 2.14 (m, 1H), 1.98 (m, 1H), 1.34 (d, 6H), 1.29-1.24 (m, 6H).

### Example 418

### N'-((2RS,3R)-3-amino-2-hydroxy-5-(isopropylsulfanyl)pentanoyl)-5-chloro-2-methoxybenzohydrazide

The desired compound was prepared by substituting 5-chloro-2-methoxybenzoic acid for o-toluic acid in Example 369.
MS (ESI) m/e 390 (M+H)⁺;
¹H NMR (500 MHz, CD₃OD) δ 7.92 (d, 0.7H), 7.89 (d, 0.3H), 7.55 (td, 1H), 7.20 (m, 1H), 4.47 (d, 0.3H), 4.44 (d, 0.7H), 4.00 (s, 2.1H), 3.98 (s, 0.9H), 3.78 (m, 1H), 3.00 (m, 1H), 2.72 (t, 2H), 2.14 (m, 1H), 1.98 (m, 1H), 1.29-1.25 (m, 6H).

### Example 419

### N'-((2RS,3R)-3-amino-2-hydroxy-5-(isopropylsulfanyl)pentanoyl)-4-(methylsulfonyl)benzohydrazide

The desired compound was prepared by substituting 4-(methylsulfonyl)benzoic acid for o-toluic acid in Example 369.
MS (ESI) m/e 404 (M+H)⁺;
¹H NMR (500 MHz, CD₃OD) δ 8.10 (s, 2.8H), 8.09 (s, 1.2H), 4.51 (d, 0.3H), 4.46 (d, 0.7H), 3.78 (m, 1H), 3.17 (s, 2.8H), 3.17 (s, 1.2H), 3.01 (m, 1H), 2.72 (t, 2H), 2.16 (m, 1H), 1.98 (m, 1H), 1.29-1.24 (m, 6H).

### Example 420

### N'-((2RS,3R)-3-amino-2-hydroxy-5-(isopropylsulfanyl)pentanoyl)-2-chloro-5-(methylsulfanyl)benzohydrazide

The desired compound was prepared by substituting 2-chloro-5-(methylthio)benzoic acid for o-toluic acid in Example 369.
MS (ESI) m/e 406 (M+H)⁺;
¹H NMR (500 MHz, CD₃OD) δ 7.49 (d, 1H), 7.43-7.35 (m, 2H), 4.48 (d, 0.3H), 4.43 (d, 0.7H), 3.78 (m, 1H), 3.00 (m, 1H), 2.72 (t, 2H), 2.52 (s, 1H), 2.15 (m, 1H), 1.97 (m, 1H), 1.29-1.24 (m, 6H).

### Example 421

### N'-((2RS,3R)-3-amino-2-hydroxy-5-(isopropylsulfanyl)pentanoyl)-3,4-diethoxybenzohydrazide

The desired compound was prepared by substituting 3,4-diethoxybenzoic acid for o-toluic acid in Example 369.
MS (ESI) m/e 414 (M+H)⁺;
¹H NMR (500 MHz, CD₃OD δ 7.51 (m, 1H), 7.48 (m, 1H), 7.02 (m, 1H), 4.47 (d, 0.3H), 4.44 (d, 0.7H), 4.12 (m, 4H), 3.77 (m, 1H), 3.00 (m, 1H), 2.72 (t, 2H), 2.15 (m, 1H), 1.98 (m, 1H), 1.43 (m, 6H), 1.29-1.24 (m, 6H).

### Example 422

### N'-((2RS,3R)-3-amino-2-hydroxy-5-(isopropylsulfanyl)pentanoyl)-2-benzylbenzohydrazide

The desired compound was prepared by substituting alpha-phenyl-o-toluic acid for o-toluic acid in Example 369.
MS (ESI) m/e 416 (M+H)⁺;
¹H NMR (500 MHz, CD₃OD) δ 7.54 (t, 1H), 7.40 (m, 1H), 7.29 (t, 1H), 7.23 (m, 5H), 7.16 (m, 1H), 4.49 (d, 0.3H), 4.43 (d, 0.7H), 4.19 (m, 1H), 3.78 (m, 1H), 3.00 (m, 1H), 2.71 (t, 2H), 2.15 (m, 1H), 1.97 (m, 1H), 1.29-1.19 (m, 6H).

### Example 423

### N'-((2RS,3R)-3-amino-2-hydroxy-5-(isopropylsulfanyl)pentanoyl)-2-anilinobenzohydrazide

The desired compound was prepared by substituting N-phenylanthranilic acid for o-toluic acid in Example 369.
MS (ESI) m/e 417 (M+H)⁺;
¹H NMR (500 MHz, CD₃OD) δ 7.68 (m, 1H), 7.32 (m, 4H), 7.16 (d, 2H), 7.02 (m, 1H), 6.83 (m, 1H), 4.49 (d, 0.3H), 4.44 (d, 0.7H), 3.78 (m, 1H), 3.00 (m, 1H), 2.71 (t, 2H), 2.16 (m, 1H), 1.98 (m, 1H), 1.28-1.19 (m, 6H).

### Example 424

### N'-((2RS,3R)-3-amino-2-hydroxy-5-(isopropylsulfanyl)pentanoyl)-2-(2 phenylethyl)benzohydrazide

The desired compound was prepared by substituting 2-bibenzylcarboxylic acid for o-toluic acid in Example 369.
MS (ESI) m/e 430 (M+H)⁺;
¹H NMR (500 MHz, CD₃OD) δ 7.53 (m, 1H), 7.40 (m, 1H), 7.28 (m, 2H), 7.23 (d, 4H), 7.15 (m, 1H), 4.50 (d, 0.3H), 4.45 (d, 0.7H), 3.81 (m, 1H), 3.10 (m, 2H), 3.01 (m, 1H), 2.93 (m, 2H), 2.72 (t, 2H), 2.17 (m, 1H), 1.99 (m, 1H), 1.29-1.20 (m, 6H).

### Example 425

### N'-((2RS,3R)-3-amino-2-hydroxy-5-(isopropylsulfanyl)pentanoyl)-3-(methylsulfanyl)benzohydrazide

The desired compound was prepared by substituting 3-(methylthio)benzoic acid for o-toluic acid in Example 369.
MS (ESI) m/e 372 (M+H)⁺;
¹H NMR (500 MHz, CD₃OD) δ 7.77 (m, 1H), 7.63 (m, 1H), 7.48 (m, 1H), 7.42 (m, 1H), 4.49 (d, 0.3H), 4.45 (d, 0.7H), 3.78 (m, 1H), 3.00 (m, 1H), 2.72 (t, 2H), 2.53 (s, 3H), 2.15 (m, 1H), 1.98 (m, 1H), 1.29-1.24 (m, 6H).

### Example 426

### N'-((2RS,3R)-3-amino-2-hydroxy-5-(isopropylsulfanyl)pentanoyl)-3-(ethylsulfanyl)benzohydrazide

The desired compound was prepared by substituting 3-(ethylthio)benzoic acid for o-toluic acid in Example 369.
MS (ESI) m/e 386 (M+H)⁺;
¹H NMR (500 MHz, CD₃OD) δ 7.83 (m, 1H), 7.67 (m, 1H), 7.54 (m, 1H), 7.43 (m, 1H), 4.49 (d, 0.3H), 4.45 (d, 0.7H), 3.78 (m, 1H), 3.00 (m, 3H), 2.72 (t, 2H), 2.15 (m, 1H), 1.98 (m, 1H), 1.33-1.25 (m, 9H).

### Example 427

### N'-((2RS,3R)-3-amino-2-hydroxy-5-(isopropylsulfanyl)pentanoyl)-3-(propylsulfanyl)benzohydrazide

The desired compound was prepared by substituting 3-(propylthio)benzoic acid for o-toluic acid in Example 369.
MS (ESI) m/e 400 (M+H)⁺;
¹H NMR (500 MHz, CD₃OD) δ 7.83 (m, 1H), 7.66 (t, 1H), 7.54 (m, 1H), 7.42 (m, 1H), 4.49 (d, 0.3H), 4.45 (d, 0.7H), 3.78 (m, 1H), 3.00 (m, 3H), 2.72 (t, 2H), 2.15 (m, 1H), 1.98 (m, 1H), 1.68 (m, 2H), 1.29-1.24 (m, 6H), 1.04 (t, 3H).

### Example 428

### N'-((2RS,3R)-3-amino-2-hydroxy-5-(isopropylsulfanyl)pentanoyl)-3- (butylsulfanyl)benzohydrazide

The desired compound was prepared by substituting 3-(butylthio)benzoic acid for o-toluic acid in Example 369.
MS (ESI) m/e 414 (M+H)⁺;
¹H NMR (500 MHz, CD₃OD δ 7.83 (m, 1H), 7.66 (t, 1H), 7.54 (t, 1H), 7.42 (m, 1H), 4.49 (d, 0.3H), 4.45 (d, 0.7H), 3.78 (m, 1H), 3.00 (m, 3H), 2.72 (t, 2H), 2.15 (m, 1H), 1.98 (m, 1H), 1.64 (m, 2H), 1.49 (m, 2H), 1.29-1.24 (m, 6H), 0.94 (t, 3H).

### Example 429

### N'-((2RS,3R)-3-amino-2-hydroxy-5-(isopropylsulfanyl)pentanoyl)-3- (hexylsulfanyl)benzohydrazide

The desired compound was prepared by substituting 3-(hexylthio)benzoic acid for o-toluic acid in Example 369.
MS (ESI) m/e 442 (M+H)⁺;
¹H NMR (500 MHz, CD₃OD) δ 7.83 (m, 1H), 7.66 (t, 1H), 7.54 (m, 1H), 7.42 (m, 1H), 4.48 (d, 0.3H), 4.44 (d, 0.7H), 3.78 (m, 1H), 3.00 (m, 3H), 2.72 (t, 2H), 2.15 (m, 1H), 1.98 (m, 1H), 1.65 (m, 2H), 1.46 (m, 2H), 1.32-1.24 (m, 10H), 0.90 (t, 3H).

### Example 430

### N'-((2RS,3R)-3-amino-2-hydroxy-5-(isopropylsulfanyl)pentanoyl)-3-(isopropylsulfanyl)benzohydrazide

The desired compound was prepared by substituting 3-(isopropylthio)benzoic acid for o-toluic acid in Example 369.
MS (ESI) m/e 400 (M+H)⁺;
¹H NMR (500 MHz, CD₃OD) δ 7.90 (m, 1H), 7.73 (m, 1H), 7.61 (m, 1H), 7.45 (m, 1H), 4.49 (d, 0.3H), 4.45 (d, 0.7H), 3.78 (m, 1H), 3.51 (m, 1H), 3.00 (m, 1H), 2.72 (t, 2H), 2.15 (m, 1H), 1.98 (m, 1H), 1.30-1.24 (m, 12H).

### Example 431

### N'-((2RS,3R)-3-amino-2-hydroxy-5-(isopropylsulfanyl)pentanoyl)-3-(isobutylsulfanyl)benzohydrazide

The desired compound was prepared by substituting 3-(2-methylpropylthio)benzoic acid for o-toluic acid in Example 369.
MS (ESI) m/e 414 (M+H)⁺;
¹H NMR (500 MHz, CD₃OD) δ 7.84 (m, 1H), 7.65 (m, 1H), 7.54 (m, 1H), 7.42 (m, 1H), 4.49 (d, 0.3H), 4.45 (d, 0.7H), 3.78 (m, 1H), 3.00 (m, 1H), 2.90 (d, 2H), 2.72 (t, 2H), 2.15 (m, 1H), 1.98 (m, 1H), 1.86 (m, 1H), 1.29-1.24 (m, 6H), 1.05 (d, 6H).

### Example 432

### N'-((2RS,3R)-3-amino-2-hydroxy-5-(isopropylsulfanyl)pentnoyl)-3-((4-methylpentyl)sulfanyl)benzohydrazide

The desired compound was prepared by substituting 3-(4-methylpentylthio)benzoic acid for o-toluic acid in Example 369.
MS (ESI) m/e 442 (M+H)⁺;
¹H NMR (500 MHz, CD₃OD) δ 7.83 (m, 1H), 7.66 (m, 1H), 7.54 (m, 1H), 7.42 (m, 1H), 4.49 (d, 0.3H), 4.44 (d, 0.7H), 3.78 (m, 1H), 3.00 (m, 3H), 2.72 (t, 2H), 2.15 (m, 1H), 1.98 (m, 1H), 1.66 (m, 2H), 1.56 (m, 1H), 1.34 (m, 2H), 1.29-1.24 (m, 6H), 0.89 (d, 6H).

### Example 433

### N'-((2RS,3R)-3-amino-2-hydroxy-5-(isopropylsulfanyl)pentanoyl)-3-(sec-butylsulfanyl)benzohydrazide

The desired compound was prepared by substituting 3-(1-methylpropylthio)benzoic acid for o-toluic acid in Example 369.
MS (ESI) m/e 414 (M+H)⁺;
¹H NMR (500 MHz, CD₃OD) δ 7.90 (m, 1H), 7.72 (m, 1H), 7.61 (m, 1H), 7.44 (m, 1H), 4.49 (d, 0.3H), 4.45 (d, 0.7H), 3.78 (m, 1H), 3.31 (m, 1H), 3.00 (m, 1H), 2.72 (t, 2H), 2.15 (m, 1H), 1.98 (m, 1H), 1.62 (m, 2H), 1.29-1.24 (m, 9H), 1.03 (t, 3H).

### Example 434

### N'-((2RS,3R)-3-amino-2-hydroxy-5-(isopropylsulfanyl)pentanoyl)-3-(neopentylsulfanyl)benzohydrazide

The desired compound was prepared by substituting 3-(2,2-dimethylpropylthio)benzoic acid for o-toluic acid in Example 369.
MS (ESI) m/e 428 (M+H)⁺;
¹H NMR (500 MHz, CD₃OD) δ 7.86 (m, 1H), 7.65 (m, 1H), 7.57 (m, 1H), 7.40 (m, 1H), 4.49 (d, 0.3H), 4.45 (d, 0.7H), 3.78 (m, 1H), 3.00 (m, 3H), 2.72 (t, 2H), 2.15 (m, 1H), 1.98 (m, 1H), 1.29-1.24 (m, 6H), 1.05 (s, 9H).

### Example 435

### N'-((2RS,3R)-3-amino-2-hydroxy-5-(isopropylsulfanyl)pentanoyl)-3-(cyclohexylsulfanyl)benzohydrazide

The desired compound was prepared by substituting 3-(cyclohexylthio)benzoic acid for o-toluic acid in Example 369.
MS (ESI) m/e 440 (M+H)⁺;
¹H NMR (500 MHz, CD₃OD) δ 7.90 (m, 1H), 7.72 (m, 1H), 7.60 (m, 1H), 7.43 (m, 1H), 4.49 (d, 0.3H), 4.45 (d, 0.7H), 3.78 (m, 1H), 3.25 (m, 1H), 3.00 (m, 1H), 2.72 (t, 2H), 2.15 (m, 1H), 1.98 (m, 3H), 1.78 (m, 2H), 1.65 (m, 2H), 1.38 (m, 4H), 1.29-1.24 (m, 6H).

### Example 436

### N'-((2RS,3R)-3-amino-2-hydroxy-5-(isopropylsulfanyl)pentanoyl)-3-((cyclohexylmethyl)sulfanyl)benzohydrazide

The desired compound was prepared by substituting 3-(2-cyclohexylethylthio)benzoic acid for o-toluic acid in Example 369.
MS (ESI) m/e 454 (M+H)⁺;
¹H NMR (500 MHz, CD₃OD) δ 7.82 (m, 1H), 7.65 (t, 1H), 7.53 (t, 1H), 7.41 (m, 1H), 4.48 (d, 0.3H), 4.44 (d, 0.7H), 3.78 (m, 1H), 3.00 (m, 1H), 2.90 (d, 2H), 2.72 (t, 2H), 2.15 (m, 1H), 1.98 (m, 1H), 1.91 (m, 2H), 1.74 (m, 2H), 1.66 (m, 2H), 1.53 (m, 1H), 1.29-1.18 (m, 8H), 1.05 (m, 2H).

### Example 437

### N'-((2RS,3R)-3-amino-2-hydroxy-5-(isopropylsulfanyl)pentanoyl)-3-(benzylsulfanyl)benzohydrazide

The desired compound was prepared by substituting 3-(benzylthio)benzoic acid for o-toluic acid in Example 369.
MS (ESI) m/e 448 (M+H)⁺;
¹H NMR (500 MHz, CD₃OD) δ 7.86 (m, 1H), 7.67 (m, 1H), 7.52 (m, 1H), 7.39 (m, 1H), 7.31 (d, 2H), 7.26 (t, 2H), 7.20 (m, 1H), 4.49 (d, 0.3H), 4.45 (d, 0.7H), 4.22 (s, 2H), 3.77 (m, 1H), 3.00 (m, 1H), 2.72 (t, 2H), 2.15 (m, 1H), 1.98 (m, 1H), 1.29-1.25 (m, 6H).

### Example 438

### N'-((2RS,3R)-3-amino-2-hydroxy-5-(isopropylsulfanyl)pentanoyl)-3-((2-phenylethyl)sulfanyl)benzohydrazide

The desired compound was prepared by substituting 3-(2-phenylethylthio)benzoic acid for o-toluic acid in Example 369.
MS (ESI) m/e 462 (M+H)⁺;
¹H NMR (500 MHz, CD₃OD) δ 7.87 (m, 1H), 7.68 (m, 1H), 7.57 (m, 1H), 7.44 (m, 1H), 7.27 (t, 2H), 7.23-7.17 (m, 3H), 4.49 (d, 0.3H), 4.45 (d, 0.7H), 3.77 (m, 1H), 3.26 (t, 2H), 3.00 (m, 1H), 2.93 (t, 2H), 2.72 (t, 2H), 2.15 (m, 1H), 1.98 (m, 1H), 1.29-1.24 (m, 6H).

### Example 439

### N'-((2RS,3R)-3-amino-2-hydroxy-5-(isopropylsulfanyl)pentanoyl)-3-((3-phenylpropyl)sulfanyl)benzohydrazide

The desired compound was prepared by substituting 3-(3-phenylpropylthio)benzoic acid for o-toluic acid in Example 369.
MS (ESI) m/e 476 (M+H)⁺;
¹H NMR (500 MHz, CD₃OD δ 7.84 (m, 1H), 7.67 (m, 1H), 7.51 (m, 1H), 7.41 (m, 1H), 7.25 (t, 2H), 7.15 (m, 3H), 4.49 (d, 0.3H), 4.44 (d, 0.7H), 3.78 (m, 1H), 3.00 (m, 3H), 2.76 (t, 2H), 2.72 (t, 2H), 2.15 (m, 1H), 1.96 (m, 3H), 1.29-1.24 (m, 6H).

### Example 440

### N'-((2RS,3R)-3-amino-2-hydroxy-5-(isopropylsulfanyl)pentanoyl)-3-(((1,1'-biphenyl)-4-ylmethyl)sulfanyl)benzohydrazide

The desired compound was prepared by substituting 3-(biphenylmethylthio)benzoic acid for o-toluic acid in Example 369.
MS (ESI) m/e 524 (M+H)⁺;
¹H NMR (500 MHz, CD₃OD δ 7.89 (s, 1H), 7.68 (m, 1H), 7.55 (m, 5H), 7.40 (m, 5H), 7.31 (m, 1H), 4.48 (d, 0.3H), 4.44 (d, 0.7H), 4.27 (m, 2H), 3.77 (m, 1H), 3.00 (m, 1H), 2.71 (t, 2H), 2.15 (m, 1H), 1.98 (m, 1H), 1.29-1.24 (m, 6H).

### Example 441

### N'-((2RS,3R)-3-amino-2-hydroxy-5-(isopropylsulfanyl)pentanoyl)-4-(methylsulfanyl)benzohydrazide

The desired compound was prepared by substituting 4-(methylthio)benzoic acid for o-toluic acid in Example 369.
MS (ESI) m/e 372 (M+H)⁺;
¹H NMR (500 MHz, CD₃OD) δ 7.81 (m, 2H), 7.34 (m, 2H), 4.49 (d, 0.3H), 4.44 (d, 0.7H), 3.77 (m, 1H), 3.00 (m, 1H), 2.72 (t, 2H), 2.53 (s, 3H), 2.15 (m, 1H), 1.98 (m, 1H), 1.29-1.25 (m, 6H).

### Example 442

### N'-((2RS,3R)-3-amino-2-hydroxy-5-(isopropylsulfanyl)pentanoyl)-4-(ethylsulfanyl)benzohydrazide

The desired compound was prepared by substituting 4-(ethylthio)benzoic acid for o-toluic acid in Example 369.
MS (ESI) m/e 386 (M+H)⁺;
¹H NMR (500 MHz, CD₃OD) δ 7.81 (m, 2H), 7.38 (m, 2H), 4.48 (d, 0.3H), 4.44 (d, 0.7H), 3.77 (m, 1H), 3.03 (m, 3H), 2.72 (t, 2H), 2.15 (m, 1H),1.98 (m, 1H), 1.35 (t, 3H), 1.29-1.25 (m, 6H).

### Example 443

### N'-((2RS,3R)-3-amino-2-hydroxy-5-(isopropylsulfanyl)pentanoyl)-4-(propylsulfanyl)benzohydrazide

The desired compound was prepared by substituting 4-(propylthio)benzoic acid for o-toluic acid in Example 369.
MS (ESI) m/e 400 (M+H)⁺;
¹H NMR (500 MHz, CD₃OD) δ 7.80 (m, 2H), 7.38 (m, 2H), 4.48 (d, 0.3H), 4.44 (d, 0.7H), 3.77 (m, 1H), 3.01 (m, 3H), 2.71 (t, 2H), 2.15 (m, 1H), 1.98 (m, 1H), 1.72 (m, 2H), 1.29-1.24 (m, 6H), 1.06 (t, 3H).

### Example 444

### N'-((2RS,3R)-3-amino-2-hydroxy-5-(isopropylsulfanyl)pentanoyl)-4-(butylsulfanyl)benzohydrazide

The desired compound was prepared by substituting 4-(butylthio)benzoic acid for o-toluic acid in Example 369.
MS (ESI) m/e 414 (M+H)⁺;
¹H NMR (500 MHz, CD₃OD) δ 7.80 (m, 2H), 7.37 (m, 2H), 4.48 (d, 0.3H), 4.44 (d, 0.7H), 3.77 (m, 1H), 3.02 (m, 3H), 2.71 (t, 2H), 2.15 (m, 1H), 1.98 (m, 1H), 1.67 (m, 2H), 1.50 (m, 2H), 1.29-1.24 (m, 6H), 0.95 (t, 3H).

### Example 445

### N'-((2RS,3R)-3-amino-2-hydroxy-5-(isopropylsulfanyl)pentanoyl)-4-(hexylsulfanyl)benzohydrazide

The desired compound was prepared by substituting 4-(hexylthio)benzoic acid for o-toluic acid in Example 369.
MS (ESI) m/e 442 (M+H)⁺;
¹H NMR (500 MHz, CD₃OD) δ 7.80 (m, 2H), 7.37 (t, 2H), 4.48 (d, 0.3H), 4.44 (d, 0.7H), 3.77 (m, 1H), 3.02 (m, 3H), 2.72 (t, 2H), 2.15 (m, 1H), 1.98 (m, 1H), 1.68 (m, 2H), 1.47 (m, 2H), 1.33 (m, 4H), 1.29-1.24 (m, 6H), 0.91 (t, 3H).

### Example 446

### N'-((2RS,3R)-3-amino-2-hydroxy-5-(isopropylsulfanyl)pentanoyl)-4-(isopropylsulfanyl)benzohydrazide

The desired compound was prepared by substituting 4-(isopropylthio)benzoic acid for o-toluic acid in Example 369.
MS (ESI) m/e 400 (M+H)⁺;
¹H NMR (500 MHz, CD₃OD) δ 7.81 (m, 2H), 7.43 (dd, 2H), 4.48 (d, 0.3H), 4.44 (d, 0.7H), 3.77 (m, 1H), 3.62 (m, 1H), 3.00 (m, I H), 2.72 (t, 2H), 2.15 (m, 1H), 1.98 (m, 1H), 1.34 (d, 6H), 1.29-1.24 (m, 6H).

### Example 447

### N'-((2RS,3R)-3-amino-2-hydroxy-5-(isopropylsulfanyl)pentanoyl)-4-(isobutylsulfanyl)benzohydrazide

The desired compound was prepared by substituting 4-(2-methylpropylthio)benzoic . acid for o-toluic acid in Example 369.
MS (ESI) m/e 414 (M+H)⁺;
¹H NMR (500 MHz, CD₃OD) δ 7.80 (m, 2H), 7.38 (m, 2H), 4.48 (d, 0.3H), 4.44 (d, 0.7H), 3.78 (m, 1H), 3.00 (m, 1H), 2.92 (d, 2H), 2.71 (t, 2H), 2.15 (m, 1H), 1.98 (m, 1H), 1.90 (m, 1H), 1.29-1.24 (m, 6H), 1.06 (d, 6H).

### Example 448

### N'-((2RS,3R)-3-amino-2-hydroxy-5-(isopropylsulfanyl)pentnoyl)-4-((4-methylpentyl)sulfanyl)benzohydrazide

The desired compound was prepared by substituting 4-(4-methylpentylthio)benzoic acid for o-toluic acid in Example 369.
MS (ESI) m/e 442 (M+H)⁺;
¹H NMR (500 MHz, CD₃OD) δ 7.80 (m, 2H), 7.38 (m, 2H), 4.45 (d, 0.3H), 4.41 (d, 0.7H), 3.71 (m, 1H), 3.01 (m, 3H), 2.71 (t, 2H), 2.13 (m, 1H), 1.96 (m, 1H), 1.69 (m, 2H), 1.57 (m, 1H), 1.36 (m, 2H), 1.29-1.24 (m, 6H), 0.89 (d, 6H).

### Example 449

### N'-((2RS,3R)-3-amino-2-hydroxy-5-(isopropylsulfanyl)pentanoyl)-4-(sec-butylsulfanyl)benzohydrazide

The desired compound was prepared by substituting 4-(1-methylpropylthio)benzoic acid for o-toluic acid in Example 369.
MS (ESI) m/e 414 (M+H)⁺;
¹H NMR (500 MHz, CD₃OD) δ 7.80 (m, 2H), 7.43 (dd, 2H), 4.49 (d, 0.3H), 4.44 (d, 0.7H), 3.77 (m, 1H), 3.00 (m, 1H), 2.72 (t, 2H), 2.15 (m, 1H), 1.98 (m, 1H), 1.65 (m, 2H), 1.33 (d, 3H), 1.29-1.24 (m, 6H), 1.04 (t, 3H).

### Example 450

### N'-((2RS,3R)-3-amino-2-hydroxy-5-(isopropylsulfanyl)pentanoyl)-4-(neopentylsulfanyl)benzohydrazide

The desired compound was prepared by substituting 4-(2, 2-dimethylpropylthio)benzoic acid for o-toluic acid in Example 369.
MS (ESI) m/e 428 (M+H)⁺;
¹H NMR (500 MHz, CD₃OD) δ 7.79 (m, 2H), 7.42 (m, 2H), 4.48 (d, 0.3H), 4.44 (d, 0.7H), 3.77 (m, 1H), 3.00 (m, 3H), 2.72 (t, 2H), 2.14 (m, 1H), 1.98 (m, 1H), 1.29-1.24 (m, 6H), 1.06 (s, 9H).

### Example 451

### N'-((2RS,3R)-3-amino-2-hydroxy-5-(isopropylsulfanyl)pentanoyl)-4-(cyclohexylsulfanyl)benzohydrazide

The desired compound was prepared by substituting 4-(cyclohexylthio)benzoic acid for o-toluic acid in Example 369.
MS (ESI) m/e 440 (M+H)⁺;
¹H NMR (500 MHz, CD₃OD δ 7.80 (m, 2H), 7.41 (m, 2H), 4.49 (d, 0.3H), 4.44 (d, 0.7H), 3.78 (m, 1H), 3.37 (m, 1H), 3.00 (m, 1H), 2.72 (t, 2H), 2.15 (m, 1H), 1.98 (m, 3H), 1.79 (m, 2H), 1.65 (m, 2H), 1.39 (m, 4H), 1.29-1.24 (m, 6H).

### Example 452

### N'-((2RS,3R)-3-amino-2-hydroxy-5-(isopropylsulfanyl)pentanoyl)-4-((cyclohexylmethyl)sulfanyl)benzohydrazide

The desired compound was prepared by substituting 4-(2-cyclohexylethylthio)benzoic acid for o-toluic acid in Example 369.
MS (ESI) m/e 454 (M+H)⁺;
¹H NMR (500 MHz, CD₃OD) δ 7.79 (m, 2H), 7.37 (m, 1H), 4.48 (d, 0.3H), 4.44 (d, 0.7H), 3.77 (m, 1H), 3.00 (m, 1H), 2.91 (d, 2H), 2.72 (t, 2H), 2.15 (m, 1H), 1.98 (m, 1H), 1.92 (m, 2H), 1.75 (m, 2H), 1.67 (m, 2H), 1.56 (m, 1H), 1.29-1.21 (m, 8H), 1.06 (m, 2H).

### Example 453

### N'-((2RS,3R)-3-amino-2-hydroxy-5-(isopropylsulfanyl)pentanoyl)-4-(benzylsulfanyl)benzohydrazide

The desired compound was prepared by substituting 4-(benzylthio)benzoic acid for o-toluic acid in Example 369.
MS (ESI) m/e 448 (M+H)⁺;
¹H NMR (500 MHz, CD₃OD) δ 7.77 (m, 2H), 7.39 (m, 4H), 7.28 (t, 2H), 7.22 (m, 1H), 4.48 (d, 0.3H), 4.43 (d, 0.7H), 4.27 (s, 0.7H), 4.26 (s, 0.3H), 3.77 (m, 1H), 3.00 (m, 1H), 2.72 (t, 2H), 2.15 (m, 1H), 1.98 (m, 1H), 1.29-1.23 (m, 6H).

### Example 454

### N'-((2RS,3R)-3-amino-2-hydroxy-5-(isopropylsulfanyl)pentanoyl)-4-((2-phenylethyl)sulfanyl)benzohydrazide

The desired compound was prepared by substituting 4-(2-phenylethylthio)benzoic acid for o-toluic acid in Example 369.
MS (ESI) m/e 462 (M+H)⁺;
¹H NMR (500 MHz, CD₃OD) δ 7.82 (m, 2H), 7.41 (m, 2H), 7.28 (m, 2H), 7.21 (m, 3H), 7.27 (t, 2H), 7.23-7.17 (m, 3H), 4.48 (d, 0.3H), 4.44 (d, 0.7H), 3.77 (m, 1H), 3.28 (m, 2H), 3.00 (m, 1H), 2.96 (t, 2H), 2.72 (t, 2H), 2.15 (m, 1H), 1.98 (m, 1H), 1.29-1.24 (m, 6H).

### Example 456

### N'-((2RS,3R)-3-amino-2-hydroxy-5-(isopropylsulfanyl)pentanoyl)-4-((3-phenylpropyl)sulfanyl)benzohydrazide

The desired compound was prepared by substituting 4-(3-phenylpropylthio)benzoic acid for o-toluic acid in Example 369.
MS (ESI) m/e 476 (M+H)⁺;
¹H NMR (500-MHz, CD₃OD) δ 7.78 (m, 2H), 7.33 (m, 2H), 7.27 (m, 2H), 7.18 (m, 3H), 4.48 (d, 0.3H), 4.44 (d, 0.7H), 3.77 (m, 1H), 3.01 (m, 3H), 2.77 (t, 2H), 2.71 (t, 2H), 2.15 (m, 1H), 1.98 (m, 3H), 1.29-1.24 (m, 6H).

### Example 457

### N'-((2RS,3R)-3-amino-2-hydroxy-5-(isopropylsulfanyl)pentanoyl)-4-(((1,1'-biphenyl)-4-ylmethyl)sulfanyl)benzohydrazide

The desired compound was prepared by substituting 4-(biphenylmethylthio)benzoic acid for o-toluic acid in Example 369.
MS (ESI) m/e 524 (M+H)⁺;
¹H NMR (500 MHz, CD₃OD) δ 7.78 (m, 2H), 7.58 (d, 2H), 7.55 (d, 2H), 7.43 (m, 6H), 7.31 (m, 1H), 4.48 (d, 0.3H), 4.43 (d, 0.7H), 4.32 (s, 1.4H), 4.31 (s, 0.6H), 3.76 (m, 1H), 3.00 (m, 1H), 2.71 (t, 2H), 2.15 (m, 1H), 1.98 (m, 1H), 1.29-1.25 (m, 6H).

### Example 458

### (2S,3R)-3-amino-N'-(3-chlorobenzoyl)-2-hydroxy-5-phenylpentanohydrazide

### Example 458A

### 4-phenylbutanal

A solution of 4-phenylbutyric acid (1.64 g, 10.0 mmol), N,O-dimethyl hydroxylamine hydrochloride (1.58 g, 16 mmol), 1-(3-dimethylaminopropyl)-3-ethylcarbodiimide hydrochloride (2.06 g, 10.7 mmol), 1-hydroxybenzotriazole (1.56 g, 11.6 mmol), and N-methylmorpholine (2.8 mL, 26 mmol) in dichloromethane (40 mL) at room temperature was stirred for 16 hours, diluted with dichloromethane, washed sequentially with aqueous NaHCO₃, brine, 10% KHSO₄, and brine, dried (MgSO₄), filtered, and concentrated. The concentrate and lithium aluminum hydride (9.0 mmol, 1 equivalent) in diethyl ether (49 mL) at room.temperature was stirred 90 minutes, treated with 1M NaHSO₄, diluted with ether, washed sequentially with 10% KHSO₄, and brine, dried (MgSO₄), filtered then concentrated to provide the desired product.
MS (ESI) m/e 148 (M+H)⁺.

### Example 458B

### (2S,3R)-3-(tert-butoxycarbonyl)amino-2-hydroxy-6-phenylhexanoic acid

The desired product was prepared by substituting Example 458A for 2-ethylhexanal in Examples 236A-236C.
MS (ESI) m/e 324 (M+H)⁺.

### Example 458C

### (2S,3R)-3-amino-N'-(3-chlorobenzoyl)-2-hydroxy-5-phenylpentanohydrazide

The desired product was prepared by substituting 3-chlorobenzoylhydrazine for O-phenyl hydroxylamine hydrochloride and Example 458B for Example 98A in Example 98B. MS (ESI) m/e 376 (M+H)⁺;
¹H NMR (300 MHz, DMSO-d₆) δ 10.69 (br s, 1H), 10.30 (br s, 1H), 8.24 (br s, 2H), 7.92 (m, 1H), 7.86 (d, 1H), 7.69 (d, 1H), 7.57 (t, 1H), 7.24 (m, 5H), 6.62 (m, 1H), 4.21 (m, 1H), 2.60 (m, 2H) 1.90-1.55 (m, 4H).

### Example 459

### (2S,3R)-3-amino-N'-(3-chlorobenzoyl)-2-hydroxy-5-(1H-indol-3-yl)pentanohydrazide

### Example 459A

### 3-(3-indolyl)propanal

The desired product was prepared by substituting 3-indolylpropionic acid for 4-phenylbutyric acid in Example 458A.
MS (ESI) m/e 174 (M+H)⁺.

### Example 459B

### (2S,3R)-3-(tert-butoxycarbonyl)amino-2-hydroxy-5-(3-indolyl)pentanoic acid

The desired product was prepared by substituting Example 459A for 2-ethylhexanal in Examples 236A-236C.
MS (ESI) m/e 349 (M+H)⁺.

### Example 459C

### (2S,3R)-3-amino-N'-(3-chlorobenzoyl)-2-hydroxy-5-(1H-indol-3-yl)pentanohydrazide

The desired product was prepared by substituting 3-chlorobenzoylhydrazine for O-phenyl hydroxylamine hydrochloride and Example 459B for Example 98A in Example 98B.
MS (ESI) m/e 400 (M+H)⁺;
¹H NMR (300 MHz, DMSO-d₆) δ 10.69 (br s, 1H), 10.33 (br s, 1H), 7.98 (br s, 2H), 7.90 (m. 1H), 7.85 (d, 1H), 7.68 (d, 1H), 7.56 (t, 1H); 7.34 (m, 2H), 7.17 (m, 1H), 7.08 (m, 1H), 6.98 (m, 1H), 6.67 (m, 1H), 4.23 (m, 1H), 2.80 (m, 2H), 2.27 (m, 1H), 1.91 (m, 1H).

### Example 460

### (2RS,3R)-3-amino-N'-(3-(2,3-dihydroxypropoxy)benzoyl)-2-hydroxy-5-(isopropylsulfanyl)pentanohydrazide

### Example 460A

### methyl 3-(prop-2-enyloxy)benzoate

Methyl-3-hydroxy-benzoate (1.09 g, 4.9 mmol), allyl bromide (0.73 g, 6.0 mmol) and potassium tert-butoxide (6.5 g, 5.8 mmol) in DMSO (15 mL) was stirred at room temperature for 16 hours. The mixture was poured into ice water, extracted with ether, washed with brine, dried (Na₂SO₄), and evaporated to give the title compound.
¹H NMR (300 MHz, DMSO-d₆) δ 7.55 (m, 1H), 7.45 (m, 2H), 7.24 (m, 1H), 6.12-5.98 (m, 1H), 5.44-5.26 (m, 2H), 4.64 (m, 2H), 3.84 (s, 3H).

### Example 460B

### methyl 3-(2,3-dihydoxypropyloxy)benzoate

A solution of Example 460A (0.3 g, 1.5 mmol), 4-methylmorpholine N-oxide (0.55 g, 4.5 mmol) and osmium tetroxide (4 wt% solution in water 0.1 mL, 0.015 mmol) in 9:1/acetone:water was stirred at room temperature for 48h. The reaction was quenched with 10% Na₂S₂O₃ and stirred for 15 minutes, extracted with ethyl acetate, washed with brine, dried (Na₂SO₄) , and concentrated to give the title compound.
MS (ESI) m/e 249 (M+Na)⁺.
¹H NMR (300 MHz, DMSO-d₆) δ 7.54 (m, 1H), 7.44 (m, 2H), 7.23 (m, 1H), 4.98 (d, 1H), 4.68 (t, 1H), 4.05 (dd, 1H), 3.51 (dd, 1H), 3.85 (s, 3H), 3.8 (m, 1H), 3.55 & 3.45 (t, 2H).

### Example 460C

### Methyl 3-(2,3-di(tertbutyldimethylsilyloxy)propoxy)benzoate

A solution of Example 460B (0.26 g, 1.15 mmol), tert-butyldimethylsilyl chloride (0.44 g, 2.87 mmol) and imidazole (0.31 g, 4.6 mmol) in DMF (10 mL) was stirred at room temperature for 18 hours. The mixture was diluted with ether, washed with brine, dried (Na₂SO₄), and concentrated to give the title compound.
MS (ESI) m/e 455 (M+H)⁺.
¹H NMR (300 MHz, DMSO-d₆) δ 7.58 (m; 1H), 7.47 (m, 2H), 7.26 (m, 1H), 4.09 (dd, 1H), 3.98 (dd, 1H), 3.89 (s, 3H), 3.85 (m, 1H), 3.67 (d, 2H), 0.91 (s, 9H), 0.89 (s, 9H), 0.08 (s, 6H), 0.05 (s, 6H).

### Example 460D

### (2RS,3R)-3-amino-N'-(3-(2,3-dihydroxypropoxy)benzoyl)-2-hydroxy-5-(isopropylsulfanyl)pentanohydrazide

Example 460C was treated with hydrazine hydrate in ethanol at reflux for 48h. After evaporation of the reaction mixture to dryness, the resulting hydrazide was reacted with Example 123B as in Example 98B.
MS (ESI) m/e 414 (M-H)⁺.
¹H NMR (300 MHz, DMSO-d₆) δ 10.57 & 10.38 (s, 1H), 10.23 & 10.12 (s, 1H), 8.05 & 7.97 (br s, 2H), 7.48-7.38(m, 3H), 7.16 (m, 1H), 6.65 & 6.56 (d, 1H), 4.98 & 4.7 (br s, 1H), 4.39 & 4.25 (t, 1H), 4.05 (dd, 1H), 3.92 (m, 1H), 3.81 (m, 1H), 3.46 (d, 2H), 3.02-2.92(m, 1H), 2.75-2.55 (m, 2H), 2.05-1.75 (m, 2H), 1.2 (m,6H).

It will be evident to one skilled in the art that the present invention is not limited to the forgoing illustrative examples, and that it can be embodied in other specific forms without departing from the essential attributes thereof. It is therefore desired that the examples be considered in all respects as illustrative and not restrictive for the invention as defined by the appended claims.

## Claims

1. A compound of formula (I), 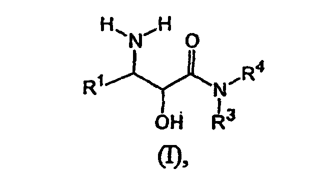 or a therapeutically acceptable salt or prodrug thereof, wherein
R¹ is selected from the group consisting of alkyl, aryl, unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, arylalkyl wherein the alkyl part of the arylalkyl can be unsubstituted or substituted with a cyano group, cycloalkyl, (cycloalkyl)alkyl, (heterocycle)alkyl, and R⁵S-(alkylene)-; wherein each group is drawn with its right-hand end being the end that is attached to the parent molecular moiety;
R³ is selected from the group consisting of hydrogen, alkyl, and arylalkyl wherein the alkyl part of the arylalkyl can be unsubstituted or substituted with a cyano group;
R⁴ is selected from the group consisting of -NR⁶R⁷, and -OR⁸; wherein each group is drawn with its left-hand end being the end that is attached to the parent molecular moiety;
R⁵ is selected from the group coasisting of alkyl, aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, arylalkyl wherein the alkyl part of the arylalkyl can be unsubstituted or substituted with a cyano group, cycloalkyl, and (cycloalkyl)alkyl;
R⁶ and R⁷ are independently selected from the group consisting of hydrogen, alkanoyl unsubstituted or substituted with one, two, or three substituents independently selected from the group consisting of alkoxycarbonyl, alkylsulfanyl, amino, and hydroxy, alkenyl, alkenyloxyalkyl, alkoxyalkyl, alkoxycarbonylalkyl, alkyl, alkylsulfanylalkyl, aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, arylalkanoyl, arylalkoxyalkyl, arylalkoxycarbonyl, arylalkyl wherein the alkyl part of the arylalkyl can be unsubstituted or substituted with a cyano group, aryloxyalkyl wherein the alkyl part of the aryloxyalkyl can be unsubstituted or substituted with a hydroxy group, (aryl)oyl, arylsulfonyl, carboxyalkyl, cycloalkyl (cycloalkyl)alkyl, (cycloalkyl)alkanoyl, (cycloalkyl)oyl, haloalkanoyl, haloalkyl, heterocycle unsubstituted or substituted with one, two, three, or four, substituents independently selected from the group consisting of alkanoyl, alkoxy, alkoxyalkyl, alkoxycarbonyl, alkyl, alkylsulfanylalkyl, amino, aminosulfonyl, carboxy, halo, haloalkoxy, haloalkyl, hydroxy, nitro, oxo, and thioxo wherein, the heterocycle groups can be further substituted with an aryl, arylalkyl, aryloxy, aryloxycarbonyl, or (heterocycle)alkenyl group, wherein the aryl, the aryl portion of the aryloxy, the arylalkyl, and the aryloxycarbonyl, and the heterocycle portion of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, (heterocycle)alkanoyl, (heterocycle)oyl, hydroxyalkyl, a nitrogen protecting group, and -C(O)NR⁹R¹⁰; or
R⁶ and R⁷ together are arylalkylidene; or
R⁶ and R⁷, together with the nitrogen atom to which they are attached, form a heterocycle unsubstituted or substituted with one, two, three, or four, substituents independently selected from the group consisting of alkanoyl, alkoxy, alkoxyalkyl, alkoxycarbonyl, alkyl, alkylsulfanylalkyl, amino, aminosulfonyl, carboxy, halo, haloalkoxy, haloalkyl, hydroxy, nitro, oxo, and thioxo wherein, the heterocycle groups can be further substituted with an aryl, arylalhyl, aryloxy, aryloxycarbonyl, or (heterocycle)alkenyl group, wherein the aryl, the aryl portion of the aryloxy, the arylalkyl, and the aryloxycarbonyl, and the heterocycle portion of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro;
R⁸ is selected from the group consisting of hydrogen, alkanoylalkyl, alkenyl, alkoxycarbonylalkyl, alkyl, amidoalkyl, aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, arylalkyl wherein the alkyl part of the arylalkyl can be unsubstituted or substituted with a cyano group, arylalkoxycarbonylalkyl, (aryl)oylalkyl, carboxyalkyl, and (cycloalkyl)alkyl; and
R⁹ and R¹⁰ are independently selected from the group consisting of hydrogen, alkyl and aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro..

2. A compound according to Claim 1, wherein R¹ is selected from the group consisting of alkyl, cycloalkyl, (cycloalkyl)alkyl, arylalkyl wherein the alkyl part of the arylalkyl can be unsubstituted or substituted with a cyano group, and (heterocycle)alkyl.

3. A compound according to Claim 2, wherein R⁴ is -OR⁸.

4. A compound according to Claim 3 selected from the group consisting of
(2RS,3R)-3-amino-4-cyclohexyl-2-hydroxy-N-phenoxybutanamide;
(2RS,3R)-3-amino-N-(benzyloxy)-4-cyclohexyl-2-hydroxybutanamide;
(2RS,3R)-3-amino-N-(methoxy)-4-cyclohexyl-2-hydroxybutanamide;
(2RS,3R)-3-amino-N-(tert-butoxy)-4-cyclohexyl-2-hydroxybutanamide;
(2RS,3R)-3-amino-4-cyclohexyl-N-ethoxy-2-hydroxybutanamide;
(2RS,3R)-N-(allyloxy)-3-amino-4-cyclohexyl-2-hydroxybutanamide;
(2RS,3R)-3-amino-4-cyclohexyl-2-hydroxy-N-isobutoxybutanamide;
(2RS,3R)-3-amino-4-cyclohexyl-N,2-dihydroxybutanamide;
(2RS,3R)-3-amino-3-cyclohexyl-2-hydroxy-N-phenoxypropanamide;
(2RS,3R)-3-amino-5-phenyl-2-hydroxy-N-phenoxypentanamide;
(2RS,3R)-3-amino-3-cyclooctyl-2-hydroxy-N-phenoxypropanamide;
(2RS,3R)-3-amino-5-cyclohexyl-2-hydroxy-N-phenoxypentanamide;
ethyl ((((2RS,3R)-3-amino-4-cyclohexyl-2-hydroxybutanoyl)amino)oxy)acetate;
benzyl ((((2RS,3R)-3-amino-4-cyclohexyl-2-hydroxybutanoyl)amino)oxy)acetate;
((((2RS,3R)-3-amino-4-cyclohexyl-2-hydroxybutanoyl)amino)oxy)acetic acid;
ethyl (2S)-2-((((((2RS,3R)-3-amino-4-cyclohexyl-2-hydroxybutanoyl)amino)oxy)acetyl)amino)propanoate;
(2RS,3R)-3-amino-4-cyclohexyl-2-hydroxy-N-(2-oxo-2-((2-phenylethyl)amino)ethoxy)butanamide;
(2RS,3R)-3-amino-N-(benzyloxy)-3-cyclohexyl-2-hydroxypropanamide; and
(2RS,3R)-3-amino-N-(benzyloxy)-2-hydroxy-5-phenylpentanamide.

5. A compound according to Claim 2 wherein R⁴ is -NR⁶R⁷.

6. A compound according to Claim 5 wherein one of R⁶ and R⁷ is hydrogen and the other is (aryl)oyl.

7. A compound according to Claim 6 selected from the group consisting of
N'-((2RS,3R)-3-amino-4-cyclohexyl-2-hydroxybutanoyl)-1-naphthohydrazide;
N'-((2RS,3R)-3-amino-4-cyclohexyl-2-hydroxybutanoyl)-4-ethoxybenzohydrazide;
N'-((2RS,3R)-3-amino-4-cyclohexyl-2-hydroxybutanoyl)-3,5-dimethoxybenzohydrazide;
N-((2RS,3R)-3-amino-4-cyclohexyl-2-hydroxybutanoyl)-2-chlorobenzohydrazide;
N'-((2RS,3R)-3-amino-4-cyclohexyl-2-hydroxybutanoyl)-3-bromobenzohydrazide;
N'-((2RS,3R)-3-amino-4-cyclohexyl-2-hydroxybutanoyl)-3-methoxybenzohydrazide;
N'-((2RS,3R)-3-amino-4-cyclohexyl-2-hydroxybutanoyl)-2,5-dichlorobenzohydrazide;
N'-((2RS,3R)-3-amino-4-cyclohexyl-2-hydroxybutanoyl)-2-methoxybenzohydrazide;
N'-((2RS,3R)-3-amino-4-cyclohexyl-2-hydroxybutanoyl)-3-chlorobenzohydrazide;
N'-((2RS,3R)-3-amino-4-cyclohexyl-2-hydroxybutanoyl)-3-methylbenzohydrazide;
N'-((2RS,3R)-3-amino-4-cyclohexyl-2-hydroxybutanoyl)-2,4-dihydroxybenzohydrazide;
N'-((2RS,3R)-3-amino-4-cyclohexyl-2-hydroxybutanoyl)-2,5-dimethoxybenzohydrazide;
N'-((2RS,3R)-3-amino-4-cyclohexyl-2-hydroxybutanoyl)-4-methylbenzohydrazide;
N'-((2RS,3R)-3-amino-4-cyclohexyl-2-hydroxybutanoyl)-4-nitrobenzohydrazide;
N'-((2RS,3R)-3-amino-4-cyclohexyl-2-hydroxybutanoyl)-2-naphthohydrazide;
N'-((2RS,3R)-3-amino-4-cyclohexyl-2-hydroxybutanoyl)-4-chlorobenzohydrazide;
(2RS,3R)-3-amino-N'-benzoyl-4-cyclohexyl-2-hydroxybutanohydrazide;
N'-((2RS,3R)-3-amino-4-cyclohexyl-2-hydroxybutanoyl)-4-bromobenzohydrazide;
N'-((2RS,3R)-3-amino-4-cyclohexyl-2-hydroxybutanoyl)-4-tert-butylbenzohydrazide;
N'-((2RS,3R)-3-amino-3-cyclohexyl-2-hydroxypropanoyl)-3-chlorobenzohydrazide;
N'-((2RS,3R)-3-amino-2-hydroxy-5-phenylpentanoyl)-3-chlorobenzohydrazide;
N'-((2RS,3R)-3-amino-3-cyclooctyl-2-hydroxypropanoyl)-3-chlorobenzohydrazide;
N'-((2RS,3R)-3-amino-5-cyclohexyl-2-hydroxypentanoyl)-3-chlorobenzohydrazide;
(2RS,3R)-3-amino-2-hydroxy-5-phenyl-N'-(1-naphthyl)pentanohydrazide;
N'-((2RS,3R)-3-amino-3-cyclohexyl-2-hydroxypropanoyl)-2-naphthohydrazide;
N'-((2RS,3R)-3-amino-4-cyclohexyl-2-hydroxybutanoyl)-1-naphthohydrazide;
N'-((2RS,3R)-3-amino-4-cyclohexyl-2-hydroxybutanoyl)-3-hydroxy-2-naphthohydrazide;
N'-((2S,3R)-3-amino-2-hydroxy-5-phenylpentanoyl)-1-naphthohydrazide;
N'-((2S,3R)-3-amino-2-hydroxy-5-phenylpentanoyl)-3-hydroxy-2-naphthohydrazide;
N'-((2S,3R,4RS)-3-amino-4-ethyl-2-hydroxyoctanoyl)-3-chlorobenzohydrazide;
N'-((2S,3R)-3-amino-5-cyclohexyl-2-hydroxypentanoyl)-3-chlorobenzohydrazide;
N'-((2RS,3R)-3-amino-4-cyclohexyl-2-hydroxybutanoyl)-2-hydroxybenzohydrazide;
N'-((2RS,3R)-3-amino-4-cyclohexyl-2-hydroxybutanoyl)-2-methylbenzohydrazide;
2-amino-N'-((2RS,3R)-3-amino-4-cyclohexyl-2-hydroxybutanoyl)benzohydrazide;
4-amino-N'-((2RS,3 R)-3-amino-4-cyclohexyl-2-hydroxybutanoyl)benzohydrazide;
3-amino-N'-((2RS,3R)-3-amino-4-cyclohexyl-2-hydroxybutanoyl)benzohydrazide;
N'-((2RS,3R)-3-amino-4-cyclohexyl-2-hydroxybutanoyl)-3-hydroxybenzohydrazide;
N'-((2RS,3R)-3-amino-4-cyclohexyl-2-hydroxybutanoyl)-4-hydroxybenzohydrazide;
N'-((2RS,3R)-3-amino-4-cyclohexyl-2-hydroxybutanoyl)-4-methoxybenzohydrazide;
N'-((2RS,3R)-3-amino-4-cyclohexyl-2-hydroxybutanoyl)-2-fluorobenzohydrazide;
N'-((2RS,3R)-3-amino-4-cyclohexyl-2-hydxoxybutanoyl)-3-fluorobenzohydrazide;
N'-((2RS,3R)-3-amino-4-cyclohexyl-2-hydroxybutanoyl)-4-fluorobenzohydrazide;
N'-((2RS,3R)-3-amino-4-cyclohexyl-2-hydroxybutanoyl)-2-bromobenzohydrazide;
N'-((2RS,3R)-3-amino-4-cyclohexyl-2-hydroxybutanoyl)-3-cyanobenzohydrazide;
N'-((2RS,3R)-3-amino-4-cyclohexyl-2-hydroxybutanoyl)-4-cyanobenzohydrazide;
N'-((2RS,3R)-3-amino-4-cyclohexyl-2-hydroxybutanoyl)-3-(dimethylamino)benzohydrazide;
N'-((2RS,3R)-3-amino-4-cyclohexyl-2-hydroxybutanoyl)-4-(dimethylamino)benzohydrazide;
N'-((2RS,3R)-3-amino-4-cyclohexyl-2-hydroxybutanoyl)-3-(trifluoromethyl)benzohydrazide;
N'-((2RS,3R)-3-amino-4-cyclohexyl-2-hydroxybutanoyl)-4-(trifluoromethyl)benzohydrazide;
N'-((2RS,3R)-3-amino-4-cyclohexyl-2-hydroxybutanoyl)-3-(trifluoromethoxy)benzohydrazide;
N'-((2RS,3R)-3-amino-4-cyclohexyl-2-hydroxybutanoyl)-4-phenoxybenzohydrazide;
N'-((2RS,3R)-3-amino-4-cyclohexyl-2-hydroxybutanoyl)-2,4-dimethylbenzohydrazide;
N'-((2RS,3R)-3-amino-4-cyclohexyl-2-hydroxybutanoyl)-2,5-dimethylbenzohydrazide;
N'-((2RS,3R)-3-amino-4-cyclohexyl-2-hydroxybutanoyl)-3,4-dimethylbenzohydrazide;
N'-((2RS,3R)-3-amino-4-cyclohexyl-2-hydroxybutanoyl)-3,5-dimethylbenzohydrazide;
N'-((2RS,3R)-3-amino-4-cyclohexyl-2-hydroxybutanoyl)-2,3-dimethoxybenzohydrazide;
N'-((2RS,3R)-3-amino-4-cyclohexyl-2-hydroxybutanoyl)-3,4-dimethoxybenzohydrazide;
N'-((2RS,3R)-3-amino-4-cyclohexyl-2-hydroxybutanoyl)-3,4,5-trimethoxybenzohydrazide;
N'-((2RS,3R)-3-amino-4-cyclohexyl-2-hydroxybutanoyl)-2,3-dichlorobenzohydrazide;
N'-((2RS,3R)-3-amino-4-cyclohexyl-2-hydroxybutanoyl)-2,4-dichlorobenzohydrazide;
N'-((2RS,3R)-3-amino-4-cyclohexyl-2-hydroxybutanoyl)-3,4-dichlorobenzohydrazide;
N'-((2RS,3R)-3-amino-4-cyclohexyl-2-hydroxybutanoyl)-3,5-dichlorobenzohydrazide;
N'-((2RS,3R)-3-amino-4-cyclohexyl-2-hydroxybutanoyl)-2,3-dimethylbenzohydrazide;
(2S,3R)-3-amino-N'-(3-chlorobenzoyl)-2-hydroxy-5-phenylpentanohydrazide; and
(2S,3R)-3-amino-N'-(3-chlorobenzoyl)-2-hydroxy-5-(1H-indol-3-yl)pentanohydrazide.

8. A compound according to Claim 5 wherein one of R⁶ and R⁷ is selected from the group consisting of hydrogen, alkyl, and aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro; and the other is selected from the group consisting of hydrogen, aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, and arylalkyl wherein the alkyl part of the arylalkyl can be unsubstituted or substituted with a cyano group.

9. A compound according to Claim 8 selected from the group consisting of
(2RS,3R)-3-amino-N'-benzyl-4-cyclohexyl-2-hydroxybutanohydrazide;
(2RS,3R)-3-amino-N'-benzyl-4-cyclohexyl-2-hydroxybutanohydrazide;
(2RS,3R)-3-amino-4-cyclohexyl-2-hydroxy-N'-(2-phenylethyl)butanohydrazide;
(2RS,3R)-3-amino-4-cyclohexyl-2-hydroxy-N'-methyl-N'-phenylbutanohydrazide;
(2RS,3R)-3-amino-4-cyclohexyl-2-hydroxy-N'-(4-methylphenyl)butanohydrazide;
(2RS,3R)-3-amino-4-cyclohexyl-2-hydroxy-N'-(4-methoxyphenyl)butanohydrazide;
(2RS,3R)-3-amino-4-cyclohexyl-2-hydroxy-N'-(1-naphthyl)butanohydrazide;
(2RS,3R)-3-amino-4-cyclohexyl-2-hydroxybutanohydrazide;
(2RS,3R)-3-amino-4-cyclohexyl-N'-(2,4-difluorophenyl)-2-hydroxybutanohydrazide;
4-(2-((2RS,3R)-3-amino-4-cyclohexyl-2-hydroxybutanoyl)hydxazino)benzenesulfonamide;
(2RS,3R)-3-amino-4-cyclohexyl-2-hydroxy-N'-phenylbutanohydrazide;
(2RS,3R)-3-amino-N-(4-bromophenyl)-4-cyclohexyl-2-hydroxybutanohydrazide;
(2RS,3R)-3-amino-4-cyclohexyl-2-hydroxy-N'-(2-methylphenyl)butanohydrazide;
(2RS,3R)-3-amino-N'-(2-chlorophenyl)-4-cyclohexyl-2-hydroxybutanohydrazide ;
(2RS,3R)-3-amino-4-cyclohexyl-2-hydroxy-N'-(3-(trifluoromethyl)phenyl)butanohydrazide;
(2RS,3R)-3-amino-4-cyclohexyl-2-hydroxy-N'-(4-iodophenyl)butanohydrazide;
(2RS,3R)-3-amino-N'-(3-chlorophenyl)-4-cyclohexyl-2-hydroxybutanohydrazide;
(2RS,3R)-3-ainino-4-cyclohexyl-2-hydroxy-N'-(3-methoxyphenyl)butanohydrazide;
(2RS,3R)-3-amino-4-cyclohexyl-N'-(3,5-dichlorophenyl)-2-hydroxybutanohydrazide;
(2RS,3R)-3-amino-N'-(3-bromophenyl)-4-cyclohexyl-2-hydroxybutanohydrazide;
(2RS,3R)-3-amino-4-cyclohexyl-2-hydroxy-N'-(4-isopropylphenyl)butanohydrazide;
(2RS,3R)-3-amino-N'-(3-chloro-4-methylphenyl)-4-cyclohexyl-2-hydroxybutanohydrazide;
(2RS,3R)-3-amino-4-cyclohexyl-2-hydroxy-N-(4-(trifluoromethoxy)phenyl)butanohydrazide;
(2RS,3R)-3-amino-4-cyclohexyl-N'-(4-fluorophenyl)-2-hydroxybutanohydrazide;
(2RS,3R)-3-amino-N'-(4-chlorophenyl)-4-cyclohexyl-2-hydroxybutanohydrazide;
(2RS,3R)-3-amino-4-cyclohexyl-N'-(2-ethylphenyl)-2-hydroxybutanohydrazide;
(2RS,3R)-3-amino-4-cyclohexyl-N'-(3-fluorophenyl)-2-hydroxybutanohydrazide;
(2RS,3R)-3-amino-N'-(4-chloro-2-methylphenyl)-4-cyclohexyl-2-hydroxybutanohydrazide;
(2RS,3R)-3-amino-4-cyclohexyl-2-hydroxy-N'-mesitylbutanohydrazide;
(2RS,3R)-3-amino-4-cyclohexyl-2-hydroxy-N'-(4-((E)-2-(2-pyridinyl)ethenyl)phenyl)butanohydrazide;
(2RS,3R)-3-amino-4-cyclohexyl-N'-(2-fluorophenyl)-2-hydroxybutanohydrazide;
(2RS,3R)-3-amino-4-cyclohexyl-2-hydroxy-N'-(4-(trifluoromethyl)phenyl)butanohydrazide;
(2RS,3R)-3-amino-N'-(2-chloro-6-fluorophenyl)-4-cyclohexyl-2-hydroxybutanohydrazide;
(2RS,3R)-3-amino-4-cyclohexyl-N'-(2,5-difluorophenyl)-2-hydroxybutanohydrazide;
(2RS,3R)-3-ammo-N'-(3-chloro-4-Suorophenyl)-4-cyclohexyl-2-hydroxybutanohydrazide;
(2RS,3R)-3-amino-N'-(2-chlorobenzyl)-4-cyclohexyl-2-hydroxybutanohydrazide;
(2RS,3R)-3-amino-4-cyclohexyl-2-hydroxy-N'-phenyl-N'-(3-phenylpropyl)butanohydrazide;
(2RS,3R)-3-amino-4-cyclohexyl-2-hydroxy-N'-isobutyl-N'-phenylbutanohydrazide;
(2RS,3R)-3-amino-4-cyclohexyl-2-hydroxy-N'-pentyl-N'-phenylbutanohydrazide;
(2RS,3R)-3-amino-4-cyclohexyl-2-hydroxy-N'-((2RS)-2-methylbutyl)-N'-phenylbutanohydrazide;
(2RS,3R)-3-amino-4-cyclohexyl-2-hydroxy-N'-isopentyl-N-phenyibutanohydrazide;
(2RS,3R)-3-amino-4-cyclohexyl-N'-hexyl-2-hydroxy-N'-phehylbutanohydrazide;
(2RS,3R)-3-amino-4-cyclohexyl-2-hydroxy-N'-((2RS)-2-methylpentyl)-N'-phenylbutanohydrazide;
(2RS,3R)-3-amino-4-cyclohexyl-2-hydroxy-N'-((3RS)-3-methylpentyl)-N'-phenylbutanohydrazide;
(2RS,3R)-3-amino-4-cyclohexyl-N'-(3,3-dimethylbutyl)-2-hydroxy-N'-phenylbutanohydrazide;
(2RS,3R)-3-amino-4-cyclohexyl-N'-(2-ethylbutyl)-2-hydroxy-N'-phenylbutanohydrazide;
(2RS,3R)-3-amino-4-cyclohexyl-N'-dodecyl 2-hydroxy-N'-phenylbutanohydrazide;
(2RS,3R)-3-amino-4-cyclohexyl-2-hydroxy-N'-phenyl-N'-((3RS)-3,5,5-trimethylhexyl)butanohydrazide;
(2RS,3R)-3-amino-4-cyclohexyl-2-hydroxy-N'-octyl-N'-phenylbutanohydrazide;
(2RS,3R)-3-amino-4-cyclohexyl-2-hydroxy-N'-phenyl-N'-propylbutanohydrazide;
(2RS,3R)-3-amino-4-cyclohexyl-N'-heptyl-2-hydroxy-N'-phenylbutanohydrazide;
(2RS,3R)-3-amino-4-cyclohexyl-N'-ethyl-2-hydroxy-N'-phenylbutanohydrazide;
(2RS,3R)-3-amino-4-cyclohexyl-2-hydroxy-N'-(2-(4-methylphenyl)ethyl)-N'-phenylbutanohydrazide;
(2RS,3R)-3-amino-4-cyclohexyl-N'-((2RS)-2-ethylhexyl)-2-hydroxy-N'-phenylbutanohydrazide;
(2RS,3R)-3-amino-N-((2RS)-2-(4-chlorophenyl)-2-cyanoethyl)-4-cyclohexyl-2-hydroxy-N'-phenylbutanohydrazide;
(2RS,3R)-3-amino-4-cyclohexyl-2-hydroxy-N'-phenyl-N'-((2RS)-2-phenylpropyl)butanohydrazide;
(2RS,3R)-3-amino-3-cyclohexyl-2-hydroxy-N'-(4-methylphenyl)propanohydrazide;
(2RS,3R)-3-amino-2-hydroxy-5-phenyl-N'-(4-methylphenyl)pentanohydrazide;
(2S,3R)-3-amino-3-cyclooctyl-2-hydroxy-N'-(4-methylphenyl)propanohydrazide;
(2RS,3S)-3-amino-4-((cyclohexylmethyl)sulfanyl)-2-hydroxy-N'-(4-methylphenyl)butanohydrazide;
(2RS,3R)-3-amino-3-cyclohexyl-2-hydroxy-N'-(1-naphthyl)propanohydrazide;
(2RS,3R)-3-amino-2-hydroxy-5-phenyl-N'-(1-naphthyl)pentanohydrazide;
(2RS,3R)-3-amino-3-cyclooctyl-2-hydroxy-N'-(1-naphthyl)propanohydrazide;
(2RS,3R)-3-amino-5-cyclohexyl-2-hydroxy-N'-(4-methylphenyl)pentanohydrazide;
(2RS,3R)-3-amino-5-cyclohexyl-2-hydroxy-N'-(1-naphthyl)pentanohydrazide; and
(2S,3R)-3-amino-4-cyclohexyl-2-hydroxybutanohydrazide.

10. A compound according to Claim 5 wherein one of R⁶ and R⁷ is selected from the group consisting of hydrogen, alkyl, and aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro; and the other is selected from the group consisting of alkanoyl unsubstituted or substituted with one, two, or three substituents independently selected from the group consisting of alkoxycarbonyl, alkylsulfanyl, amino, and hydroxy, alkenyl, alkenyloxyalkyl, alkoxyalkyl, alkoxycarbonylalkyl, alkylsulfanylalkyl, arylalkoxyalkyl, arylalkoxycarbonyl, arylsulfonyl; carboxyalkyl, -C(O)R⁹R¹⁰, cycloalkyl, (cycloalkyl)alkyl, haloalkanoyl, haloalkyl, heterocycle unsubstituted or substituted with one, two, three, or four, substituents independently selected from the group consisting of alkanoyl, alkoxy, alkoxyalkyl, alkoxycarbonyl, alkyl, alkylsulfanylalkyl, amino, aminosulfonyl, carboxy, halo, haloalkoxy, haloalkyl, hydroxy, nitro, oxo, and thioxo wherein, the heterocycle groups can be further substituted with an aryl, arylalhyl, aryloxy, aryloxycarbonyl, or (heterocycle)alkenyl group, wherein the aryl, the aryl portion of the aryloxy, the arylalkyl, and the aryloxycarbonyl, and the heterocycle portion of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro, (heterocycle)oyl, and hydroxyalkyl.

11. A compound according to Claim 10 selected from the group consisting of
(2RS,3R)-3-amino-*N*-(7-chloro-4-quinolinyl)-4-cyclohexyl-2-hydroxybutanohydrazide;
2-((2RS,3R)-3-amino-4-cyclohexyl-2-hydroxybutanoyl)-N-(4-iodophenyl)hydrazinecarboxamide;
N'-((2RS,3R)-3-amino-4-cyclohexyl-2-hydroxybutanoyl)-2,4,6-trimethylbenzenesulfonohydrazide;
ethyl (2-((2RS,3R)-3-amino-4-cyclohexyl-2-hydroxybutanoyl)hydrazino)acetate trifluoroacetate;
benzyl 2-((2RS,3R)-3-amino-4-cyclohexyl-2-hydroxybutanoyl)hydrazinecarboxylate;
ethyl 3-(2-((2RS,3R)-3-amino-4-cyclohexyl-2-hydroxybutanoyl)hydrazino)-3-oxopropanoate;
(2RS,3R)-3-amino-4-cyclohexyl-2-hydroxy-N'-(2,2,2-trifluoroethyl)butanohydrazide;
(2RS,3R)-3-amino-4-cyclohexyl-2-hydroxy-N'-methyl-N'-(3-nitro-2-pyridinyl)butanohydrazide;
(2RS,3R)-3-amino-N',4-dicyclohexyl-2-hydroxybutanohydrazide;
(2RS,3R)-3-amino-4-cyclohexyl-2-hydroxy-N'-(2-pyridiayl)butanohydrazide;
(2RS,3R)-3-amino-N'-(6-chloro-3-pyridazinyl)-4-cyclohexyl-2-hydroxy-N'-methylbutanohydrazide;
(2RS,3R)-3-amino-N'-(5-chloro-1-methyl-6-oxo-1,6-dihydro-4-pyridazinyl)-4-cyclohexyl-2-hydroxy-N'-methylbutanohydrazide;
N'-((2RS,3R)-3-amino-4-cyclohexyl-2-hydroxybutanoyl)-1,3-benzodioxole-5-carbohydrazide;
methyl 2-(2-((2RS,3R)-3-amino-4-cyclohexyl-2-hydroxybutanoyl)hydrazino)-4-(trifluoromethyl)-5-pyrimidinecarboxylate;
(2RS,3R)-3-amino-4-cyclohexyl-2-hydroxy-N'-((2RS)-2-hydroxy-3-(3-(trifluoromethyl)phenoxy)propyl)-N'-methylbutanohydrazide;
methyl 3-(2-((2RS,3R)-3-amino-4-cyclohexyl-2-hydroxybutanoyl)hydrazino)-2-thiophenecarboxylate;
(2RS,3R)-3-amino-4-cyclohexyl-2-hydroxy-N'-(2-pyridinylcarbonyl)butanohydtazide;
ethyl 3-(2-((2RS,3R)-3-amino4-cyclohexyl-2-hydroxybutanoyl)hydrazino)-6,6-dimethyl-4-oxo-4,5,6,7-tetrahydro-2-benzothiophene-1-carboxylate;
(2RS,3R)-3-amino-N'-(1,3-benzothiazol-2-yl)-4-cyclohexyl-2-hydroxybutanohydrazide;
(2RS,3R)-3-amino-4-cyclohexyl-2-hydroxy-N'-(trifluoroacetyl)butanohydrazide;
N'-((2RS,3R)-3-amino-4-cyclohexyl-2-hydroxybutanoyl)-3-chloro-1-benzothiophene-2-carbohydrazide;
(2RS,3R)-3-amino-4-cyclohexyl-2-hydroxy-N'-(2-quinoxalinyl)butanohydrazide;
(2RS,3R)-3-amino-4-cyclohexyl-2-hydroxy-N'-methyl-N'-(5-(trifluoromethyl)-2-pyridinyl)butanohydrazide;
(2RS,3R)-3-amino-4-cyclohexyl-N'-(1,3-dimethyl-4-nitro-1H-pyrazol-5-yl)-2-hydroxybutanohydrazide;
2-((2RS,3R)-3-amino-4-cyclohexyl-2-hydroxybutanoyl)-N-phenylhydrazinecarboxamide;
2-((2RS,3R)-3-amino-4-cyclohexyl-2-hydroxybutanoyl)-N-(4-chloro-2-methoxyphenyl)hydrazinecarboxamide;
2-((2RS,3R)-3-amino-4-cyclohexyl-2-hydroxybutanoyl)-N-(3-fluorophenyl)hydrazinecarboxamide;
N-((1R)-1-((2-((2RS,3R)-3-amino-4-cyclohexyl-2-hydroxybutanoyl)hydrazino)carbonyl)-3-(methylsulfanyl)propyl)-4-(trifluoromethyl)benzamide;
(2RS,3R)-3-amino-4-cyclohexyl-2-hydroxy-N'-(2-thienylcarbonyl)butanohydrazide;
4-chlorobenzyl 2-(2-((2RS,3R)-3-amino-4-cyclohexyl-2-hydroxybutanoyl)hydrazino)-4-(trifluoromethyl)-5-pyrimidinecarboxylate;
(2RS,3R)-3-amino-N'-(6-chloro-3-pyridazinyl)-4-cyclohexyl-2-hydroxybutanohydrazide;
(2RS,3R)-3-amino-4-cyclohexyl-2-hydroxy-N'-((3RS)-3-(methylsulfanyl)butyl)-N'-phenylbutanohydrazide;
(2RS,3R)-3-amino-4-cyclohexyl-N'-(cyclopropylmethyl)-2-hydroxy-N'-phenylbutanohydrazide;
(2RS,3R)-3-amino-N'-(2-(benzyloxy)ethyl)-4-cyclohexyl-2-hydroxy-N'-phenylbutanohydrazide;
(2RS,3R)-3-amino-4-cyclohexyl-2-hydroxy-N'-phenyl-N'-(2,2,5-trichloropentyl)butanohydrazide;
(2RS,3R)-3-amino-4-cyclohexyl-2-hydroxy-N'-(3-(methylsulfanyl)propyl)-N'-phenylbutanohydrazide;
(2RS,3R)-3-amino-4-cyclohexyl-N'-(cyclopentylmethyl)-2-hydroxy-N'-phenylbutanohydrazide;
(2RS,3R)-3-amino-4-cyclohexyl-2-hydroxy-N'-(5-hydroxypentyl)-N'-phenylbutanohydrazide;
(2RS,3R)-3-amino-4-cyclohexyl-N'-((2R)-2,3-dihydroxypropyl)-2-hydroxy-N'-phenylbutanohydrazide;
(2RS,3R)-3-amino-4-cyclohexyl-N'-(2,2-dichlorohexyl)-2-hydroxy-N'-phenylbutanohydrazide;
(2RS,3R)-3-amino-4-cyclohexyl-2-hydroxy-N'-((3RS)-7-methoxy-3,7-dimethyloctyl)-N'-phenylbutanohydrazide;
(2RS,3R)-3-amino-4-cyclohexyl-N'-(cyclooctylmethyl)-2-hydroxy-N'-phenylbutanohydrazide;
(2RS,3R)-3-amino-4-cyclohexyl-N'-((11Z)-11-hexadecenyl)-2-hydroxy-N'-phenylbutanohydrazide;
(2RS,3R)-3-amino-4-cyclohexyl-2-hydroxy-N'-phenyl-N'-tridecylbutanohydrazide;
4-(2-((2RS,3R)-3-amino-4-cyclohexyl-2-hydroxybutanoyl)-1-phenylhydrazino)butanoic acid;
(2RS,3R)-3-amino-4-cyclohexyl-2-hydroxy-N'-((6Z)-6-nonenyl)-N-phenylbutanohydrazide;
(2RS,3R)-3-amino-4-cyclohexyl-N'-((4Z)-4-decenyl)-2-hydroxy-N'-phenylbutanohydrazide;
(2RS,3R)-3-amino-4-cyclohexyl-2-hydroxy-N'-(4-pentenyl)-N'-phenylbutanohydrazide;
(2RS,3R)-3-amino-4-cyclohexyl-N'-((3RS)-3,7-dimethyl-6-octenyl)-2-hydroxy-N'-phenylbutanohydrazide;
(2RS,3R)-3-amino-4-cyclohexyl-2-hydroxy-N'-phenyl-N'-(4,4,4-trifluorobutyl)butanohydrazide;
(2RS,3R)-3-amino-4-cyclohexyl-2-hydroxy-N'-((3RS)-3-hydroxybutyl)-N'-phenylbutanohydrazide;
(2RS,3R)-3-amino-4-cyclohexyl-N'-(2-(((3RS)-3,7-dimethyl-6-octenyl)oxy)entyl)-2-hydroxy-N'-phenylbutanohydrazide;
(2RS,3R)-3-amino-4-cyclohexyl-N'-(2-((1RS)-3,3-dimethylcyclohexyl)ethyl)-2-hydroxy-N'-phenylbutanohydrazide;
(2RS,3R)-3-amino-N-((4S)-6-bromo-4-methylhexyl)-4-cyclohexyl-2-hydroxy-N'-phenylbutanohydrazide; and
(2RS,3R)-3-amino-4-cyclohexyl-N'-(cyclohexylmethyl)-2-hydroxy-N'-phenylbutanohydrazide.

12. A compound according to Claim 5 wherein R⁶ and R⁷ together are arylalkylidene.

13. A compound according to Claim 12 which is
(2RS,3R)-3-amino-N'-((E)-(4-chlorophenyl)methylidene)-4-cyclohexyl-2-hydroxybutanohydrazide.

14. A compound according to Claim 5 wherein R⁶ and R⁷, together with the nitrogen atom to which they are attached, form a heterocycle unsubstituted or substituted with one, two, three, or four, substituents independently selected from the group consisting of alkanoyl, alkoxy, alkoxyalkyl, alkoxycarbonyl, alkyl, alkylsulfanylalkyl, amino, aminosulfonyl, carboxy, halo, haloalkoxy, haloalkyl, hydroxy, nitro, oxo, and thioxo wherein, the heterocycle groups can be further substituted with an aryl, arylalhyl, aryloxy, aryloxycarbonyl, or (heterocycle)alkenyl group, wherein the aryl, the aryl portion of the aryloxy, the arylalkyl, and the aryloxycarbonyl, and the heterocycle portion of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro..

15. A compound according to Claim 14 selected from the group consisiting of
(2RS,3R)-3-amino-4-cyclohexyl-2-hydroxy-N-((2S)-2-(methoxymethyl)pyrrolidinyl)butanamide;
(2RS,3R)-3-amino-4-cyclohexyl-2-hydroxy-N-(1-pyrrolidinyl)butanamide;
(2RS,3R)-3-amino-4-cyclohexyl-2-hydroxy-N-(4-oxo-2-thioxo-1,3-thiazolidin-3-yl)butanamide;
(2RS,3R)-3-amino-4-cyclohexyl-2-hydroxy-N-(1-piperidinyl)butanamide; and
(2RS,3R)-3-amino-4-cyclohexyl-2-hydroxy-N-((2R)-2-(methoxymethyl)pyrrolidinyl)butanamide.

16. A compound according to Claim 1 wherein R¹ is R⁵S-(alkylene)-.

17. A compound according to Claim 16 wherein R⁴ is -OR⁸.

18. A compound according to Claim 17 selected from the group consisting of
(2RS,3R)-3-amino-5-(ethylsulfanyl)-2-hydroxy-N-phenoxypentamide;
(2RS,3S)-3-amino-4-(ethylsulfanyl)-2-hydroxy-N-phenoxybutanamide;
(2RS,3S)-3-amino-2-hydroxy-N-phenoxy-4-(propylsulfanyl)butanamide;
(2RS,3R)-3-amino-2-hydroxy-5-(isopropylsulfanyl)-N-phenoxypentanamide;
(2RS,3S)-3-amino-2-hydroxy-4-(isobutylsulfanyl)-N-phenoxybutanamide;
(2RS,3S)-3-amino-4-((cyclohexylmethyl)sulfanyl)-2-hydroxy-N-phenoxybutanamide;
(2RS,3R)-3-amino-5-(ethylsulfanyl)-2-hydroxy-N-benzyloxypentanamide;
(2RS,3R)-3-amino-N-(benzyloxy)-2-hydroxy-5-(isopropylsulfanyl)pentanamide;
(2RS,3S)-3-amino-N-(benzyloxy)-2-hydroxy-4-(isobutylsulfanyl)butanamide;
(2S,3S)-3-amino-N-(cyclohexylmethoxy)-2-hydroxy-4-(isobutylsulfanyl)butanamide;
(2S,3S)-3-amino-2-hydroxy-4-(isobutylsulfanyl)-N-(mesitylmethoxy)butanamide;
(2RS,3R)-3-amino-2-hydroxy-5-(ethylsulfanyl)-N-((1RS)-1-phenylethoxy)pentanamide;
(2S,3S)-3-amino-N-(benzyloxy)-2-hydroxy-4-(isobutylsulfanyl)-N-methylbutanamide;
(2RS,3S)-3-amino-N-(benzyloxy)-2-hydroxy-5-(isopropylsulfanyl)-N-methylpentanamide; and
(2RS,3R)-3-amino-2-hydroxy-5-(isopropylsulfanyl)-N-((1RS)-1-phenylethoxy)pentanamide.

19. A compound according to Claim 18 wherein R⁴ is -NR⁶R⁷.

20. A compound according to Claim 19 wherein one of R⁶ and R⁷ is hydrogen and the other is (aryl)oyl.

21. A compound according to Claim 20 wherein the aryl(oyl) is unsubstituted.

22. A compound according to Claim 21 selected from the group consisting of
N'-((2RS,3R)-3-amino-5-(ethylsulfanyl)-2-hydroxypentanoyl)-2-naphthohydrazide;
N'-((2RS,3R)-3-amino-5-(eihylsulfanyl)-2-hydroxypentanoyl)benzohydrazide;
N'-((2RS,3R)-3-amino-2-hydroxy-5-isopropylsulfanylpentanoyl)-2-naphthohydrazide;
N'-((2RS,3S)-3-amino-2-hydroxy-4-(isobutylsulfanyl)butanoyl)-2-naphthohydrazide;
N'-((2RS,3R)-3-amino-5-(ethylsulfanyl)-2-hydroxypentanoyl)-1 -naphthohydrazide; and
N'-((2RS,3R)-3-amino-2-hydroxy-5-(isopropylsulfanyl)pentanoyl)-1-naphthohydrazide.

23. A compound according to Claim 20 wherein the aryl(oyl) is substituted with one substituent.

24. A compound according to Claim 23 wherein the substituent is at the 2-position.

25. A compound according to Claim 24 selected from the group consisting of
N'-((2RS,3R)-3-amino-5-(ethylsulfanyl)-2-hydroxypentanoyl)-2-chlorobenzohydrazide;
N'-((2RS,3R)-3-amino-5-(ethylsulfanyl)-2-hydroxypentanoyl)-3-hydroxy-2-naphthohydrazide;
N'-((2RS,3R)-3-amino-5-(ethylsulfanyl)-2-hydroxypentanoyl)-2-methylbenzohydrazide;
N'-((2RS,3R)-3-amino-5-(ethylsulfanyl)-2-hydroxypentanoyl)-2-aminobenzohydrazide;
N'-((2RS,3R)-3-amino-5-(ethylsulfanyl)-2-hydroxypentanoyl)-2-hydroxybenzohydrazide;
N'-((2RS,3R)-3-amino-5-(ethylsulfanyl)-2-hydroxypentanoyl)-2-fluorobenzohydrazide;
N'-((2RS,3R)-3-amino-5-(ethylsulfanyl)-2-hydroxypentanoyl)-2-bromobenzohydrazide;
N'-((2RS,3R)-3-amino-2-hydroxy-5-(isopropylsulfanyl)pentanoyl)-2-methylbenzohydrazide;
N'-((2RS,3R)-3-amino-2-hydroxy-5-(isopropylsulfanyl)pentanoyl)-2-methoxybenzohydrazide;
N'-((2RS,3R)-3-amino-2-hydroxy-5-(isopropylsulfanyl)pentanoyl)-2-fluorobenzohydrazide;
N'-((2RS,3R)-3-amino-2-hydroxy-5-(isopropylsulfanyl)pentanoyl)-2-chlorobenzohydrazide;
N'-((2RS,3R)-3-amino-2-hydroxy-5-(isopropylsulfanyl)pentanoyl)-2-bromobenzohydrazide;
N'-((2RS,3R)-3-amino-2-hydroxy-5-(isopropylsulfanyl)pentanoyl)-2-ethoxybenzohydrazide;
N'-((2RS,3R)-3-amino-2-hydroxy-5-(isopropylsulfanyl)pentanoyl)-2-benzylbenzohydrazide;
N'-((2RS,3R)-3-amino-2-hydroxy-5-(isopropylsulfanyl)pentanoyl)-2-anilinobenzohydrazide; and
N'-((2RS,3R)-3-amino-2-hydroxy-5-(isopropylsulfanyl)pentanoyl)-2-(2-phenylethyl)benzohydrazide.

26. A compound according to Claim 23 wherein the substituent is at the 3-position.

27. A compound according to Claim 23 selected from the group consisting of
N'-((2RS,3R)-3-amino-5-(ethylsulfanyl)-2-hydroxypentanoyl)-3-chlorobenzohydrazide;
N'-((2RS,3S)-3-amino-4-(ethylsulfanyl)-2-hydroxybutanoyl)-3-chlorobenzohydrazide;
N'-((2RS,3S)-3-amino-2-hydroxy-4-(propylsulfanyl)butanoyl)-3-chlorobenzohydrazide;
N'-((2RS,3R)-3-amino-2-hydroxy-5-(isopropylsulfanyl)pentanoyl)-3-chlorobenzohydrazide;
N'-((2RS,3S)-3-amino-2-hydroxy-4-(isobutylsulfanyl)butanoyl)-3-chlorobenzohydrazide;
N'-((2RS,3S)-3-amino-4-((cyclohexylmethyl)sulfanyl)-2-bydroxybutanoyl)-3-chlorobenzohydrazide;
N'-((2S,3R)-3-amino-2-hydroxy-5-(isopropylsulfanyl)pentanoyl-3-chlorobenzohydrazide
N'-((2S,3R)-3-amino-5-(ethylsulfanyl)-2-hydroxypentanoyl)-3-chlorobenzohydrazide;
N'-((2R,3R)-3-amino-5-(ethylsulfanyl)-2-hydroxypentanoyl)-3-chlorobenzohydrazide;
N'-((2R,3R)-3-amino-2-hydroxy-5-(isopropylsulfanyl)pentanoyl)-3-chlorobenzohydrazide;
3-(2-aminoethyl)-N'-((2S,3S)-3-amino-2-hydroxy-4-(isobutylsulfanyl)butanoyl)benzohydrazide;
N'-((2S,3S)-3-amino-2-hydroxy-4-(isobutylsulfanyl)butanoyl)-3-propoxybenzohydrazide;
N'-((2RS,3R)-3-amino-5-(ethylsulfanyl)-2-hydroxypentanoyl)-3-trifluoromethylsulfanylbenzohydrazide;
N'-((2RS,3R)-3-amino-5-(ethylsulfanyl)-2-hydroxypentanoyl)-3-methylbenzohydrazide;
N'-((2RS,3R)-3-amino-5-(ethylsulfanyl)-2-hydroxypentanoyl)-3-aminobenzohydrazide;
N'-((2RS,3R)-3-amino-5-(ethylsulfanyl)-2-hydroxypentanoyl)-3-hydroxybenzohydrazide;
N'-((2RS,3R)-3-amino-5-(ethylsulfanyl)-2-hydroxypentanoyl)-3-methoxybenzohydrazide;
N'-((2RS,3R)-3-amino-5-(ethylsulfanyl)-2-hydroxypentanoyl)-3-fluorobenzohydrazide;
N'-((2RS,3R)-3-amino-5-(ethylsulfanyl)-2-hydroxypentanoyl)-3-(dimethylamino)benzohydrazide;
N'-((2RS,3R)-3-amino-5-(ethylsulfanyl)-2-hydroxypentanoyl)-3-(trifluoromethyl)benzohydrazide;
N'-((2RS,3R)-3-amino-5-(ethylsulfanyl)-2-hydroxypentanoyl)-3-(trifluoromethoxy)benzohydrazide;
(2RS,3R)-3-amino-N'-(3-(2-aminoethoxy)phenyl)-2-hydroxy-5-(isopropylsulfanyl)pentanohydrazide;
N'-((2RS,3R)-3-amino-5-(ethylsulfanyl)-2-hydroxypentanoyl)-3-bromobenzohydrazide;
N'-((2RS,3R)-3-amino-5-(ethylsulfanyl)-2-hydroxypentanoyl)-3-cyanobenzohydrazide;
N'-((2RS,3R)-3-amino-2-hydroxy-5-(isopropylsulfanyl)pentanoyl)-3-hydroxybenzohydrazide;
N'-((2RS,3R)-3-amino-2-hydroxy-5-(isopropylsulfanyl)pentanoyl)-3-methylbenzohydrazide;
N'-((2RS,3R)-3-amino-2-hydroxy-5-(isopropylsulfanyl)pentanoyl)-3-methoxybenzohydrazide;
N'-((2RS,3R)-3-ammo-2-hydroxy-5-(isopropylsulfanyl)pentanoyl)-3-fluorobenzohydrazide;
N'-((2RS,3R)-3-amino-2-hydroxy-5-(isopropylsulfanyl)pentanoyl)-3-bromobenzohydrazide;
N'-((2RS,3R)-3-amino-2-hydroxy-5-(isopropylsulfanyl)pentanoyl)-3-(methylsulfanyl)benzohydrazide;
N'-((2RS,3R)-3-amino-2-hydroxy-5-(isopropylsulfanyl)pentanoyl)-3-(ethylsulfanyl)benzohydrazide;
N'-((2RS,3R)-3-amino-2-hydroxy-5-(isopropylsulfanyl)pentanoyl)-3-(propylsulfanyl)benzohydrazide;
N'-((2RS,3R)-3-amino-2-hydroxy-5-(isopropylsulfanyl)pentanoyl)-3-(butylsulfanyl)benzohydrazide;
N'-((2RS,3R)-3-amino-2-hydroxy-5-(isopropylsulfanyl)pentanoyl)-3-(hexylsulfanyl)benzohydrazide;
N'-((2RS,3R)-3-amino-2-hydroxy-5-(isopropylsulfanyl)pentanoyl)-3-(isopropylsulfanyl)benzohydrazide;
N'-((2RS,3R)-3-amino-2-hydroxy-5-(isopropylsulfanyl)pentanoyl)-3-(isobutylsulfanyl)benzohydrazide;
N'-((2RS,3R)-3-amino-2-hydroxy-5-(isopropylsulfanyl)pentnoyl)-3-((4-methylpentyl)sulfanyl)benzohydrazide;
N'-((2RS,3R)-3-amino-2-hydroxy-5-(isopropylsulfanyl)pentanoyl)-3-(sec-butylsulfanyl)benzohydrazide;
N-((2RS,3R)-3-amino-2-hydroxy-5-(isopropylsulfanyl)pentanoyl)-3-(neopentylsulfanyl)benzohydrazide;
N'-((2RS,3R)-3-amino-2-hydroxy-5-(isopropylsulfanyl)pentanoyl)-3-(cyclohexylsulfanyl)benzohydrazide;
N'-((2RS,3R)-3-amino-2-hydroxy-5-(isopropylsulfanyl)pentanoyl)-3-((cyclohexylmethyl)sulfanyl)benzohydrazide;
N'-((2RS,3R)-3-amino-2-hydroxy-5-(isopropylsulfanyl)pentanoyl)-3-(benzylsulfanyl)benzohydrazide;
N'-((2RS,3R)-3-amino-2-hydroxy-5-(isopropylsulfanyl)pentanoyl)-3-((2-phenylethyl)sulfanyl)benzohydrazide;
N'-((2RS,3R)-3-amino-2-hydroxy-5-(isopropylsulfanyl)pentanoyl)-3-((3-phenylpropyl)sulfanyl)benzohydrazide;
N'-((2RS,3R)-3-amino-2-hydroxy-5-(isopropylsulfanyl)pentanoyl)-3-(((1,1'-biphenyl)-4-ylmethyl)sulfanyl)benzohydrazide; and
(2RS,3R)-3-amino-N'-(3-(2,3-dihydroxypropoxy)benzoyl)-2-hydroxy-5-(isopropylsulfanyl)pentanohydrazide.

28. A compound according to Claim 23 wherein the substituent is at the 4-position.

29. A compound according to Claim 28 selected from the group consisting of
N'-((2RS,3R)-3-amino-5-(ethylsulfanyl)-2-hydroxypentanoyl)-4-methylbenzohydrazide;
N'-((2RS,3R)-3-amino-5-(ethylsulfanyl)-2-hydroxypentanoyl)-4-aminobenzohydrazide;
N'-((2RS,3R)-3-amino-5-(ethylsulfanyl)-2-hydroxypentanoyl)-4-hydroxybenzohydrazide;
N'-((2RS,3R)-3-amino-5-(ethylsulfanyl)-2-hydroxypentanoyl)-4-methoxybenzohydrazide;
N'-((2RS,3R)-3-amino-5-(ethylsulfanyl)-2-hydroxypentanoyl)-4-fluorobenzohydrazide;
N'-((2RS,3R)-3-amino-5-(ethylsulfanyl)-2-hydroxypentanoyl)-4-bromobenzohydrazide;N'-((2RS,3R)-3-amino-5-(ethylsulfanyl)-2-hydroxypentanoyl)-4-isopropylbenzohydrazide;
N'-((2RS,3R)-3-amino-5-(ethylsulfanyl)-2-hydroxypentanoyl)-4-propoxybenzohydrazide;
N'-((2RS,3R)-3-amino-5-(ethylsulfanyl)-2-hydroxypentanoyl)-4-(methylsulfanyl)benzohydrazide;
N'-((2RS,3R)-3-amino-5-(ethylsulfanyl)-2-hydroxypentanoyl)-4-isopropoxybenzohydrazide;
N'-((2RS,3R)-3-amino-5-(ethylsulfanyl)-2-hydroxypentanoyl)-4-(diethylamino)benzohydrazide;
N'-((2RS,3R)-3-amino-5-(ethylsulfanyl)-2-hydroxypentanoyl)-4-butoxybenzohydrazide;
N'-((2RS,3R)-3-amino-5-(ethylsulfanyl)-2-hydroxypentanoyl)-4-chlorobenzohydrazide;
N'-((2RS,3R)-3-amino-5-(ethylsulfanyl)-2-hydroxypentanoyl)-4-(dimethylamino)benzohydrazide;
N'-((2RS,3R)-3-amino-5-(ethylsulfanyl)-2-hydroxypentanoyl)-4-(trifluoromethyl)benzohydrazide;
N'-((2RS,3R)-3-amino-5-(ethylsulfanyl)-2-hydroxypentanoyl)-4-phenoxybenzohydrazide;
N-((2RS,3R)-3-amino-5-(ethylsulfanyl)-2-hydroxypentanoyl)-4-(phenoxymethyl)benzohydrazide;
N'-((2RS,3R)-3-amino-5-(ethylsulfanyl)-2-hydroxypentanoyl)-4-cyanobenzohydrazide;
N'-((2RS,3R)-3-amino-2-hydroxy-5-(isopropylsulfanyl)pentanoyl)-4-methylbenzohydrazide;
N'-((2RS,3R)-3-amino-2-hydroxy-5-(isopropylsulfanyl)pentanoyl)-4-methoxybenzohydrazide;
N'-((2RS,3R)-3-amino-2-hydroxy-5-(isopropylsulfanyl)pentanoyl)-4-fluorobenzohydrazide;
N'-((2RS,3R)-3-amino-2-hydroxy-5-(isopropylsulfanyl)pentanoyl)-4-chlorobenzohydrazide;
N'-((2RS,3R)-3-amino-2-hydroxy-5-(isopropylsulfanyl)pentanoyl)-4-bromobenzohydrazide;
N'-((2RS,3R)-3-amino-2-hydroxy-5-(isopropylsulfanyl)pentanoyl)-4-ethylbenzohydrazide;
N'-((2RS,3R)-3-amino-2-hydroxy-5-(isopropylsulfanyl)pentanoyl)-4-propylbenzohydrazide;
N'-((2RS,3R)-3-amino-2-hydroxy-5-(isopropylsulfanyl)pentanoyl)-4-isopropylbenzohydrazide;
N'-((2RS,3R)-3-amino-2-hydroxy-5-(isopropylsulfanyl)pentanoyl)-4-ethoxybenzohydrazide;
N'-((2RS,3R)-3-amino-2-hydroxy-5-(isopropylsulfanyl)pentanoyl)-4-tert-butylbenzohydrazide;
N'-((2RS,3R)-3-amino-2-hydroxy-5-(isopropylsulfanyl)pentanoyl)-4-propoxybenzohydrazide;
N'-((2RS,3R)-3-amino-2-hydroxy-5-(isopropylsulfanyl)pentanoyl)-4-isopropoxybenzohydrazide;
N'-((2RS,3R)-3-amino-2-hydroxy-5-(isopropylsulfanyl)pentanoyl)-4-(methylsulfonyl)benzohydrazide;
N'-((2RS,3R)-3-amino-2-hydroxy-5-(isopropylsulfanyl)pentanoyl)-4-(methylsulfanyl)benzohydrazide;
N'-((2RS,3R)-3-amino-2-hydroxy-5-(isopropylsulfanyl)pentanoyl)-4-(ethylsulfanyl)benzohydrazide;
N'-((2RS,3R)-3-amino-2-hydroxy-5-(isopropylsulfanyl)pentanoyl)-4-(propylsulfanyl)benzohydrazide;
N'-((2RS,3R)-3-amino-2-hydroxy-5-(isopropylsulfanyl)pentanoyl)-4-(butylsulfanyl)benzohydrazide;
N'-((2RS,3R)-3-amino-2-hydroxy-5-(isopropylsulfanyl)pentanoyl)-4-(hexylsulfanyl)benzohydrazide;
N'-((2RS,3R)-3-amino-2-hydroxy-5-(isopropylsulfanyl)pentanoyl)-4-(isopropylsulfanyl)benzohydrazide;
N'-((2RS,3R)-3-amino-2-hydroxy-5-(isopropylsulfanyl)pentanoyl)-4-(isobutylsulfanyl)benzohydrazide;
N'-((2RS,3R)-3-amino-2-hydroxy-5-(isopropylsulfanyl)pentanoyl)-4-((4-methylpentyl)sulfanyl)benzohydrazide;
N'-((2RS,3R)-3-amino-2-hydroxy-5-(isopropylsulfanyl)pentanoyl)-4-(sec-butylsulfanyl)benzohydrazide;
N'-((2RS,3R)-3-amino-2-hydroxy-5-(isopropylsulfanyl)pentanoyl)-4-(neopentylsulfanyl)benzohydrazide;
N'-((2RS,3R)-3-amino-2-hydroxy-5-(isopropylsulfanyl)pentanoyl)-4-(cyclohexylsulfanyl)benzohydrazide;
N'-((2RS,3R)-3-amino-2-hydroxy-5-(isopropylsulfanyl)pentanoyl)-4-((cyclohexylmethyl)sulfanyl)benzohydrazide;
N'-((2RS,3R)-3-amino-2-hydroxy-5-(isopropylsulfanyl)pentanoyl)-4-(benzylsulfanyl)benzohydrazide;
N-((2RS,3R)-3-amino-2-hydroxy-5-(isopropylsulfanyl)pentanoyl)-4-((2-phenylethyl)sulfanyl)benzohydrazide;
N'-((2RS,3R)-3-amino-2-hydroxy-5-(isopropylsulfanyl)pentanoyl)-4-((3-phenylpropyl)sulfanyl)benzohydrazide; and
N'-((2RS,3R)-3-amino-2-hydroxy-5-(isopropylsulfanyl)pentanoyl)-4-(((1,1'-biphenyl)-4-ylmethyl)sulfanyl)benzohydrazide.

30. A compound according to Claim 20 wherein the aryl(oyl) is substituted with two or three substituents.

31. A compound according to Claim 30 selected from the group consisting of
N'-((2RS,3R)-3-amino-5-(ethylsulfanyl)-2-hydroxypentanoyl)-3,4-diethoxybenzohydrazide;
N'-((2RS,3R)-3-amino-5-(ethylsulfanyl)-2-hydroxypentanoyl)-2,3-dimethylbenzohydrazide;
N'-((2RS,3R)-3-amino-5-(ethylsulfanyl)-2-hydroxypentanoyl)-2,4-dimethylbenzohydrazide;
N'-((2RS,3R)-3-amino-5-(ethylsulfanyl)-2-hydroxypentanoyl)-2,5-dimethylbenzohydrazide;
N'-((2RS,3R)-3-amino-5-(ethylsulfanyl)-2-hydroxypentanoyl)-4,5-dimethylbenzohydrazide;
N'-((2RS,3R)-3-amino-5-(ethylsulfanyl)-2-hydroxypentanoyl)-3,5-dimethylbenzohydrazide;
N'-((2RS,3R)-3-amino-5-(ethylsulfanyl)-2-hydroxypentanoyl)-2,3-dimethoxybenzohydrazide;
N'-((2RS,3R)-3-amino-5-(ethylsulfanyl)-2-hydroxypentanoyl)-2,4-dimethoxybenzohydrazide;
N'-((2RS,3R)-3-amino-5-(ethylsulfanyl)-2-hydroxypentanoyl)-2,5-dimethoxybenzohydrazide;
N'-((2RS,3R)-3-amino-5-(ethylsulfanyl)-2-hydroxypentanoyl)-3,4-dimethoxybenzohydrazide;
N'-((2RS,3R)-3-amino-5-(ethylsulfanyl)-2-hydroxypentanoyl)-3,5-dimethoxybenzohydrazide;
N'-((2RS,3R)-3-amino-5-(ethylsulfanyl)-2-hydroxypentanoyl)-3,4,5-trimethoxybenzohydrazide;
N'-((2RS,3R)-3-amino-5-(ethylsulfanyl)-2-hydroxypentanoyl)-2,3-dichlorobenzohydrazide;
N'-((2RS,3R)-3-amino-5-(ethylsulfanyl)-2-hydroxypentanoyl)-2,4-dichlorobenzohydrazide;
N'-((2RS,3R)-3-amino-5-(ethylsulfanyl)-2-hydroxypentanoyl)-2,5-dichlorobenzohydrazide;
N'-((2RS,3R)-3-amino-5-(ethylsulfanyl)-2-hydroxypentanoyl)-3,4-dichlorobenzohydrazide;
N'-((2RS,3R)-3-amino-5-(ethylsulfanyl)-2-hydroxypentanoyl)-3,5-dichlorobenzohydrazide;
N'-((2RS,3R)-3-amino-2-hydroxy-5-(isopropylsulfanyl)pentanoyl)-2,3-dichlorobenzohydrazide;N'-((2RS,3R)-3-amino-2-hydroxy-5-(isopropylsulfanyl)pentanoyl)-3-fluoro-2-methylbenzohydrazide;
N'-((2RS,3R)-3-amino-2-hydroxy-5-(isopropylsulfanyl)pentanoyl)-2,3-difluorobenzohydrazide;
N'-((2RS,3R)-3-amino-2-hydroxy-5-(isopropylsulfanyl)pentanoyl)-5-chloro-2-methoxybenzohydrazide;
N'-((2RS,3R)-3-amino-2-hydroxy-5-(isopropylsulfanyl)pentanoyl)-2-chloro-5-(methylsulfanyl)benzohydrazide; and
N'-((2RS,3R)-3-amino-2-hydroxy-5-(isopropylsulfanyl)pentanoyl)-3,4-diethoxybenzohydrazide.

32. A compound according to Claim 19 wherein one of R⁶ and R⁷ is hydrogen; and the other is selected from the group consisting of aryl unsubstituted or substituted with one, two, three, four, or five substituents independently selected from the group consisting of alkanoyl, alkoxy, alkoxyalkyl, alkoxycarbonyl, alkyl, alkylsulfonyl, alkylsulfanyl, alkylsulfanylalkyl, amino, aminoalkoxy, aminoalkyl, aminosulfonyl, carboxy, cyano, (cycloalkyl)alkylsulfanyl, cycloalkylsulfanyl, halo, haloalkoxy, haloalkyl, haloalkylsulfanyl, (heterocycle)alkenyl, hydroxy, hydroxyalkoxy, nitro, oxo, and thioxo, wherein the aryl groups can be further substituted with an additional aryl group or an arylalkyl, arylalkylsulfanyl, aryloxy, aryloxyalkyl, heterocycle, or (heterocycle)alkenyl group, wherein the aryl, the aryl part of the arylalkyl, the arylalkylsulfanyl, the aryloxy, and the aryloxyalkyl, the heterocycle, and the heterocycle part of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro; arylalkanoyl, arylalkoxycarbonyl, and arylsulfonyl.

33. A compound according to Claim 32 selected from the group consisting of
benzyl 2-((2RS,3S)-3-amino-4-((cyclohexylmethyl)sulfanyl)-2-hydxoxybutanoyl)hydrazinecarboxylate;
benzyl 2-((2RS,3S)-3-amino-2-hydroxy-4-(propylsulfanyl)butanoyl)hydrazinecarboxylate;
(2RS,3R)-3-amino-5-(ethylsulfanyl)-2-hydroxy-N'-(4-methylphenyl)pentanohydrazide;
(2RS,3R)-3-amino-5-(ethylsulfanyl)-2-hydroxy-N'-(4-methoxyphenyl)pentanohydrazide;
(2RS,3R)-3-amino-5-(ethylsulfanyl)-2-hydroxy-N'-(1-naphthyl)pentanohydrazide;
(2RS,3R)-3-amino-5-(ethylsulfanyl)-2-hydroxy-N'-(4-iodophenyl)pentanohydrazide;
(2RS,3R)-3-amino-5-(ethylsulfanyl)-2-hydroxy-N'-(3-chlorophenyl)pentanohydrazide;
(2RS,3R)-3-amino-5-(ethylsulfanyl)-2-hydroxy-N'-(3-methoxyphenyl)pentanohydrazide;
(2RS,3R)-3-amino-5-(ethylsulfanyl)-2-hydroxy-N'-(2-chlorophenyl)pentanohydrazide;
(2RS,3R)-3-amino-5-(ethylsulfanyl)-2-hydroxy-N'-(3-trifluoromethylphenyl)pentanohydrazide;
(2RS,3R)-3-amino-5-(ethylsulfanyl)-2-hydroxy-N'-(4-isopropyl)pentanohydrazide;
(2RS,3R)-3-amino-5-(ethylsulfanyl)-2-hydroxy-N'-(3-chloro-4-methylphenyl)pentanohydrazide;
(2RS,3R)-3-amino-5-(ethylsulfanyl)-2-hydroxy-N'-(3-fluorophenyl)pentanohydrazide;
(2RS,3R)-3-amino-5-(ethylsulfanyl)-2-hydroxy-N'-(2-ethylphenyl)pentanohydrazide;
(2RS,3R)-3-amino-5-(ethylsulfanyl)-2-hydroxy-N'-(4-fluorophenyl)pentanohydrazide;
(2RS,3R)-3-amino-5-(ethylsulfanyl)-2-hydroxy-N'-(4-trifluoromethoxyphenyl)pentanohydrazide;
(2RS,3S)-3-amino-4-(ethylsulfanyl)-2-hydroxy-N'-(4-methylphenyl)butanohydrazide;
(2RS,3S)-3-amino-2-hydroxy-N'-(4-methylphenyl)-4-(propylsulfanyl)butanohydrazide;
(2RS,3R)-3-amino-2-hydroxy-5-(isopropylsulfanyl)-N'-(4-methylphenyl)pentanohydrazide;
(2RS,3S)-3-amino-2-hydroxy-4-(isobutylsulfanyl)-N'-(4-methylphenyl)butanohydrazide;
(2RS,3S)-3-amino-4-(ethylsulfanyl)-2-hydroxy-N'-(1-naphthyl)butanohydrazide;
(2RS,3R)-3-amino-2-hydroxy-5-(isopropylsulfanyl)-N'-(1-naphthyl)pentanohydrazide;
(2RS,3S)-3-amino-2-hydroxy-4-(isobutylsulfanyl)-N'-(1-naphthyl)butanohydrazide;
(2RS,3S)-3-amino-2-hydroxy-N'-(1-naphthyl)-4-(propylsulfanyl)butanohydrazide;
(2RS,3S)-3-amino-4-((cyclohexylmethyl)sulfanyl)-2-hydroxy-N'-(1-naphthyl)butanohydrazide;
(2RS,3R)-3-amino-5-(ethylsulfanyl)-2-hydroxy-N'-(phenylacetyl)pentanohydrazide;
(2RS,3R)-3-amino-5-(ethylsulfanyl)-2-hydroxy-N'-((2-methoxyphenyl)acetyl)pentanohydrazide;
(2RS,3R)-3-amino-5-(ethylsulfanyl)-2-hydroxy-N'-((3-methoxyphenyl)acetyl)pentanohydrazide;
(2RS,3R)-3-amino-5-(ethylsulfanyl)-2-hydroxy-N'-((4-methoxyphenyl)acetyl)pentanohydrazide;
(2RS,3R)-3-amino-5-(ethylsulfanyl)-2-hydroxy-N'-((2-chlorophenyl)acetyl)pentanohydrazide;
(2RS,3R)-3-amino-5-(ethylsulfanyl)-2-hydroxy-N'-((3-chlorophenyl)acetyl)pentanohydrazide;
(2RS,3R)-3-amino-5-(ethylsulfanyl)-2-hydroxy-N'-((4-chlorophenyl)acetyl)pentanohydrazide;
N'-((2RS,3R)-3-amino-5-(ethylsulfanyl)-2-hydroxypentanoyl)-2-(1,1'-biphenyl)-4-ylacetohydrazide;
N'-((2RS,3R)-3-amino-5-(ethylsulfanyl)-2-hydroxypentanoyl)-2-(4-dimethylaminophenyl)acetohydrazide;
N'-((2RS,3R)-3-amino-5-(ethylsulfanyl)-2-hydroxypentanoyl)-2-(1-naphthyl)acetohydrazide;
N'-((2RS,3R)-3-amino-5-(ethylsulfanyl)-2-hydroxypentanoyl)-2-(2-naphthyl)acetohydrazide;
N'-((2RS,3R)-3-amino-5-(ethylsulfanyl)-2-hydroxypentanoyl)benzenesulfonohydrazide;
N'-((2RS,3R)-3-amino-5-(ethylsulfanyl)-2-hydroxypentanoyl)-3-chlorobenzenesulfonohydrazide;
N'-((2RS,3R)-3-amino-5-(ethylsulfanyl)-2-hydroxypentanoyl)-1-naphthalenesulfonohydrazide; and
N'-((2RS,3R)-3-amino-5-(ethylsulfanyl)-2-hydroxypentanoyl)-4-methylbenzenesulfonohydrazide.

34. A compound according to Claim 19 wherein one of R⁶ and R⁷ is hydrogen; and the other is selected from the group consisting of alkanoyl unsubstituted or substituted with one, two, or three substituents independently selected from the group consisting of alkoxycarbonyl, alkylsulfanyl, amino, and hydroxy, cycloalkyl, cycloalkylalkanoyl, (cycloalkyl)oyl, (heterocycle)alkanoyl, and (heterocycle)oyl.

35. A compound according to Claim 34 selected from the group consisting of
(2RS,3R)-3-amino-5-(ethylsulfanyl)-2-hydroxy-N'-cyclopentylpentanohydrazide;
(2RS,3R)-N'-acetyl-3-amino-5-(ethylsulfanyl)-2-hydroxypentanohydrazide;
(2RS,3R)-3-amino-5-(ethylsulfanyl)-2-hydroxy-N'-isobutyrylpentanohydrazide;
(2RS,3R)-3-amino-5-(ethylsulfanyl)-2-hydroxy-N'-(3-methylbutanoyl)pentanohydrazide;
(2RS,3R)-3-amino-5-(ethylsulfanyl)-N'-heptanoyl-2-hydroxypentanohydrazide;
(2RS,3R)-3-amino-5-(ethylsulfanyl)-2-hydroxy-N'-(tetrahydro-2-furanylcarbonyl)pentanohydrazide;
(2RS,3R)-3-amino-N'-(cyclohexylacetyl)-5-(ethylsulfanyl)-2-hydroxyheptanohydrazide;
N'-((2RS,3R)-3-amino-5-(ethylsulfanyl)-2-hydroxypentanoyl)-2-furohydrazide;
N'-((2RS,3R)-3-amino-5-(ethylsulfanyl)-2-hydroxypentanoyl)-3-furohydrazide;
N'-((2RS,3R)-3-amino-5-(ethylsulfanyl)-2-hydroxypentanoyl)-2-thiophenecarbohydrazide;
N'-((2RS,3R)-3-amino-5-(ethylsulfanyl)-2-hydroxypentanoyl)-3-thiophenecarbohydrazide;
N'-((2RS,3R)-3-amino-5-(ethylsulfanyl)-2-hydroxypentanoyl)-1H-pyrrole-2-carbohydrazide;
N'-((2RS,3R)-3-amino-5-(ethylsulfanyl)-2-hydroxypentanoyl)-1,3-thiazole-2-carbohydrazide;
N'-((2RS,3R)-3-amino-5-(ethylsulfanyl)-2-hydroxypentanoyl)-1,3-thiazole-4-carbohydrazide;
N'-((2RS,3R)-3-amino-5-(ethylsulfanyl)-2-hydroxypentanoyl)-1,3-thiazole-5-carbohydrazide;
N'-((2RS,3R)-3-amino-5-(ethylsulfanyl)-2-hydroxypentanoyl)-1H-pyrazole-5-carbohydrazide;
N'-((2RS,3R)-3-amino-5-(ethylsulfanyl)-2-hydroxypentanoyl)-1H-pyrazole-4-carbohydrazide;
N'-((2RS,3R)-3-amino-5-(ethylsulfanyl)-2-hydroxypentanoyl)-5-isoxazolecarbohydrazide;
N'-((2RS,3R)-3-amino-5-(ethylsulfanyl)-2-hydroxypentanoyl)-2-pyridinecarbohydrazide;
N'-((2RS,3R)-3-amino-5-(ethylsulfanyl)-2-hydroxypentanoyl)-2-(2-pyridinyl)acetohydrazide;
N'-((2RS,3R)-3-amino-5-(ethylsulfanyl)-2-hydroxypentanoyl)-3-pyridinecarbohydrazide;
N'-((2RS,3R)-3-amino-5-(ethylsulfanyl)-2-hydroxypentanoyl)-2-(3-pyridinyl)acetohydrazide;
N'-((2RS,3R)-3-amino-5-(ethylsulfanyl)-2-hydroxypentanoyl)-4-pyridinecarbohydrazide;
N'-((2RS,3R)-3-amino-5-(ethylsulfanyl)-2-hydroxypentanoyl)-2-(4-pyridinyl)acetohydrazide;
N'-((2RS,3R)-3-amino-5-(ethylsulfanyl)-2-hydroxypentanoyl)-3-pyridazinecarbohydrazide;
N'-((2RS,3R)-3-amino-5-(ethylsulfanyl)-2-hydroxypentanoyl)-4-pyrimidinecarbohydrazide;
N'-((2RS,3R)-3-amino-5-(ethylsulfanyl)-2-hydroxypentanoyl)-2-pyrazinecarbohydrazide;
(2RS,3R)-3-amino-N'-((2RS,3S)-3-amino-2-hydroxy-5-(isopropylsulfanyl)pentanoyl)-2-hydroxy-5-(isopropylsulfanyl)pentanohydrazide;N'-((2RS,3R)-3-amino-5-(ethylsulfanyl)-2-hydroxypentanoyl)-1,3-benzodioxole-5-carbohydrazide;(2RS,3R)-3-amino-2-hydroxy-5-(isopropylsulfanyl)-N'-(tetrahydro-2-furanylcarbonyl)pentanohydrazide;
(2R8,3R)-3-amino-2-hydroxy-5-(isopropylsulfanyl)-N'-(tetrahydro-3-furanylcarbonyl)pentanohydrazide;
(2RS,3R)-3-amino-2-hydroxy-5-(isopropylsulfanyl)-N'-cyclopentylpentanohydrazide;
N'-((2RS,3R)-3-amino-2-hydroxy-5-(isopropylsulfanyl)pentanoyl)-2-cyclopentylacetohydrazide;
(2RS,3R)-3-amino-2-hydroxy-5-(isopropylsulfanyl)-N'-cyclohexylpentanohydrazide;
N'-((2RS,3R)-3-amino-2-hydroxy-5-(isopropylsulfanyl)pentanoyl)-2-cyclohexylacetohydrazide;
N'-((2RS,3R)-3-amino-2-hydroxy-5-(isopropylsulfanyl)pentanoyl)-2-furohydrazide;
N'-((2RS,3R)-3-amino-2-hydroxy-5-(isopropylsulfanyl)pentanoyl)-3-furohydrazide;
N'-((2RS,3R)-3-amino-2-hydroxy-5-(isopropylsulfanyl)pentanoyl)-2,5-dimethyl-3-furohydrazide;
N'-((2RS,3R)-3-amino-2-hydroxy-5-(isopropylsulfanyl)pentanoyl)-2-thiophenecarbohydrazide;
N'-((2RS,3R)-3-amino-2-hydroxy-5-(isopropylsulfanyl)pentanoyl)-3-thiophenecarbohydrazide;
N'-((2RS,3R)-3-amino-2-hydroxy-5-(isopropylsulfanyl)pentanoyl)-3-methyl-2-thiophenecarbohydrazide;
N'-((2RS,3R)-3-amino-2-hydroxy-5-(isopropylsulfanyl)pentanoyl)-5-methyl-2-thiophenecarbohydrazide;
N'-((2RS,3R)-3-amino-2-hydroxy-5-(isopropylsulfanyl)pentanoyl)-1H-pyrrole-2-carbohydrazide;
N'-((2RS,3R)-3-amino-2-hydroxy-5-(isopropylsulfanyl)pentanoyl)-1-methyl-1H-pyrrole-2-carbohydrazide;
N'-((2RS,3R)-3-amino-2-hydroxy-5-(isopropylsulfanyl)pentanoyl)-1,3-thiazole-2-carbohydrazide;
N-((2RS,3R)-3-amino-2-hydroxy-5-(isopropylsulfanyl)pentanoyl)-1,3-thiazole-4-carbohydrazide;
N'-((2RS,3R)-3-amino-2-hydroxy-5-isopropylpentanoyl)-2-pyridinecarbohydrazide;
N'-((2RS,3R)-3-amino-2-hydroxy-5-(isopropylsulfanyl)pentanoyl)-6-chloro-2H-chromene-3-carbohydrazide;
N'-((2RS,3R)-3-amino-2-hydroxy-5-(isopropylsulfanyl)pentanoyl)-2-(4-morpholinyl)acetohydrazide;
N'-((2RS,3R)-3-amino-2-hydroxy-5-(isopropylsulfanyl)pentanoyl)-2-(4-methyl-1-piperazinyl)acetohydrazide; and
1-acetyl-N'-((2RS,3R)-3-amino-2-hydroxy-5-(isopropylsulfanyl)pentanoyl)-4-piperidinecarbohydrazide.

36. A compound according to Claim 19 wherein R⁶ and R⁷, together with the nitrogen atom to which they are attached, form a heterocycle unsubstituted or substituted with one, two, three, or four, substituents independently selected from the group consisting of alkanoyl, alkoxy, alkoxyalkyl, alkoxycarbonyl, alkyl, alkylsulfanylalkyl, amino, aminosulfonyl, carboxy, halo, haloalkoxy, haloalkyl, hydroxy, nitro, oxo, and thioxo wherein, the heterocycle groups can be further substituted with an aryl, arylalhyl, aryloxy, aryloxycarbonyl, or (heterocycle)alkenyl group, wherein the aryl, the aryl portion of the aryloxy, the arylalkyl, and the aryloxycarbonyl, and the heterocycle portion of the (heterocycle)alkenyl can be further substituted with one, two, three, four, or five substituents independently selected from the group consisting of alkanoyl, alkoxy, alkoxycarbonyl, alkyl, carboxy, cyano, haloalkoxy, haloalkyl, and nitro.

37. A compound according to Claim 36 which is
(2RS,3R)-3-amino-5-(ethylsulfanyl)-2-hydroxy-N-(1-piperidinyl)pentanamide.

38. A pharmaceutical composition comprising a compound of Claim I or a therapeutically acceptable salt or prodrug thereof, in combination with a therapeutically acceptable carrier.

39. A method of inhibiting angiogenesis in a mammal in recognized need of such treatment comprising administering to the mammal a therapeutically acceptable amount of a compound of Claim 1.

40. A compound which is
N'-((2S,3R)-3-amino-2-hydroxy-5-(isopropylsulfanyl)pentanoyl)-3-chlorobenzoyl hydrazide.

## Patentansprüche

1. Eine Verbindung mit der Formel (I). 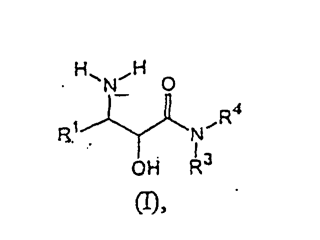 oder ein therapeutisch verträgliches Salz oder Prodrug davon, worin
R¹ gewählt ist aus der Gruppe bestehend aus Alkyl, Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, Arylalkyl, worin der Alkylteil von dem Arylalkyl unsubstituiert oder mit einer Cyanogruppe substituiert sein kann, Cycloalkyl, (Cycloalkyl)alkyl, (Heterocyclus)alkyl und R⁵S-(alkylen)-;
worin jede Gruppe so gezeichnet ist, dass ihr rechtes Ende das Ende ist, das an den Stammmolekülteil angelagert ist;
R³ ist gewählt aus der Gruppe bestehend aus Wasserstoff, Alkyl und Arylalkyl, worin der Alkylteil von dem Arylalkyl unsubstituiert oder mit einer Cyanogruppe substituiert sein kann,
R⁴ ist gewählt aus der Gruppe bestehend aus -NR⁶R⁷ und -OR⁸;
worin jede Gruppe so gezeichnet ist, dass ihr linkes Ende das Ende ist, das an den Stammmolekülteil angelagert ist;
R⁵ ist gewählt aus der Gruppe bestehend aus Alkyl, Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, Arylalkyl, worin der Alkylteil von dem Arylalkyl unsubstituiert oder mit einer Cyanogruppe substituiert sein kann, Cycloalkyl und (Cycloalkyl)alkyl;
R⁶ und R⁷ sind unabhängig gewählt aus der Gruppe bestehend aus Wasserstoff, Alkanoyl, unsubstituiert oder substituiert mit ein, zwei oder drei Substituenten, die unabhängig gewählt sind aus der Gruppe bestehend aus Alkoxycarbonyl, Alkylsulfanyl, Amino und Hydroxy, Alkenyl, Alkenyloxyalkyl, Alkoxyalkyl, Alkoxycarbonylalkyl, Alkyl, Alkylsulfanylalkyl, Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, Arylalkanoyl, Arylalkoxyalkyl, Arylalkoxycarbonyl, Arylalkyl, worin der Alkylteil von dem Arylalkyl unsubstituiert oder mit einer Cyanogruppe substituiert sein kann, Aryloxyalkyl, worin der Alkylteil von dem Aryloxyalkyl unsubstituiert oder mit einer Hydroxygruppe substituiert sein kann, (Aryl)oyl, Arylsulfonyl, Carboxyalkyl, Cycloalkyl, (Cycloalkyl)alkyl, (Cycloalkyl)alkanoyl, (Cycloalkyl)oyl, Haloalkanoyl, Haloalkyl, Heterocyclus, unsubstituiert oder substitutiert mit ein, zwei, drei oder vier Substituenten, die unabhängig gewählt sind aus der Gruppe bestehend aus Alkanoyl, Alkoxy, Alkoxyalkyl, Alkoxycarbonyl, Alkyl, Alkylsulfanylalkyl, Amino, Aminosulfonyl, Carboxy, Halo, Haloalkoxy, Haloalkyl, Hydroxy, Nitro, Oxo und Thioxo, worin die Heterocyclusgruppen weiter substituiert sein können mit einer Aryl-, Arylalkyl-, Aryloxy-, Aryloxycarbonyl- oder (Heterocyclus)alkenylgruppe, worin das Aryl, der Arylteil von dem Aryloxy, das Arylalkyl und das Aryloxycarbonyl, und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, (Heterocyclus)alkanoyl, (Heterocyclus)oyl, Hydroxyalkyl, einer Stickstoffschutzgruppe und -C(O)NR⁹R¹⁰; oder
R⁶ und R⁷ sind zusammen Arylalkyliden; oder
R⁶ und R⁷ bilden zusammen mit dem Stickstoffatom, an das sie angelagert sind, einen Heterocyclus, unsubstituiert oder substitutiert mit ein, zwei, drei oder vier Substituenten, die unabhängig gewählt sind aus der Gruppe bestehend aus Alkanoyl, Alkoxy, Alkoxyalkyl, Alkoxycarbonyl, Alkyl, Alkylsulfanylalkyl, Amino, Aminosulfonyl, Carboxy, Halo, Haloalkoxy, Haloalkyl, Hydroxy, Nitro, Oxo und Thioxo, worin die Heterocyclusgruppen weiter substituiert sein können mit einer Aryl-, Arylalkyl-, Aryloxy-, Aryloxycarbonyl- oder (Heterocyclus)alkenylgruppe, worin das Aryl, der Arylteil von dem Aryloxy, das Arylalkyl und das Aryloxycarbonyl, und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro;
R⁸ ist gewählt aus der Gruppe bestehend aus Wasserstoff, Alkanoylalkyl, Alkenyl, Alkoxycarbonylalkyl, Alkyl, Amidoalkyl, Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, Arylalkyl, worin der Alkylteil von dem Arylalkyl unsubstituiert oder mit einer Cyanogruppe substituiert sein kann, Arylalkoxycarbonylalkyl, (Aryl)oylalkyl, Carboxyalkyl und (Cycloalkyl)alkyl; und
R⁹ und R¹⁰ sind unabhängig gewählt aus der Gruppe bestehend aus Wasserstoff, Alkyl und Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro.

2. Eine Verbindung gemäß Anspruch 1, worin R¹ gewählt ist aus der Gruppe bestehend aus Alkyl, Cycloalkyl, (Cycloalkyl)alkyl, Arylalkyl, worin der Alkylteil von dem Arylalkyl unsubstituiert oder mit einer Cyanogruppe substituiert sein kann, und (Heterocyclus)alkyl.

3. Eine Verbindung gemäß Anspruch 2, worin R⁴ -OR⁸ ist.

4. Eine Verbindung gemäß Anspruch 3, gewählt aus der Gruppe bestehend aus
(2RS,3R)-3-Amino-4-cyclohexyl-2-hydroxy-N-phenoxybutanamid;
(2RS,3R)-3-Amino-N-(benzyloxy)-4-cyclohexyl-2-hydroxybutanamid;
(2RS,3R)-3-Amino-N-(methoxy)-4-cyclohexyl-2-hydroxybutanamid;
(2RS,3R)-3-Amino-N-(tert-butoxy)-4-cyclohexyl-2-hydroxybutanamid;
(2RS,3R)-3-Amino-4-cyclohexyl-N-ethoxy-2-hydroxybutanamid;
(2RS,3R)-N-(Allyloxy)-3-amino-4-cyclohexyl-2-hydroxybutanamid;
(2RS,3R)-3-Amino-4-cyclohexyl-2-hydroxy-N-isobutoxybutanamid;
(2RS,3R)-3-Amino-4-cyclohexyl-N,2-dihydroxybutanamid;
(2RS,3R)-3-Amino-3-cyclohexyl-2-hydroxy-N-phenoxypropanamid;
(2RS,3R)-3-Amino-5-phenyl-2-hydroxy-N-phenoxypentanamid;
(2RS,3R)-3-Amino-3-cyclooctyl-2-hydroxy-N-phenoxypropanamid;
(2RS,3R)-3-Amino-5-cyclohexyl-2-hydroxy-N-phenoxypentanamid;
Ethyl-((((2RS,3R)-3-amino-4-cyclohexyl-2-hydroxybutanoyl)amino)oxy)acetat;
Benzyl-((((2RS,3R)-3-amino-4-cyclohexyl-2-hydroxybutanoyl)amino)oxy)acetat;
((((2RS,3R)-3-Amino-4-cyclohexyl-2-hydroxybutanoyl)amino)oxy)essigsäure;
Ethyl-(2S)-2-((((((2RS,3R)-3-amino-4-cyclohexyl-2-hydroxybutanoyl)amino)oxy)acetyl)amino)propanoat;
(2RS,3R)-3-Amino-4-cyclohexyl-2-hydroxy-N-(2-oxo-2-((2-phenylethyl)amino)ethoxy)butanamid;
(2RS,3R)-3-Amino-N-(benzyloxy)-3-cyclohexyl-2-hydroxypropanamid; und
(2RS,3R)-3-Amino-N-(benzyloxy)-2-hydroxy-5-phenylpentanamid.

5. Eine Verbindung gemäß Anspruch 2, worin R⁴ -NR⁶R⁷ ist.

6. Eine Verbindung gemäß Anspruch 5, worin eines von R⁶ und R⁷ Wasserstoff ist und das andere (Aryl)oyl ist.

7. Eine Verbindung gemäß Anspruch 6, gewählt aus der Gruppe bestehend aus
N'-((2RS,3R)-3-Amino-4-cyclohexyl-2-hydroxybutanoyl)-1-naphthohydrazid;
N'-((2RS,3R)-3-Amino-4-cyclohexyl-2-hydroxybutanoyl)-4-ethoxybenzohydrazid;
N'-((2RS,3R)-3-Amino-4-cyclohexyl-2-hydroxybutanoyl)-3,5-dimethoxybenzohydrazid;
N'-((2RS,3R)-3-Amino-4-cyclohexyl-2-hydroxybutanoyl)-2-chlorbenzohydrazid;
N'-((2RS,3R)-3-Amino-4-cyclohexyl-2-hydroxybutanoyl)-3-brombenzohydrazid;
N'-((2RS,3R)-3-Amino-4-cyclohexyl-2-hydroxybutanoyl)-3-methoxybenzohydrazid;
N'-((2RS,3R)-3-Amino-4-cyclohexyl-2-hydroxybutanoyl)-2,5-dichlorbenzohydrazid;
N'-((2RS,3R)-3-Amino-4-cyclohexyl-2-hydroxybutanoyl)-2-methoxybenzohydrazid;
N'-((2RS,3R)-3-Amino-4-cyclohexyl-2-hydroxybutanoyl)-3-chlorbenzohydrazid;
N'-((2RS,3R)-3-Amino-4-cyclohexyl-2-hydroxybutanoyl)-3-methylbenzohydrazid;
N'-((2RS,3R)-3-Amino-4-cyclohexyl-2-hydroxybutanoyl)-2,4-dihydroxybenzohydrazid;
N'-((2RS,3R)-3-Amino-4-cyclohexyl-2-hydroxybutanoyl)-2,5-dimethoxybenzohydrazid;
N'-((2RS,3R)-3-Amino-4-cyclohexyl-2-hydroxybutanoyl)-4-methylbenzohydrazid;
N'-((2RS,3R)-3-Amino-4-cyclohexyl-2-hydroxybutanoyl)-4-nitrobenzohydrazid;
N'-((2RS,3R)-3-Amino-4-cyclohexyl-2-hydroxybutanoyl)-2-naphthohydrazid;
N'-((2RS,3R)-3-Amino-4-cyclohexyl-2-hydroxybutanoyl)-4-chlorbenzohydrazid;
(2RS,3R)-3-Amino-N'-benzoyl-4-cyclohexyl-2-hydroxybutanohydrazid;
N'-((2RS,3R)-3-Amino-4-cyclohexyl-2-hydroxybutanoyl)-4-brombenzohydrazid;
N'-((2RS,3R)-3-Amino-4-cyclohexyl-2-hydroxybutanoyl)-4-tertbutylbenzohydrazid;
N'-((2RS,3R)-3-Amino-3-cyclohexyl-2-hydroxypropanoyl)-3-chlorbenzohydrazid;
N'-((2RS,3R)-3-Amino-2-hydroxy-5-phenylpentanoyl)-3-chlorbenzohydrazid;
N'-((2RS,3R)-3-Amino-3-cyclooctyl-2-hydroxypropanoyl)-3-chlorbenzohydrazid;
N'-((2RS,3R)-3-Amino-5-cyclohexyl-2-hydroxypentanoyl)-3-chlorbenzohydrazid;
(2RS,3R)-3-Amino-2-hydroxy-5-phenyl-N'-(1-naphthyl)pentanohydrazid;
N'-((2RS,3R)-3-Amino-3-cyclohexyl-2-hydroxypropanoyl)-2-naphthohydrazid;
N'-((2RS,3R)-3-Amino-4-cyclohexyl-2-hydroxybutanoyl)-1-naphthohydrazid;
N'-((2RS,3R)-3-Amino-4-cyclohexyl-2-hydroxybutanoyl)-3-hydroxy-2-naphthohydrazid;
N'-((2S,3R)-3-Amino-2-hydroxy-5-phenylpentanoyl)-1-naphthohydrazid;
N'-((2S,3R)-3-Amino-2-hydroxy-5-phenylpentanoyl)-3-hydroxy-2-naphthohydrazid;
N'-((2S,3R,4RS)-3-Amino-4-ethyl-2-hydroxyoctanoyl)-3-chlorbenzohydrazid;
N'-((2S,3R)-3-Amino-5-cyclohexyl-2-hydroxypentanoyl)-3-chlorbenzohydrazid;
N'-((2RS,3R)-3-Amino-4-cyclohexyl-2-hydroxybutanoyl)-2-hydroxybenzohydrazid;
N'-((2RS,3R)-3-Amino-4-cyclohexyl-2-hydroxybutanoyl)-2-methylbenzohydrazid;
2-Amino-N'-((2RS,3R)-3-amino-4-cyclohexyl-2-hydroxybutanoyl)benzohydrazid;
4-Amino-N'-((2RS,3R)-3-amino-4-cyclohexyl-2-hydroxybutanoyl)benzohydrazid;
3-Amino-N'-((2RS,3R)-3-amino-4-cyclohexyl-2-hydroxybutanoyl)benzohydrazid;
N'-((2RS,3R)-3-Amino-4-cyclohexyl-2-hydroxybutanoyl)-3-hydroxybenzohydrazid;
N'-((2RS,3R)-3-Amino-4-cyclohexyl-2-hydroxybutanoyl)-4-hydroxybenzohydrazid;
N'-((2RS,3R)-3-Amino-4-cyclohexyl-2-hydroxybutanoyl)-4-methoxybenzohydrazid;
N'-((2RS,3R)-3-Amino-4-cyclohexyl-2-hydroxybutanoyl)-2-fluorbenzohydrazid;
N'-((2RS,3R)-3-Amino-4-cyclohexyl-2-hydroxybutanoyl)-3-fluorbenzohydrazid;
N'-((2RS,3R)-3-Amino-4-cyclohexyl-2-hydroxybutanoyl)-4-fluorbenzohydrazid;
N'-((2RS,3R)-3-Amino-4-cyclohexyl-2-hydroxybutanoyl)-2-brombenzohydrazid;
N'-((2RS,3R)-3-Amino-4-cyclohexyl-2-hydroxybutanoyl)-3-cyanobenzohydrazid;
N'-((2RS,3R)-3-Amino-4-cyclohexyl-2-hydroxybutanoyl-4-cyanobenzohydrazid;
N'-((2RS,3R)-3-Amino-4-cyclohexyl-2-hydroxybutanoyl)-3-(dimethylamino)benzohydrazid;
N'-((2RS,3R)-3-Amino-4-cyclohexyl-2-hydroxybutanoyl)-4-(dimethylamino)benzohydrazid;
N'-((2RS,3R)-3-Amino-4-cyclohexyl-2-hydroxybutanoyl)-3-(trifluormethyl)benzohydrazid;
N'-((2RS,3R)-3-Amino-4-cyclohexyl-2-hydroxybutanoyl)-4-(trifluormethyl)benzohydrazid;
N'-((2RS,3R)-3-Amino-4-cyclohexyl-2-hydroxybutanoyl)-3-(trifluormethoxy)benzohydrazid;
N'-((2RS,3R)-3-Amino-4-cyclohexyl-2-hydroxybutanoyl)-4-phenoxybenzohydrazid;
N'-((2RS,3R)-3-Amino-4-cyclohexyl-2-hydroxybutanoyl)-2,4-dimethylbenzohydrazid;
N'-((2RS,3R)-3-Amino-4-cyclohexyl-2-hydroxybutanoyl)-2,5-dimethylbenzohydrazid;
N'-((2RS,3R)-3-Amino-4-cyclohexyl-2-hydroxybutanoyl)-3,4-dimethylbenzohydrazid;
N'-((2RS,3R)-3-Amino-4-cyclohexyl-2-hydroxybutanoyl)-3,5-dimethylbenzohydrazid;
N'-((2RS,3R)-3-Amino-4-cyclohexyl-2-hydroxybutanoyl)-2,3-dimethoxybenzohydrazid;
N'-((2RS,3R)-3-Amino-4-cyclohexyl-2-hydroxybutanoyl)-3,4-dimethoxybenzohydrazid;
N'-((2RS,3R)-3-Amino-4-cyclohexyl-2-hydroxybutanoyl)-3,4,5-trimethoxybenzohydrazid;
N'-((2RS,3R)-3-Amino-4-cyclohexyl-2-hydroxybutanoyl)-2,3-dichlorbenzohydrazid;
N'-((2RS,3R)-3-Amino-4-cyclohexyl-2-hydroxybutanoyl)-2,4-dichlorbenzohydrazid;
N'-((2RS,3R)-3-Amino-4-cyclohexyl-2-hydroxybutanoyl)-3,4-dichlorbenzohydrazid;
N'-((2RS,3R)-3-Amino-4-cyclohexyl-2-hydroxybutanoyl)-3,5-dichlorbenzohydrazid;
N'-((2RS,3R)-3-Amino-4-cyclohexyl-2-hydroxybutanoyl)-2,3-dimethylbenzohydrazid;
(2S,3R)-3-Amino-N'-(3-chlorbenzoyl)-2-hydroxy-5-phenylpentanohydrazid; und
(2S,3R)-3-Amino-N'-(3-chlorbenzoyl)-2-hydroxy-5-(1H-indol-3-yl)pentanohydrazid.

8. Eine Verbindung gemäß Anspruch 5, worin eines von R⁶ und R⁷ gewählt ist aus der Gruppe bestehend aus Wasserstoff, Alkyl und Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro; und das andere ist gewählt aus der Gruppe bestehend aus Wasserstoff, Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, und Arylalkyl, worin der Alkylteil von dem Arylalkyl unsubstituiert oder mit einer Cyanogruppe substituiert sein kann.

9. Eine Verbindung gemäß Anspruch 8, gewählt aus der Gruppe bestehend aus
(2RS,3R)-3-Amino-N'-benzyl-4-cyclohexyl-2-hydroxybutanohydrazid;
(2RS,3R)-3-Amino-N'-benzyl-4-cyclohexyl-2-hydroxybutanohydrazid;
(2RS,3R)-3-Amino-4-cyclohexyl-2-hydroxy-N'-(2-phenylethyl)butanohydrazid;
(2RS,3R)-3-Amino-4-cyclohexyl-2-hydroxy-N'-methyl-N'phenylbutanohydrazid;
(2RS,3R)-3-Amino-4-cyclohexyl-2-hydroxy-N'-(4-methylphenyl)butanohydrazid;
(2RS,3R)-3-Amino-4-cyclohexyl-2-hydroxy-N'-(4-methoxyphenyl)butanohydrazid;
(2RS,3R)-3-Amino-4-cyclohexyl-2-hydroxy-N'-(1-naphthyl)butanohydrazid;
(2RS,3R)-3-Amino-4-cyclohexyl-2-hydroxybutanohydrazid;
(2RS,3R)-3-Amino-4-cyclohexyl-N'-(2,4-difluorphenyl)-2-hydroxybutanohydrazid;
4-(2-((2RS,3R)-3-Amino-4-cyclohexyl-2-hydroxybutanoyl)hydrazino)benzensulfonamid;
(2RS,3R)-3-Amino-4-cyclohexyl-2-hydroxy-N'-phenylbutanohydrazid;
(2RS,3R)-3-Amino-N'-(4-bromphenyl)-4-cyclohexyl-2-hydroxybutanohydrazid;
(2RS,3R)-3-Amino-4-cyclohexyl-2-hydroxy-N'-(2-methylphenyl)butanohydrazid;
(2RS,3R)-3-Amino-N'-(2-chlorphenyl)-4-cyclohexyl-2-hydroxybutanohydrazid;
(2RS,3R)-3-Amino-4-cyclohexyl-2-hydroxy-N'-(3-(trifluormethyl)phenyl)butanohydrazid;
(2RS,3R)-3-Amino-4-cyclohexyl-2-hydroxy-N'-(4-iodophenyl)butanohydrazid;
(2RS,3R)-3-Amino-N'-(3-chlorphenyl)-4-cyclohexyl-2-hydroxybutanohydrazid;
(2RS,3R)-3-Amino-4-cyclohexyl-2-hydroxy-N'-(3-methoxyphenyl)butanohydrazid;
(2RS,3R)-3-Amino-4-cyclohexyl-N'-(3,5-dichlorphenyl)-2-hydroxybutanohydrazid;
(2RS,3R)-3-Amino-N'-(3-bromphenyl)-4-cyclohexyl-2-hydroxybutanohydrazid;
(2RS,3R)-3-Amino-4-cyclohexyl-2-hydroxy-N'-(4-isopropylphenyl)butanohydrazid;
(2RS,3R)-3-Amino-N'-(3-chlor-4-methylphenyl)-4-cyclohexyl-2-hydroxybutanohydrazid;
(2RS,3R)-3-Amino-4-cyclohexyl-2-hydroxy-N'-(4-(trifluormethoxy)phenyl)butanohydrazid;
(2RS,3R)-3-Amino-4-cyclohexyl-N'-(4-fluorphenyl)-2-hydroxybutanohydrazid;
(2RS,3R)-3-Amino-N'-(4-chlorphenyl)-4-cyclohexyl-2-hydroxybutanohydrazid;
(2RS,3R)-3-Amino-4-cyclohexyl-N'-(2-ethylphenyl)-2-hydroxybutanohydrazid;
(2RS,3R)-3-Amino-4-cyclohexyl-N'-(3-fluorphenyl)-2-hydroxybutanohydrazid;
(2RS,3R)-3-Amino-N'-(4-chlor-2-methylphenyl)-4-cyclohexyl-2-hydroxybutanohydrazid;
(2RS,3R)-3-Amino-4-cyclohexyl-2-hydroxy-N'mesitylbutanohydrazid;
(2RS,3R)-3-Amino-4-cyclohexyl-2-hydroxy-N'-(4-((E)-2-(2-pyridinyl)ethenyl)phenyl)butanohydrazid;
(2RS,3R)-3-Amino-4-cyclohexyl-N'-(2-fluorphenyl)-2-hydroxybutanohydrazid;
(2RS,3R)-3-Amino-4-cyclohexyl-2-hydroxy-N'-(4-(trifluormethyl)phenyl)butanohydrazid;
(2RS,3R)-3-Amino-N'-(2-chlor-6-fluorphenyl)-4-cyclohexyl-2-hydroxybutanohydrazid;
(2RS,3R)-3-Amino-4-cyclohexyl-N'-(2,5-difluorphenyl)-2-hydroxybutanohydrazid;
(2RS,3R)-3-Amino-N'-(3-chlor-4-fluorphenyl)-4-cyclohexyl-2-hydroxybutanohydrazid;
(2RS,3R)-3-Amino-N'-(2-chlorbenzyl)-4-cyclohexyl-2-hydroxybutanohydrazid;
(2RS,3R)-3-Amino-4-cyclohexyl-2-hydroxy-N'-phenyl-N'-(3-phenylpropyl)butanohydrazid;
(2RS,3R)-3-Amino-4-cyclohexyl-2-hydroxy-N'-isobutyl-N'phenylbutanohydrazid;
(2RS,3R)-3-Amino-4-cyclohexyl-2-hydroxy-N'-pentyl-N'phenylbutanohydrazid;
(2RS,3R)-3-Amino-4-cyclohexyl-2-hydroxy-N'-((2RS)-2-methylbutyl)-N'-phenylbutanohydrazid;
(2RS,3R)-3-Amino-4-cyclohexyl-2-hydroxy-N'-isopentyl-N'phenylbutanohydrazid;
(2RS,3R)-3-Amino-4-cyclohexyl-N'-hexyl-2-hydroxy-N'phenylbutanohydrazid;
(2RS,3R)-3-Amino-4-cyclohexyl-2-hydroxy-N'-((2RS)-2-methylpentyl)-N'-phenylbutanohydrazid;
(2RS,3R)-3-Amino-4-cyclohexyl-2-hydroxy-N'-((3RS)-3-methylpentyl)-N'-phenylbutanohydrazid;
(2RS,3R)-3-Amino-4-cyclohexyl-N'-(3,3-dimethylbutyl)-2-hydroxy-N'-phenylbutanohydrazid;
(2RS,3R)-3-Amino-4-cyclohexyl-N'-(2-ethylbutyl)-2-hydxoxy-N'phenylbutanohydrazid;
(2RS,3R)-3-Amino-4-cyclohexyl-N'-dodecyl-2-hydroxy-N'phenylbutanohydrazid;
(2RS,3R)-3-Amino-4-cyclohexyl-2-hydroxy-N'-phenyl-N'-((3RS)-3,5,5-trimethylhexyl)butanohydrazid;
(2RS,3R)-3-Amino-4-cyclohexyl-2-hydroxy-N'-octyl-N'phenylbutanohydrazid;
(2RS,3R)-3-Amino-4-cyclohexyl-2-hydroxy-N'-phenyl-N'propylbutanohydrazid;
(2RS,3R)-3-Amino-4-cyclohexyl-N'-heptyl-2-hydroxy-N'phenylbutanohydrazid;
(2RS,3R)-3-Amino-4-cyclohexyl-N'-ethyl-2-hydroxy-N'phenylbutanohydrazid;
(2RS,3R)-3-Amino-4-cyclohexyl-2-hydroxy-N'-(2-(4-methylphenyl)ethyl-N'-phenylbutanohydrazid;
(2RS,3R)-3-Amino-4-cyclohexyl-N'-((2RS)-2-ethylhexyl)-2-hydroxy-N'-phenylbutanohydrazid;
(2RS,3R)-3-Amino-N'-((2RS)-2-(4-chlorphenyl)-2-cyanoethyl)-4-cyclohexyl-2-hydroxy-N'-phenylbutanohydrazid;
(2RS,3R)-3-Amino-4-cyclohexyl-2-hydroxy-N'-phenyl-N'-((2RS)-2-phenylpropyl)butanohydrazid;
(2RS,3R)-3-Amino-3-cyclohexyl-2-hydroxy-N'-(4-methylphenyl)propanohydrazid;
(2RS,3R)-3-Amino-2-hydroxy-5-phenyl-N'-(4-methylphenyl)pentanohydrazid;
(2S,3R)-3-Amino-3-cyclooctyl-2-hydroxy-N'-(4-methylphenyl)propanohydrazid;
(2RS,3S)-3-Amino-4-(cyclohexylmethyl)sulfanyl)-2-hydroxy-N'-(4-methylphenyl)butanohydrazid;
(2RS,3R)-3-Amino-3-cyclohexyl-2-hydroxy-N'-(1-naphthyl)propanohydrazid;
(2RS,3R)-3-Amino-2-hydroxy-5-phenyl-N'-(1-naphthyl)pentanohydrazid;
(2RS,3R)-3-Amino-3-cyclooctyl-2-hydroxy-N'-(1-naphthyl)propanohydrazid;
(2RS,3R)-3-Amino-5-cyclohexyl-2-hydroxy-N'-(4-methylphenyl)pentanohydrazid;
(2RS,3R)-3-Amino-5-cyclohexyl-2-hydroxy-N'-(1-naphthyl)pentanohydrazid; und
(2S,3R)-3-Amino-4-cyclohexyl-2-hydroxybutanohydrazid.

10. Eine Verbindung gemäß Anspruch 5, worin eines von R⁶ und R⁷ gewählt ist aus der Gruppe bestehend aus Wasserstoff, Alkyl und Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro; und das andere ist gewählt aus der Gruppe bestehend aus Alkanoyl, unsubstituiert oder substituiert mit ein, zwei oder drei Substituenten, die unabhängig gewählt sind aus der Gruppe bestehend aus Alkoxycarbonyl, Alkylsulfanyl, Amino und Hydroxy, Alkenyl, Alkenyloxyalkyl, Alkoxyalkyl, Alkoxycarbonylalkyl, Alkylsulfanylalkyl, Arylalkoxyalkyl, Arylalkoxycarbonyl, Arylsulfonyl, Carboxyalkyl, -C(O)R⁹R¹⁰, Cycloalkyl, (Cycloalkyl)alkyl, Haloalkanoyl, Haloalkyl, Heterocyclus, unsubstituiert oder substitutiert mit ein, zwei, drei oder vier Substituenten, die unabhängig gewählt sind aus der Gruppe bestehend aus Alkanoyl, Alkoxy, Alkoxyalkyl, Alkoxycarbonyl, Alkyl, Alkylsulfanylalkyl, Amino, Aminosulfonyl, Carboxy, Halo, Haloalkoxy, Haloalkyl, Hydroxy, Nitro, Oxo und Thioxo, worin die Heterocyclusgruppen weiter substituiert sein können mit einer Aryl-, Arylalkyl-, Aryloxy-, Aryloxycarbonyl- oder (Heterocyclus)alkenylgruppe, worin das Aryl, der Arylteil von dem Aryloxy, das Arylalkyl und das Aryloxycarbonyl, und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, (Heterocyclus)oyl und Hydroxyalkyl.

11. Eine Verbindung gemäß Anspruch 10, gewählt aus der Gruppe bestehend aus
(2RS,3R)-3-Amino-N'-(7-chlor-4-chinolinyl)-4-cyclohexyl-2-hydroxybutanohydrazid;
2-((2RS,3R)-3-Amino-4-cyclohexyl-2-hydroxybutanoyl)-N-(4-iodophenyl)hydrazincarboxamid;
N'-((2RS,3R)-3-Amino-4-cyclohexyl-2-hydroxybutanoyl)-2,4,6-trimethylbenzensulfonohydrazid;
Ethyl-(2-((2RS,3R)-3-amino-4-cyclohexyl-2-hydroxybutanoyl)hydrazino)acetattrifluoracetat;
Benzyl-2-((2RS,3R)-3-amino-4-cyclohexyl-2-hydroxybutanoyl)hydrazincarboxylat;
Ethyl-3-(2-((2RS,3R)-3-amino-4-cyclohexyl-2-hydroxybutanoyl)hydrazino)-3-oxopropanoat;
(2RS,3R)-3-Amino-4-cyclohexyl-2-hydroxy-N'-(2,2,2-trifluorethyl)butanohydrazid;
(2RS,3R)-3-Amino-4-cyclohexyl-2-hydroxy-N'-methyl-N'-(3-nitro-2-pyridinyl)butanohydrazid;
(2RS,3R)-3-Amino-N',4-dicyclohexyl-2-hydroxybutanohydrazid;
(2RS,3R)-3-Amino-4-cyclohexyl-2-hydroxy-N'-(2-pyridinyl)butanohydrazid;
(2RS,3R)-3-Amino-N'-(6-chlor-3-pyridazinyl)-4-cyclohexyl-2-hydroxy-N'-methylbutanohydrazid;
(2RS,3R)-3-Amino-N'-(5-chlor-1-methyl-6-oxo-1,6-dihydro-4-pyridazinyl)-4-cyclohexyl-2-hydroxy-N'-methylbutanohydrazid;
N'-((2RS,3R)-3-Amino-4-cyclohexyl-2-hydroxybutanoyl)-1,3-benzodioxol-5-carbohydrazid;
Methyl-2-(2-((2RS,3R)-3-amino-4-cyclohexyl-2-hydroxybutanoyl)hydrazino)-4-(trifluormethyl)-5-pyrimidincarboxylat;
(2RS,3R)-3-Amino-4-cyclohexyl-2-hydroxy-N'-((2RS)-2-hydroxy-3-(3-(trifluormethyl)phenoxy)propyl)-N'-methylbutanohydrazid;
Methyl-3-(2-((2RS,3R)-3-amino-4-cyclohexyl-2-hydroxybutanoyl)hydrazino)-2-thiophencarboxylat;
(2RS,3R)-3-Amino-4-cyclohexyl-2-hydroxy-N'-(2-pyridinylcarbonyl)butanohydrazid;
Ethyl-3-(2-((2RS,3R)-3-amino-4-cyclohexyl-2-hydroxybutanoyl)hydrazino)-6,6-dimethyl-4-oxo-4,5,6,7-tetrahydro-2-benzothiophen-1-carboxylat;
(2RS,3R)-3-Amino-N'-(1,3-benzothiazol-2-yl)-4-cyclohexyl-2-hydroxybutanohydrazid;
(2RS,3R)-3-Amino-4-cyclohexyl-2-hydroxy-N'-(trifluoracetyl)butanohydrazid;
N'-((2RS,3R)-3-Amino-4-cyclohexyl-2-hydroxybutanoyl)-3-chlor-1-benzothiophen-2-carbohydrazid;
(2RS,3R)-3-Amino-4-cyclohexyl-2-hydroxy-N'-(2-chinoxalinyl)butanohydrazid;
(2RS,3R)-3-Amino-4-cyclohexyl-2-hydroxy-N'-methyl-N'-(5-(trifluormethyl)-2-pyridinyl)butanohydrazid;
(2RS,3R)-3-Amino-4-cyclohexyl-N'-(1,3-dimethyl-4-nitro-1H-pyrazol-5-yl)-2-hydroxybutanohydrazid;
2-((2RS,3R)-3-Amino-4-cyclohexyl-2-hydroxybutanoyl)-N-phenylhydrazincarboxamid;
2-((2RS,3R)-3-Amino-4-cyclohexyl-2-hydroxybutanoyl)-N-(4-chlor-2-methoxyphenyl)hydrazincarboxamid;
2-((2RS,3R)-3-Amino-4-cyclohexyl-2-hydroxybutanoyl)-N-(3-fluorphenyl)hydrazincarboxamid;
N-((1R)-1-((2-((2RS,3R)-3-Amino-4-cyclohexyl-2-hydroxybutanoyl)hydrazino)carbonyl)-3-(methylsulfanyl)propyl)-4-(trifluormethyl)benzamid;
(2RS,3R)-3-Amino-4-cyclohexyl-2-hydroxy-N'-(2-thienylcarbonyl)butanohydrazide;
4-Chlorbenzyl-2-(2-((2RS,3R)-3-amino-4-cyclohexyl-2-hydroxybutanoyl)hydrazino)-4-(trifluormethyl)-5-pyrimidincarboxylat;
(2RS,3R)-3-Amino-N'-(6-chloro-3-pyridazinyl)-4-cyclohexyl-2-hydroxybutanohydrazid;
(2RS,3R)-3-Amino-4-cyclohexyl-2-hydroxy-N'-((3RS)-3-(methylsulfanyl)butyl)-N'-phenylbutanohydrazid;
(2RS,3R)-3-Amino-4-cyclohexyl-N'-(cyclopropylmethyl)-2-hydroxy-N'-phenylbutanohydrazid;
(2RS,3R)-3-Amino-N'-(2-(benzyloxy)ethyl)-4-cyclohexyl-2-hydroxy-N'-phenylbutanohydrazid;
(2RS,3R)-3-Amino-4-cyclohexyl-2-hydroxy-N'-phenyl-N'-(2,2,5-trichlorpentyl)butanohydrazid;
(2RS,3R)-3-Amino-4-cyclohexyl-2-hydroxy-N'-(3-(methylsulfanyl)propyl)-N'-phenylbutanohydrazid;
(2RS,3R)-3-Amino-4-cyclohexyl-N'-(cyclopentylmethyl)-2-hydroxy-N'-phenylbutanohydrazid;
(2RS,3R)-3-Amino-4-cyclohexyl-2-hydroxy-N'-(5-hydroxypentyl)-N'phenylbutanohydrazid;
(2RS,3R)-3-Amino-4-cyclohexyl-N'-((2R)-2,3-dihydroxypropyl)-2-hydroxy-N'-phenylbutanohydrazid;
(2RS,3R)-3-Amino-4-cyclohexyl-N'-(2,2-dichlorhexyl)-2-hydroxy-N'-phenylbutanohydrazid;
(2RS,3R)-3-Amino-4-cyclohexyl-2-hydroxy-N'-((3RS)-7-methoxy-3,7-dimethyloctyl)-N'-phenylbutanohydrazid;
(2RS,3R)-3-Amino-4-cyclohexyl-N'-(cyclooctylmethyl)-2-hydroxy-N'-phenylbutanohydrazid;
(2RS,3R)-3-Amino-4-cyclohexyl-N'-((11Z)-11-hexadecenyl)-2-hydroxy-N'-phenylbutanohydrazid;
(2RS,3R)-3-Amino-4-cyclohexyl-2-hydroxy-N'-phenyl-N'-tridecylbutanohydrazid;
4-(2-((2RS,3R)-3-Amino-4-cyclohexyl-2-hydroxybutanoyl)-1-phenylhydrazino)butansäure;
(2RS,3R)-3-Amino-4-cyclohexyl-2-hydroxy-N'-((6Z)-6-nonenyl)-N'phenylbutanohydrazid;
(2RS,3R)-3-Amino-4-cyclohexyl-N'-((4Z)-4-decenyl)-2-hydroxy-N'phenylbutanohydrazid;
(2RS,3R)-3-Amino-4-cyclohexyl-2-hydroxy-N'-(4-pentenyl)-N'phenylbutanohydrazid;
(2RS,3R)-3-Amino-4-cyclohexyl-N'-((3RS)-3,7-dimethyl-6-octenyl)-2-hydroxy-N'-phenylbutanohydrazid;
(2RS,3R)-3-Amino-4-cyclohexyl-2-hydroxy-N'-phenyl-N'-(4,4,4-trifluorbutyl)butanohydrazid;
(2RS,3R)-3-Amino-4-cyclohexyl-2-hydroxy-N'-((3RS)-3-hydroxybutyl)-N'-phenylbutanohydrazid;
(2RS,3R)-3-Amino-4-cyclohexyl-N'-(2-(((3RS)-3,7-dimethyl-6-octenyl)oxy)ethyl)-2-hydroxy-N'-phenylbutanohydrazid;
(2RS,3R)-3-Amino-4-cyclohexyl-N'-(2-((1RS)-3,3-dimethylcyclohexyl)ethyl)-2-hydroxy-N'-phenylbutanohydrazid;
(2RS,3R)-3-Amino-N'-((4S)-6-bromo-4-methylhexyl)-4-cyclohexyl-2-hydroxy-N'-phenylbutanohydrazid; und
(2RS,3R)-3-Amino-4-cyclohexyl-N'-(cyclohexylmethyl)-2-hydroxy-N'-phenylbutanohydrazid.

12. Eine Verbindung gemäß Anspruch 5, worin R⁶ und R⁷ zusammen Arylalkyliden sind.

13. Eine Verbindung gemäß Anspruch 12, welche (2RS,3R)-3-Amino-N'-((E)-(4-chlorphenyl)methyliden)-4-cyclohexyl-2-hydroxybutanohydrazid ist.

14. Eine Verbindung gemäß Anspruch 5, worin R⁶ und R⁷ zusammen mit dem Stickstoffatom, an welches sie angelagert sind, einen Heterocyclus bilden, unsubstituiert oder substitutiert mit ein, zwei, drei oder vier Substituenten, die unabhängig gewählt sind aus der Gruppe bestehend aus Alkanoyl, Alkoxy, Alkoxyalkyl, Alkoxycarbonyl, Alkyl, Alkylsulfanylalkyl, Amino, Aminosulfonyl, Carboxy, Halo, Haloalkoxy, Haloalkyl, Hydroxy, Nitro, Oxo und Thioxo, worin die Heterocyclusgruppen weiter substituiert sein können mit einer Aryl-, Arylalkyl-, Aryloxy-, Aryloxycarbonyl- oder (Heterocyclus)alkenylgruppe, worin das Aryl, der Arylteil von dem Aryloxy, das Arylalkyl und das Aryloxycarbonyl, und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro.

15. Eine Verbindung gemäß Anspruch 14, gewählt aus der Gruppe bestehend aus
(2RS,3R)-3-Amino-4-cyclohexyl-2-hydroxy-N-((2S)-2-(methoxymethyl)pyrrolidinyl)butanamid;
(2RS,3R)-3-Amino-4-cyclohexyl-2-hydroxy-N-(1-pyrrolidinyl)butanamid;
(2RS,3R)-3-Amino-4-cyclohexyl-2-hydroxy-N-(4-oxo-2-thioxo-1,3-thiazolidin-3-yl)butanamid;
(2RS,3R)-3-Amino-4-cyclohexyl-2-hydroxy-N-(1-piperidinyl)butanamid; und
(2RS,3R)-3-Amino-4-cyclohexyl-2-hydroxy-N-((2R)-2-(methoxymethyl)pyrrolidinyl)butanamid.

16. Eine Verbindung gemäß Anspruch 1, worin R¹ R⁵S-(Alkylen)-ist.

17. Eine Verbindung gemäß Anspruch 16, worin R⁴ -OR⁸ ist.

18. Eine Verbindung gemäß Anspruch 17, gewählt aus der Gruppe bestehend aus
(2RS,3R)-3-Amino-5-(ethylsulfanyl)-2-hydroxy-N-phenoxypentamid;
(2RS,3S)-3-Amino-4-(ethylsulfanyl)-2-hydroxy-N-phenoxybutanamid;
(2RS,3S)-3-Amino-2-hydroxy-N-phenoxy-4-(propylsulfanyl)butanamid;
(2RS,3R)-3-Amino-2-hydroxy-5-(isopropylsulfanyl)-N-phenoxypentanamid;
(2RS,3S)-3-Amino-2-hydroxy-4-(isobutylsulfanyl)-N-phenoxybutanamid;
(2RS,3S)-3-Amino-4-((cyclohexylmethyl)sulfanyl)-2-hydroxy-N-phenoxybutanamid;
(2RS,3R)-3-Amino-5-(ethylsulfanyl)-2-hydroxy-N-benzyloxypentanamid;
(2RS,3R)-3-Amino-N-(benzyloxy)-2-hydroxy-5-(isopropylsulfanyl)pentanamid;
(2RS,3S)-3-Amino-N-(benzyloxy)-2-hydroxy-4-(isobutylsulfanyl)butanamid;
(2S,3S)-3-Amino-N-(cyclohexylmethoxy)-2-hydroxy-4-(isobutylsulfanyl)butanamid;
(2S,3S)-3-Amino-2-hydroxy-4-(isobutylsulfanyl)-N-(mesitylmethoxy)butanamid;
(2RS,3R)-3-Amino-2-hydroxy-5-(ethylsulfanyl)-N-((1RS)-1-phenylethoxy)pentanamid;
(2S,3S)-3-Amino-N-(benzyloxy)-2-hydroxy-4-(isobutylsulfanyl)-N-methylbutanamid;
(2RS,3S)-3-Amino-N-(benzyloxy)-2-hydroxy-5-(isopropylsulfanyl)-N-methylpentanamid;
und
(2RS,3R)-3-Amino-2-hydroxy-5-(isopropylsulfanyl)-N-((1RS)-1-phenylethoxy)pentanamid.

19. Eine Verbindung gemäß Anspruch 18, worin R⁴ -NR⁶R⁷ ist.

20. Eine Verbindung gemäß Anspruch 19, worin eines von R⁶ und R⁷ Wasserstoff und das andere (Aryl)oyl ist.

21. Eine Verbindung gemäß Anspruch 20, worin das Aryl(oyl) unsubstituiert ist.

22. Eine Verbindung gemäß Anspruch 21, gewählt aus der Gruppe bestehend aus
N'-((2RS,3R)-3-Amino-5-(ethylsulfanyl)-2-hydroxypentanoyl)-2-naphthohydrazid;
N'-((2RS,3R)-3-Amino-5-(ethylsulfanyl)-2-hydroxypentanoyl)benzohydrazid;
N'-((2RS,3R)-3-Amino-2-hydroxy-5-isopropylsulfanylpentanoyl)-2-naphthohydrazid;
N'-((2RS,3S)-3-Amino-2-hydroxy-4-(isobutylsulfanyl)butanoyl)-2-naphthohydrazid;
N'-((2RS,3R)-3-Amino-5-(ethylsulfanyl)-2-hydroxypentanoyl)-1-naphthohydrazid; und
N'-((2RS,3R)-3-Amino-2-hydroxy-5-(isopropylsulfanyl)pentanoyl)-1-naphthohydrazid.

23. Eine Verbindung gemäß Anspruch 20, worin das Aryl(oyl) mit einem Substituenten substituiert ist.

24. Eine Verbindung gemäß Anspruch 23, worin der Substituent an der 2-Position ist.

25. Eine Verbindung gemäß Anspruch 24, gewählt aus der Gruppe bestehend aus
N'-((2RS,3R)-3-Amino-5-(ethylsulfanyl)-2-hydroxypentanoyl)-2-chlorbenzohydrazid;
N'-((2RS,3R)-3-Amino-5-(ethylsulfanyl)-2-hydroxypentanoyl)-3-hydroxy-2-naphthohydrazid;
N'-((2RS,3R)-3-Amino-5-(ethylsulfanyl)-2-hydroxypentanoyl)-2-methylbenzohydrazid;
N'-((2RS,3R)-3-Amino-5-(ethylsulfanyl)-2-hydroxypentanoyl)-2-aminobenzohydrazid;
N'-((2RS,3R)-3-Amino-5-(ethylsulfanyl)-2-hydroxypentanoyl)-2-hydroxybenzohydrazid;
N'-((2RS,3R)-3-Amino-5-(ethylsulfanyl)-2-hydroxypentanoyl)-2-fluorbenzohydrazid;
N'-((2RS,3R)-3-Amino-5-(ethylsulfanyl)-2-hydroxypentanoyl)-2-brombenzohydrazid;
N'-((2RS,3R)-3-Amino-2-hydroxy-5-(isopropylsulfanyl)pentanoyl)-2-methylbenzohydrazid;
N'-((2RS,3R)-3-Amino-2-hydroxy-5-(isopropylsulfanyl)pentanoyl)-2-methoxybenzohydrazid;
N'-((2RS,3R)-3-Amino-2-hydroxy-5-(isopropylsulfanyl)pentanoyl)-2-fluorbenzohydrazid;
N'-((2RS,3R)-3-Amino-2-hydroxy-5-(isopropylsulfanyl)pentanoyl)-2-chlorbenzohydrazid;
N'-((2RS,3R)-3-Amino-2-hydroxy-5-(isopropylsulfanyl)pentanoyl)-2-brombenzohydrazid;
N'-((2RS,3R)-3-Amino-2-hydroxy-5-(isopropylsulfanyl)pentanoyl)-2-ethoxybenzohydrazid;
N'-((2RS,3R)-3-Amino-2-hydroxy-5-(isopropylsulfanyl)pentanoyl)-2-benzylbenzohydrazid;
N'-((2RS,3R)-3-Amino-2-hydroxy-5-(isopropylsulfanyl)pentanoyl)-2-anilinobenzohydrazid;
und
N'-((2RS,3R)-3-Amino-2-hydroxy-5-(isopropylsulfanyl)pentanoyl)-2-(2-phenylethyl)benzohydrazid.

26. Eine Verbindung gemäß Anspruch 23, worin der Substituent an der 3-Position ist.

27. Eine Verbindung gemäß Anspruch 23, gewählt aus der Gruppe bestehend aus
N'-((2RS,3R)-3-Amino-5-(ethylsulfanyl)-2-hydroxypentanoyl)-3-chlorbenzohydrazid;
N'-((2RS,3S)-3-Amino-4-(ethylsulfanyl)-2-hydroxybutanoyl)-3-chlorbenzohydrazid;
N'-((2RS,3S)-3-Amino-2-hydroxy-4-(propylsulfanyl)butanoyl)-3-chlorbenzohydrazid;
N'-((2RS,3R)-3-Amino-2-hydroxy-5-(isopropylsulfanyl)pentanoyl)-3-chlorbenzohydrazid;
N'-((2RS,3S)-3-Amino-2-hydroxy-4-(isobutylsulfanyl)butanoyl)-3-chlorbenzohydrazid;
N'-((2RS,3S)-3-Amino-4-((cyclohexylmethyl)sulfanyl)-2-hydroxybutanoyl)-3-chlorbenzohydrazid;
N'-((2S,3R)-3-Amino-2-hydroxy-5-(isopropylsulfanyl)pentanoyl-3-chlorbenzohydrazid;
N'-((2S,3R)-3-Amino-5-(ethylsulfanyl)-2-hydroxypentanoyl)-3-chlorbenzohydrazid;
N'-((2R,3R)-3-Amino-5-(ethylsulfanyl)-2-hydroxypentanoyl)-3-chlorbenzohydrazid;
N'-((2R,3R)-3-Amino-2-hydroxy-5-(isopropylsulfanyl)pentanoyl)-3-chlorbenzohydrazid;
3-(2-Aminoethyl)-N'-((2S,3S)-3-amino-2-hydroxy-4-(isobutylsulfanyl)butanoyl)benzohydrazid;
N'-((2S,3S)-3-Amino-2-hydroxy-4-(isobutylsulfanyl)butanoyl)-3-propoxybenzohydrazid;
N'-((2RS,3R)-3-Amino-5-(ethylsulfanyl)-2-hydroxypentanoyl)-3-trifluormethylsulfanylbenzohydrazid;
N'-((2RS,3R)-3-Amino-5-(ethylsulfanyl)-2-hydroxypentanoyl)-3-methylbenzohydrazid;
N'-((2RS,3R)-3-Amino-5-(ethylsulfanyl)-2-hydroxypentanoyl)-3-aminobenzohydrazid;
N'-((2RS,3R)-3-Amino-S-(ethylsulfanyl)-2-hydroxypentanoyl)-3-hydroxybenzohydrazid;
N'-((2RS,3R)-3-Amino-5-(ethylsulfanyl)-2-hydroxypentanoyl)-3-methoxybenzohydrazid;
N'-((2RS,3R)-3-Amino-5-(ethylsulfanyl)-2-hydroxypentanoyl)-3-fluorbenzohydrazid;
N'-((2RS,3R)-3-Amino-5-(ethylsulfanyl)-2-hydroxypentanoyl)-3-(dimethylamino)benzohydrazid;
N'-((2RS,3R)-3-Amino-5-(ethylsulfanyl)-2-hydroxypentanoyl)-3-(trifluormethyl)benzohydrazid;
N'-((2RS,3R)-3-Amino-5-(ethylsulfanyl)-2-hydroxypentanoyl)-3-(trifluormethoxy)benzohydrazid;
(2RS,3R)-3-Amino-N'-(3-(2-aminoethoxy)phenyl)-2-hydroxy-5-(isopropylsulfanyl)pentanohydrazid;
N'-((2RS,3R)-3-Amino-5-(ethylsulfanyl)-2-hydroxypentanoyl)-3-brombenzohydrazid;
N'-((2RS,3R)-3-Amino-5-(ethylsulfanyl)-2-hydroxypentanoyl)-3-cyanobenzohydrazid;
N'-((2RS,3R)-3-Amino-2-hydroxy-5-(isopropylsulfanyl)pentanoyl)-3-hydroxybenzohydrazid;
N'-((2RS,3R)-3-Amino-2-hydroxy-5-(isopropylsulfanyl)pentanoyl)-3-methylbenzohydrazid;
N'-((2RS,3R)-3-Amino-2-hydroxy-5-(isopropylsulfanyl)pentanoyl)-3-methoxybenzohydrazid;
N'-((2RS,3R)-3-Amino-2-hydroxy-5-(isopropylsulfanyl)pentanoyl)-3-fluorbenzohydrazid;
N'-((2RS,3R)-3-Amino-2-hydroxy-5-(isopropylsulfanyl)pentanoyl)-3-brombenzohydrazid;
N'-((2RS,3R)-3-Amino-2-hydroxy-5-(isopropylsulfanyl)pentanoyl)-3-(methylsulfanyl)benzohydrazid;
N'-((2RS,3R)-3-Amino-2-hydroxy-5-(isopropylsulfanyl)pentanoyl)-3-(ethylsulfanyl)benzohydrazid;
N'-((2RS,3R)-3-Amino-2-hydroxy-5-(isopropylsulfanyl)pentanoyl)-3-(propylsulfanyl)benzohydrazid;
N'-((2RS,3R)-3-Amino-2-hydroxy-5-(isopropylsulfanyl)pentanoyl)-3-(butylsulfanyl)benzohydrazid;
N'-((2RS,3R)-3-Amino-2-hydroxy-5-(isopropylsulfanyl)pentanoyl)-3-(hexylsulfanyl)benzohydrazid;
N'-((2RS,3R)-3-Amino-2-hydroxy-5-(isopropylsulfanyl)pentanoyl)-3-(isopropylsulfanyl)benzohydrazid;
N'-((2RS,3R)-3-Amino-2-hydroxy-5-(isopropylsulfanyl)pentanoyl)-3-(isobutylsulfanyl)benzohydrazid;
N'-((2RS,3R)-3-Amino-2-hydroxy-5-(isopropylsulfanyl)pentanoyl)-3-((4-methylpentyl)sulfanyl)benzohydrazid;
N'-((2RS,3R)-3-Amino-2-hydroxy-5-(isopropylsulfanyl)pentanoyl)-3-(secbutylsulfanyl)benzohydrazid;
N'-((2RS,3R)-3-Amino-2-hydroxy-5-(isopropylsulfanyl)pentanoyl)-3-(neopentylsulfanyl)benzohydrazid;
N'-((2RS,3R)-3-Amino-2-hydroxy-5-(isopropylsulfanyl)pentanoyl)-3-(cyclohexylsulfanyl)benzohydrazid;
N'-((2RS,3R)-3-Amino-2-hydroxy-5-(isopropylsulfanyl)pentanoyl)-3-((cyclohexylmethyl)sulfanyl)benzohydrazid;
N'-((2RS,3R)-3-Amino-2-hydroxy-5-(isopropylsulfanyl)pentanoyl)-3-(benzylsulfanyl)benzohydrazid;
N'-((2RS,3R)-3-Amino-2-hydroxy-5-(isopropylsulfanyl)pentanoyl)-3-((2-phenylethyl)sulfanyl)benzohydrazid;
N'-((2RS,3R)-3-Amino-2-hydroxy-5-(isopropylsulfanyl)pentanoyl)-3-((3-phenylpropyl)sulfanyl)benzohydrazid;
N'-((2RS,3R)-3-Amino-2-hydroxy-5-(isopropylsulfanyl)pentanoyl)-3-(((1,1'-biphenyl)-4-ylmethyl)sulfanyl)benzohydrazid; und
(2RS,3R)-3-Amino-N'-(3-(2,3 -dihydroxypropoxy)benzoyl)-2-hydroxy-5-(isopropylsulfanyl)pentanohydrazid.

28. Eine Verbindung gemäß Anspruch 23, worin der Substituent an der 4-Position ist.

29. Eine Verbindung gemäß Anspruch 28, gewählt aus der Gruppe bestehend aus
N'-((2RS,3R)-3-Amino-5-(ethylsulfanyl)-2-hydroxypentanoyl)-4-methylbenzohydrazid;
N'-((2RS,3R)-3-Amino-5-(ethylsulfanyl)-2-hydroxypentanoyl)-4-aminobenzohydrazid;
N'-((2RS,3R)-3-Amino-5-(ethylsulfanyl)-2-hydroxypentanoyl)-4-hydroxybenzohydrazid;
N'-((2RS,3R)-3-Amino-5-(ethylsulfanyl)-2-hydroxypentanoyl)-4-methoxybenzohydrazid;
N'-((2RS,3R)-3-Amino-5-(ethylsulfanyl)-2-hydroxypentanoyl)-4-fluorbenzohydrazid;
N'-((2RS,3R)-3-Amino-5-(ethylsulfanyl)-2-hydroxypentanoyl)-4-brombenzohydrazid;
N'-((2RS,3R)-3-Amino-5-(ethylsulfanyl)-2-hydroxypentanoyl)-4-isopropylbenzohydrazid;
N'-((2RS,3R)-3-Amino-5-(ethylsulfanyl)-2-hydroxypentanoyl)-4-propoxybenzohydrazid;
N'-((2RS,3R)-3-Amino-5-(ethylsulfanyl)-2-hydroxypentanoyl)-4-(methylsulfanyl)benzohydrazid;
N'-((2RS,3R)-3-Amino-5-(ethylsulfanyl)-2-hydroxypentanoyl)-4-isopropoxybenzohydrazid;
N'-((2RS,3R)-3-Amino-5-(ethylsulfanyl)-2-hydroxypentanoyl)-4-(diethylamino)benzohydrazid;
N'-((2RS,3R)-3-Amino-5-(ethylsulfanyl)-2-hydroxypentanoyl)-4-butoxybenzohydrazid;
N'-((2RS,3R)-3-Amino-5-(ethylsulfanyl)-2-hydroxypentanoyl)-4-chlorbenzohydrazid;
N'-((2RS,3R)-3-Amino-5-(ethylsulfanyl)-2-hydroxypentanoyl)-4-(dimethylamino)benzohydrazid;
N'-((2RS,3R)-3-Amino-5-(ethylsulfanyl)-2-hydroxypentanoyl)-4-(trifluormethyl)benzohydrazid;
N'-((2RS,3R)-3-Amino-5-(ethylsulfanyl)-2-hydroxypentanoyl)-4-phenoxybenzohydrazid;
N'-((2RS,3R)-3-Amino-5-(ethylsulfanyl)-2-hydroxypentanoyl)-4-(phenoxymethyl)benzohydrazid;
N'-((2RS,3R)-3-Amino-5-(ethylsulfanyl)-2-hydroxypentanoyl)-4-cyanobenzohydrazid;
N'-((2RS,3R)-3-Amino-2-hydroxy-5-(isopropylsulfanyl)pentanoyl)-4-methylbenzohydrazid;
N'-((2RS,3R)-3-Amino-2-hydroxy-5-(isopropylsulfanyl)pentanoyl)-4-methoxybenzohydrazid;
N'-((2RS,3R)-3-Amino-2-hydroxy-5-(isopropylsulfanyl)pentanoyl)-4-fluorbenzohydrazid;
N'-((2RS,3R)-3-Amino-2-hydroxy-5-(isopropylsulfanyl)pentanoyl)-4-chlorbenzohydrazid;
N'-((2RS,3R)-3-Amino-2-hydroxy-5-(isopropylsulfanyl)pentanoyl)-4-brombenzohydrazid;
N'-((2RS,3R)-3-Amino-2-hydroxy-5-(isopropylsulfanyl)pentanoyl)-4-ethylbenzohydrazid;
N'-((2RS,3R)-3-Amino-2-hydroxy-5-(isopropylsulfanyl)pentanoyl)-4-propylbenzohydrazid;
N'-((2RS,3R)-3-Amino-2-hydroxy-5-(isopropylsulfanyl)pentanoyl)-4-isopropylbenzohydrazid;
N'-((2RS,3R)-3-Amino-2-hydroxy-5-(isopropylsulfanyl)pentanoyl)-4-ethoxybenzohydrazid;
N'-((2RS,3R)-3-Amino-2-hydroxy-5-(isopropylsulfanyl)pentanoyl)-4-tert-butylbenzohydrazid;
N'-((2RS,3R)-3-Amino-2-hydroxy-5-(isopropylsulfanyl)pentanoyl)-4-propoxybenzohydrazid;
N'-((2RS,3R)-3-Amino-2-hydroxy-5-(isopropylsulfanyl)pentanoyl)-4-isopropoxybenzohydrazid;
N'-((2RS,3R)-3-Amino-2-hydroxy-5-(isopropylsulfanyl)pentanoyl)-4-(methylsulfonyl)benzohydrazid;
N'-((2RS,3R)-3-Amino-2-hydroxy-5-(isopropylsulfanyl)pentanoyl)-4-(methylsulfanyl)benzohydrazid;
N'-((2RS,3R)-3-Amino-2-hydroxy-5-(isopropylsulfanyl)pentanoyl)-4-(ethylsulfanyl)benzohydrazid;
N'-((2RS,3R)-3-Amino-2-hydroxy-5-(isopropylsulfanyl)pentanoyl)-4-(propylsulfanyl)benzohydrazid;
N'-((2RS,3R)-3-Amino-2-hydroxy-5-(isopropylsulfanyl)pentanoyl)-4-(butylsulfanyl)benzohydrazid;
N'-((2RS,3R)-3-Amino-2-hydroxy-5-(isopropylsulfanyl)pentanoyl)-4-(hexylsulfanyl)benzohydrazid;
N'-((2RS,3R)-3-Amino-2-hydroxy-5-(isopropylsulfanyl)pentanoyl)-4-(isopropylsulfanyl)benzohydrazid;
N'-((2RS,3R)-3-Amino-2-hydroxy-5-(isopropylsulfanyl)pentanoyl)-4-(isobutylsulfanyl)benzohydrazid;
N'-((2RS,3R)-3-Amino-2-hydroxy-5-(isopropylsulfanyl)pentanoyl)-4-((4-methylpentyl)sulfanyl)benzohydrazid;
N'-((2RS,3R)-3-Amino-2-hydroxy-5-(isopropylsulfanyl)pentanoyl)-4-(secbutylsulfanyl)benzohydrazid;
N'-((2RS,3R)-3-Amino-2-hydroxy-5-(isopropylsulfanyl)pentanoyl)-4-(neopentylsulfanyl)benzohydrazid;
N'-((2RS,3R)-3-Amino-2-hydroxy-5-(isopropylsulfanyl)pentanoyl)-4-(cyclohexylsulfanyl)benzohydrazid;
N'-((2RS,3R)-3-Amino-2-hydroxy-5-(isopropylsulfanyl)pentanoyl)-4-((cyclohexylmethyl)sulfanyl)benzohydrazid;
N'-((2RS,3R)-3-Amino-2-hydroxy-5-(isopropylsulfanyl)pentanoyl)-4-(benzylsulfanyl)benzohydrazid;
N'-((2RS,3R)-3-Amino-2-hydroxy-5-(isopropylsulfanyl)pentanoyl)-4-((2-phenylethyl)sulfanyl)benzohydrazid;
N'-((2RS,3R)-3-Amino-2-hydroxy-5-(isopropylsulfanyl)pentanoyl)-4-((3-phenylpropyl)sulfanyl)benzohydrazid; und
N'-((2RS,3R)-3-Amino-2-hydroxy-5-(isopropylsulfanyl)pentanoyl)-4-(((1,1'-biphenyl)-4-ylmethyl)sulfanyl)benzohydrazid.

30. Eine Verbindung gemäß Anspruch 20, worin das Aryl(oyl) mit zwei oder drei Substituenten substituiert ist.

31. Eine Verbindung gemäß Anspruch 30, gewählt aus der Gruppe bestehend aus
N'-((2RS,3R)-3-Amino-5-(ethylsulfanyl)-2-hydroxypentanoyl)-3,4-diethoxybenzohydrazid;
N'-((2RS,3R)-3-Amino-5-(ethylsulfanyl)-2-hydroxypentanoyl)-2,3-dimethylbenzohydrazid;
N'-((2RS,3R)-3-Amino-5-(ethylsulfanyl)-2-hydroxypentanoyl)-2,4-dimethylbenzohydrazid;
N'-((2RS,3R)-3-Amino-5-(ethylsulfanyl)-2-hydroxypentanoyl)-2,5-dimethylbenzohydrazid;
N'-((2RS,3R)-3-Amino-5-(ethylsulfanyl)-2-hydroxypentanoyl)-4,5-dimethylbenzohydrazid;
N'-((2RS,3R)-3-Amino-5-(ethylsulfanyl)-2-hydroxypentanoyl)-3,5-dimethylbenzohydrazid;
N'-((2RS,3R)-3-Amino-5-(ethylsulfanyl)-2-hydroxypentanoyl)-2,3-dimethoxybenzohydrazid;
N'-((2RS,3R)-3-Amino-5-(ethylsulfanyl)-2-hydroxypentanoyl)-2,4-dimethoxybenzohydrazid;
N'-((2RS,3R)-3-Amino-5-(ethylsulfanyl)-2-hydroxypentanoyl)-2,5-dimethoxybenzohydrazid;
N'-((2RS,3R)-3-Amino-5-(ethylsulfanyl)-2-hydroxypentanoyl)-3,4-dimethoxybenzohydrazid;
N'-((2RS,3R)-3-Amino-5-(ethylsulfanyl)-2-hydroxypentanoyl)-3,5-dimethoxybenzohydrazid;
N'-((2RS,3R)-3-Amino-5-(ethylsulfanyl)-2-hydroxypentanoyl-3,4,5-trimethoxybenzohydrazid;
N'-((2RS,3R)-3-Amino-5-(ethylsulfanyl)-2-hydroxypentanoyl)-2,3-dichlorbenzohydrazid;
N'-((2RS,3R)-3-Amino-5-(ethylsulfanyl)-2-hydroxypentanoyl)-2,4-dichlorbenzohydrazid;
N'-((2RS,3R)-3-Amino-5-(ethylsulfanyl)-2-hydroxypentanoyl)-2,5-dichlorbenzohydrazid;
N'-((2RS,3R)-3-Amino-5-(ethylsulfanyl)-2-hydroxypentanoyl)-3,4-dichlorbenzohydrazid;
N'-((2RS,3R)-3-Amino-5-(ethylsulfanyl)-2-hydroxypentanoyl)-3,5-dichlorbenzohydrazid;
N'-((2RS,3R)-3-Amino-2-hydroxy-5-(isopropylsulfanyl)pentanoyl)-2,3-dichlorbenzohydrazid;
N'-((2RS,3R)-3-Amino-2-hydroxy-5-(isopropylsulfanyl)pentanoyl)-3-fluor-2-methylbenzohydrazid;
N'-((2RS,3R)-3-Amino-2-hydroxy-5-(isopropylsulfanyl)pentanoyl)-2,3-difluorbenzohydrazid;
N'-((2RS,3R)-3-Amino-2-hydroxy-5-(isopropylsulfanyl)pentanoyl)-5-chlor-2-methoxybenzohydrazid;
N'-((2RS,3R)-3-Amino-2-hydroxy-5-(isopropylsulfanyl)pentanoyl)-2-chlor-5-(methylsulfanyl)benzohydrazid; und
N'-((2RS,3R)-3-Amino-2-hydroxy-5-(isopropylsulfanyl)pentanoyl)-3,4-diethoxybenzohydrazid.

32. Eine Verbindung gemäß Anspruch 19, worin eines von R⁶ und R⁷ Wasserstoff ist; und das andere ist gewählt aus der Gruppe bestehend aus Aryl, unsubstituiert oder substituiert mit ein, zwei, drei, vier oder fünf Substituenten, die unabhängig gewählt sind aus der Gruppe bestehend aus Alkanoyl, Alkoxy, Alkoxyalkyl, Alkoxycarbonyl, Alkyl, Alkylsulfonyl, Alkylsulfanyl, Alkylsulfanylalkyl, Amino, Aminoalkoxy, Aminoalkyl, Aminosulfonyl, Carboxy, Cyano, (Cycloalkyl)alkylsulfanyl, Cycloalkylsulfanyl, Halo, Haloalkoxy, Haloalkyl, Haloalkylsulfanyl, (Heterocyclus)alkenyl, Hydroxy, Hydroxyalkoxy, Nitro, Oxo und Thioxo, worin die Arylgruppen weiter substituiert sein können mit einer zusätzlichen Arylgruppe oder einer Arylalkyl-, Arylalkylsulfanyl-, Aryloxy-, Aryloxyalkyl-, Heterocyclus- oder (Heterocyclus)alkenylgruppe, worin das Aryl, der Arylteil von dem Arylalkyl, das Arylalkylsulfanyl, das Aryloxy und das Aryloxyalkyl, der Heterocyclus und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro, Arylalkanoyl, Arylalkoxycarbonyl und Arylsulfonyl.

33. Eine Verbindung gemäß Anspruch 32, gewählt aus der Gruppe bestehend ,aus
Benzyl-2-((2RS,3S)-3-amino-4-((cyclohexylmethyl)sulfanyl)-2-hydroxybutanoyl)hydrazincarboxylat;
Benzyl-2-((2RS,3S)-3-amino-2-hydroxy-4-(propylsulfanyl)butanoyl)hydrazincarboxylat;
(2RS,3R)-3-Amino-5-(ethylsulfanyl)-2-hydroxy-N'-(4-methylphenyl)pentanohydrazid;
(2RS,3R)-3-Amino-5-(ethylsulfanyl)-2-hydroxy-N'-(4-methoxyphenyl)pentanohydrazid;
(2RS,3R)-3-Amino-5-(ethylsulfanyl)-2-hydroxy-N'-(1-naphthyl)pentanohydrazid;
(2RS,3R)-3-Amino-5-(ethylsulfanyl)-2-hydroxy-N'-(4-iodophenyl)pentanohydrazid;
(2RS,3R)-3-Amino-5-(ethylsulfanyl)-2-hydroxy-N'-(3-chlorphenyl)pentanohydrazid;
(2RS,3R)-3-Amino-5-(ethylsulfanyl)-2-hydroxy-N'-(3-methoxyphenyl)pentanohydrazid;
(2RS,3R)-3-Amino-5-(ethylsulfanyl)-2-hydroxy-N'-(2-chlorphenyl)pentanohydrazid;
(2RS,3R)-3-Amino-5-(ethylsulfanyl)-2-hydroxy-N'-(3-trifluormethylphenyl)pentanohydrazid;
(2RS,3R)-3-Amino-5-(ethylsulfanyl)-2-hydroxy-N'-(4-isopropyl)pentanohydrazid;
(2RS,3R)-3-Amino-5-(ethylsulfanyl)-2-hydroxy-N'-(3-chlor-4-methylphenyl)pentanohydrazid;
(2RS,3R)-3-Amino-5-(ethylsulfanyl)-2-hydroxy-N'-(3-fluorphenyl)pentanohydrazid;
(2RS,3R)-3-Amino-5-(ethylsulfanyl)-2-hydroxy-N'-(2-ethylphenyl)pentanohydrazid;
(2RS,3R)-3-Amino-5-(ethylsulfanyl)-2-hydroxy-N'-(4-fluorphenyl)pentanohydrazid;
(2RS,3R)-3-Amino-5-(ethylsulfanyl)-2-hydroxy-N'-(4-trifluormethoxyphenyl)pentanohydrazid;
(2RS,3S)-3-Amino-4-(ethylsulfanyl)-2-hydroxy-N'-(4-methylphenyl)butanohydrazid;
(2RS,3S)-3-Amino-2-hydroxy-N'-(4-methylphenyl)-4-(propylsulfanyl)butanohydrazid;
(2RS,3R)-3-Amino-2-hydroxy-5-(isopropylsulfanyl)-N'-(4-methylphenyl)pentanohydrazid;
(2RS,3S)-3-Amino-2-hydroxy-4-(isobutylsulfanyl)-N'-(4-methylphenyl)butanohydrazid;
(2RS,3S)-3-Amino-4-(ethylsulfanyl)-2-hydroxy-N'-(1-naphthyl)butanohydrazid;
(2RS,3R)-3-Amino-2-hydroxy-5-(isopropylsulfanyl)-N'-(1-naphthyl)pentanohydrazid;
(2RS,3S)-3-Amino-2-hydroxy-4-(isobutylsulfanyl)-N'-(1-naphthyl)butanohydrazid;
(2RS,3S)-3-Amino-2-hydroxy-N'-(1-naphthyl)-4-(propylsulfanyl)butanohydrazid;
(2RS,3S)-3-Amino-4-((cyclohexylmethyl)sulfanyl)-2-hydroxy-N'-(1-naphthyl)butanohydrazid;
(2RS,3R)-3-Amino-5-(ethylsulfanyl)-2-hydroxy-N'-(phenylacetyl)pentanohydrazid;
(2RS,3R)-3-Amino-5-(ethylsulfanyl)-2-hydroxy-N'-((2-methoxyphenyl)acetyl)pentanohydrazid;
(2RS,3R)-3-Amino-5-(ethylsulfanyl)-2-hydroxy-N'-((3-methoxyphenyl)acetyl)pentanohydrazid;
(2RS,3R)-3-Amino-5-(ethylsulfanyl)-2-hydroxy-N'-((4-methoxyphenyl)acetyl)pentanohydrazid;
(2RS,3R)-3-Amino-5-(ethylsulfanyl)-2-hydroxy-N'-((2-chlorphenyl)acetyl)pentanohydrazid;
(2RS,3R)-3-Amino-5-(ethylsulfanyl)-2-hydroxy-N'-((3-chlorphenyl)acetyl)pentanohydrazid;
(2RS,3R)-3-Amino-5-(ethylsulfanyl)-2-hydroxy-N'-((4-chlorphenyl)acetyl)pentanohydrazid;
N'-((2RS,3R)-3-Amino-5-(ethylsulfanyl)-2-hydroxypentanoyl)-2-(1,1'-biphenyl)-4-ylacetohydrazid;
N'-((2RS,3R)-3-Amino-5-(ethylsulfanyl)-2-hydroxypentanoyl)-2-(4-dimethylaminophenyl)acetohydrazid;
N'-((2RS,3R)-3-Amino-5-(ethylsulfanyl)-2-hydroxypentanoyl)-2-(1-naphthyl)acetohydrazid;
N'-((2RS,3R)-3-Amino-5-(ethylsulfanyl)-2-hydroxypentanoyl)-2-(2-naphthyl)acetohydrazid;
N'-((2RS,3R)-3-Amino-5-(ethylsulfanyl)-2-hydroxypentanoyl)benzensulfonohydrazid;
N'-((2RS,3R)-3-Amino-5-(ethylsulfanyl)-2-hydroxypentanoyl)-3-chlorbenzensulfonohydrazid;
N'-((2RS,3R)-3-Amino-5-(ethylsulfanyl)-2-hydroxypentanoyl)-1-naphthalensulfonohydrazid; und
N'-((2RS,3R)-3-Amino-5-(ethylsulfanyl)-2-hydroxypentanoyl)-4-methylbenzensulfonohydrazid.

34. Eine Verbindung gemäß Anspruch 19, worin eines von R⁶ und R⁷ Wasserstoff ist; und das andere ist gewählt aus der Gruppe bestehend aus Alkanoyl, unsubstituiert oder substituiert mit ein, zwei oder drei Substituenten, die unabhängig gewählt sind aus der Gruppe bestehend aus Alkoxycarbonyl, Alkylsulfanyl, Amino und Hydroxy, Cycloalkyl, Cycloalkylalkanoyl, (Cycloalkyl)oyl, (Heterocyclus)alkanoyl und (Heterocyclus)oyl.

35. Eine Verbindung gemäß Anspruch 34, gewählt aus der Gruppe bestehend aus
(2RS,3R)-3-Amino-5-(ethylsulfanyl)-2-hydroxy-N'cyclopentylpentanohydrazid;
(2RS,3R)-N'-Acetyl-3-amino-5-(ethylsulfanyl)-2-hydroxypentanohydrazid;
(2RS,3R)-3-Amino-5-(ethylsulfanyl)-2-hydroxy-N'isobutyrylpentanohydrazid;
(2RS,3R)-3-Amino-5-(ethylsulfanyl)-2-hydroxy-N'-(3-methylbutanoyl)pentanohydrazid;
(2RS,3R)-3-Amino-5-(ethylsulfanyl)-N'-heptanoyl-2-hydroxypentanohydrazid;
(2RS,3R)-3-Amino-5-(ethylsulfanyl)-2-hydroxy-N'-(tetrahydro-2-furanylcarbonyl)pentanohydrazid;
(2RS,3R)-3-Amino-N'-(cyclohexylacetyl)-5-(ethylsulfanyl)-2-hydroxyheptanohydrazid;
N'-((2RS,3R)-3-Amino-5-(ethylsulfanyl)-2-hydroxypentanoyl)-2-furohydrazid;
N'-((2RS,3R)-3-Amino-5-(ethylsulfanyl)-2-hydroxypentanoyl)-3-furohydrazid;
N'-((2RS,3R)-3-Amino-5-(ethylsulfanyl)-2-hydroxypentanoyl)-2-thiophencarbohydrazid;
N'-((2RS,3R)-3-Amino-5-(ethylsulfanyl)-2-hydroxypentanoyl)-3-thiophencarbohydrazid;
N'-((2RS,3R)-3-Amino-5-(ethylsulfanyl)-2-hydroxypentanoyl)-1Hpyrrol-2-carbohydrazid;
N'-((2RS,3R)-3-Amino-5-(ethylsulfanyl)-2-hydroxypentanoyl)-1,3-thiazol-2-carbohydrazid;
N'-((2RS,3R)-3-Amino-5-(ethylsulfanyl)-2-hydroxypentanoyl)-1,3-thiazol-4-carbohydrazid;
N'-((2RS,3R)-3-Amino-5-(ethylsulfanyl)-2-hydroxypentanoyl)-1,3-thiazol-5-carbohydrazid;
N'-((2RS,3R)-3-Amino-5-(ethylsulfanyl)-2-hydroxypentanoyl)-1Hpyrazol-5-carbohydrazid;
N'-((2RS,3R)-3-Amino-5-(ethylsulfanyl)-2-hydroxypentanoyl)-1Hpyrazol-4-carbohydrazid;
N'-((2RS,3R)-3-Amino-5-(ethylsulfanyl)-2-hydroxypentanoyl)-5-isoxazolcarbohydrazid;
N'-((2RS,3R)-3-Amino-5-(ethylsulfanyl)-2-hydroxypentanoyl)-2-pyridincarbohydrazid;
N'-((2RS,3R)-3-Amino-5-(ethylsulfanyl)-2-hydroxypentanoyl)-2-(2-pyridinyl)acetohydrazid;
N'-((2RS,3R)-3-Amino-5-(ethylsulfanyl)-2-hydroxypentanoyl)-3-pyridincarbohydrazid;
N'-((2RS,3R)-3-Amino-5-(ethylsulfanyl)-2-hydroxypentanoyl)-2-(3-pyridinyl)acetohydrazid;
N'-((2RS,3R)-3-Amino-5-(ethylsulfanyl)-2-hydroxypentanoyl)-4-pyridincarbohydrazid;
N'-((2RS,3R)-3-Amino-5-(ethylsulfanyl)-2-hydroxypentanoyl)-2-(4-pyridinyl)acetohydrazid;
N'-((2RS,3R)-3-Amino-5-(ethylsulfanyl)-2-hydroxypentanoyl)-3-pyridazincarbohydrazid;
N'-((2RS,3R)-3-Amino-5-(ethylsulfanyl)-2-hydroxypentenoyl)-4-pyrimidincarbohydrazid;
N'-((2RS,3R)-3-Amino-5-(ethylsulfanyl)-2-hydroxypentanoyl)-2-pyrazincarbohydrazid;
(2RS,3R)-3-Amino-N'-((2RS,3S)-3-amino-2-hydroxy-5-(isopropylsulfanyl)pentanoyl)-2-hydroxy-5-(isopropylsulfanyl)pentanohydrazid;
N'-((2RS,3R)-3-Amino-5-(ethylsulfanyl)-2-hydroxypentanoyl)-1,3-benzodioxol-5-carbohydrazid;
(2RS,3R)-3-Amino-2-hydroxy-5-(isopropylsulfanyl)-N'-(tetrahydro-2-furanylcarbonyl)pentanohydrazid;
(2RS,3R)-3-Amino-2-hydroxy-5-(isopropylsulfanyl)-N'-(tetrahydro-3-furanylcarbonyl)pentanohydrazid;
(2RS,3R)-3-Amino-2-hydroxy-5-(isopropylsulfanyl)-N'cyclopentylpentanohydrazid;
N'-((2RS,3R)-3-Amino-2-hydroxy-5-(isopropylsulfanyl)pentanoyl)-2-cyclopentylacetohydrazid;
(2RS,3R)-3-Amino-2-hydroxy-5-(isopropylsulfanyl)-N'cyclohexylpentanohydrazid;
N'-((2RS,3R)-3-Amino-2-hydroxy-5-(isopropylsulfanyl)pentanoyl)-2-cyclohexylacetohydrazid;
N'-((2RS,3R)-3-Amino-2-hydroxy-5-(isopropylsulfanyl)pentanoyl)-2-furohydrazid;
N'-((2RS,3R)-3-Amino-2-hydroxy-5-(isopropylsulfanyl)pentanoyl)-3-furohydrazid;
N'-((2RS,3R)-3-amino-2-hydroxy-5-(isopropylsulfanyl)pentanoyl)-2,5-dimethyl-3-furohydrazid;
N'-((2RS,3R)-3-Amino-2-hydroxy-5-(isopropylsulfanyl)pentanoyl)-2-thiophencarbohydrazid;
N'-((2RS,3R)-3-Amino-2-hydroxy-5-(isopropylsulfanyl)pentanoyl)-3-thiophencarbohydrazid;
N'-((2RS,3R)-3-Amino-2-hydroxy-5-(isopropylsulfanyl)pentanoyl)-3-methyl-2-thiophencarbohydrazid;
N'-((2RS,3R)-3-Amino-2-hydroxy-5-(isopropylsulfanyl)pentanoyl)-5-methyl-2-thiophencarbohydrazid;
N'-((2RS,3R)-3-Amino-2-hydroxy-5-(isopropylsulfanyl)pentanoyl)-1H-pyrrol-2-carbohydrazid;
N'-((2RS,3R)-3-Amino-2-hydroxy-5-(isopropylsulfanyl)pentanoyl)-1-methyl-1H-pyrrol-2-carbohydrazid;
N'-((2RS,3R)-3-Amino-2-hydroxy-5-(isopropylsulfanyl)pentanoyl)-1,3-thiazol-2-carbohydrazid;
N'-((2RS,3R)-3-Amino-2-hydroxy-5-(isopropylsulfanyl)pentanoyl)-1,3-thiazol-4-carbohydrazid;
N'-((2RS,3R)-3-Amino-2-hydroxy-5-isopropylpentanoyl)-2-pyridincarbohydrazid;
N'-((2RS,3R)-3-Amino-2-hydroxy-5-(isopropylsulfanyl)pentanoyl)-6-chlor-2H-chromen-3-carbohydrazid;
N'-((2RS,3R)-3-Amino-2-hydroxy-5-(isopropylsulfanyl)pentanoyl)-2-(4-morpholinyl)acetohydrazid;
N'-((2RS,3R)-3-Amino-2-hydroxy-5-(isopropylsulfanyl)pentanoyl)-2-(4-methyl-1-piperazinyl)acetohydrazid; und
1-Acetyl-N'-((2RS,3R)-3-amino-2-hydroxy-5-(isopropylsulfanyl)pentanoyl)-4-piperidincarbohydrazid.

36. Eine Verbindung gemäß Anspruch 19, worin R⁶ und R⁷ zusammen mit dem Stickstoffatom, an welches sie angelagert sind, einen Heterocyclus bilden, unsubstituiert oder substituiert mit ein, zwei, drei oder vier Substituenten, die unabhängig gewählt sind aus der Gruppe bestehend aus Alkanoyl, Alkoxy, Alkoxyalkyl, Alkoxycarbonyl, Alkyl, Alkylsulfanylalkyl, Amino, Aminosulfonyl, Carboxy, Halo, Haloalkoxy, Haloalkyl, Hydroxy, Nitro, Oxo und Thioxo, worin die Heterocyclusgruppen weiter substituiert sein können mit einer Aryl-, Arylalkyl-, Aryloxy-, Aryloxycarbonyl- oder (Heterocyclus)alkenylgruppe, worin das Aryl, der Arylteil von dem Aryloxy, das Arylalkyl und das Aryloxycarbonyl, und der Heterocyclusteil von dem (Heterocyclus)alkenyl weiter substituiert sein können mit ein, zwei, drei, vier oder fünf Substituenten, die unabhängig gewählt sind aus der Gruppe bestehend aus Alkanoyl, Alkoxy, Alkoxycarbonyl, Alkyl, Carboxy, Cyano, Haloalkoxy, Haloalkyl und Nitro.

37. Eine Verbindung gemäß Anspruch 36, welche (2RS,3R)-3-Amino-5-(ethylsulfanyl)-2-hydroxy-N-(1-piperidinyl)pentanamid ist.

38. Eine pharmazeutische Zusammensetzung, die eine Verbindung von Anspruch 1 oder ein therapeutisch verträgliches Salz oder Prodrug davon umfasst, in Kombination mit einem therapeutisch verträglichen Träger.

39. Ein Verfahren zum Hemmen von Angiogenese in einem Säugetier mit erkanntem Bedarf für eine solche Behandlung, welches das Verabreichen einer therapeutisch verträglichen Menge einer Verbindung von Anspruch 1 an ein Säugetier umfasst.

40. Eine Verbindung, welche N'-((2S,3R)-3-Amino-2-hydroxy-5-(isopropylsulfanyl)pentanoyl)-3-chlorbenzoylhydrazid ist.

## Revendications

1. Composé répondant à la formule (I) 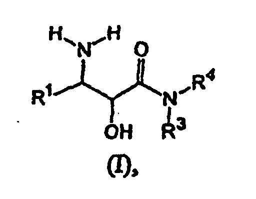 ou sel ou promédicament thérapeutiquement acceptable de celui-ci, dans lequel
R¹ est choisi dans le groupe constitué par alkyle, aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; arylalkyle, où la partie alkyle de l'arylalkyle peut être non substituée ou substituée par un groupe cyano ; cycloalkyle, (cycloalkyl)alkyle, (hétérocyle)alkyle, et R⁵S-(alkylène)- ;
dans lequel chaque groupe est tiré par son extrémité droite, celle-ci étant l'extrémité qui est attachée au groupement moléculaire de base ;
R³ est choisi dans le groupe constitué par hydrogène, alkyle et arylalkyle, où la partie alkyle de l'arylalkyle peut être non substituée ou substituée par un groupe cyano ;
R⁴ est choisi dans le groupe constitué par -NR⁶R⁷, et -OR⁸ ;
dans lequel chaque groupe est tiré par son extrémité gauche, celle-ci étant l'extrémité qui est attachée au groupement moléculaire de base ;
R⁵ est choisi dans le groupe constitué par alkyle, aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; arylakyle, où la partie alkyle de l'arylalkyle peut être non substituée ou substituée par un groupe cyano ; cycloalkyle et (cycloalkyl)alkyle ;
R⁶ et R⁷ sont indépendamment choisis dans le groupe constitué par hydrogène, alcanoyle non substitué ou substitué par un, deux ou trois substituants indépendamment choisis dans le groupe constitué par alkoxycarbonyle, alkylsulfanyle, amino et hydroxy ; alcényle, alcényloxyalkyle, alkoxyalkyle, alkoxycarbonylalkyle, alkyle, alkylsulfanylalkyle, aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; arylalcanoyle, arylalkoxyalkyle, arylalkoxycarbonyle, arylalkyle, où la partie alkyle de l'arylalkyle peut être non substituée ou substituée par un groupe cyano ; aryloxyalkyle, où la partie alkyle de l'aryloxyalkyle peut être non substituée ou substituée par un groupe hydroxy ; (aryl)oyle ; arylsulfonyle, carboxyalkyle, cycloalkyle, (cycloalkyl)alkyle, (cycloalkyl)alcanoyle, (cycloalkyl)oyle, haloalcanoyle, haloalkyle, hétérocycle non substitué or substitué par un, deux, trois, ou quatre substituants indépendamment choisis dans le groupe constitué par alcanoyle, alkoxy, alkoxyalkyle, alkoxycarbonyle, alkyle, alkylsulfanylalkyle, amino, aminosulfonyle, carboxy, halo, haloalkoxy, haloalkyle, hydroxy, nitro, oxo, et thioxo, où les groupes hétérocycle peuvent être également substitués par un groupe aryle, arylalkyle, aryloxy, aryloxycarbonyle, ou (hétérocycle)alcényle, où l'aryle, la portion aryle de l'aryloxy, l'arylalkyle, et l'aryloxycarbonyle, et la portion hétérocycle de l'(hétérocycle)acényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle et nitro ; (hétérocyle)alcanoyle, (hétérocycle)oyle, hydroxyalkyle, un groupe protecteur azote et C(O)NR⁹R¹⁰ ; ou
R⁶ et R⁷ ensemble sont arylalkylidène ; ou
R⁶ et R⁷, ensemble avec l'atome d'azote auquel ils sont attachés, forment un hétérocycle non substitué or substitué par un, deux, trois, ou quatre substituants indépendamment choisis dans le groupe constitué par alcanoyle, alkoxy, alkoxyalkyle, alkoxycarbonyle, alkyle, alkylsulfanylalkyle, amino, aminosulfonyle, carboxy, halo, haloalkoxy, haloalkyle, hydroxy, nitro, oxo, et thioxo, où les groupes hétérocycle peuvent être également substitués par un groupe aryle, arylalkyle, aryloxy, aryloxycarbonyle, ou (hétérocycle)alcényle, où l'aryle, la portion aryle de l'aryloxy, l'arylalkyle, et l'aryloxycarbonyle, et la portion hétérocycle de l'(hétérocycle)acényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle et nitro ;
R⁸ est choisi dans le groupe constitué par hydrogène, alcanoylalkyle, alcényle, alkoxycarbonylalkyle, alkyle, amidoalkyle, aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkyisulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; arylalkyle où la partie alkyle de l'arylalkyle peut être non substituée ou substituée par un groupe cyano ; arylalkoxycarbonylalkyle, (aryl)oylalkyle, carboxyalkyle et (cycloalkyl)alkyle ; et
R⁹ et R¹⁰ sont indépendamment choisis dans le groupe constitué par hydrogène, alkyle et aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro.

2. Composé selon la revendication 1, dans lequel R¹ est choisi dans le groupe constitué par alkyle, cycloalkyle, (cycloalkyl)alkyle, arylalkyle où la partie alkyle de l'arylalkyle peut être non substituée ou substituée par un groupe cyano ; et (hétérocycle)alkyle.

3. Composé selon la revendication 2, dans lequel R⁴ est -OR⁸.

4. Composé selon la revendication 3, choisi dans le groupe constitué par
(2RS,3R)-3-amino-4-cyclohexyl-2-hydroxy-N-phénoxybutanamide ;
(2RS,3R)-3-amino-N-(benryloxy)-4-cyclohexyl-2-hydroxybutanamide ;
(2RS,3R)-3-amino-N-(méthoxy)-4-cyclohexyl-2-hydroxybutanamide ;
(2RS,3R)-3-amino-N-(tert-butoxy)-4-cyclohexyl-2-hydroxybutanamide ;
(2RS,3R)-3-amino-4-cyclohexyl-N-éthoxy-2-hydroxybutanamide ;
(2RS,3R)-N-(allyloxy)-3-amino-4-cyctohexyl-2-hydroxybutanamide ;
(2RS,3R)-3-amino-4-cyclohexyl-2-hydroxy-N-isobutoxybutanamide ;
(2RS,3R)-3-amino-4-cyclohexyl-N,2-dihydroxybutanamide ;
(2RS,3R)-3-amino-3-cyclohexyl-2-hydroxy-N-phénoxypropanamide ;
(2RS,3R)-3-amino-5-phényl-2-hydroxy-N-phénoxypentanamide ;
(2RS,3R)-3-amino-3-cyclooctyl-2-hydroxy-N-phénoxypropanamide ;
(2RS,3R)-3-amino-5-cyclohexyl-2-hydroxy-N-phénoxypentanamide ;
((((2RS,3R)-3-amino-4-cyclohexyl-2-hydroxybutanoyl)amino)oxy)acétate d'éthyle ;
((((2RS,3R)-3-amino-4-cyclohexyl-2-hydroxybutanoyl)amino)oxy)acétate de benzyle ;
acide ((((2RS,3R)-3-amino-4-cyctohexyl-2-hydroxybutanoyl)amino)oxy)acétique ;
(2S)-2-((((((2RS,3R)-3-amino-4-cyctohexy)-2-hydroxybutanoyl)amino)oxy)acétyl)amino)propanoate d'éthyle ;
(2RS,3R)-3-amino-4-cyclohexyl-2-hydroxy-N-(2-oxo-2-((2-phényléthyl)amino)éthoxy)butanamide ;
(2RS,3R)-3-amino-N-(benzyloxy)-3-cyclohexyl-2-hydroxypropanamide; et
(2RS,3R)-3-amino-N-(benzyloxy)-2-hydroxy-5-phénylpentanamide.

5. Composé selon la revendication 2, dans lequel R⁴ est -NR⁶R⁷.

6. Composé selon la revendication 5, dans lequel l'un de R⁶ et R⁷ est hydrogène et l'autre est (aryl)oyle.

7. Composé selon la revendication 6, choisi dans le groupe constitué par
N'-((2RS,3R)-3-amino-4-cyclohexyl-2-hydroxybutanoyl)-1-naphtohydrazide ;
N'-((2RS,3R)-3-amino-4-cyclohexyl-2-hydroxybutanoyl)-4-éthoxybenzohydrazide ;
N'-((2RS,3R)-3-amino-4-cyclohexyl-2-hydroxybutanoyl)-3,5-diméthoxybenzohydrazide ;
N'-((2RS,3R)-3-amino-4-cyclohexyl-2-hydroxybutanoyl)-2-chlorobenzohydrazide ;
N'-((2RS,3R)-3-amino-4-cyclohexyl-2-hydroxybutanoyl)-3-bromobenzohydrazide ;
N'-((2RS,3R)-3-amino-4-cyclohexyl-2-hydroxybutanoyl)-3-méthoxybenzohydrazide ;
N'-((2RS,3R)-3-amino-4-cyclohexyl-2-hydroxybutanoyl)-2,5-dichlorobenzohydrazide ;
N'-((2RS,3R)-3-amino-4-cyclohexyl-2-hydroxybutanoyl)-2-méthoxybenzohydrazide ;
N'-((2RS,3R)-3-amino-4-cyclohexyl-2-hydroxybutanoyl)-3-chlorobenzohydrazide ;
N'-((2RS,3R)-3-amino-4-cyclohexyl-2-hydroxybutanoyl)-3-méthylbenzohydrazide ;
N'-((2RS,3R)-3-amino-4-cyclohexyl-2-hydroxybutanoyl)-2,4-dihydroxybenzohydrazide ;
N'-((2RS,3R)-3-amino-4-cyclohexyl-2-hydroxybutanoyl)-2,5-diméthoxybenzohydrazide ;
N'-((2RS,3R)-3-amino-4-cyclohexyl-2-hydroxybutanoyl)-4-méthylbenzohydrazide ;
N'-((2RS,3R)-3-amino-4-cyclohexyl-2-hydroxybutanoyl)-4-nitrobenzohydrazide ;
N'-((2RS,3R)-3-amino-4-cyclohexyl-2-hydroxybutanoyl)-2-naphtohydrazide ;
N'-((2RS,3R)-3-amino-4-cyclohexyl-2-hydroxybutanoyl)-4-chlorobenzohydrazide ;
(2RS,3R)-3-amino-N'-benzoyl-4-cyclohexyl-2-hydroxybutanohydrazide ;
N'-((2RS,3R)-3-amino-4-cyclohexyl-2-hydroxybutanoyl)-4-bromobenzohydrazide ;
N'-((2RS,3R)-3-amino-4-cyclohexyl-2-hydroxybutanoyl)-4-tert-butylbenzohydrazide ;
N'-((2RS,3R)-3-amino-3-cyclohexyl-2-hydroxypropanoyl)-3-chlorobenzohydrazide ;
N'-((2RS,3R)-3-amino-2-hydroxy-5-phénylpentanoyl)-3-chlorobenzohydrazide ;
N'-((2RS,3R)-3-amino-3-cyclooctyl-2-hydroxypropanoyl)-3-chlorobenzohydrazide ;
N'-((2RS,3R)-3-amino-5-cyclohexyl-2-hydroxypentanoyl)-3-chlorobenzohydrazide ;
(2RS,3R)-3-amino-2-hydroxy-5-phényl-N'-(1-naphtyl)pentanohydrazide ;
N'-((2RS,3R)-3-amino-3-cyclohexyl-2-hydroxypropanoyl)-2-naphtohydrazide ;
N'-((2RS,3R)-3-amino-4-cyclohexyl-2-hydroxybutanoyl)-1-naphtohydrazide ;
N'-((2RS,3R)-3-amino-4-cyclohexyl-2-hydroxybutanoyl)-3-hydroxy-2-naphtohydrazide ;
N'-((2S,3R)-3-amino-2-hydroxy-5-phénylpentanoyl)-1-naphtohydrazide ;
N'-((2S,3R)-3-amino-2-hydroxy-5-phénylpentanoyl)-3-hydroxy-2-naphtohydrazide ;
N'-((2S,3R,4RS)-3-amino-4-éthyl-2-hydroxyoctanoyl}-3-chlorobenzohydrazide ;
N'-((2S,3R)-3-amino-5-cyclohexyl-2-hydroxypentanoyl)-3-chlorobenzohydrazide ;
N'-((2RS,3R)-3-amino-4-cyclohexyl-2-hydroxybutanoyl)-2-hydroxybenzohydrazide ;
N'-((2RS,3R)-3-amino-4-cyclohexyl-2-hydroxybutanoyl)-2-méthylbenzohydrazide ;
2-amino-N'-((2RS,3R)-3-amino-4-cyclohexyl-2-hydroxybutanoyl)benzohydrazide ;
4-amino-N'-((2RS,3R)-3-amino-4-cyclohexyl-2-hydroxybutanoyl)benzohydrazide ;
3-amino-N'-((2RS,3R)-3-amino-4-cyclohexyl-2-hydroxybutanoyl)benzohydrazide ;
N'-((2RS,3R)-3-amino-4-cyclohexyl-2-hydroxybutanoyl)-3-hydroxybenzohydrazide ;
N'-((2RS,3R)-3-amino-4-cyclohexyl-2-hydroxybutanoyl)-4-hydroxybenzohydrazide ;
N'-((2RS,3R)-3-amino-4-cyclohexyl-2-hydroxybutanoyl)-4-méthoxybenzohydrazide ;
N'-((2RS,3R)-3-amino-4-cyclohexyl-2-hydroxybutanoyl)-2-fluorobenzohydrazide ;
N'-((2RS,3R)-3-amino-4-cyclohexyl-2-hydroxybutanoyl)-3-fluorobenzohydrazide ;
N'-((2RS,3R)-3-amino-4-cyclohexyl-2-hydroxybutanoyl)-4-fluorobenzohydrazide ;
N'-((2RS,3R)-3-amino-4-cyclohexyl-2-hydroxybutanoyl)-2-bromobenzohydrazide ;
N'((2RS,3R)-3-amino-4-cyclohexyl-2-hydroxybutanoyl)-3-cyanobenzohydrazide ;
N'-((2RS,3R)-3-amino-4-cyclohexyl-2-hydroxybutanoyl)-4-cyanobenzohydrazide ;
N'-((2RS,3R)-3-amino-4-cyclohexyl-2-hydroxybutanoyl)-3-(diméthylamino)benzohydrazide ;
N'-((2RS,3R)-3-amino-4-cyclohexyl-2-hydroxybutanoyl)-4-(diméthylamino)benzohydrazide ;
N'-((2RS,3R)-3-amino-4-cyclohexyl-2-hydroxybutanoyl)-3-(trifluorométhyl)benzohydrazide ;
N'-((2RS,3R)-3-amino-4-cyclohexyl-2-hydroxybutanoyl)-4-(trifluorométhyl)benzohydrazide ;
N'-((2RS,3R)-3-amino-4-cyclohexyl-2-hydroxybutanoyl)-3-(trifluorométhoxy)benzohydrazide ;
N'-((2RS,3R)-3-amino-4-cyclohexyl-2-hydroxybutanoyl)-4-phénoxybenzohydrazide ;
N'-((2RS,3R)-3-amino-4-cyclohexyl-2-hydroxybutanoyl)-2,4-diméthylbenzohydrazide ;
N'-((2RS,3R)-3-amino-4-cyclohexyl-2-hydroxybutanoyl)-2,5-diméthylbenzohydrazide ;
N'-((2RS,3R)-3-amino-4-cyclohexyl-2-hydroxybutanoyl)-3,4-diméthylbenzohydrazide ;
N'-((2RS,3R)-3-amino-4-cyclohexyl-2-hydroxybutanoyl)-3,5-diméthylbenzohydrazide ;
N'-((2RS,3R)-3-amino-4-cyclohexyl-2-hydroxybutanoyl)-2,3-diméthoxybenzohydrazide ;
N'-((2RS,3R)-3-amino-cyclohexyl-2-hydroxybutanoyl)-3,4-diméthoxybenzohydrazide ;
N'-((2RS,3R)-3-amino-4-cyclohexyl-2-hydroxybutanoyl)-3,4,5-triméthoxybenzohydrazide ;
N'-((2RS,3R)-3-amino-4-cyclohexyl-2-hydroxybutanoyl)-2,3-dichlorobenzohydrazide ;
N'-((2RS,3R)-3-amino-4-cyclohexyl-2-hydroxybutanoyl)-2,4-dichlorobenzohydrazide ;
N'-((2RS,3R)-3-amino-4-cyclohexyt-2-hydroxybutanoyl)-3,4-dichlorobenzohydrazide ;
N'-((2RS,3R)-3-amino-4-cyclohexyl-2-hydroxybutanoyl)-3,5-dichlorobenzohydrazide ;
N'-((2RS,3R)-3-amino-4-cyclohexyl-2-hydroxybutanoyl)-2,3-diméthylbenzohydrazide ;
(2S,3R)-3-amino-N'-(3-chlorobenzoyl)-2-hydroxy-5-phénylpentanohydrazide ; et
(2S,3R)-3-amino-N'-(3-chlorobenzoyl)-2-hydroxy-5-(1H-indol-3-yl)pentanohydrazide.

8. Composé selon la revendication 5, dans lequel l'un de R⁶ et R⁷ est choisi dans le groupe constitué par hydrogène, alkyle, et aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haioalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; et l'autre est choisi dans le groupe constitué par hydrogène, aryle non substitué ou substitué par un, deux, trois, quatre ou cinq substituants indépendamment choisis dans le groupe constitué par alcanoyle, alkoxy, alkoxyalkyle, alkaxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; et arylalkyle, où la partie alkyle de l'arylalkyle peut être non substituée ou substituée par un groupe cyano.

9. Composé selon la revendication 8, choisi dans le groupe constitué par
(2RS,3R)-3-amino-N'-benzyl-4-cyclohexyl-2-hydroxybutanohydrazide ;
(2RS,3R)-3-amino-N'-benzyl-4-cyclohexyl-2-hydroxybutanohydrazide ;
(2RS,3R)-3-amino-4-cyclohexyl-2-hydroxy-N'-(2-phényléthyl)butanohydrazide ;
(2RS,3R)-3-amino-4-cyclohexyl-2-hydroxy-N'-méthyl-N'-phénylbutanohydrazide ;
(2RS,3R)-3-amino-4-cyclohexyl-2-hydroxy-N'-(4-méthylphényl)butanohydrazide ;
(2RS,3R)-3-amino-4-cyclohexyl-2-hydroxy-N'-(4-méthoxyphényl)butanohydrazide ;
(2RS,3R)-3-amino-4-cyclohexyl-2-hydroxy-N'-(1-naphtyl)butanohydrazide ;
(2RS,3R)-3-amino-4-cyclohexyl-2-hydroxybutanohydrazide ;
(2RS,3R)-3-amino-4-cyclohexyl-N'-(2,4-difluorophényl)-2-hydroxybutanohydrazide ;
4-(2-((2RS,3R)-3-amino-4-cyclohexyl-2-hydroxybutanoyl)hydrazino)benzènesulfonamide;
(2RS,3R)-3-amino-4-cyclohexyl-2-hydroxy-N'-phénylbutanohydrazide ;
(2RS,3R)-3-amino-N'-(4-bromophényl)-4-cyclohexyl-2-hydroxybutanohydrazide ;
(2RS,3R)-3-amino-4-cyclohexyl-2-hydroxy-N'-(2-méthylphényl)butanohydrazide ;
(2RS,3R)-3-amino-N'-(2-chlorophényl)-4-cycloheoyl-2-hydroxybutanohydrazide ;
(2RS,3R)-3-amino-4-cyclohexyl-2-hydroxy-N'-(3-(trifluorométhyl)phényl)butanohydrazide ;
(2RS,3R)-3-amino-4-cyclohexyl-2-hydroxy-N'-(4-iodophényl)butanohydrazide ;
(2RS,3R)-3-amino-N'-(3-chlorophényl)-4-cyclohexyl-2-hydroxybutanohydrazide ;
(2RS,3R)-3-amino-4-cyclohexyl-2-hydroxy-N'-(3-méthoxyphényl)butanohydrazide ;
(2RS,3R)-3-amino-4-cyclohexyl-N'-(3,5-dichlorophényl)-2-hydroxybutanohydrazide ;
(2RS,3R)-3-amino-N'-(3-bromophényl)-4-cyclohexyl-2-hydroxybutanohydrazide ;
(2RS,3R)-3-amino-4-cyclohexyl-2-hydroxy-N'-(4-isopropylphényl)butanohydrazide ;
(2RS,3R)-3-amino-N'-(3-chloro-4-méthylphényl)-4-cyclohexyl-2-hydroxybutanohydrazide ;
(2RS,3R)-3-amino-4-cyclohexyl-2-hydroxy-N'-(4-(trifluorométhoxy)phényl)butanohydrazide ;
(2RS,3R)-3-amino-4-cyclohexyl-N'-(4-fluorophényl)-2-hydroxybutanohydrazide ;
(2RS,3R)-3-amino-N'-(4-chlorophényl)-4-cyclohexyl-2-hydroxybutanohydrazide ;
(2RS,3R)-3-amino-4-cyclohexyl-N'-(2-éthylphényl)-2-hydroxybutanohydrazide ;
(2RS,3R)-3-amino-4-cyclohexyl-N'-(3-fluorophényl)-2-hydroxybutanohydrazide ;
(2RS,3R)-3-amino-N'-(4-chloro-2-méthylphényl)-4-cyclohexyl-2-hydroxybutanohydrazide ;
(2RS,3R)-3-amino-4-cyclohexyl-2-hydroxy-N'-mésitylbutanohydrazide ;
(2RS,3R)-3-amino-4-cyclohexyl-2-hydroxy-N'-(4-((E)-2-(2-pyridinyl)éthényl)phényl)butanohydrazide ;
(2RS,3R)-3-amino-4-cyclohexyl-N'-(2-fluorophényl)-2-hydroxybutanohydrazide ;
(2RS,3R)-3-amino-4-cyclohexyl-2-hydroxy-N'-(4-(trifluorométhyl)phényl)butanohydrazide ;
(2RS,3R)-3-amino-N'-(2-chloro-6-fluorophényl)-4-cyclohexyl-2-hydroxybutanohydrazide ;
(2RS,3R)-3-amino-4-cyclohexyl-N'-(2,5-difluorophényl)-2-hydroxybutanohydrazide ;
(2RS,3R)-3-amino-N'-(3-chloro-4-fluorophényl)-4-cyclohexyl-2-hydroxybutanohydrazide ;
(2RS,3R)-3-amino-N'-(2-chlorobenzyl)-4-cyclohexyl-2-hydroxybutanohydrazide ;
(2RS,3R)-3-amino-4-cyclohexyl-2-hydroxy-N'-phényl-N'-(3-phénylpropyl)butanohydrazide ;
(2RS,3R)-3-amino-4-cyclohexyl-2-hydroxy-N'-isobutyl-N'-phénylbutanohydrazide ;
(2RS,3R)-3-amino-4-cyclohexyl-2-hydroxy-N'-pentyl-N'-phénylbutanohydrazide ;
(2RS,3R)-3-amino-4-cyclohexyl-2-hydroxy-N'-((2RS)-2-méthylbutyl)-N'-phénylbutanohydrazide ;
(2RS,3R)-3-amino-4-cyclohexyl-2-hydroxy-N'-isopentyl-N'-phénylbutanohydrazide ;
(2RS,3R)-3-amino-4-cyclohexyl-N'-hexyl-2-hydroxy-N'-phénylbutanohydrazide ;
(2RS,3R)-3-amino-4-cyclohexyl-2-hydroxy-N'-((2RS)-2-méthylpentyl)-N'-phénylbutanohydrazide ;
(2RS,3R)-3-amino-4-cyclohexyl-2-hydroxy-N'-((3RS)-3-méthylpentyl)-N'-phénylbutanohydrazide ;
(2RS,3R)-3-amino-4-cyclohexyl-N'-(3,3-diméthylbutyl)-2-hydroxy-N'-phénylbutanohydrazide ;
(2RS,3R)-3-amino-4-cyclohexyl-N'-(2-éthylbutyl)-2-hydroxy-N'-phénylbutanohydrazide ;
(2RS,3R)-3-amino-cyclohexyl-N'-dodécyl-2-hydroxy-N'-phénylbutanohydrazide ;
(2RS,3R)-3-amino-4-cyclohexyl-2-hydroxy-N'-phényl-N'-((3RS)-3,5,5-triméthylhexyl)butanohydrazide ;
(2RS,3R)-3-amino-4-cyclohexyl-2-hydroxy-N'-octyl-N'-phénylbutanohydrazide ;
(2RS,3R)-3-amino-4-cyclohexyl-2-hydroxy-N'-phényl-N'-propylbutanohydrazide ;
(2RS,3R)-3-amino-4-cyclohexyl-N'-heptyl-2-hydroxy-N'-phénylbutanohydrazide ;
(2RS,3R)-3-amino-4-cyclohexyl-N'-éthyl-2-hydroxy-N'-phénylbutanohydrazide ;
(2RS,3R)-3-amino-4-cyclohexyl-2-hydroxy-N'-(2-(4-méthylphényl)éthyl)-N'-phénylbutanohydrazide ;
(2RS,3R)-3-amino-4-cyclohexyl-N'-((2RS)-2-éthylhexyl)-2-hydroxy-N'-phénylbutanohydrazide ;
(2RS,3R)-3-amino-N'-((2RS)-2-(4-chlorophényl)-2-cyanoéthyl)-4-cyclohexyl-2-hydroxy-N'-phénylbutanohydrazide ;
(2RS,3R)-3-amino-4-cyclohexyl-2-hydroxy-N'-phényl-N'-((2RS)-2-phénylpropyl)butanohydrazide ;
(2RS,3R)-3-amino-3-cyclohexyl-2-hydroxy-N'-(4-méthylphényl)propanohydrazide ;
(2RS,3R)-3-amino-2-hydroxy-5-phényl-N'-(4-méthylphényl)pentanohydrazide ;
(2S,3R)-3-amino-3-cyclooctyl-2-hydroxy-N'-(4-méthylphényl)propanohydrazide ;
(2RS,3S)-3-amino-4-((cyclohexylméthyl)sulfanyl)-2-hydroxy-N'-(4-méthylphényl)butanohydrazide ;
(2RS,3R)-3-amino-3-cyclohexyl-2-hydroxy-N'-(1-naphtyl)propanohydrazide ;
(2RS,3R)-3-amino-2-hydroxy-5-phényl-N'-(1-naphtyl)pentanohydrazide ;
(2RS,3R)-3-amino-3-cyclooctyl-2-hydroxy-N'-(1-naphtyl)propanohydrazide ;
(2RS,3R)-3-amino-5-cyclohexyl-2-hydroxy-N'-(4-méthylphényl)pentanohydrazide ;
(2RS,3R)-3-amino-5-cyclohexyl-2-hydroxy-N'-(1-naphtyl)pentanohydrazide ; et
(2S,3R)-3-amino-4-cyclohexyl-2-hydroxybutanohydrazide.

10. Composé selon la revendication 5, dans lequel l'un de R⁶ et R⁷ est choisi dans le groupe constitué par hydrogène, alkyle et aryle non substitué ou substitué par un, deux, trois, quatre ou cinq substituants indépendamment choisis dans le groupe constitué par alcanoyle, alkoxy, alkoxyalkyle, alkoxycarbonyle, alkyle, atkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycte)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; et l'autre est choisi dans le groupe constitué par alcanoyle non substitué ou substitué par un, deux ou trois substituants indépendamment choisis dans le groupe constitué par alkoxycarbonyle, alkylsulfanyle, amino et hydroxy ; alcényle, alcényleoxyalkyle, alkoxyalkyle, alkoxycarbonylalkyle, alkylsulfanylalkyle, arylalkoxyalkyle, arylalkoxycarbonyle, arylsulfonyle, carboxyalkyle, -C(O)R⁹R¹⁰, cycloalkyle, (cycloalkyl)alkyle, haloatcanoyle, haloalkyle; hétérocycle non substitué or substitué par un, deux, trois, ou quatre substituants indépendamment choisis dans le groupe constitué par alcanoyle, alkoxy, alkoxyalkyle, alkoxycarbonyle, alkyle, alkylsulfanylalkyle, amino, aminosulfonyle, carboxy, halo, haloalkoxy, haloalkyle, hydroxy, nitro, oxo, et thioxo, où les groupes hétérocycle peuvent être également substitués par un groupe aryle, arylalkyle, aryloxy, aryloxycarbonyle, ou (hétérocycle)alcényle, où l'aryle, la portion aryle de l'aryloxy, l'arylalkyle, et l'aryloxycarbonyle, et la portion hétérocycle de l'(hétérocycle)acényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle et nitro ; (hétérocycle)oyle, et hydroxyalkyle.

11. Composé selon la revendication 10, choisi dans le groupe constitué par
(2RS,3R)-3-amino-N'-(7-chloro-4-quinolinyl)-4-cyclohexyl-2-hydroxybutanohydrazide ;
2-((2RS,3R)-3-amino-4-cyclohexyl-2-hydroxybutanoyl)-N-(4-iodophényl)hydrazinecarboxamide ;
N'-((2RS,3R)-3-amino-4-cyclohexyl-2-hydroxybutanoyl)-2,4,6-triméthylbenzènesulfonohydrazide ;
trifluoroacétate de (2-((2RS,3R)-3-amino-4-cyclohexyl-2-hydroxybutanoyl)hydrazino)acétate d'éthyle ;
2-((2RS,3R)-3-amino-4-cyclohexyl-2- hydroxybutanoyl)hydrazinecarboxylate de benzyle ;
3-(2-((2RS,3R)-3-amino-4-cyclohexyl-2-hydroxybutanoyl)hydrazino)-3-oxopropanoate d'éthyle ;
(2RS,3R)-3-amino-4-cyclohexyl-2-hydroxy-N'-(2,2,2-trifluoroéthyl)butanohydrazide ;
(2RS,3R)-3-amino-4-cyclohexyl-2-hydroxy-N'-méthyl-N'-(3-nitro-2-pyridinyl)butanohydrazide ;
(2RS,3R)-3-amino-N',4-dicyclohexyl-2-hydroxybutanohydrazide ;
(ZRS,3R)-3-amino-4-cyclohexyl-Z-hydroxy-N'-(Z-pyridinyl)butanohydrazide ;
(ZRS,3R)-3-amino-N'-(6-chloro-3-pyridazinyl)-4-cyclohexyl-Z-hydroxy-N'-méthylbutanohydrazide ;
(2RS,3R)-3-amino-N'-(5-chloro-1-méthyl-6-oxo-1,6-dihydro-4-pyridazinyl)-4-cyclohexyl-2-hydroxy-N'-méthylbutanohydrazide ;
N'-((2RS,3R)-3-amino-4-cyclohexyl-2-hydroxybutanoyl)-1,3-benzodioxole-5-carbohydrazide ;
2-(2-((2RS,3R)-3-amino-4-cyclohexyl-2-hydroxybutanoyl)hydrazino)-4-(trifluorométhyl)-5-pyrimidinecarboxylate de méthyle ;
(2RS,3R)-3-amino-4-cyclohexyl-2-hydroxy-N'-((2RS)-2-hydroxy-3-(3-(trifluorométhyl)phénoxy)propyl)-N'-méthylbutanohydrazide ;
3-(2-((2RS,3R)-3-amino-4-cyclohexyl-2-hydroxybutanoyl)hydrazino)-2-thiophènecarboxylate de méthyle ;
(2RS,3R)-3-amino-4-cyclohexyl-2-hydroxy-N'-(2-pyridinylcarbonyl)butanohydrazide ;
3-(2-((2RS,3R)-3-amino-4-cyclohexyl-2-hydroxybutanoyl)hydrazino)-6,6-diméthyl-4-oxo-4,5,6,7-tétrahydro-2-benzothiophène-1-carboxylate d'éthyle ;
(2RS,3R)-3-amino-N'-(1,3-benzothiazoi-2-yl)-4-cyclohexyl-2-hydroxybutanohydrazide ;
(2RS,3R)-3-amino-4-cyclohexyl-2-hydroxy-N'-(trifluoroacétyl)butanohydrazide ;
N'-((2RS,3R)-3-amino-4-cyclohexyl-2-hydroxybutanoyl)-3-chloro-1-benzothiophène-2-carbohydrazide ;
(2RS,3R)-3-amino-4-cyclohexyl-2-hydroxy-N'-(2-quinoxalinyl)butanohydrazide ;
(2RS,3R)-3-amino-4-cyclohexyl-2-hydroxy-N'-méthyl-N'-(5-(trifluorométhyl)-2-pyridinyl)butanohydrazide ;
(2RS,3R)-3-amino-4-cyclohexyl-N'-(1,3-diméthyl-4-nitro-1H-pyrazol-5-yl)-2-hydroxybutanohydrazide ;
2-((2RS,3R)-3-amino-4-cyclohexyl-2-hydroxybutanoyl)-N-phénylhydrazinecarboxamide ;
2-((2RS,3R)-3-amino-4-cyclohexyl-2-hydroxybutanoyl)-N-(4-chloro-2-méthoxyphényl)hydrazinecarboxamide ;
2-((2RS,3R)-3-amino-4-cyclohexyl-2-hydroxybutanoyl)-N-(3-fluorophényl)hydrazinecarboxamide ;
N-((1R)-1-((2-((2RS,3R)-3-amino-4-cyclohexyl-2-hydroxybutanoyl)hydrazino)carbonyl)-3-(méthylsulfanyl)propyl)-4-(trifluorométhyl)benzamide ;
(2RS,3R)-3-amino-4-cyclohexyl-2-hydroxy-N'-(2-thiénylcarbonyl)butanohydrazide ;
4-chlorobenzyl 2-(2-((2RS,3R)-3-amino-4-cyclohexyl-2-hydroxybutanoyl)hydrazino)-4-(trifluorométhyl)-5-pyrimidinecarboxylate ;
(2RS,3R)-3-amino-N'-(6-chloro-3-pyridazinyl)-4-cyclohexyl-2-hydroxybutanohydrazide ;
(2RS,3R)-3-amino-4-cyclohexyl-2-hydroxy-N'-((3RS)-3-(méthylsulfanyl)butyl)-N'-phénylbutanohydrazide ;
(2RS,3R)-3-amino-4-cyclohexyl-N'-(cyclopropylméthyl)-2-hydroxy-N'-phénylbutanohydrazide ;
(2RS,3R)-3-amino-N'-(2-(benzyloxy)éthyl)-4-cyclohexyl-2-hydroxy-N'-phénylbutanohydrazide ;
(2RS,3R)-3-amino-4-cyclohexyl-2-hydroxy-N'-phényl-N'-(2,2,5-trichloropentyl)butanohydrazide ;
(2RS,3R)-3-amino-4-cyclohexyl-2-hydroxy-N'-(3-(méthylsulfanyl)propyl)-N'-phénylbutanohydrazide ;
(2RS,3R)-3-amino-4-cyclohexyl-N'-(cyclopentylméthyl)-2-hydroxy-N'-phénylbutanohydrazide ;
(2RS,3R)-3-amino-4-cyclohexyl-2-hydroxy-N'-(5-hydroxypentyl)-N'-phénylbutanohydrazide ;
(2RS,3R)-3-amino-4-cyclohexyl-N'-((2R)-2,3-dihydroxypropyl)-2-hydroxy-N'-phénylbutanohydrazide ;
(2RS,3R)-3-amino-4-cyclohexyl-N'-(2,2-dichlorohexyl)-2-hydroxy-N'-phénylbutanohydrazide ;
(2RS,3R)-3-amino-4-cyclohexyl-2-hydroxy-N'-((3RS)-7-méthoxy-3,7-diméthyloctyl)-N'-phénylbutanohydrazide ;
(2RS,3R)-3-amino-4-cyclohexyl-N'-(cyclooctylméthyl)-2-hydroxy-N'-phénylbutanohydrazide ;
(2RS,3R)-3-amino-4-cyclohexyl-N'-((11Z)-11-hexadécényl)-2-hydroxy-N'-phénylbutanohydrazide ;
(2RS,3R)-3-amino-4-cyclohexyl-2-hydroxy-N'-phényl-N'-tridécylbutanohydrazide ;
acide 4-(2-((2RS,3R)-3-amino-4-cyclohexyl-2-hydroxybutanoyl)-1-phénylhydrazino)butanoïque ;
(2RS,3R)-3-amino-4-cyclohexyl-2-hydroxy-N'-((6Z)-6-nonényl)-N'-phénylbutanohydrazide ;
(2RS,3R)-3-amino-4-cyclohexyl-N'-((4Z)-4-décényl)-2-hydroxy-N'-phénylbutanohydrazide ;
(2RS,3R)-3-amino-4-cyclohexyl-2-hydroxy-N'-(4-pentényl)-N'-phénylbutanohydrazide ;
(2RS,3R)-3-amino-4-cyclohexyl-N'-((3RS)-3,7-diméthyl-6-octényl)-2-hydroxy-N'-phénylbutanohydrazide ;
(2RS,3R)-3-amino-4-cyclohexyl-2-hydroxy-N'-phényl-N'-(4,4,4-trifluorobutyl)butanohydrazide ;
(2RS,3R)-3-amino-4-cyclohexyl-2-hydroxy-N'-((3RS)-3-hydroxybutyl)-N'-phénylbutanohydrazide ;
(2RS,3R)-3-amino-4-cyclohexyl-N'-(2-(((3RS)-3,7-diméthyl-6-octényl)oxy)éthyl)-2-hydroxy-N'-phénylbutanohydrazide ;
(2RS,3R)-3-amino-4-cyclohexyl-N'-(2-((1RS)-3,3-diméthylcyclohexyl)éthyl)-2-hydroxy-N'-phénylbutanohydrazide ;
(2RS,3R)-3-amino-N'-((4S)-6-bromo-4-méthylhexyl)-4-cyclohexyl-2-hydroxy-N'-phénylbutanohydrazide ; et
(2RS,3R)-3-amino-4-cyclohexyl-N'-(cyclohexylméthyl)-2-hydroxy-N'-phénylbutanohydrazide.

12. Composé selon la revendication 5, dans lequel R⁶ et R⁷ ensemble sont arylalkylidène.

13. Composé selon la revendication 12, qui est (2RS,3R)-3-amino-N'-((E)-(4-chlorophényl)méthylidène)-4-cyclohexyl-2-hydroxybutanohydrazide.

14. Composé selon la revendication 5, dans lequel R⁶ et R⁷, ensemble avec l'atome d'azote auquel ils sont attachés, forment un hétérocycle non substitué or substitué par un, deux, trois, ou quatre substituants indépendamment choisis dans le groupe constitué par alcanoyle, alkoxy, alkoxyalkyle, alkoxycarbonyle, alkyle, alkylsulfanylalkyle, amino, aminosulfonyle, carboxy, halo, haloalkoxy, haloalkyle, hydroxy, nitro, oxo, et thioxo, où les groupes hétérocycle peuvent être également substitués par un groupe aryle, arylalkyle, aryloxy, aryloxycarbonyle, ou (hétérocycle)alcényle, où l'aryle, la portion aryle de l'aryloxy, l'arylalkyle, et l'aryloxycarbonyle, et la portion hétérocycle de l'(hétérocycle)acényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle et nitro.

15. Composé selon la revendication 14, choisi dans le groupe constitué par
(2RS,3R)-3-amino-4-cyclohexyl-2-hydroxy-N-((2S)-2-(méthoxyméthyl)pyrrolidinyl)butanamide ;
(2RS,3R)-3-amino-4-cyclohexyl-2-hydroxy-N-( 1-pyrrolidinyl)butanamide ;
(2RS,3R)-3-amino-4-cyclohexyl-2-hydroxy-N-(4-oxo-2-thioxo-1,3-thiazolidin-3-yl)butanamide ;
(2RS,3R)-3-amino-4-cyclohexyl-2-hydroxy-N-(1-pipéridinyl)butanamide ; et
(2RS,3R)-3-amino-4-cyclohexyl-2-hydroxy-N-((2R)-2-(méthoxyméthyl)pyrrolidinyl)butanamide.

16. Composé selon la revendication 1, dans lequel R¹ est R⁵S-(alkylène)-.

17. Composé selon la revendication 16, dans lequel R⁴ est -OR⁸.

18. Composé selon la revendication 17, choisi dans le groupe constitué par
(2RS,3R)-3-amino-5-(éthylsulfanyl)-2-hydroxy-N-phénoxypentamide ;
(2RS,3S)-3-amino-4-(éthylsulfanyl)-2-hydroxy-N-phénoxybutanamide ;
(2RS,3S)-3-amino-2-hydroxy-N-phénoxy-4-(propylsulfanyl)butanamide ;
(2RS,3R)-3-amino-2-hydroxy-5-(isopropylsulfanyl)-N-phénoxypentanamide ;
(2RS,3S)-3-amino-2-hydroxy-4-(isobutylsulfanyl)-N-phénoxybutanamide ;
(2RS,3S)-3-amino-4-((cyclohexyiméthyl)sulfanyl)-2-hydroxy-N-phénoxybutanamide ;
(2RS,3R)-3-amino-5-(éthylsulfanyl)-2-hydroxy-N-benzyloxypentanamide ;
(2RS,3R)-3-amino-N-(benzyloxy)-2-hydroxy-5-(isopropylsulfanyl)pentanamide ;
(2RS,3S)-3-amino-N-(benzyloxy)-2-hydroxy-4-(isobutylsulfanyl)butanamide ;
(2S,3S)-3-amino-N-(cyclohexylméthoxy)-2-hydroxy-4-(isobutylsulfanyl)butanamide ;
(2S,3S)-3-amino-2-hydroxy-4-(isobutylsulfanyl)-N-(mésitylméthoxy)butanamide ;
(2RS,3R)-3-amino-2-hydroxy-5-(éthylsulfanyl)-N-((1RS)-1-phényléthoxy)pentanamide ;
(2S,3S)-3-amino-N-(benzyloxy)-2-hydroxy-4-(Isobutylsulfanyl)-N-méthylbutanamide ;
(2RS,3S)-3-amino-N-(benzyloxy)-2-hydroxy-5-(isopropylsulfanyl)-N-méthylpentanamide ; et
(2RS,3R)-3-amino-2-hydroxy-5-(isopropylsulfanyl)-N-((1RS)-1-phényléthoxy)pentanamide.

19. Composé selon la revendication 18, dans lequel R⁴ est -NR⁶R⁷.

20. Composé selon la revendication 19, dans lequel l'un de R⁶ et R⁷ est hydrogène et l'autre est (aryl)oyle.

21. Composé selon la revendication 20, dans lequel l'aryl(oyle) est non substitué.

22. Composé selon la revendication 21, choisi dans le groupe constitué par
N'-((2RS,3R)-3-amino-5-(éthylsulfanyl)-2-hydroxypentanoyl)-2-naphtohydrazide ;
N'-((2RS,3R)-3-amino-5-(éthylsulfanyl)-2-hydroxypentanoyl)benzohydrazide ;
N'-((2RS,3R)-3-amino-2-hydroxy-5-isopropylsulfanylpentanoyl)-2-naphtohydrazide ;
N'-((2RS,3S)-3-amino-2-hydroxy-4-(isobutylsulfanyl)butanoyl)-2-naphtohydrazide ;
N'-((2RS,3R)-3-amino-5-(éthylsulfanyl)-2-hydroxypentanoyl)-1-naphtohydrazide ; et
N'-((2RS,3R)-3-amino-2-hydroxy-5-(isopropylsulfanyl)pentanoyl)-1-naphtohydrazide.

23. Composé selon la revendication 20, dans lequel l'aryl(oyle) est substitué par un substituant.

24. Composé selon la revendication 23, dans lequel le substituant est à la position 2.

25. Composé selon la revendication 24, choisi dans le groupe constitué par
N'-((2RS,3R)-3-amino-5-(éthylsulfanyl)-2-hydroxypentanoyl)-2-chlorobenzohydrazide ;
N'-((2RS,3R)-3-amino-5-(éthylsulfanyl)-2-hydroxypentanoyl)-3-hydroxy-2-naphtohydrazide ;
N'-((2RS,3R)-3-amino-5-(éthylsulfanyl)-2-hydroxypentanoyl)-2-méthylbenzohydrazide ;
N'-((2RS,3R)-3-amino-5-(éthylsulfanyl)-2-hydroxypentanoyl)-2-aminobenzohydrazide ;
N'-((2RS,3R)-3-amino-5-(éthylsulfanyl)-2-hydroxypentanoyl)-2-hydroxybenzohydrazide ;
N'-((2RS,3R)-3-amino-5-(éthylsulfanyl)-2-hydroxypentanoyl)-2-fluorobenzohydrazide ;
N'-((2RS,3R)-3-amino-5-(éthylsulfanyl)-2-hydroxypentanoyl)-2-bromobenzohydrazide ;
N'-((2RS,3R)-3-amino-2-hydroxy-5-(isopropy(sulfanyl)pentanoyl)-2-méthylbenzohydrazide ;
N'-((2RS,3R)-3-amino-2-hydroxy-5-(isopropylsulfanyl)pentanoyl)-2-méthoxybenzohydrazide ;
N'-((2RS,3R)-3-amino-2-hydroxy-5-(isopropylsulfanyl)pentanoyl)-2-fluorobenzohydrazide ;
N'-((2RS,3R)-3-amino-2-hydroxy-5-(isopropylsulfanyl)pentanoyl)-2-chlorobenzohydrazide ;
N'-((2RS,3R)-3-amino-2-hydroxy-5-(isopropylsulfanyl)pentanoyl)-2-bromobenzohydrazide ;
N'-((2RS,3R)-3-amino-2-hydroxy-5-(isopropylsulfanyl)pentanoyl)-2-éthoxybenzohydrazide ;
N'-((2RS,3R)-3-amino-2-hydroxy-5-(isopropylsulfanyl)pentanoyl)-2-benzylbenzohydrazide ;
N'-((2RS,3R)-3-amino-2-hydroxy-5-(isopropylsulfanyl)pentanoyl)-2-anilinobenzohydrazide ;
et
N'-((2RS,3R)-3-amino-2-hydroxy-5-(isopropylsulfanyl)pentanoyl)-2-(2-phényléthyl)benzohydrazide.

26. Composé selon la revendication 23, dans lequel le substituant est à la position 3.

27. Composé selon la revendication 23, choisi dans le groupe constitué par
N'-((2RS,3R)-3-amino-5-(éthylsulfanyl)-2-hydroxypentanoyl)-3-chlorobenzohydrazide ;
N'-((2RS,3S)-3-amino-4-(éthylsulfanyl)-2-hydroxybutanoyl)-3-chlorobenzohydrazide ;
N'-((2RS,3S)-3-amino-2-hydroxy-4-(propylsulfanyl)butanoyl)-3-chlorobenzohydrazide ;
N'-((2RS,3R)-3-amino-2-hydroxy-5-(isopropylsulfanyl)pentanoyl)-3-chlorobenzohydrazide ;
N'-((2RS,3S)-3-amino-2-hydroxy-4-(isobutylsulfanyl)butanoyl)-3-chlorobenzohydrazide ;
N'-((2RS,3S)-3-amino-4-((cyclohexylméthyl)sulfanyl)-2-hydroxybutanoyl)-3-chlorobenzohydrazide ;
N'-((2S,3R)-3-amino-2-hydroxy-5-(isopropylsulfanyl)pentanoyl-3-chlorobenzohydrazide ;
N'-((2S,3R)-3-amino-5-(éthylsulfanyl)-2-hydroxypentanoyl)-3-chlorobenzohydrazide ;
N'-((2R,3R)-3-amino-5-(éthylsulfanyl)-2-hydroxypentanoyl)-3-chlorobenzohydrazide ;
N'-((2R,3R)-3-amino-2-hydroxy-5-(isopropylsulfanyl)pentanoyl)-3-chlorobenzohydrazide ;
3-(2-aminoéthyl)-N'-((2S,3S)-3-amino-2-hydroxy-4-(isobutylsulfanyl)butanoyl)benzohydrazide ;
N'-((2S,3S)-3-amino-2-hydroxy-4-(isobutylsulfanyl)butanoyl)-3-propoxybenzohydrazide ;
N'-((2RS,3R)-3-amino-5-(éthylsulfanyl)-2-hydroxypentanoyl)-3-trifluorométhylsulfanylbenzohydrazide ;
N'-((2RS,3R)-3-amino-5-(éthylsulfanyl)-2-hydroxypentanoyl)-3-méthylbenzohydrazide ;
N'-((2RS,3R)-3-amino-5-(éthylsulfanyl)-2-hydroxypentanoyl)-3-aminobenzohydrazide ;
N'-((2RS,3R)-3-amino-5-(éthylsulfanyl)-2-hydroxypentanoyl)-3-hydroxybenzohydrazide ;
N'-((2RS,3R)-3-amino-5-(éthylsulfanyl)-2-hydroxypentanoyl)-3-méthoxybenzohydrazide ;
N'-((2RS,3R)-3-amino-5-(éthylsulfanyl)-2-hydroxypentanoyl)-3-fluorobenzohydrazide ;
N'-((2RS,3R)-3-amino-5-(éthylsulfanyl)-2-hydroxypentanoyl)-3-(diméthylamino)benzohydrazide ;
N'-((2RS,3R)-3-amino-5-(éthylsulfanyl)-2-hydroxypentanoyl)-3-(trifluorométhyl)benzohydrazide ;
N'-((2RS,3R)-3-amino-5-(éthylsulfanyl)-2-hydroxypentanoyl)-3-(trifluorométhoxy)benzohydrazide ;
(2RS,3R)-3-amino-N'-(3-(2-aminoéthoxy)phényl)-2-hydroxy-5-(isopropylsulfanyl)pentanohydrazide ;
N'-((2RS,3R)-3-amino-5-(éthylsulfanyl)-2-hydroxypentanoyl)-3-bromobenzohydrazide ;
N'-((2RS,3R)-3-amino-5-(éthylsulfanyl)-2-hydroxypentanoyl)-3-cyanobenzohydrazide ;
N'-((2RS,3R)-3-amino-2-hydroxy-5-(isopropylsulfanyl)pentanoyl)-3-hydroxybenzohydrazide ;
N'-((2RS,3R)-3-amino-2-hydroxy-5-(isopropylsulfanyl)pentanoyl)-3-méthylbenzohydrazide ;
N'-((2RS,3R)-3-amino-2-hydroxy-5-(isopropylsulfanyl)pentanoyl)-3-méthoxybenzohydrazide ;
N'-((2RS,3R)-3-amino-2-hydroxy-5-(isopropylsulfanyl)pentanoyl)-3-fluorobenzohydrazide ;
N'-((2RS,3R)-3-amino-2-hydroxy-5-(isopropylsulfanyl)pentanoyl)-3-bromobenzohydrazide ;
N'-((2RS,3R)-3-amino-2-hydroxy-5-(isopropylsulfanyl)pentanoyl)-3-(méthylsulfanyl)benzohydrazide ;
N'-((2RS,3R)-3-amino-2-hydroxy-5-(isopropylsulfanyl)pentanoyl)-3-(éthylsulfanyl)benzohydrazide ;
N'-((2RS,3R)-3-amino-2-hydroxy-5-(isopropylsulfanyl)pentanoyl)-3-(propylsulfanyl)benzohydrazide ;
N'-((2RS,3R)-3-amino-2-hydroxy-5-(isopropylsulfanyl)pentanoyl)-3-(butylsulfanyl)benzohydrazide ;
N'-((2RS,3R)-3-amino-2-hydroxy-5-(isopropylsulfanyl)pentanoyl)-3-(hexylsulfanyl)benzohydrazide ;
N'-((2RS,3R)-3-amino-2-hydroxy-5-(isopropylsulfanyl)pentanoyl)-3-(isopropylsulfanyl)benzohydrazide ;
N'-((2RS,3R)-3-amino-2-hydroxy-5-(isopropylsulfanyl)pentanoyl)-3-(isobutylsulfanyl)benzohydrazide ;
N'-((2RS,3R)-3-amino-2-hydroxy-5-(isopropylsulfanyl)pentanoyl)-3-((4-méthylpentyl)sulfanyl)benzohydrazide ;
N'-((2RS,3R)-3-amino-2-hydroxy-5-(isopropylsulfanyl)pentanoyl)-3-(sec-butylsulfanyl)benzohydrazide ;
N'-((2R5,3R)-3-amino-2-hydroxy-5-(isopropylsulfanyl)pentanoyl)-3-(néopentylsulfanyl)benzohydrazide ;
N'-((2RS,3R)-3-amino-2-hydroxy-5-(isopropylsulfanyl)pentanoyl)-3-(cyclohexylsulfanyl)benzohydrazide ;
N'-((2RS,3R)-3-amino-2-hydroxy-5-(isopropylsulfanyl)pentanoyl)-3-((cyclohexylméthyl)sulfanyl)benzohydrazide ;
N'-((2RS,3R)-3-amino-2-hydroxy-5-(isopropylsulfanyl)pentanoyl)-3-(benzylsulfanyl)benzohydrazide ;
N'-((2RS,3R)-3-amino-2-hydroxy-5-(isopropylsulfanyl)pentanoyl)-3-((2-phényléthyl)sulfanyl)benzohydrazide ;
N'-((2RS,3R)-3-amino-2-hydroxy-5-(isopropylsulfanyl)pentanoyl)-3-((3-phénylpropyl)sulfanyl)benzohydrazide ;
N'-((2RS,3R)-3-amino-2-hydroxy-5-(isopropylsulfanyl)pentanoyl)-3-(((1,1'-biphényl)-4-ylméthyl)sulfanyl)benzohydrazide ; et
(2RS,3R)-3-amino-N'-(3-(2,3-dihydroxypropoxy)benzoyl)-2-hydroxy-5-(isopropylsulfanyl)pentanohydrazide.

28. Composé selon la revendication 23, dans lequel le substituant est à la position 4.

29. Composé selon la revendication 28, choisi dans le groupe constitué par
N'-((2RS,3R)-3-amino-5-(éthylsulfanyl)-2-hydroxypentanoyl)-4-méthylbenzohydrazide ;
N'-((2RS,3R)-3-amino-5-(éthylsulfanyl)-2-hydroxypentanoyl)-4-aminobenzohydrazide ;
N'-((2RS,3R)-3-amino-5-(éthylsulfanyl)-2-hydroxypentanoyl)-4-hydroxybenzohydrazide ;
N'-((2RS,3R)-3-amino-5-(éthylsulfanyl)-2-hydroxypentanoyl)-4-méthoxybenzohydrazide ;
N'-((2RS,3R)-3-amino-5-(éthylsulfanyl)-2-hydroxypentanoyl)-4-fluorobenzohydrazide ;
N'-((2RS,3R)-3-amino-5-(éthylsulfanyl)-2-hydroxypentanoyl)-4-bromobenzohydrazide ;
N'-((2RS,3R)-3-amino-5-(éthylsulfanyl)-2-hydroxypentanoyt)-4-isopropylbenzohydrazide ;
N'-((2RS,3R)-3-amino-5-(éthylsulfanyl)-2-hydroxypentanoyl)-4-propoxybenzohydrazide ;
N'-((2RS,3R)-3-amino-5-(éthylsulfanyl)-2-hydroxypentanoyl)-4-(méthylsulfanyl)benzohydrazide ;
N'-((2RS,3R)-3-amino-5-(éthyisulfanyl)-2-hydroxypentanoyl)-4-isopropoxybenzohydrazide ;
N'-((2RS,3R)-3-amino-5-(éthylsulfanyl)-2-hydroxypentanoyl)-4-(diéthylamino)benzohydrazide ;
N'-((2RS,3R)-3-amino-5-(éthy)sutfany))-2-hydroxypentanoyt)-4-butoxybenzohydrazide ;
N'-((2RS,3R)-3-amino-5-(éthylsulfanyl)-2-hydroxypentanoyl)-4-chlorobenzohydrazide ;
N'-((2RS,3R)-3-amino-5-(éthylsulfanyl)-2-hydroxypentanoyl)-4-(diméthylamino)benzohydrazide ;
N'-((2RS,3R)-3-amino-5-(éthylsulfanyl)-2-hydroxypentanoyl)-4-(trifluorométhyl)benzohydrazide ;
N'-((2RS,3R)-3-amino-5-(éthylsulfanyl)-2-hydroxypentanoyl)-4-phénoxybenzohydrazide ;
N'-((2RS,3R)-3-amino-5-(éthylsulfanyl)-2-hydroxypentanoyl)-4-(phénoxyméthyl)benzohydrazide ;
N'-((2RS,3R)-3-amino-5-(éthylsulfanyl)-2-hydroxypentanoyl)-4-cyanobenzohydrazide ;
N'-((2RS,3R)-3-amino-2-hydroxy-5-(isopropylsulfanyl)pentanoyl)-4-méthylbenzohydrazide ;
N'-((2RS,3R)-3-amino-2-hydroxy-5-(isopropylsulfanyl)pentanoyl)-4-méthoxybenzohydrazide ;
N'-((2RS,3R)-3-amino-2-hydroxy-5-(isopropylsulfanyl)pentanoyl)-4-fluorobenzohydrazide ;
N'-((2RS,3R)-3-amino-2-hydroxy-5-(isopropylsulfanyl)pentanoyl)-4-chlorobenzohydrazide ;
N'-((2RS,3R)-3-amino-2-hydroxy-5-(isopropylsulfanyl)pentanoyl)-4-bromobenzohydrazide ;
N'-((2RS,3R)-3-amino-2-hydroxy-5-(isopropylsulfanyl)pentanoyl)-4-éthylbenzohydrazide ;
N'-((2RS,3R)-3-amino-2-hydroxy-5-(isopropylsulfanyl)pentanoyl)-4-propylbenzohydrazide ;
N'-((2RS,3R)-3-amino-2-hydroxy-5-(isopropylsulfanyl)pentanoyl)-4-isopropylbenzohydrazide ;
N'-((2RS,3R)-3-amino-2-hydroxy-5-(isopropylsulfanyl)pentanoyl)-4-éthoxybenzohydrazide ;
N'-((2RS,3R)-3-amino-2-hydroxy-5-(isopropylsulfanyl)pentanoyl)-4-tert-butylbenzohydrazide ;
N'-((2RS,3R)-3-amino-2-hydroxy-5-(isopropylsulfanyl)pentanoyl)-4-propoxybenzohydrazide ;
N'-((2RS,3R)-3-amino-2-hydroxy-5-(isopropylsulfanyl)pentanoyl)-4-isopropoxybenzohydrazide ;
N'-((2RS,3R)-3-amino-2-hydroxy-5-(isopropylsulfanyl)pentanoyl)-4-(méthylsulfonyl)benzohydrazide ;
N'-((2RS,3R)-3-amino-2-hydroxy-5-(isopropylsulfanyl)pentanoyl)-4-(méthylsulfanyl)benzohydrazide ;
N'-((2RS,3R)-3-amino-2-hydroxy-5-(isopropylsulfanyl)pentanoyl)-4-(éthylsulfanyl)benzohydrazide ;
N'-((2RS,3R)-3-amino-2-hydroxy-5-(isopropylsulfanyl)pentanoyl)-4-(propylsulfanyl)benzohydrazide ;
N'-((2RS,3R)-3-amino-2-hydroxy-5-(isopropylsulfanyl)pentanoyl)-4-(butylsulfanyl)benzohydrazide ;
N'-((2RS,3R)-3-amino-2-hydroxy-5-(isopropylsulfanyl)pentanoyl)-4-(hexylsulfanyl)benzohydrazide ;
N'-((2RS,3R)-3-amino-2-hydroxy-5-(isopropylsulfanyl)pentanoyl)-4-(isopropylsulfanyl)benzohydrazide ;
N'-((2RS,3R)-3-amino-2-hydroxy-5-(isopropylsulfanyl)pentanoyl)-4-(isobutylsulfanyl)benzohydrazide ;
N'-((2RS,3R)-3-amino-2-hydroxy-5-(isopropylsulfanyl)pentanoyl)-4-((4-méthylpentyl)sulfanyl)benzohydrazide ;
N'-((2RS,3R)-3-amino-2-hydroxy-5-(isopropylsulfanyl)pentanoyl)-4-(sec-butylsulfanyl)benzohydrazide ;
N'-((2RS,3R)-3-amino-2-hydroxy-5-(isopropylsulfanyl)pentanoyl)-4-(néopentylsulfanyl)benzohydrazide ;
N'-((2RS,3R)-3-amino-2-hydroxy-5-(isopropylsulfanyl)pentanoyl)-4-(cyclohexylsulfanyl)benzohydrazide ;
N'-((2RS,3R)-3-amino-2-hydroxy-5-(isopropylsulfanyl)pentanoyl)-4-((cyclohexylméthyl)sulfanyl)benzohydrazide ;
N'-((2RS,3R)-3-amino-2-hydroxy-5-(isopropylsulfanyl)pentanoyl)-4-(benzylsulfanyl)benzohydrazide ;
N'-((2RS,3R)-3-amino-2-hydroxy-5-(isopropylsulfanyl)pentanoyl)-4-((2-phényléthyl)sulfanyl)benzohydrazide ;
N'-((2RS,3R)-3-amino-2-hydroxy-5-(isopropylsulfanyl)pentanoyl)-4-((3-phénylpropyl)sulfanyl)benzohydrazide ; et
N'-((2RS,3R)-3-amino-2-hydroxy-5-(isopropylsulfanyl)pentanoyl)-4-(((1,1'-biphényl)-4-ylméthyl)sulfanyl)benzohydrazide.

30. Composé selon la revendication 20, dans lequel l'aryl(oyle) est substitué par deux ou trois substituants.

31. Composé selon la revendication 30, choisi dans le groupe constitué par
N'-((2RS,3R)-3-amino-5-(éthylsulfanyl)-2-hydroxypentanoyl)-3,4-diéthoxybenzohydrazide ;
N'-((2RS,3R)-3-amino-5-(éthylsulfanyl)-2-hydroxypentanoyl)-2,3-diméthylbenzohydrazide ;
N'-((2RS,3R)-3-amino-5-(éthylsulfanyl)-2-hydroxypentanoyl)-2,4-diméthylbenzohydrazide ;
N'-((2RS,3R)-3-amino-5-(éthylsulfanyl)-2-hydroxypentanoyl)-2,5-diméthylbenzohydrazide ;
N'-((2RS,3R)-3-amino-5-(éthylsulfanyl)-2-hydroxypentanoyl)-4,5-diméthylbenzohydrazide ;
N'-((2RS,3R)-3-amino-5-(éthylsulfanyl)-2-hydroxypentanoyl)-3,5-diméthylbenzohydrazide ;
N'-((2RS,3R)-3-amino-5-(éthylsulfanyl)-2-hydroxypentanoyl)-2,3-diméthoxybenzohydrazide ;
N'-((2RS,3R)-3-amino-5-(éthylsulfanyl)-2-hydroxypentanoyl)-2,4-diméthoxybenzohydrazide ;
N'-((2RS,3R)-3-amino-5-(éthylsulfanyl)-2-hydroxypentanoyl)-2,5-diméthoxybenzohydrazide ;
N'-((2RS,3R)-3-amino-5-(éthylsulfanyl)-2-hydroxypentanoyl)-3,4-diméthoxybenzohydrazide ;
N'-((2RS,3R)-3-amino-5-(éthylsulfanyl)-2-hydroxypentanoyl)-3,5-diméthoxybenzohydrazide ;
N'-((2RS,3R)-3-amino-5-(éthylsulfanyl)-2-hydroxypentanoyl)-3,4,5-triméthoxybenzohydrazide ;
N'-((2RS,3R)-3-amino-5-(éthylsulfanyl)-2-hydroxypentanoyl)-2,3-dichlorobenzohydrazide ;
N'-((2RS,3R)-3-amino-5-(éthylsulfanyl)-2-hydroxypentanoyl)-2,4-dichlorobenzohydrazide ;
N'-((2RS,3R)-3-amino-5-(éthylsulfanyl)-2-hydroxypentanoyl)-2,5-dichlorobenzohydrazide ;
N'-((2RS,3R)-3-amino-5-(éthylsulfanyl)-2-hydroxypentanoyl)-3,4-dichlorobenzohydrazide ;
N'-((2RS,3R)-3-amino-5-(éthylsulfanyl)-2-hydroxypentanoyl)-3,5-dichlorobenzohydrazide ;
N'-((2RS,3R)-3-amino-2-hydroxy-5-(isopropylsulfanyl)pentanoyl)-2,3-dichlorobenzohydrazide ;
N'-((2RS,3R)-3-amino-2-hydroxy-5-(isopropylsulfanyl)pentanoyl)-3-fluoro-2-méthylbenzohydrazide ;
N'-((2RS,3R)-3-amino-2-hydroxy-5-(isopropylsulfanyl)pentanoyl)-2,3-difluorobenzohydrazide ;
N'-((2RS,3R)-3-amino-2-hydroxy-5-(isopropylsulfanyl)pentanoyl)-5-chloro-2-méthoxybenzohydrazide ;
N'-((2RS,3R)-3-amino-2-hydroxy-5-(isopropylsulfanyl)pentanoyl)-2-chloro-5-(méthylsulfanyl)benzohydrazide ; et
N'-((2RS,3R)-3-amino-2-hydroxy-5-(isopropylsulfanyl)pentanoyl)-3,4-diéthoxybenzohydrazide.

32. Composé selon la revendication 19, dans lequel l'un de R⁶ et R⁷ est hydrogène, et l'autre est choisi dans le groupe constitué par aryle non substitué ou substitué par un, deux, trois, quatre ou cinq substituants indépendamment choisis dans le groupe constitué par alcanoyle, alkoxy, alkoxyalkyle, alkoxycarbonyle, alkyle, alkylsulfonyle, alkylsulfanyle, alkylsulfanylalkyle, amino, aminoalkoxy, aminoalkyle, aminosulfonyle, carboxy, cyano, (cycloalkyl)alkylsulfanyle, cycloalkylsulfanyle, halo, haloalkoxy, haloalkyle, haloalkylsulfanyle, (hétérocycle)alcényle, hydroxy, hydroxyalkoxy, nitro, oxo, et thioxo, où les groupes aryle peuvent être également substitués par un groupe aryle supplémentaire ou un groupe arylalkyle, arylalkylsulfanyle, aryloxy, aryloxyalkyle, hétérocycle, ou (hétérocycle)alcényle, où l'aryle, la partie aryle de l'arylalkyle, l'arylalkylsulfanyle, l'aryloxy, et l'aryloxyalkyle, l'hétérocycle, et la partie hétérocycle de l'(hétérocycle)alcényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle, et nitro ; arylalcanoyle, arylalkoxycarbonyle et arylsulfonyle.

33. Composé selon la revendication 32, choisi dans le groupe constitué par
2-((2RS,3S)-3-amino-4-((cyclohexylméthyl)sulfanyl)-2-hydroxybutanoyl)hydrazinecarboxylate de benzyle ;
2-((2RS,3S)-3-amino-2-hydroxy-4-(propylsulfanyl)butanoyl)hydrazinecarboxylate de benzyle ;
(2RS,3R)-3-amino-5-(éthylsulfanyl)-2-hydroxy-N'-(4-méthylphényl)pentanohydrazide ;
(2RS,3R)-3-amino-5-(éthylsulfanyl)-2-hydroxy-N'-(4-méthoxyphényl)pentanohydrazide ;
(2RS,3R)-3-amino-5-(éthylsulfanyl)-2-hydroxy-N'-(1-naphtyl)pentanohydrazide ;
(2RS,3R)-3-amino-5-(éthylsulfanyl)-2-hydroxy-N'-(4-iodophényl)pentanohydrazide ;
(2RS,3R)-3-amino-5-(éthylsulfanyl)-2-hydroxy-N'-(3-chlorophényl)pentanohydrazide ;
(2RS,3R)-3-amino-5-(éthylsulfanyl)-2-hydroxy-N'-(3-méthoxyphényl)pentanohydrazide ;
(2RS,3R)-3-amino-5-(éthylsulfanyl)-2-hydroxy-N'-(2-chlorophényl)pentanohydrazide ;
(2RS,3R)-3-amino-5-(éthylsulfanyl)-2-hydroxy-N'-(3-trifluorométhylphényl)pentanohydrazide ;
(2RS,3R)-3-amino-5-(éthylsulfanyl)-2-hydroxy-N'-(4-isopropyl)pentanohydrazide ;
(2RS,3R)-3-amino-5-(éthylsulfanyl)-2-hydroxy-N'-(3-chloro-4-méthylphényl)pentanohydrazide ;
(2RS,3R)-3-amino-5-(éthylsulfanyl)-2-hydroxy-N'-(3-fluorophényl)pentanohydrazide ;
(2RS,3R)-3-amino-5-(éthylsulfanyl)-2-hydroxy-N'-(2-éthylphényl)pentanohydrazide ;
(2RS,3R)-3-amino-5-(éthylsulfanyl)-2-hydroxy-N'-(4-fluorophényl)pentanohydrazide ;
(2RS,3R)-3-amino-5-(éthylsulfanyl)-2-hydroxy-N'-(4-trifluorométhoxyphényl)pentanohydrazide ;
(2RS,3S)-3-amino-4-(éthylsulfanyl)-2-hydroxy-N'-(4-méthylphényl)butanohydrazide ;
(2RS,3S)-3-amino-2-hydroxy-N'-(4-méthylphényl)-4-(propylsulfanyl)butanohydrazide ;
(2RS,3R)-3-amino-2-hydroxy-5-(isopropylsulfanyl)-N'-(4-méthylphényl)pentanohydrazide ;
(2RS,3S)-3-amino-2-hydroxy-4-(isobutylsulfanyl)-N'-(4-méthylphényl)butanohydrazide ;
(2RS,3S)-3-amino-4-(éthylsulfanyl)-2-hydroxy-N'-(1-naphtyl)butanohydrazide ;
(2RS,3S)-3-amino-2-hydroxy-5-(isopropylsulfanyl)-N'-(1-naphtyl)pentanohydrazide ;
(2RS,3S)-3-amino-2-hydroxy-4-(isobutylsulfanyl)-N'-(1-naphtyl)butanohydrazide ;
(2RS,3S)-3-amino-2-hydroxy-N'-(1-naphtyl)-4-(propylsulfanyl)butanohydrazide ;
(2RS,3S)-3-amino-4-((cyclohexylméthyl)sulfanyl)-2-hydroxy-N'-(1-naphtyl)butanohydrazide ;
(2RS,3R)-3-amino-5-(éthylsulfanyl)-2-hydroxy-N'-(phénylacétyl)pentanohydrazide ;
(2RS,3R)-3-amino-5-(éthylsulfanyl)-2-hydroxy-N'-((2-méthoxyphényl)acétyl)pentanohydrazide ;
(2RS,3R)-3-amino-5-(éthylsulfanyl)-2-hydroxy-N'-((3-méthoxyphényl)acétyl)pentanohydrazide ;
(2RS,3R)-3-amino-5-(éthylsulfanyl)-2-hydroxy-N'-((4-méthoxyphényl)acétyl)pentanohydrazide ;
(2RS,3R)-3-amino-5-(éthylsulfanyl)-2-hydroxy-N'-((2-chlorophényl)acétyl)pentanohydrazide ;
(2RS,3R)-3-amino-5-(éthylsulfanyl)-2-hydroxy-N'-((3-chlorophényl)acétyl)pentanohydrazide ;
(2RS,3R)-3-amino-5-(éthylsutfanyl)-2-hydroxy-N'-((4-chlorophényl)acétyl)pentanohydrazide ;
N'-((2RS,3R)-3-amino-5-éthylsulfanyl)-2-hydroxypentanoyl)-2-(1,1'-biphényl)-4-ylacétohydrazide ;
N'-((2RS,3R)-3-amino-5-(éthylsulfanyl)-2-hydroxypentanoyl)-2-(4-diméthylaminophényl)acétohydrazide ;
N'-((2RS,3R)-3-amino-5-(éthylsulfanyl)-2-hydroxypentanoyl)-2-(1-naphtyl)acétohydrazide ;
N'-((2RS,3R)-3-amino-5-(éthylsulfanyl)-2-hydroxypentanoyl)-2-(2-naphtyl)acétohydrazide ;
N'-((2RS,3R)-3-amino-5-(éthylsulfanyl)-2-hydroxypentanoyl)benzènesulfonohydrazide ;
N'-((2RS,3R)-3-amino-5-(éthylsulfanyl)-2-hydroxypentanoyl)-3-chlorobenzènesulfonohydrazide ;
N'-((2RS,3R)-3-amino-5-(éthylsulfanyl)-2-hydroxypentanoyl)-1-naphtalènesulfonohydrazide ; et
N'-((2RS,3R)-3-amino-5-(éthylsulfanyl)-2-hydroxypentanoyl)-4-méthylbenzènesulfonohydrazide.

34. Composé selon la revendication 19, dans lequel l'un de R⁶ et R⁷ est hydrogène ; et l'autre est choisi dans le groupe constitué par alcanoyle non substitué ou substitué par un, deux ou trois substituants indépendamment choisis dans le groupe constitué par alkoxycarbonyle, alkylsulfanyle, amino et hydroxy ; cycloalkyle, cycloalkylalcanoyle, (cycloalkyl)oyle, (hétérocycle)alcanoyle et (hétérocycle)oyle.

35. Composé selon la revendication 34, choisi dans le groupe constitué par
(2RS,3R)-3-amino-5-(éthylsulfanyl)-2-hydroxy-N'-cyclopentylpentanohydrazide ;
(2RS,3R)-N'-acétyl-3-amino-5-(éthylsulfanyl)-2-hydroxypentanohydrazide ;
(2RS,3R)-3-amino-5-(éthylsulfanyl)-2-hydroxy-N'-isobutyrylpentanohydrazide ;
(2RS,3R)-3-amino-5-(éthylsulfanyl)-2-hydroxy-N'-(3-méthylbutanoyl)pentanohydrazide ;
(2RS,3R)-3-amino-5-(éthylsulfanyl)-N'-heptanoyl-2-hydroxypentanohydrazide ;
(2RS,3R)-3-amino-5-(éthylsulfanyl)-2-hydroxy-N'-(tétrahydro-2-furanylcarbonyl)pentanohydrazide ;
(2RS,3R)-3-amino-N'-(cyclohexylacétyl)-5-(éthylsulfanyl)-2-hydroxyheptanohydrazide ;
N'-((2RS,3R)-3-amino-5-(éthylsulfanyl)-2-hydroxypentanoyl)-2-furohydrazide ;
N'-((2RS,3R)-3-amino-5-(éthylsutfanyl)-2-hydroxypentanoyl)-3-furohydrazide ;
N'-((2RS,3R)-3-amino-5-(éthylsulfanyl)-2-hydroxypentanoyl)-2-thiophènecarbohydrazide ;
N'-((2RS,3R)-3-amino-5-(éthylsulfanyl)-2-hydroxypentanoyl)-3-thiophènecarbohydrazide ;
N'-((2RS,3R)-3-amino-5-(éthylsulfanyl)-2-hydroxypentanoyl)-1H-pyrrole-2-carbohydrazide ;
N'-((2RS,3R)-3-amino-5-(éthylsulfanyl)-2-hydroxypentanoyl)-1,3-thiazole-2-carbohydrazide ;
N'-((2RS,3R)-3-amino-5-(éthylsulfanyl)-2-hydroxypentanoyl)-1,3-thiazole-4-carbohydrazide ;
N'-((2RS,3R)-3-amino-5-(éthylsulfanyl)-2-hydroxypentanoyl)-1,3-thiazole-5-carbohydrazide ;
N'-((2RS,3R)-3-amino-5-(éthylsulfanyl)-2-hydroxypentanoyl)-1H-pyrazole-5-carbohydrazide ;
N'-((2RS,3R)-3-amino-5-(éthylsulfanyl)-2-hydroxypentanoyl)-1H-pyrazole-4-carbohydrazide ;
N'-((2RS,3R)-3-amino-5-(éthylsulfanyl)-2-hydroxypentanoyl)-5-isoxazolecarbohydrazide ;
N'-((2RS,3R)-3-amino-5-(éthylsulfanyl)-2-hydroxypentanoyl)-2-pyridinecarbohydrazide ;
N'-((2RS,3R)-3-amino-5-(éthylsulfanyl)-2-hydroxypentanoyl)-2-(2-pyridinyl)acétohydrazide ;
N'-((2RS,3R)-3-amino-5-(éthylsulfanyl)-2-hydroxypentanoyl)-3-pyridinecarbohydrazide ;
N'-((2RS,3R)-3-amino-5-(éthylsulfanyl)-2-hydroxypentanoyl)-2-(3-pyridinyl)acétohydrazide ;
N'-((2RS,3R)-3-amino-5-(éthylsulfanyl)-2-hydroxypentanoyl)-4-pyridinecarbohydrazide ;
N'-((2RS,3R)-3-amino-5-(éthylsulfanyl)-2-hydroxypentanoyl)-2-(4-pyridinyl)acétohydrazide ;
N'-((2R5,3R)-3-amino-5-(éthylsulfanyl)-2-hydroxypentanoyl)-3-pyridazinecarbohydrazide ;
N'-((2RS,3R)-3-amino-5-(éthylsulfanyl)-2-hydroxypentanoyl)-4-pyrimidinecarbohydrazide ;
N'-((2RS,3R)-3-amino-5-(éthylsulfanyl)-2-hydroxypentanoyl)-2-pyrazinecarbohydrazide ;
(2RS,3R)-3-amino-N'-((2RS,3S)-3-amino-2-hydroxy-5-(isopropylsulfanyl)pentanoyl)-2-hydroxy-5-(isopropylsulfanyl)pentanohydrazide ;
N'-((2RS,3R)-3-amino-5-(éthylsulfanyl)-2-hydroxypentanoyl)-1,3-benzodioxole-5-carbohydrazide ;
(2RS,3R)-3-amino-2-hydroxy-5-(isopropylsulfanyl)-N'-(tétrahydro-2-furanylcarbonyl)pentanohydrazide ;
(2RS,3R)-3-amino-2-hydroxy-5-(isopropylsulfanyl)-N'-(tétrahydro-3-furanylcarbonyl)pentanohydrazide ;
(2RS,3R)-3-amino-2-hydroxy-5-(isopropylsulfanyl)-N'-cyclopentylpentanohydrazide ;
N'-((2RS,3R)-3-amino-2-hydroxy-5-(isopropylsulfanyl)pentanoyl)-2-cyclopentylacétohydrazide ;
(2RS,3R)-3-amino-2-hydroxy-5-(isopropylsulfanyl)-N'-cyclohexylpentanohydrazide ;
N'-((2RS,3R)-3-amino-2-hydroxy-5-(isopropylsulfanyl)pentanoyl)-2-cyclohexylacétohydrazide ;
N'-((2RS,3R)-3-amino-2-hydroxy-5-(isopropylsulfanyl)pentanoyl)-2-furohydrazide ;
N'-((2RS,3R)-3-amino-2-hydroxy-5-(isopropylsulfanyl)pentanoyl)-3-furohydrazide ;
N'-((2RS,3R)-3-amino-2-hydroxy-5-(isopropylsulfanyl)pentanoyl)-2,5-diméthyl-3-furohydrazide ;
N'-((2RS,3R)-3-amino-2-hydroxy-5-(isopropylsulfanyl)pentanoyl)-2-thiophènecarbohydrazide ;
N'-((2RS,3R)-3-amino-2-hydroxy-5-(isopropylsulfanyl)pentanoyl)-3-thiophènecarbohydrazide ;
N'-((2RS,3R)-3-amino-2-hydroxy-5-(isopropylsulfanyl)pentanoyl)-3-méthyl-2-thiophènecarbohydrazide ;
N'-((2RS,3R)-3-amino-2-hydroxy-5-(isopropylsulfanyl)pentanoyl)-5-méthyl-2-thiophènecarbohydrazide ;
N'-((2RS,3R)-3-amino-2-hydroxy-5-(isopropylsulfanyl)pentanoyl)-1H-pyrrole-2-carbohydrazide ;
N'-((2RS,3R)-3-amino-2-hydroxy-5-(isopropylsulfanyl)pentanoyl)-1-méthyl-1H-pyrrole-2-carbohydrazide ;
N'-((2RS,3R)-3-amino-2-hydroxy-5-(isopropylsulfanyl)pentanoyl)-1,3-thiazole-2-carbohydrazide ;
N'-((2RS,3R)-3-amino-2-hydroxy-5-(isopropylsulfanyl)pentanoyl)-1,3-thiazole-4-carbohydrazide ;
N'-((2RS,3R)-3-amino-2-hydroxy-5-isopropylpentanoyl)-2-pyridinecarbohydrazide ;
N'-((2RS,3R)-3-amino-2-hydroxy-5-(isopropylsulfanyl)pentanoyl)-6-chloro-2H-chromène-3-carbohydrazide ;
N'-((2RS,3R)-3-amino-2-hydroxy-5-(isopropylsulfanyl)pentanoyl)-2-(4-morpholinyl)acétohydrazide ;
N'-((2RS,3R)-3-amino-2-hydroxy-5-(isopropylsulfanyl)pentanoyl)-2-(4-méthyl-1-pipérazinyl)acétohydrazide ; et
1-acétyl-N'-((2RS,3R)-3-amino-2-hydroxy-5-(isopropylsulfanyl)pentanoyl)-4-pipéridinecarbohydrazide.

36. Composé selon la revendication 19, dans lequel R⁶ et R⁷, ensemble avec l'atome d'azote auquel ils sont attachés, forment un hétérocycle non substitué or substitué par un, deux, trois, ou quatre substituants indépendamment choisis dans le groupe constitué par alcanoyle, alkoxy, alkoxyalkyle, alkoxycarbonyle, alkyle, alkylsulfanylalkyle, amino, aminosulfonyle, carboxy, halo, haloalkoxy, haloalkyle, hydroxy, nitro, oxo, et thioxo, où les groupes hétérocycle peuvent être également substitués par un groupe aryle, arylalkyle, aryloxy, aryloxycarbonyle, ou (hétérocycle)alcényle, où l'aryle, la portion aryle de l'aryloxy, l'arylalkyle, et l'aryloxycarbonyle, et la portion hétérocycle de l'(hétérocycle)acényle peuvent être également substitués par un, deux, trois, quatre ou cinq substituants indépendamment choisis dans le groupe constitué par alcanoyle, alkoxy, alkoxycarbonyle, alkyle, carboxy, cyano, haloalkoxy, haloalkyle et nitro.

37. Composé selon la revendication 36, qui est
(2RS,3R)-3-amino-5-(éthylsulfanyl)-2-hydroxy-N-(1-pipéridinyl)pentanamide.

38. Composition pharmaceutique comprenant un composé selon la revendication 1 ou un sel ou promédicament thérapeutiquement acceptable de celui-ci, en combinaison avec un support thérapeutiquement acceptable.

39. Procédé d'inhibition d'angiogénèse chez un mammifère, en cas de nécessité reconnue d'un traitement de cette nature, consistant à administrer au mammifère une quantité thérapeutiquement acceptable d'un composé selon la revendication 1.

40. Composé qui est
N'-((2S,3R)-3-amino-2-hydroxy-5-(isopropylsulfanyl)pentanoyl)-3-chlorobenzoyl hydrazide.
